(19) Europäisches Patentamt
European Patent Office
Office européen des brevets

(11) EP 2 885 297 B1

(12) **FASCICULE DE BREVET EUROPEEN**

(45) Date de publication et mention de la délivrance du brevet:
**11.10.2017 Bulletin 2017/41**

(21) Numéro de dépôt: **13750072.4**

(22) Date de dépôt: **16.08.2013**

(51) Int Cl.:
***C07D 413/14*** *(2006.01)* ***A61K 31/506*** *(2006.01)*

(86) Numéro de dépôt international:
**PCT/EP2013/067129**

(87) Numéro de publication internationale:
**WO 2014/027081 (20.02.2014 Gazette 2014/08)**

(54) **PYRIDO[3,2-D]PYRIMIDINES TRISUBSTITUÉES, LEURS PROCÉDÉS DE PRÉPARATION ET LEURS UTILISATIONS EN THÉRAPEUTIQUE**

TRISUBSTITUIERTE PYRIDO[3,2-D]PYRIMIDINE, DEREN VERFAHREN ZUR HERSTELLUNG UND DEREN THERAPEUTISCHE ANWENDUNGEN

TRISUBSTITUTED PYRIDO[3,2-D]PYRIMIDINES, THEIR PREPARATION PROCESS AND THEIR THERAPEUTICAL USES

(84) Etats contractants désignés:
**AL AT BE BG CH CY CZ DE DK EE ES FI FR GB GR HR HU IE IS IT LI LT LU LV MC MK MT NL NO PL PT RO RS SE SI SK SM TR**

(30) Priorité: **17.08.2012 FR 1257856**

(43) Date de publication de la demande:
**24.06.2015 Bulletin 2015/26**

(73) Titulaires:
- **Centre National de la Recherche Scientifique (C.N.R.S.)
75016 Paris (FR)**
- **Universite D'Orleans
45067 Orleans Cedex 2 (FR)**

(72) Inventeurs:
- **ROUTIER, Sylvain
F-45510 Tigy (FR)**
- **BENEDETTI, Hélène
F-45800 Saint-Jean-de-Bray (FR)**
- **BURON, Frédéric
F-45100 Orléans (FR)**
- **HIEBEL, Marie-Aude
F-45160 Olivet (FR)**
- **SAURAT, Thibault
78120 Rambouillet (FR)**
- **GUILLAUMET, Gérald
F-45650 Saint-Jean-le-Blanc (FR)**

(74) Mandataire: **Lavoix
2, place d'Estienne d'Orves
75441 Paris Cedex 09 (FR)**

(56) Documents cités:
**WO-A1-2011/101429 WO-A1-2011/135259
WO-A1-2012/058671**

Remarques:
Le dossier contient des informations techniques présentées postérieurement au dépôt de la demande et ne figurant pas dans le présent fascicule.

Il est rappelé que: Dans un délai de neuf mois à compter de la publication de la mention de la délivrance du brevet européen au Bulletin européen des brevets, toute personne peut faire opposition à ce brevet auprès de l'Office européen des brevets, conformément au règlement d'exécution. L'opposition n'est réputée formée qu'après le paiement de la taxe d'opposition. (Art. 99(1) Convention sur le brevet européen).

EP 2 885 297 B1

## Description

La présente invention concerne de nouveaux dérivés de type pyrido[3,2-d]pyrimidine et leurs procédés de préparation. Elle a également pour objet les utilisations thérapeutiques desdits nouveaux dérivés notamment en tant qu'inhibiteurs de kinases.

L'inhibition des enzymes composants des cascades de signalisation cellulaire fait l'objet d'intenses efforts. Parmi elles, une des plus universellement sur-régulée est la cascade PI3K-Akt-mTor.

La « mammalian target of rapamycin » (mTOR) est membre de la famille des kinases PIKK. Elle apparaît comme une famille non conventionnelle de sérine/thréonine kinase à haut poids moléculaire dont les séquences sont similaires à celle de la PI3K. Dans le cancer, mTOR est fréquemment suractivée validant ainsi cette enzyme comme cible thérapeutique. Deux alcaloïdes d'origine naturelle sont actuellement utilisés comme médicament en clinique. Il s'agit du temsirolimus et de l'évérolimus.

La « phosphatidyl-inositol 3-kinase » (PI3K), quant à elle, est une enzyme ubiquitaire qui phosphoryle le groupement hydroxyle situé en position 3 du Phosphatidyl-inositol 4,5 biphosphate (PIP2). Cette réaction, qui génère du phosphatidyl-inositol 3,4,5 triphosphate (PIP3), va par la suite déclencher une cascade de réactions qui favoriseront l'apoptose, la prolifération cellulaire, l'angiogenèse ou la progression du cycle cellulaire. La PI3K est un hétérodimère composé d'une sous-unité catalytique p110 et d'une sous-unité régulatrice p85. On compte 4 types d'isoformes $\alpha$, $\beta$, $\gamma$ et $\delta$ pour la sous-unité p110. Cependant, chaque isoforme de la sous-unité catalytique va avoir un rôle plus ou moins prédominant selon certains types de pathologies. PI3K $\alpha$ va intervenir dans de nombreux cancers, tandis que PI3K $\beta$ semble être impliquée uniquement dans des cancers spécifiques et aussi dans les phénomènes de thrombose. Les isoformes PI3K $\gamma$ et PI3K $\delta$ sont impliquées dans les réponses immunitaires à savoir les mécanismes inflammatoires et les maladies auto-immunes.

Inhiber simultanément ces deux enzymes peut donner naissance à des traitements efficaces contre le cancer.

La présente invention a pour but de fournir de nouveaux inhibiteurs de PI3K.

La présente invention a pour but de fournir de nouveaux inhibiteurs de mTOR.

La présente invention a pour but de fournir de nouveaux inhibiteurs simultanés de PI3K et de mTOR.

La présente invention a pour but de fournir de nouveaux inhibiteurs des kinases PI3K et mTOR ciblant directement et sélectivement lesdites kinases.

La présente invention concerne des composés de formule générale (I) suivante :

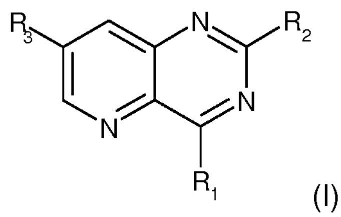

(I)

dans laquelle :

- $R_1$ est choisi dans le groupe constitué :

    - des (hétéro)aryles comprenant de 5 à 30 atomes, éventuellement substitués, et
    - des groupes $-NR_aR_b$, $R_a$ et $R_b$ formant ensemble avec l'atome d'azote sur lequel ils sont reliés, un hétérocycle comprenant de 5 à 30 atomes, comprenant éventuellement un radical $-SO_2-$ ou un autre hétéroatome choisi parmi N, O et S, ledit hétérocycle étant éventuellement substitué ;

- $R_2$ est choisi dans le groupe constitué :

    - des atomes d'halogène,
    - des (hétéro)aryles comprenant de 5 à 30 atomes, éventuellement substitués,
    - des groupes $-NR'_aR'_b$, $R'_a$ et $R'_b$ formant ensemble avec l'atome d'azote sur lequel ils sont reliés, un hétérocycle comprenant de 5 à 30 atomes, comprenant éventuellement un radical $-SO_2-$ ou un autre hétéroatome choisi parmi N, O et S, ledit hétérocycle étant éventuellement substitué ; et

- $R_3$ est choisi dans le groupe constitué :

    - des atomes d'halogène ;

- des alcényles comprenant de 1 à 20 atomes de carbone, éventuellement substitués,
- des groupes -C(O)$R_c$, $R_c$ étant choisi dans le groupe constitué d'un atome d'hydrogène ou d'un groupe alkyle comprenant de 1 à 10 atomes de carbone, ledit groupe alkyle étant éventuellement substitué,
- des groupes -C(O)OR'$_c$, R'$_c$ étant choisi dans le groupe constitué d'un atome d'hydrogène ou d'un groupe alkyle comprenant de 1 à 10 atomes de carbone, ledit groupe alkyle étant éventuellement substitué,
- des groupes -C($R_e$)=N-(O$R_d$), $R_d$ et $R_e$ étant indépendamment choisi parmi le groupe constitué d'un atome d'hydrogène ou d'un groupe alkyle comprenant de 1 à 10 atomes de carbone,
- des hétérocycloalkyles comprenant de 3 à 20 atomes, éventuellement substitués,
- des alkyles, comprenant de 1 à 20 atomes de carbone, éventuellement substitués par au moins un substituant ;

à condition qu'au moins un de $R_1$ et $R_2$ représente respectivement un groupe -N$R_aR_b$ ou un groupe -NR'$_a$R'$_b$,
ainsi que ses sels pharmaceutiquement acceptables, notamment chlorhydrates, ses hydrates, ses racémates, diastéréoisomères ou énantiomères.

La présente invention concerne des composés de formule générale (I) suivante :

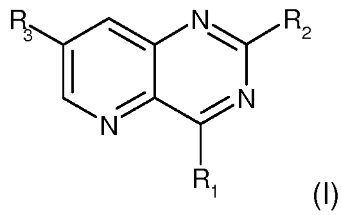

(I)

dans laquelle :

- $R_1$ est choisi dans le groupe constitué :
  - des (hétéro)aryles comprenant de 5 à 30 atomes, éventuellement substitués, et
  - des groupes -N$R_aR_b$, $R_a$ et $R_b$ formant ensemble avec l'atome d'azote sur lequel ils sont reliés, un hétérocycle comprenant de 5 à 30 atomes, comprenant éventuellement un radical -$SO_2$- ou un autre hétéroatome choisi parmi N, O et S, ledit hétérocycle étant éventuellement substitué ;
- $R_2$ est choisi dans le groupe constitué :
  - des atomes d'halogène choisis parmi F, Br et I,
  - des aryles comprenant de 5 à 30 atomes, éventuellement substitués,
  - des groupes -NR'$_a$R'$_b$, R'$_a$ et R'$_b$ formant ensemble avec l'atome d'azote sur lequel ils sont reliés, un hétérocycle comprenant de 5 à 30 atomes, comprenant éventuellement un radical -$SO_2$- ou un autre hétéroatome choisi parmi N, O et S, ledit hétérocycle étant éventuellement substitué ; et
- $R_3$ est choisi dans le groupe constitué :
  - des atomes d'halogène choisis parmi F, Cl et I,
  - des alcényles comprenant de 1 à 20 atomes de carbone, éventuellement substitués,
  - des groupes -C(O)$R_c$, $R_c$ étant choisi dans le groupe constitué d'un atome d'hydrogène ou d'un groupe alkyle comprenant de 1 à 10 atomes de carbone, ledit groupe alkyle étant éventuellement substitué,
  - des groupes -C(O)OR'$_c$, R'$_c$ étant choisi dans le groupe constitué d'un atome d'hydrogène ou d'un groupe alkyle comprenant de 1 à 10 atomes de carbone, ledit groupe alkyle étant éventuellement substitué,
  - des groupes -C($R_e$)=N-(O$R_d$), $R_d$ et $R_e$ étant indépendamment choisi parmi le groupe constitué d'un atome d'hydrogène ou d'un groupe alkyle comprenant de 1 à 10 atomes de carbone,
  - des hétérocycloalkyles comprenant de 3 à 20 atomes, éventuellement substitués,
  - des alkyles, comprenant de 1 à 20 atomes de carbone, éventuellement substitués par au moins un substituant ;

à condition qu'au moins un de $R_1$ et $R_2$ représente respectivement un groupe -N$R_aR_b$ ou un groupe -NR'$_a$R'$_b$,
ainsi que ses sels pharmaceutiquement acceptables, notamment chlorhydrates, ses hydrates, ses racémates, diastéréoisomères ou énantiomères.

En particulier, les composés de formule (I) de l'invention sont utilisés sous forme libre ou sous forme salifiée, notamment des chlorhydrates.

Selon un mode de réalisation, la présente invention concerne des composés de formule générale (I) suivante :

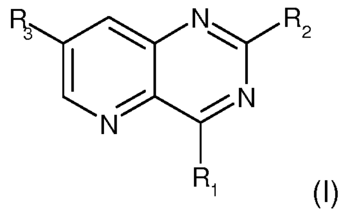

(I)

dans laquelle :

- $R_1$ est choisi dans le groupe constitué :
  - des (hétéro)aryles comprenant de 5 à 30 atomes, éventuellement substitués, et
  - des groupes $-NR_aR_b$, $R_a$ et $R_b$ formant ensemble avec l'atome d'azote sur lequel ils sont reliés, un hétérocycle comprenant de 5 à 30 atomes, comprenant éventuellement un radical $-SO_2-$ ou un autre hétéroatome choisi parmi N, O et S,ledit hétérocycle étant éventuellement substitué ;
- $R_2$ est choisi dans le groupe constitué :
  - des aryles comprenant de 5 à 30 atomes de carbone, éventuellement substitués,
  - des groupes $-NR'_aR'_b$, $R'_a$ et $R'_b$ formant ensemble avec l'atome d'azote sur lequel ils sont reliés, un hétérocycle comprenant de 5 à 30 atomes, comprenant éventuellement un radical $-SO_2-$ ou un autre hétéroatome choisi parmi N, O et S, ledit hétérocycle étant éventuellement substitué ; et
- $R_3$ est choisi dans le groupe constitué :
  - des alcényles comprenant de 1 à 20 atomes de carbone, éventuellement substitués,
  - des groupes $-C(O)R_c$, $R_c$ étant choisi dans le groupe constitué d'un atome d'hydrogène ou d'un groupe alkyle comprenant de 1 à 10 atomes de carbone, ledit groupe alkyle étant éventuellement substitué,
  - des groupes $-C(O)OR'_c$, $R'_c$ étant choisi dans le groupe constitué d'un atome d'hydrogène ou d'un groupe alkyle comprenant de 1 à 10 atomes de carbone, ledit groupe alkyle étant éventuellement substitué,
  - des groupes $-C(R_e)=N-(OR_d)$, $R_d$ et $R_e$ étant indépendamment choisi parmi le groupe constitué d'un atome d'hydrogène ou d'un groupe alkyle comprenant de 1 à 10 atomes de carbone,
  - des hétérocycloalkyles comprenant de 3 à 20 atomes, éventuellement substitués,
  - des alkyles, comprenant de 1 à 20 atomes de carbone, éventuellement substitués par au moins un substituant ;

à condition qu'au moins un de $R_1$ et $R_2$ représente respectivement un groupe $-NR_aR_b$ ou un groupe $-NR'_aR'_b$, ainsi que ses sels pharmaceutiquement acceptables, notamment chlorhydrates, ses hydrates, ses racémates, diastéréoisomères ou énantiomères.

Selon un mode de réalisation, la présente invention concerne des composés de formule générale (I) suivante :

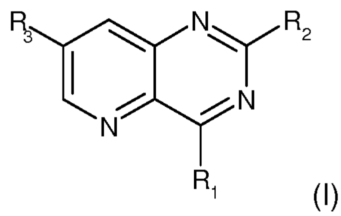

(I)

dans laquelle :

- $R_1$ est choisi dans le groupe constitué :
  - des (hétéro)aryles comprenant de 5 à 30 atomes, éventuellement substitués, et
  - des groupes $-NR_aR_b$, $R_a$ et $R_b$ formant ensemble avec l'atome d'azote sur lequel ils sont reliés, un hétérocycle comprenant de 5 à 30 atomes, comprenant éventuellement un autre hétéroatome choisi parmi N, O et S, ledit

hétérocycle étant éventuellement substitué ;

- $R_2$ est choisi dans le groupe constitué :
  - des atomes d'halogène choisis parmi F, Br et I,
  - des aryles comprenant de 5 à 30 atomes de carbone, éventuellement substitués,
  - des groupes $-NR'_aR'_b$, $R'_a$ et $R'_b$ formant ensemble avec l'atome d'azote sur lequel ils sont reliés, un hétérocycle comprenant de 5 à 30 atomes, comprenant éventuellement un autre hétéroatome choisi parmi N, O et S, ledit hétérocycle étant éventuellement substitué ; et

- $R_3$ est choisi dans le groupe constitué :
  - des atomes d'halogène choisis parmi F, Cl et I,
  - des alcényles comprenant de 1 à 20 atomes de carbone, éventuellement substitués,
  - des groupes $-C(O)R_c$, $R_c$ étant choisi dans le groupe constitué d'un atome d'hydrogène ou d'un groupe alkyle comprenant de 1 à 10 atomes de carbone, ledit groupe alkyle étant éventuellement substitué,
  - des groupes $-C(O)OR'_c$, $R'_c$ étant choisi dans le groupe constitué d'un atome d'hydrogène ou d'un groupe alkyle comprenant de 1 à 10 atomes de carbone, ledit groupe alkyle étant éventuellement substitué,
  - des groupes $-C(R_e)=N-(OR_d)$, $R_d$ et $R_e$ étant indépendamment choisi parmi le groupe constitué d'un atome d'hydrogène ou d'un groupe alkyle comprenant de 1 à 10 atomes de carbone,
  - des hétérocycloalkyles comprenant de 3 à 20 atomes de carbone, éventuellement substitués,
  - des alkyles, comprenant de 1 à 20 atomes de carbone, éventuellement substitués par au moins un substituant ;

à condition qu'au moins un de $R_1$ et $R_2$ représente respectivement un groupe $-NR_aR_b$ ou un groupe $-NR'_aR'_b$, ainsi que ses sels pharmaceutiquement acceptables, ses hydrates, ses racémates, diastéréoisomères ou énantiomères.

Selon un mode de réalisation, la présente invention concerne des composés de formule générale (I) suivante :

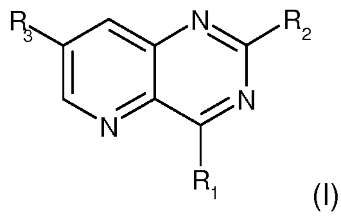

(I)

dans laquelle :

- $R_1$ est choisi dans le groupe constitué :
  - des (hétéro)aryles comprenant de 5 à 30 atomes, éventuellement substitués, et
  - des groupes $-NR_aR_b$, $R_a$ et $R_b$ formant ensemble avec l'atome d'azote sur lequel ils sont reliés, un hétérocycle comprenant de 5 à 30 atomes, comprenant éventuellement un autre hétéroatome choisi parmi N, O et S, ledit hétérocycle étant éventuellement substitué ;

- $R_2$ est choisi dans le groupe constitué :
  - des aryles comprenant de 5 à 30 atomes de carbone, éventuellement substitués,
  - des groupes $-NR'_aR'_b$, $R'_a$ et $R'_b$ formant ensemble avec l'atome d'azote sur lequel ils sont reliés, un hétérocycle comprenant de 5 à 30 atomes de, comprenant éventuellement un autre hétéroatome choisi parmi N, O et S, ledit hétérocycle étant éventuellement substitué ; et

- $R_3$ est choisi dans le groupe constitué :
  - des alcényles comprenant de 1 à 20 atomes de carbone, éventuellement substitués,
  - des groupes $-C(O)R_c$, $R_c$ étant choisi dans le groupe constitué d'un atome d'hydrogène ou d'un groupe alkyle comprenant de 1 à 10 atomes de carbone, ledit groupe alkyle étant éventuellement substitué,
  - des groupes $-C(O)OR'_c$, $R'_c$ étant choisi dans le groupe constitué d'un atome d'hydrogène ou d'un groupe alkyle comprenant de 1 à 10 atomes de carbone, ledit groupe alkyle étant éventuellement substitué,

- des groupes -$C(R_e)=N-(OR_d)$, $R_d$ et $R_e$ étant indépendamment choisi parmi le groupe constitué d'un atome d'hydrogène ou d'un groupe alkyle comprenant de 1 à 10 atomes de carbone,
- des hétérocycloalkyles comprenant de 3 à 20 atomes, éventuellement substitués,
- des alkyles, comprenant de 1 à 20 atomes de carbone, éventuellement substitués par au moins un substituant ;

à condition qu'au moins un de $R_1$ et $R_2$ représente respectivement un groupe $-NR_aR_b$ ou un groupe $-NR'_aR'_b$, ainsi que ses sels pharmaceutiquement acceptables, ses hydrates, ses racémates, diastéréoisomères ou énantiomères.

Selon un mode de réalisation, dans le groupe $-NR_aR_b$, $R_a$ et $R_b$ forment ensemble avec l'atome d'azote sur lequel ils sont reliés, un hétérocycle comprenant de 5 à 30 atomes, comprenant éventuellement un radical $SO_2$ ou un autre hétéroatome choisi parmi N, O et S, ledit hétérocycle n'étant pas substitué.

De préférence, dans le groupe $-NR_aR_b$, $R_a$ et $R_b$ forment ensemble avec l'atome d'azote sur lequel ils sont reliés, une morpholine non substituée, et notamment le groupe suivant :

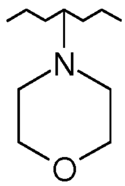

Selon un mode de réalisation, dans le groupe $-NR'_aR'_b$, $R'_a$ et $R'_b$ forment ensemble avec l'atome d'azote sur lequel ils sont reliés, un hétérocycle comprenant de 5 à 30 atomes, comprenant éventuellement un radical $SO_2$ ou un autre hétéroatome choisi parmi N, O et S, ledit hétérocycle n'étant pas substitué.

De préférence, dans le groupe $-NR'_aR'_b$, $R'_a$ et $R'_b$ forment ensemble avec l'atome d'azote sur lequel ils sont reliés, une morpholine non substituée, et notamment le groupe suivant :

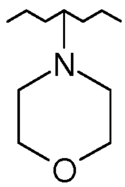

Selon un mode de réalisation, parmi les composés de formule (I), on peut citer ceux pour lesquels $R_3$ représente un halogène, et notamment un chlore.

Selon un mode de réalisation, parmi les composés de formule (I), on peut citer ceux pour lesquels $R_3$ représente un alcényle, et de préférence un allyle.

Selon un mode de réalisation, parmi les composés de formule (I), on peut citer ceux pour lesquels $R_3$ représente un groupe -C(O)H.

Selon un autre mode de réalisation, parmi les composés de formule (I), on peut citer ceux pour lesquels $R_3$ représente un groupe -C(O)OH.

Selon un autre mode de réalisation, parmi les composés de formule (I), on peut citer ceux pour lesquels $R_3$ représente un groupe $-C(O)OR'_c$, dans lequel $R'_c$ représente un alkyle comprenant de 1 à 10 atomes de carbone.

Selon un autre mode de réalisation, parmi les composés de formule (I), on peut citer ceux pour lesquels $R_3$ représente un hétérocycloalkyle comprenant de 3 à 20 atomes de carbone, éventuellement substitué. En particulier, $R_3$ représente un hétérocycloalkyle comprenant de 3 à 5 atomes, éventuellement substitué par au moins un groupe alkyle, et notamment deux groupes méthyle.

Selon un autre mode de réalisation, parmi les composés de formule (I), on peut citer ceux pour lesquels $R_3$ représente un groupe choisi parmi -CH=N-(OH) ou un groupe $-CH=N-(OCH_3)$.

Selon un mode de réalisation, la présente invention concerne des composés de formule (I) dans laquelle $R_3$ représente un groupe alkyle comprenant de 1 à 20 atomes de carbone, ledit groupe alkyle étant éventuellement substitué par au moins un substituant choisi parmi le groupe constitué de :

- $OR_f$, $R_f$ représentant un groupe choisi parmi un atome d'hydrogène ou un alkyle comprenant de 1 à 10 atomes de carbone,
- $NHR_g$, $R_g$ représentant un groupe cycloalkyle comprenant de 3 à 12 atomes de carbone, éventuellement substitué,
- $NR_hR_i$, avec $R_h$ et $R_i$ formant un hétérocycle, comprenant de 5 à 30 atomes, avec l'atome d'azote sur lequel ils sont reliés, ledit hétérocycle comprenant éventuellement un autre hétéroatome choisi parmi N, O et S, et étant éventuellement substitué par au moins un substituant choisi dans le groupe constitué de : méthyle, cyclohexyle, phényle et $-SO_2Me$ ;

- un halogène, tel que F, Cl, Br ou I ;
- un groupe C(O)H,
- $-N_3$,
- -CN, et
- un groupe (hétéro)aryle comprenant de 5 à 30 atomes, tel qu'un isoxazole ou un triazole, ledit groupe (hétéro)aryle étant éventuellement substitué par au moins un substituant choisi dans le groupe constitué de :$-CH_2OH$, $-CH_2OMe$, $-CH_2NMe_2$, $-CH_2F$, $-CH_2OCH_2OMe$.

De préférence, dans les composés de formule (I), $R_3$ représente un groupe alkyle comprenant de 1 à 20 atomes de carbone, de préférence de 1 à 5 atomes de carbone. En particulier, $R_3$ représente un groupe méthyle.

De préférence, dans les composés de formule (I), $R_3$ représente un groupe alkyle comprenant de 1 à 20 atomes de carbone, de préférence de 1 à 5 atomes de carbone, ledit groupe alkyle étant substitué par au moins un substituant choisi dans le groupe constitué de :

- OH ;
- NH-cyclopropyle ;
- NH-cyclohexyle ;
- N-morpholine ;
- N-pipérazine, éventuellement substitué par au moins un substituant choisi dans le groupe constitué de : méthyle, $-SO_2Me$, cyclohexyle et phényle ;
- OMe ;
- $N_3$ ;
- hetéroaryle, et notamment 1,2,3-triazole ou isoxazole, substitué par au moins un substituant choisi dans le groupe constitué de : $-CH_2OH$ , - $CH_2OMe$, $-CH_2NMe_2$, $-CH_2F$, $-CH_2OCH_2OMe$ ;
- CN ; et
- C(O)H.

Selon la présente invention, les radicaux "alkyle" représentent des radicaux hydrocarbonés saturés, en chaîne droite ou ramifiée, comprenant de 1 à 20 atomes de carbone, de préférence de 1 à 10 atomes de carbone, et préférentiellement de 1 à 5 atomes de carbone (ils peuvent typiquement être représentés par la formule $C_nH_{2n+1}$, n représentant le nombre d'atomes de carbone). On peut notamment citer, lorsqu'ils sont linéaires, les radicaux méthyle, éthyle, propyle, butyle, pentyle, hexyle, octyle, nonyle, décyle et dodécyle. On peut notamment citer, lorsqu'ils sont ramifiés ou substitués par un ou plusieurs radicaux alkyles, les radicaux isopropyle, tert-butyle, 2-éthylhexyle, 2-méthylbutyle, 2-méthylpentyle, 1-méthylpentyle et 3-méthylheptyle.

Selon la présente invention, les radicaux "alcènyle" représentent des radicaux hydrocarbonés, en chaîne droite ou ramifiée, comprenant une ou plusieurs insaturations éthyléniques. Lorsqu'ils comprennent une seule double liaison, ils peuvent typiquement être représentés par la formule $C_nH_{2n}$, n représentant le nombre d'atomes de carbone. Parmi les radicaux alcényles, on peut notamment citer les radicaux allyle ou vinyle.

Selon la présente invention, le radical "cycloalkyle" est un radical hydrocarboné mono- ou bicyclique saturé ou partiellement insaturé, non aromatique, comprenant de 3 à 20 atomes de carbone, et de préférence de 3 à 12 atomes de carbone, tel que notamment le cyclopropyle, cyclopentyle ou cyclohexyle.

Selon la présente invention, les radicaux "hétérocycloalkyles" ou "hétérocycles" désignent les systèmes mono ou bicycliques, saturés ou partiellement insaturés, non aromatiques, comprenant de 3 à 20 atomes de carbone, de préférence de 3 à 8, comprenant un ou plusieurs hétéroatomes, de préférence de 1 à 2 hétéroatomes, choisis parmi N, O ou S. On peut notamment citer à titre d'hétérocycloalkyle, la morpholine, le dioxolane ou la pipérazine. Lorsque l'hétérocycle est substitué, on peut notamment citer la N-méthylpipérazine, la N-méthylsulfonylpipérazine, la N-cyclohexylpipérazine, la N-phénylpipérazine et le 2,2-diméthyl-[1,3]dioxolane.

Selon la présente invention, on entend par « halogène », un atome choisi dans le groupe constitué de F, Cl, Br et I.

Le terme "aryle" désigne un système aromatique hydrocarboné, mono ou bicyclique comprenant de 5 à 30, de préférence de 6 à 10, atomes de carbone. Parmi les radicaux aryle, on peut notamment citer le radical phényle ou naphtyle.

Lorsque le radical aryle comprend au moins un hétéroatome, on parle de radical "hétéroaryle". Ainsi, le terme "hétéroaryle" désigne un système aromatique comprenant un ou plusieurs hétéroatomes choisis parmi l'azote, l'oxygène ou le soufre, mono ou bicyclique, comprenant de 5 à 30, et de préférence de 5 à 10, atomes. Parmi les radicaux hétéroaryles, on pourra citer le 1,3,4-oxadiazolyle, l'isoxazole, le triazolyle, le 4-H-[1,2,4]triazolyle, le tétrazolyle, le 2H-tétrazolyle, le 1,3,4-thiadiazolyle, le 1,2,3-triazolyle, le 1H-[1,2,3]-triazolyle, l'indolyle, l'indolizinyle, le pyrimidinyle, , ainsi que les groupes correspondants, issus de leur fusion ou de la fusion avec le noyau phényle.

Les radicaux "alkyle", "aryle", "hétéroaryle", "alcényle", "hétérocycle", "hétérocycloalkyle" et "cycloalkyle" susmentionnés peuvent être substitués par un ou plusieurs substituants, de préférence par un à cinq substituants, et préférentiellement de un à deux. Parmi ces substituants, on peut notamment citer les groupes suivants : $-N_3$, CHO, amino, amine, hydroxy, halogène, carboxyle, alkyle (éventuellement substitué tel que par un halogène), $CH_2OH$, $CH_2F$, $CF_3$, $SO_2$alkyle, $CH_2OMe$, alkaryle, alkoxy, alkylcarbonyle, aryle (éventuellement substitué), aminocarbonyle, alkylcarboxyle, alkylamino, -NH-hétérocycloalkyle, hétérocycloalkyle (éventuellement substitué par un groupe Me, $SO_2Me$ cyclohexyle ou phényle), hétéroaryle (éventuellement substitué par un groupe $-CH_2NMe_2$, $CH_2F$, $CH_2OCH_2OMe$ ou $CH_2OMe$), aryloxy, arylalkoxy, cyano, trifluorométhyle, -NHC(O)NH-aryle (avec aryle éventuellement substitué par $-CH_2OH$), -NHC(O)NH-alkyle (avec alkyle éventuellement substitué par halogène), carboxyalkyle, alkoxyalkoxy ou nitro.

Les radicaux "alkoxy" selon la présente invention sont des radicaux de formule - O-alkyle, le groupe alkyle étant tel que défini précédemment.

Les radicaux « alkoxyalkoxy » selon la présente invention sont des radicaux de formule -O-alkyle-O-alkyle, le groupe alkyle étant tel que défini précédemment.

Le terme "alkylamino" désigne un groupe -NH-alkyle, le groupe alkyle étant tel que défini ci-dessus.

Le terme "alkylcarbonyle" désigne un groupe -CO-alkyle, le groupe alkyle étant tel que défini ci-dessus.

Le terme "alkylcarboxyle" désigne un groupe -COO-alkyle, le groupe alkyle étant tel que défini ci-dessus.

Parmi les atomes d'halogène, on cite plus particulièrement les atomes de fluor, de chlore, de brome et d'iode.

Le terme "aryloxy" désigne un groupe -O-aryle, le groupe aryle étant tel que défini ci-dessus.

Le terme "arylalkoxy" désigne un groupe aryl-alkoxy-, les groupes aryles et alkoxy étant tels que définis ci-dessus.

Le terme "carboxyalkyle" désigne un groupe HOOC-alkyle-, le groupe alkyle étant tel que défini ci-dessus. Comme exemple de groupes carboxyalkyles, on peut citer notamment le carboxyméthyle ou le carboxyéthyle.

Lorsqu'un radical alkyle est substitué par un groupe aryle, on parle de radical "arylalkyle" ou "aralkyle". Les radicaux « arylalkyles » ou « aralkyles » sont des radicaux aryl-alkyl-, les groupes aryles et alkyles étant tels que définis ci-dessus. Parmi les radicaux arylalkyles, on peut notamment citer le radical benzyle ou phénéthyle.

Parmi les groupes aryles ou hétéroaryles, substitués ou non, on peut plus particulièrement citer les groupes suivants :

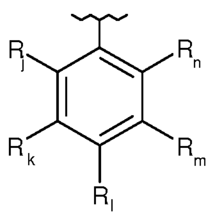
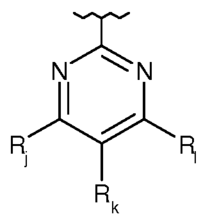
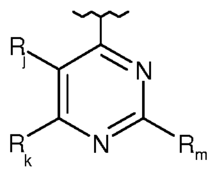
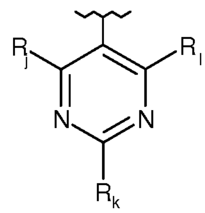

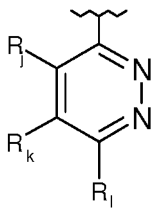
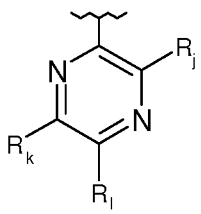

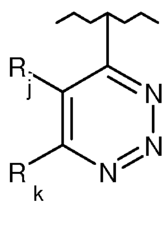
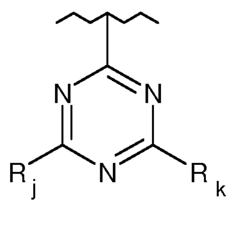
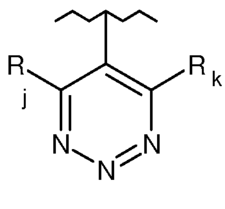

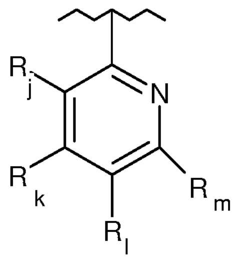
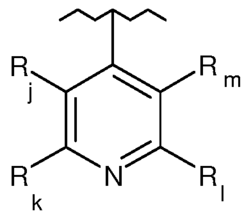
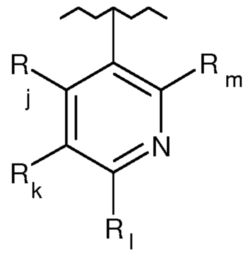

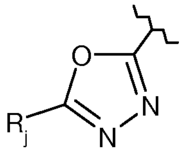
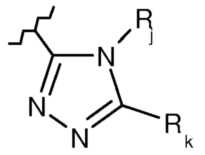
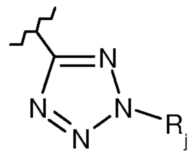

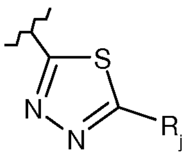
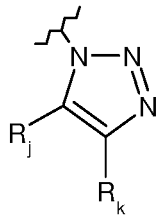
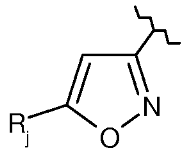
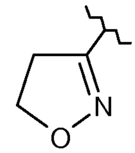

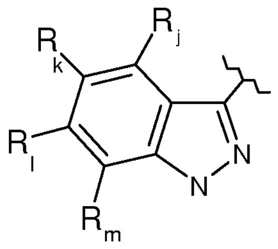
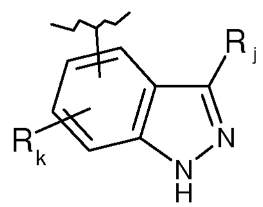

les groupes $R_j$, $R_k$, $R_l$, $R_m$ et $R_n$, étant choisis, indépendamment les uns des autres, dans le groupe constitué des substituants suivants :

- un atome d'hydrogène,
- un atome d'halogène,
- un groupe alkyle comprenant de 1 à 10 atomes de carbone, et étant de préférence un groupe méthyle,
  ledit groupe alkyle étant éventuellement substitué, notamment par un ou plusieurs substituants choisis dans le groupe constitué de :

  . un atome d'halogène,
  . un groupe $OR'_\alpha$, $R'_\alpha$ représentant un atome d'hydrogène ou un groupe alkyle comprenant de 1 à 10 atomes de carbone, de préférence de 1 à 5 atomes de carbone,
  . un groupe $NR'_\beta R'_\gamma$, $R'_\beta$ et $R'_\gamma$ représentant un groupe alkyle comprenant de 1 à 10 atomes de carbone,
  . un groupe $NR_aR_b$, $R_a$ et $R_b$ étant tels que définis précédemment,
  . un groupe $COR'_\alpha$, $R'_\alpha$ étant tel que défini ci-dessus,
  . un groupe $COOR'_\alpha$, $R'_\alpha$ étant tel que défini ci-dessus,

- un groupe $-NO_2$,
- un groupe $OR'_\alpha$, $R'_\alpha$ étant tel que défini ci-dessus,
- un groupe $-O-(CH_2)_n-O-R'_\alpha$, $R'_\alpha$ étant tel que défini ci-dessus, et représentant de préférence un groupe alkyle, et n représentant un nombre entier compris de 1 à 10, de préférence égal à 1, notamment un groupe $-OCH_2OCH_3$,
- un groupe $-NR'_\beta R'_\gamma$, $R'_\beta$ et $R'_\gamma$ étant tels que définis ci-dessus, notamment un groupe $NH_2$,
- un groupe $-NH-C(O)-NHR'_\lambda$, dans lesquels $R'_\lambda$ peut être choisi dans le groupe constitué de l'atome d'hydrogène,

des groupes alkyles comprenant de 1 à 10 atomes de carbone, des groupes aryles comprenant de 5 à 30 atomes de carbone et des hétéroaryles comprenant de 5 à 30 atomes, lesdits groupes alkyles, aryles et hétéroaryles étant éventuellement substitués ;

- un groupe $-SO_2NHR''\gamma$, $R''\gamma$ étant tel qu'il peut être choisi dans le groupe constitué de l'atome d'hydrogène, des groupes alkyles comprenant de 1 à 10 atomes de carbone, des groupes aryles comprenant de 5 à 30 atomes de carbone et des hétéroaryles comprenant de 5 à 30 atomes, lesdits groupes alkyles, aryles et hétéroaryles étant éventuellement substitués.

Selon un mode de réalisation, le radical $R'_\lambda$ est choisi dans le groupe constitué des groupes aryle, tel qu'un phényle, et alkyle, tel qu'un méthyle ou un éthyle ; lesdits groupes aryle et alkyle étant éventuellement substitués par un halogène ou un groupe - $CH_2OH$.

Selon un mode de réalisation, le radical $R'_\lambda$ est choisi dans le groupe constitué de : alkyle tel que méthyle, $-CH_2CF_3$,

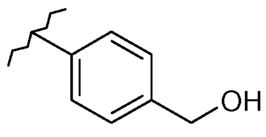

et

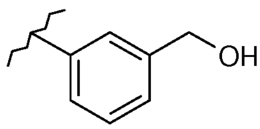

.

Parmi les groupes aryles, on peut citer :

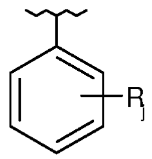

$R_j$ étant tel que défini ci-dessus, et étant de préférence choisi dans le groupe constitué de : H, $OCH_2OCH_3$, OH, OMe, $CH_2OH$, $NO_2$ $NH_2$ et NH-C(O)NHR'$_\lambda$, $R'_\lambda$ étant tel que défini ci-dessus. En particulier, $R_j$ représente l'un des groupes suivants : -NH-C(O)NHalkyle, $NH-C(O)NHCH_2CF_3$,

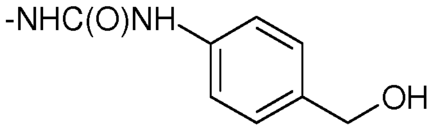

ou

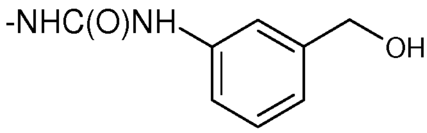

.

Parmi les groupes hétéroaryles, on peut citer :

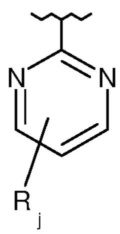

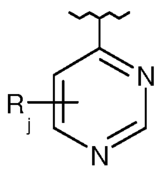

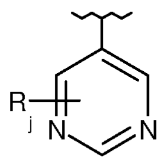

$R_j$ étant tel que défini ci-dessus, et étant de préférence choisi dans le groupe constitué de : un atome d'hydrogène ; un halogène, de préférence un atome de chlore ou de fluor ; $CF_3$ ; un groupe $NR'_\beta R'_\gamma$, $R'_\beta$ et $R'_\gamma$ étant tels que définis ci-dessus, notamment un groupe $NH_2$ ; un groupe $-O-(CH_2)_n-O-R'_\alpha$, n et $R'_\alpha$ étant tels que définis ci-dessus ; un groupe

-NH-C(O)-NHR'$_{\lambda}$, R'$_{\lambda}$ étant tel que défini précédemment, et notamment -NH-C(O)-$NH_2$ ; et un groupe -$SO_2$NHR"γ, R"$_{\gamma}$ étant tel que défini ci-dessus, et notamment -$SO_2NH_2$.

Parmi les groupes hétéroaryles, on peut citer :

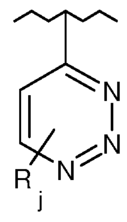 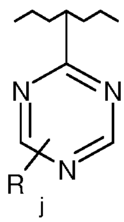

$R_j$ étant tel que défini ci-dessus, et étant de préférence choisi dans le groupe constitué de : un atome d'hydrogène ; un halogène, de préférence un atome de chlore ou de fluor ; $CF_3$ ; un groupe NR'$_{\beta}$R'$_{\gamma}$, R'$_{\beta}$ et R'$_{\gamma}$ étant tels que définis ci-dessus, notamment un groupe $NH_2$ ; un groupe -O-$(CH_2)_n$-O-R'$_{\alpha}$, R'$_{\alpha}$ étant tel que défini ci-dessus ; un groupe -NH-C(O)-NHR'$_{\lambda}$, R\ étant tel que défini précédemment, et notamment -NH-C(O)-$NH_2$ ; et un groupe -$SO_2$NHR"γ, R"γ étant tel que défini ci-dessus, et notamment -$SO_2NH_2$.

Parmi les groupes hétéroaryles, on peut citer :

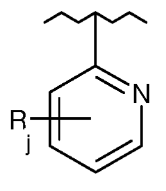 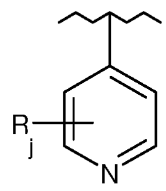 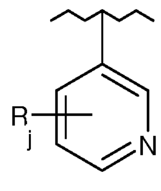

$R_j$ étant tel que défini ci-dessus, et étant de préférence choisi dans le groupe constitué de : un atome d'hydrogène ; un halogène, de préférence un atome de chlore ou de fluor ; $CF_3$ ; un groupe NR'$_{\beta}$R'$_{\gamma}$, R'$_{\beta}$ et R'$_{\gamma}$ étant tels que définis ci-dessus, notamment un groupe $NH_2$ ; un groupe -O-$(CH_2)_n$-O-R'$_{\alpha}$, R'$_{\alpha}$ étant tel que défini ci-dessus ; un groupe -NH-C(O)-NHR'$_{\lambda}$, R\ étant tel que défini précédemment, et notamment -NH-C(O)-$NH_2$ ; et un groupe -$SO_2$NHR"γ, R"γ étant tel que défini ci-dessus, et notamment -$SO_2NH_2$.

Parmi les groupes hétéroaryles, on peut également citer :

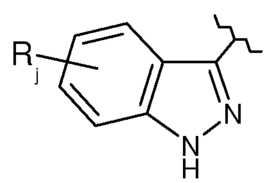 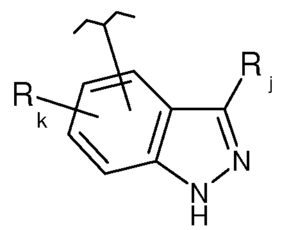

$R_j$ et $R_k$ étant tels que définis ci-dessus, et et étant de préférence indépendamment l'un de l'autre choisis dans le groupe constitué de : un atome d'hydrogène ; un halogène, de préférence un atome de chlore ou de fluor ; $CF_3$ ; un groupe NR'$_{\beta}$R'$_{\gamma}$, R'$_{\beta}$ et R'$_{\gamma}$ étant tels que définis ci-dessus, notamment un groupe $NH_2$ ; un groupe -O-$(CH_2)_n$-O-R'$_{\alpha}$, R'$_{\alpha}$ étant tel que défini ci-dessus ;un groupe -NH-C(O)-NHR\, R\ étant tel que défini précédemment, et notamment -NH-C(O)-$NH_2$ ; et un groupe -$SO_2$NHR"γ, R"γ étant tel que défini ci-dessus, et notamment -$SO_2NH_2$.

Parmi les hétéroaryles, on peut également citer :

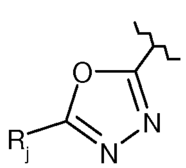 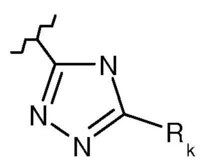 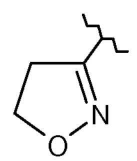

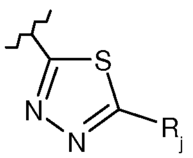

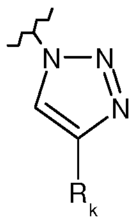

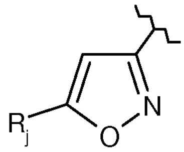

$R_j$ et $R_k$ étant tels que définis ci-dessus, et de préférence $R_j$ et $R_k$ représentant indépendamment l'un de l'autre un atome d'hydrogène ou un groupe alkyle comprenant de 1 à 10 atomes de carbone, éventuellement substitué, de préférence par un substituant choisi dans le groupe suivant :

- un atome d'halogène, tel que F ;
- un groupe $OR'_\alpha$, $R'_\alpha$ étant tel que défini ci-dessus, de préférence OH ou $OCH_3$,
- un groupe $NR_aR_b$, $R_a$ et $R_b$ étant tels que définis précédemment, de préférence choisi parmi une morpholine, une pipéridine, une N-méthylpipéridine, une pipérazine ou une N-méthylpipérazine,
- un groupe COOH ou $COOCH_3$ ;
- un groupe $NR'_\beta R'_\gamma$, avec $R'_\beta$ et $R'_\gamma$ étant tels que définis précédemment, de préférence $R'_\beta$ et $R'_\gamma$ représentant Me ;
- un groupe $-O-(CH_2)_n-OR'_\alpha$, avec n et $R'_\alpha$ étant tel que définis précédemment.

Parmi les hétéroaryles, on peut également citer :

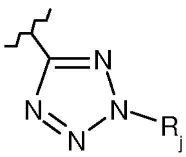

$R_j$ étant tel que défini ci-dessus, et de préférence $R_j$ représente un groupe alkyle.

L'expression "sels pharmaceutiquement acceptables" fait référence aux sels d'addition acide relativement non toxiques, inorganiques et organiques, et les sels d'addition de base, des composés de la présente invention. Ces sels peuvent être préparés *in situ* pendant l'isolement final et la purification des composés. En particulier, les sels d'addition acide peuvent être préparés en faisant réagir séparément le composé purifié sous sa forme épurée avec un acide organique ou inorganique et en isolant le sel ainsi formé. Parmi les exemples de sels d'addition acide, on trouve les sels bromhydrate, chlorhydrate, sulfate, bisulfate, phosphate, nitrate, acétate, oxalate, valérate, oléate, palmitate, stéarate, laurate, borate, benzoate, lactate, phosphate, tosylate, citrate, maléate, fumarate, succinate, tartrate, naphthylate, mésylate, glucoheptanate, lactobionate, sulfamates, malonates, salicylates, propionates, méthylènebis-b-hydroxynaphtoates, acide gentisique, iséthionates, di-p-toluoyltartrates, méthanesulfonates, éthanesulfonates, benzènesulfonates, p-toluènesulfonates, cyclohexylsulfamates et quinateslaurylsulfonate, et analogues (Voir par exemple S.M. Berge et al. « Pharmaceutical Salts » J. Pharm. Sci, 66: p.1-19 (1977)). Les sels d'addition acide peuvent également être préparés en faisant réagir séparément le composé purifié sous sa forme acide avec une base organique ou inorganique et en isolant le sel ainsi formé. Les sels d'addition acide comprennent les sels aminés et métalliques. Les sels métalliques adaptés comprennent les sels de sodium, potassium, calcium, baryum, zinc, magnésium et aluminium. Les sels de sodium et de potassium sont préférés. Les sels d'addition inorganiques de base adaptés sont préparés à partir de bases métalliques qui comprennent hydrure de sodium, hydroxyde de sodium, hydroxyde de potassium, hydroxyde de calcium, hydroxyde d'aluminium, hydroxyde de lithium, hydroxyde de magnésium, hydroxyde de zinc. Les sels d'addition aminés de base adaptés sont préparés à partir d'amines qui ont une alcalinité suffisante pour former un sel stable, et de préférence comprennent les amines qui sont souvent utilisées en chimie médicinale en raison de leur faible toxicité et de leur acceptabilité pour l'usage médical : ammoniaque, éthylènediamine, N-méthyl-glucamine, lysine, arginine, ornithine, choline, N,N'-dibenzylethylenediamine, chloroprocaïne, diéthanolamine, procaïne, N-benzylphénéthylamine, diéthylamine, pipérazine, tris(hydroxyméthyl)-aminométhane, hydroxyde de tétraméthyl-ammonium, triéthylamine, dibenzylamine, éphénamine, dehydroabiétylamine, N-éthylpipéridine, benzylamine, tétra-méthylammonium, tétraéthylammonium, méthylamine, diméthylamine, triméthyl-amine, éthylamine, acides aminés de base, par exemple lysine et arginine, et dicyclohexylamine, et analogues.

L'invention se rapporte également aux formes tautomères, aux énantiomères, diastéréoisomères, épimères et aux sels organiques ou minéraux des composés de formule générale (I).

Une famille selon la présente invention est constituée de composés de formule (I) dans laquelle $R_1$ représente

un (hétéro)aryle comprenant de 5 à 30 atomes de carbone, éventuellement substitué.

Selon un mode de réalisation, la présente invention concerne des composés de formule (I) telle que définie ci-dessus, dans laquelle $R_1$ représente un hétéroaryle comprenant de 5 à 30 atomes, éventuellement substitué.

De préférence, $R_1$ représente un indazole ou une pyrimidine, éventuellement substitué par un substituant choisi dans le groupe constitué d'un atome d'halogène, notamment un atome de chlore, ou d'un groupe $-NR'_\beta R'_\gamma$, $R'_\beta$ et $R'_\gamma$ étant tels que définis ci-dessus, notamment un groupe $NH_2$.

Préférentiellement, $R_1$ représente l'un des hétéroaryles suivants :

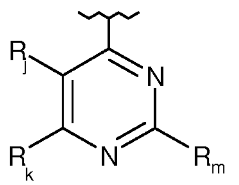

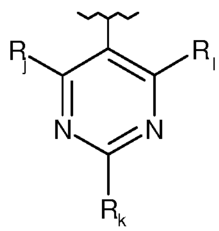

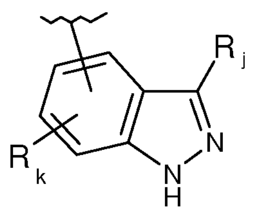

dans lesquelles, $R_j$, $R_k$, $R_l$ et $R_m$ sont tels que définis ci-dessus.

En particulier, $R_j$, $R_k$, $R_l$ et $R_m$ sont indépendamment choisis parmi un atome d'hydrogène, un chlore et un groupe $-NH_2$.

De préférence, $R_1$ représente une pyridazine ou une pyrazine éventuellement substituée. En particulier, les pyridazines et pyrazines sont substituées par un substituant choisi dans le groupe constitué d'un atome d'halogène, notamment un atome de chlore, ou d'un groupe $-NR'_\beta R'_\gamma$, $R'_\beta$ et $R'_\gamma$ étant tels que définis ci-dessus, notamment un groupe $NH_2$.

Préférentiellement, $R_1$ représente l'un des hétéroaryles suivants :

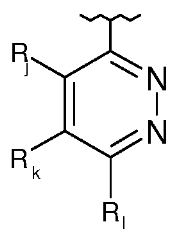

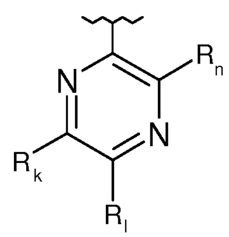

dans lesquels, $R_j$, $R_k$, $R_l$ et $R_m$ sont tels que définis ci-dessus.

De préférence, $R_1$ représente une triazine ou une pydirine, éventuellement substituée. En particulier, les triazines et pyridines sont substituées par un substituant choisi dans le groupe constitué d'un atome d'halogène, notamment un atome de chlore, ou d'un groupe $-NR'_\beta R'_\gamma$, $R'_\beta$ et $R'_\gamma$ étant tels que définis ci-dessus, notamment un groupe $NH_2$.

Préférentiellement, $R_1$ représente l'un des hétéroaryles suivants :

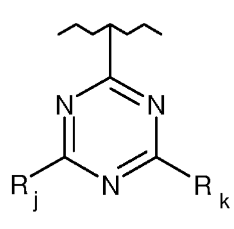

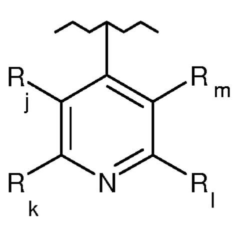

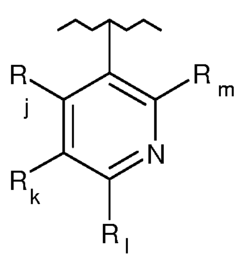

dans lesquels, $R_j$, $R_k$, $R_l$ et $R_m$ sont tels que définis ci-dessus. En particulier, $R_j$, $R_k$, $R_l$ et $R_m$ sont indépendamment choisis parmi un atome d'hydrogène, un chlore et un groupe $-NH_2$.

Selon un autre mode de réalisation, la présente invention concerne des composés de formule (I) telle que définie ci-dessus, dans laquelle $R_1$ représente un aryle comprenant de 5 à 30 atomes de carbone, éventuellement substitué.

De préférence, dans la formule (I), $R_1$ représente un groupe phényle, éventuellement substitué. En particulier, $R_1$ représente un groupe phényle, substitué par au moins un substituant choisi parmi le groupe constitué de :

- un groupe alkyle comprenant de 1 à 10 atomes de carbone, et étant de préférence un groupe méthyle, ledit groupe alkyle étant éventuellement substitué notamment par un ou plusieurs substituants $OR'_\alpha$, $R'_\alpha$ représentant notamment un atome d'hydrogène ou un groupe méthyle,
- un groupe $-NO_2$,
- un groupe $OR'_\alpha$, $R'_\alpha$ étant tel que défini ci-dessus, et représentant de préférence un atome d'hydrogène ou un méthyle,
- un groupe $-O-(CH_2)_n-O-R'_\alpha$, $R'_\alpha$ et n étant tels que définis ci-dessus, et $R'_\alpha$ représentant de préférence un groupe méthyle, et n représentant de préférence 1,
- un groupe $-NR'_\beta R'_\gamma$, $R'_\beta$ et $R'_\gamma$ étant tels que définis ci-dessus, notamment un groupe $NH_2$,
- un groupe $-NH-C(O)-NHR'_\lambda$, R\ étant tel que défini ci-dessus.

En particulier, la présente invention concerne les composés de formule (I), dans laquelle $R_1$ représente un groupe aryle suivant :

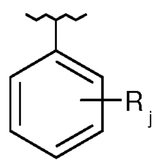

(E)

$R_j$ étant tel que défini ci-dessus, et étant de préférence choisi dans le groupe constitué de : H, $OCH_2OCH_3$, OH, OMe, $CH_2OH$, $NO_2$ $NH_2$ et $NH-C(O)NHR'_\lambda$, $R'_\lambda$, étant tel que défini ci-dessus.

Parmi les composés selon l'invention, on peut citer ceux pour lesquels $R_1$ représente un groupe (E), dans lequel $R_j$ représente un groupe -OH, de préférence en position méta.

Parmi les composés selon l'invention, on peut citer ceux pour lesquels $R_1$ représente un groupe (E), dans lequel $R_j$ représente un groupe -OH, de préférence en position orto.

Parmi les composés selon l'invention, on peut citer ceux pour lesquels $R_1$ représente un groupe (E), dans lequel $R_j$ représente un groupe -OH, de préférence en position para.

**[0075]** Parmi les composés selon l'invention, on peut citer ceux pour lesquels $R_1$ représente un groupe (E), dans lequel $R_j$ représente un groupe $-CH_2OH$, de préférence en position méta.

Parmi les composés selon l'invention, on peut citer ceux pour lesquels $R_1$ représente un groupe (E), dans lequel $R_j$ représente un groupe $-CH_2OH$, de préférence en position para.

**[0077]** Parmi les composés selon l'invention, on peut citer ceux pour lesquels $R_1$ représente un groupe (E), dans lequel $R_j$ représente un groupe $-NHC(O)NHR'_\lambda$, de préférence en position para.

**[0078]** Parmi les composés selon l'invention, on peut citer ceux pour lesquels $R_1$ représente un groupe (E), dans lequel $R_j$ représente un groupe $-NO_2$, de préférence en position para.

**[0079]** Parmi les composés selon l'invention, on peut citer ceux pour lesquels $R_1$ représente un groupe (E), dans lequel $R_j$ représente un groupe $-NH_2$, de préférence en position para.

**[0080]** Selon un mode de réalisation particulier, $R_1$ représente l'un des groupes aryles suivants :

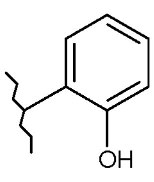
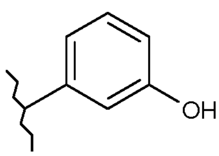
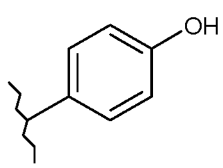

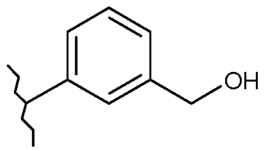
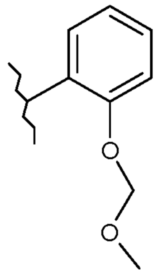
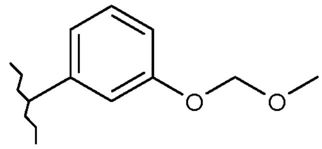
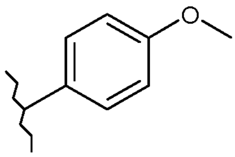
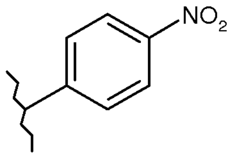
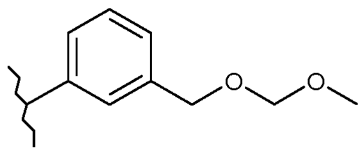
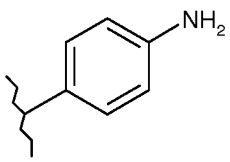
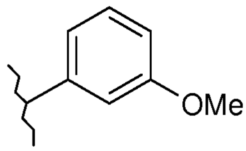
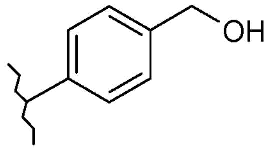
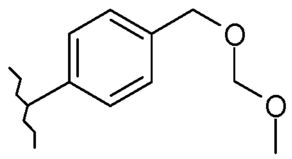
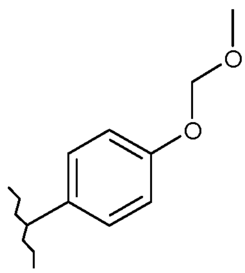

Selon un mode de réalisation particulier, $R_1$ représente le groupe aryle suivant :

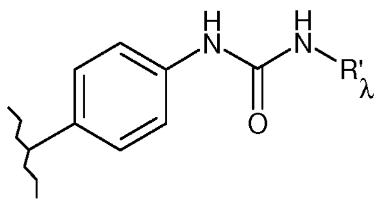

R\ étant tel que défini ci-desus. De préférence, R\ représente un groupe aryle, et notamment un phényle, éventuellement substitué par un groupe $CH_2OH$.

En particulier, $R_1$ représente le groupe aryle suivant :

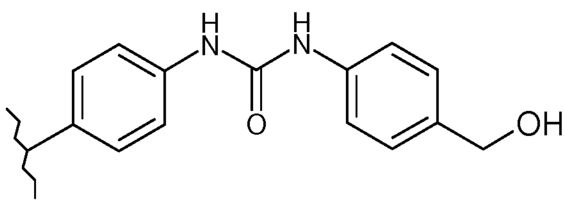

Une autre famille selon la présente invention est constituée de composés de formule (I) dans laquelle $R_1$

représente des groupes $-NR_aR_b$, $R_a$ et $R_b$ étant tels que définis ci-dessus.

Selon un mode de réalisation, la présente invention concerne des composés de formule (I) telle que définie ci-dessus, dans laquelle $R_1$ représente l'un des groupes suivants :

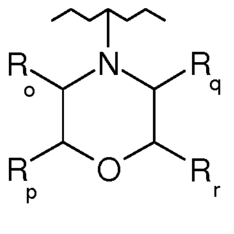

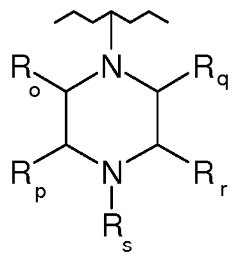

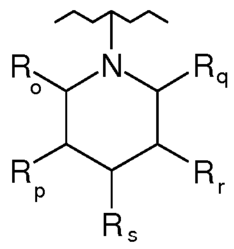

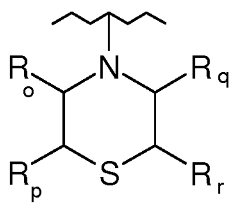

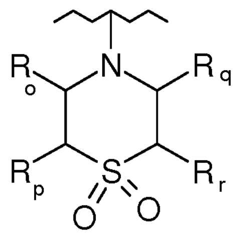

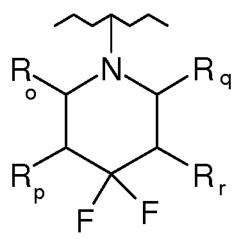

dans lesquelles, $R_o$, $R_p$, $R_q$, $R_r$ et $R_s$ représentent indépendamment les uns des autres, un substituant choisi dans le groupe constitué de : un atome d'hydrogène, un groupe alkyle comprenant de 1 à 10 atomes de carbone, un groupe $-OR'_\alpha$, $R'_\alpha$ étant tel que défini ci-dessus et un groupe $-NR'_\beta R'_\gamma$, $R'_\beta$ et $R'_\gamma$ étant tels que définis ci-dessus, ou $R_r$ et $R_p$ forment ensemble un cycle comprenant de 2 à 3 atomes de carbone.

En particulier, parmi les composés de formule (I), on peut citer ceux pour lesquels $R_o$, $R_p$, $R_q$, $R_r$ et $R_s$ représentent un atome d'hydrogène.

De préférence, $R_1$ représente le groupe suivant :

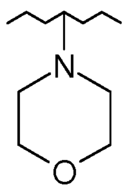

Une famille selon la présente invention est constituée de composés de formule (I) dans laquelle $R_2$ représente un hétéroaryle comprenant de 5 à 30 atomes, éventuellement substitué.

De préférence, $R_2$ représente un indazole ou une pyrimidine, éventuellement substitué par un substituant choisi dans le groupe constitué d'un atome d'halogène, notamment un atome de chlore, ou d'un groupe $-NR'_\beta R'_\gamma$, $R'_\beta$ et $R'_\gamma$ étant tels que définis ci-dessus, notamment un groupe $NH_2$,

Préférentiellement, $R_2$ représente l'un des hétéroaryles suivants :

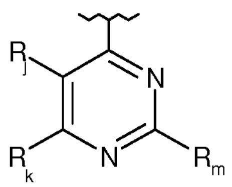

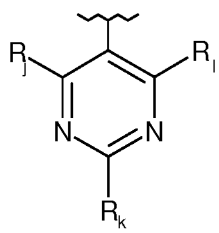

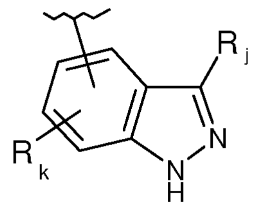

dans lesquels, $R_j$, $R_k$, $R_l$ et $R_m$ sont tels que définis ci-dessus. En particulier, $R_j$, $R_k$, $R_l$ et $R_m$ sont indépendamment choisis parmi un atome d'hydrogène, un chlore et un groupe $-NH_2$.

De préférence, $R_2$ représente une pyridazine ou une pyrazine éventuellement substituée. En particulier, les pyridazines et pyrazines sont substituées par un substituant choisi dans le groupe constitué d'un atome d'halogène, notamment un atome de chlore, ou d'un groupe $-NR'_\beta R'_\gamma$, $R'_\beta$ et $R'_\gamma$ étant tels que définis ci-dessus, notamment un groupe $NH_2$.

Préférentiellement, $R_2$ représente l'un des hétéroaryles suivants :

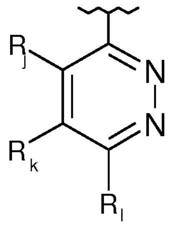

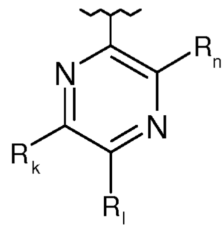

dans lesquels, $R_j$, $R_k$, $R_l$ et $R_m$ sont tels que définis ci-dessus.

De préférence, $R_2$ représente une triazine ou une pyridine, éventuellement substituée. En particulier, les triazines et pyridines sont substituées par un substituant choisi dans le groupe constitué d'un atome d'halogène, notamment un atome de chlore, ou d'un groupe $-NR'_\beta R'_\gamma$, $R'_\beta$ et $R'_\gamma$ étant tels que définis ci-dessus, notamment un groupe $NH_2$.

Préférentiellement, $R_2$ représente l'un des hétéroaryles suivants :

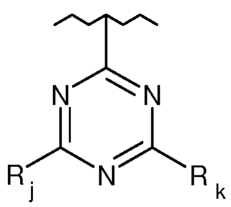

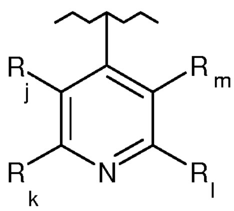

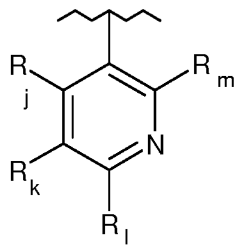

dans lesquels, $R_j$, $R_k$, $R_l$ et $R_m$ sont tels que définis ci-dessus. En particulier, $R_j$, $R_k$, $R_l$ et $R_m$ sont indépendamment choisis parmi un atome d'hydrogène, un chlore et un groupe $-NH_2$.

Selon un autre mode de réalisation, la présente invention concerne des composés de formule (I) telle que définie ci-dessus, dans laquelle $R_2$ représente des aryles comprenant de 5 à 30 atomes de carbone, éventuellement substitués.

De préférence, dans la formule (I), $R_2$ représente un groupe phényle, éventuellement substitué. En particulier, $R_2$ représente un groupe phényle, substitué par un substituant choisi parmi le groupe constitué de :

- un groupe alkyle comprenant de 1 à 10 atomes de carbone, et étant de préférence un groupe méthyle, ledit groupe alkyle étant éventuellement substitué notamment par un ou plusieurs substituants $OR'_\alpha$, $R'_\alpha$ représentant notamment un atome d'hydrogène ou un groupe méthyle,
- un groupe $-NO_2$,
- un groupe $OR'_\alpha$, $R'_\alpha$ étant tel que défini ci-dessus, et représentant de préférence un atome d'hydrogène ou un méthyle,
- un groupe $-O-(CH_2)_n-O-R'_\alpha$, $R'_\alpha$ et n étant tel que défini ci-dessus, et $R'_\alpha$ représentant de préférence un groupe méthyle, et n représentant de préférence 1,
- un groupe $-NR'_\beta R'_\gamma$, $R'_\beta$ et $R'_\gamma$ étant tels que définis ci-dessus, notamment un groupe $NH_2$,
- un groupe $-NH-C(O)-NHR'_\lambda$, R\ étant tel que défini ci-dessus.

En particulier, la présente invention concerne les composés de formule (I), dans laquelle $R_2$ représente un groupe aryle suivant :

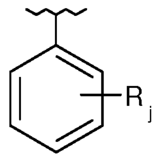

(E)

$R_j$ étant tel que défini ci-dessus, et étant de préférence choisi dans le groupe constitué de : H, $OCH_2OCH_3$, OH, OMe, $CH_2OH$, $NO_2$ $NH_2$ et NH-C(O)NHR'$_\lambda$, R'$_\lambda$, étant tel que défini ci-dessus.

Parmi les composés selon l'invention, on peut citer ceux pour lesquels $R_2$ représente un groupe (E), dans lequel $R_j$ représente un groupe -OH, de préférence en position méta.

Parmi les composés selon l'invention, on peut citer ceux pour lesquels $R_2$ représente un groupe (E), dans lequel $R_j$ représente un groupe -OH, de préférence en position orto.

Parmi les composés selon l'invention, on peut citer ceux pour lesquels $R_2$ représente un groupe (E), dans lequel $R_j$ représente un groupe -OH, de préférence en position para.

**[0100]** Parmi les composés selon l'invention, on peut citer ceux pour lesquels $R_2$ représente un groupe (E), dans lequel $R_j$ représente un groupe -$CH_2OH$, de préférence en position méta.

Parmi les composés selon l'invention, on peut citer ceux pour lesquels $R_2$ représente un groupe (E), dans lequel $R_j$ représente un groupe -$CH_2OH$, de préférence en position para.

**[0102]** Parmi les composés selon l'invention, on peut citer ceux pour lesquels $R_2$ représente un groupe (E), dans lequel $R_j$ représente un groupe -NHC(O)NHR'$_\lambda$, de préférence en position para. On peut notamment citer les composés pour lesquels $R_2$ représente un groupe (E), dans lequel $R_j$ représente un groupe -NHC(O)NHR'$_\lambda$, avec R'$_\lambda$ représentant alkyle ou aryle éventuellement substitué. De préférence, $R_j$ représente l'un des groupes suivants : NH-C(O)NH$CH_2CF_3$,

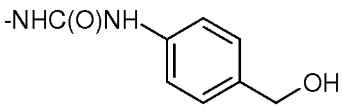 ou 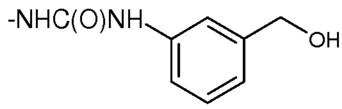 .

Parmi les composés selon l'invention, on peut citer ceux pour lesquels $R_2$ représente un groupe (E), dans lequel $R_j$ représente un groupe -$NO_2$, de préférence en position para.

**[0104]** Parmi les composés selon l'invention, on peut citer ceux pour lesquels $R_2$ représente un groupe (E), dans lequel $R_j$ représente un groupe -$NH_2$, de préférence en position para.

**[0105]** Selon un mode de réalisation particulier, $R_2$ représente l'un des groupes aryles suivants :

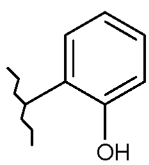
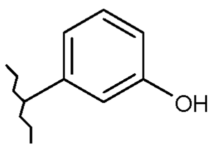
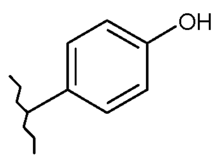

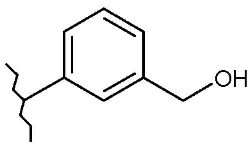
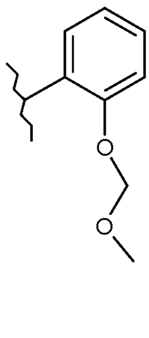
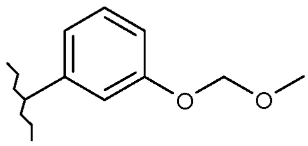

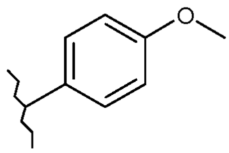

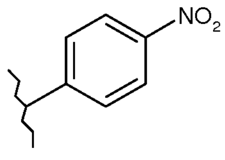

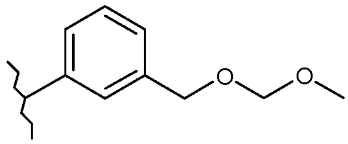

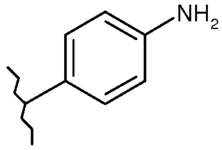

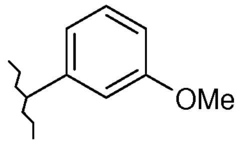

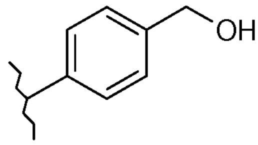

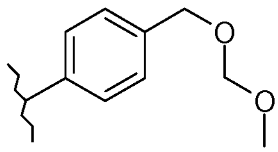

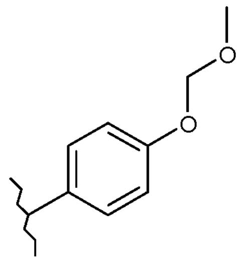

Selon un mode de réalisation particulier, $R_2$ représente le groupe aryle suivant :

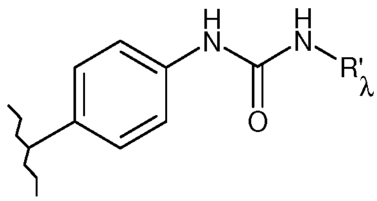

R'$_\lambda$ étant tel que défini ci-dessus.

De préférence, R'$_\lambda$ représente un groupe aryle, et notamment un phényle, éventuellement substitué par un groupe $CH_2OH$.

En particulier, $R_2$ représente le groupe aryle suivant :

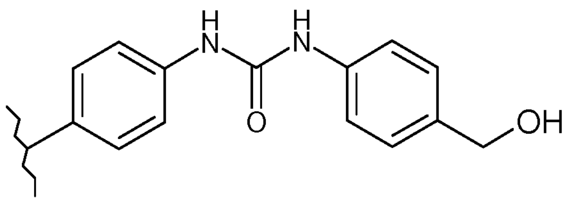

En particulier, $R_2$ représente le groupe aryle suivant :

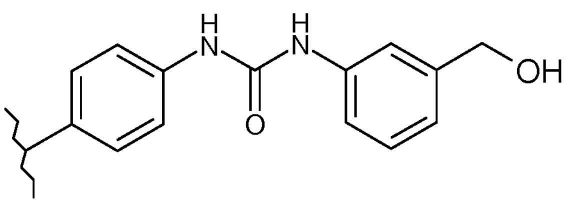

De préférence, R\ représente un groupe alkyle, et notamment un méthyle ou un éthyle, éventuellement substitué

par un halogène. En particulier, $R'_\lambda$ représente $-CH_2CF_3$.

En particulier, $R_2$ représente le groupe aryle suivant :

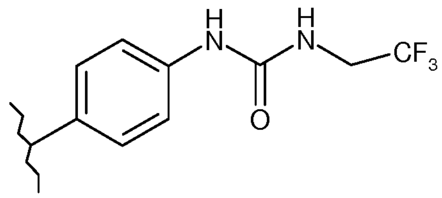

Une autre famille selon la présente invention est constituée de composés de formule (I) dans laquelle $R_2$ représente des groupes $-NR_aR_b$, $R_a$ et $R_b$ étant tels que définis ci-dessus.

Selon un mode de réalisation, la présente invention concerne des composés de formule (I) telle que définie ci-dessus, dans laquelle $R_2$ représente l'un des groupes suivants :

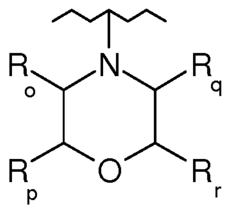

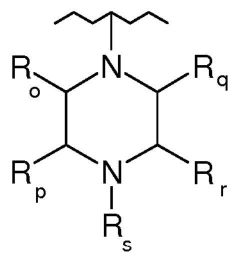

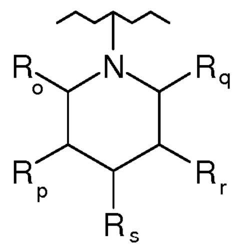

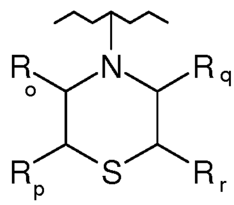

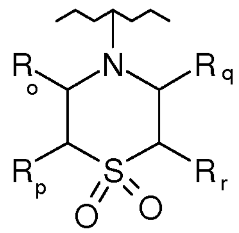

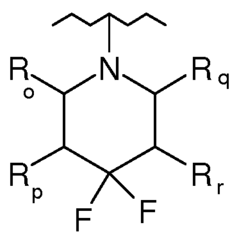

dans lesquels, $R_o$, $R_p$, $R_q$, $R_r$ et $R_s$ représentent indépendamment les uns des autres, un substituant choisi dans le groupe constitué de : un atome d'hydrogène, un groupe alkyle comprenant de 1 à 10 atomes de carbone, un groupe $-OR'_\alpha$, $R'_\alpha$ étant tel que défini ci-dessus et un groupe $-NR'_\beta R'_\gamma$, $R'_\beta$ et $R'_\gamma$ étant tels que définis ci-dessus, ou $R_r$ et $R_p$ forment ensemble un cycle comprenant de 2 à 3 atomes de carbone.

En particulier, parmi les composés de formule (I), on peut citer ceux pour lesquels $R_o$, $R_p$, $R_q$, $R_r$ et $R_s$ représentent un atome d'hydrogène.

De préférence, $R_2$ représente le groupe suivant :

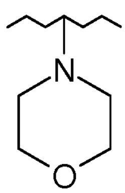

Une autre famille selon la présente invention est constituée de composés de formule (I) dans laquelle $R_2$ représente un atome d'halogène choisi parmi F, Br et I.

Parmi les composés de l'invention, on peut notamment citer les composés de formule (I-1) suivante :

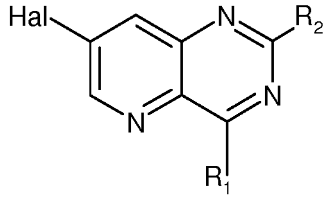

(I-1)

dans laquelle Hal représente un halogène, et $R_1$ et $R_2$ étant tels que définis précédemment.

Les composés de formule (I-1) correspondent à des composés de formule (I), dans lesquels $R_3$ représente un halogène.

Parmi les composés de formule (I-1), on peut notamment citer les composés de formules (I-1-1) et (I-1-2) suivantes :

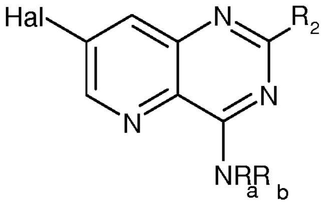

(I-1-1)

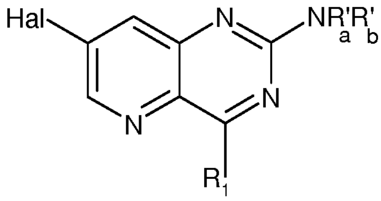

(I-1-2)

dans lesquelles Hal, $R_1$, $R_2$, $R_a$, $R_b$, $R'_a$ et $R'_b$ sont tels que définis précédemment,

Selon un mode de réalisation, dans les composés de formules (I-1), (I-1-1) et (I-1-2), Hal représente un halogène et de préférence un atome de chlore.

Selon un autre mode de réalisation, dans les composés de formules (I-1) et (I-1-2), $R_1$ est choisi dans le groupe constitué des (hétéro)aryles comprenant de 5 à 30 atomes, éventuellement substitués.

Selon un autre mode de réalisation, dans les composés de formules (I-1) et (I-1-1), $R_2$ est choisi dans le groupe constitué :

- des halogènes choisis parmi F, Br et I,
- des aryles comprenant de 5 à 30 atomes de carbone, éventuellement substitués.

Parmi les composés de l'invention, on peut également citer les composés de formule (I-2) suivante :

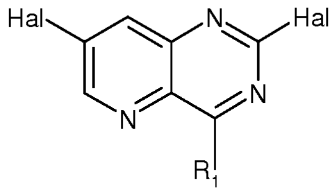

(I-2)

dans laquelle $R_1$ représente un groupe $-NR_aR_b$, $R_a$ et $R_b$ étant tels que définis ci-dessus et Hal représente un atome d'halogène, et notamment un atome de chlore.

Selon un mode de réalisation, dans la formule (I-2), $R_1$ est choisi parmi l'un des groupes suivants :

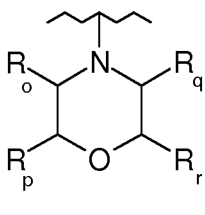

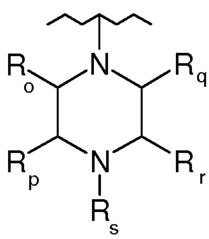

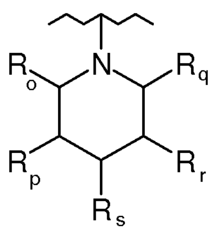

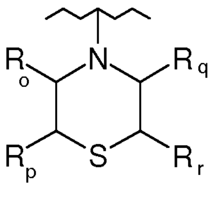

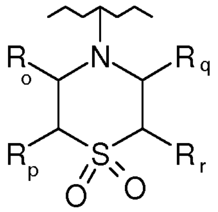

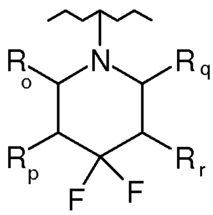

$R_o$, $R_p$, Rq, $R_r$ et $R_s$ étant tels que définis ci-dessus.

Il est décrit des composés de formule (I-2-1) suivante :

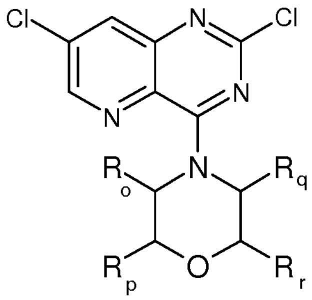

(I-2-1)

$R_o$, $R_p$, $R_q$ et $R_r$ étant tels que définis ci-dessus.

De préférence, $R_o$, $R_p$, $R_q$ et $R_r$ représentent un atome d'hydrogène. Ainsi, il est décrit un composé répondant à la formule suivante :

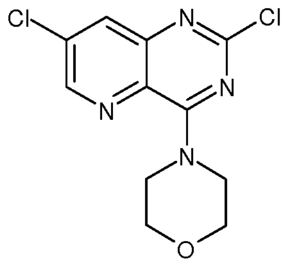

Une classe de composés selon l'invention est constituée de composés de formule (I-3) suivante :

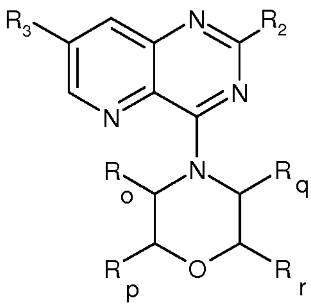

(I-3)

$R_2$, $R_3$, $R_a$, $R_p$, $R_q$ et $R_r$ étant tels que définis ci-dessus.

Parmi les composés de formule (I-3), la présente invention concerne les composés de formule (I-3-1) suivante :

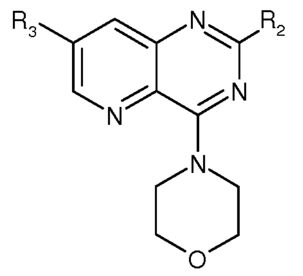

dans laquelle, $R_2$ et $R_3$ sont tels que définis ci-dessus.

Les composés de formule (I-3-1) selon l'invention, correspondent aux composés de formule (I-3) dans laquelle $R_o$, $R_p$, $R_q$ et $R_r$ représentent un atome d'hydrogène.

Selon un mode de réalisation, dans les composés de formules (I-3) et (I-3-1), $R_2$ représente un atome d'halogène choisi parmi F, Br et I.

Selon un autre mode de réalisation, dans les composés de formules (I-3) et (I-3-1), $R_2$ représente un groupe aryle comprenant de 5 à 30 atomes de carbone, ledit groupe aryle étant éventuellement substitué.

De préférence, le groupe aryle est le groupe suivant :

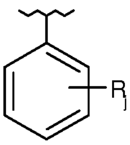

$R_j$ étant tel que défini ci-dessus, et de préférence choisi parmi : H, $OCH_2OCH_3$, OH, OMe, $CH_2OH$, $NO_2$ $NH_2$ et NH-C(O)NHR'$_{\lambda}$, R'$_{\lambda}$ étant tel que défini ci-dessus. En particulier, R'$_{\lambda}$ représente un groupe alkyle, aryle ou hétéroaryle, lesdits alkyle, aryle ou hétéroaryle étant éventuellement substitués par au moins un substituant choisi parmi un groupe $-NH_2$, un halogène, -OH, $-CH_2OH$, $CF_3$, alcoxy, $-O-(CH_2)_xOCH_3$, x étant un entier compris de 1 à 10, et -COOH.

Selon un mode de réalisation, dans les composés de formules (I-3) et (I-3-1) selon l'invention, $R_2$ représente un des groupes suivants :

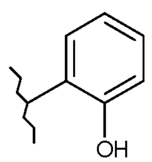
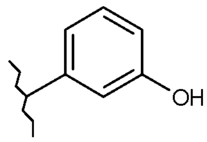
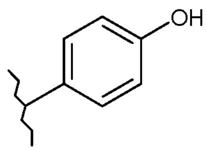

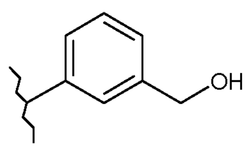
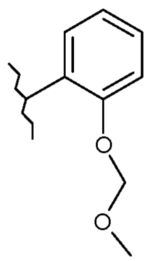
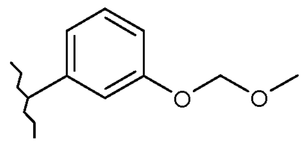

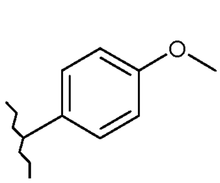
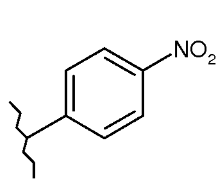
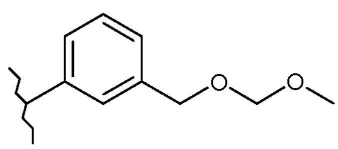

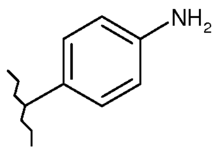

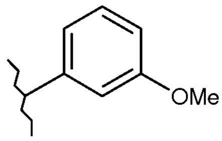

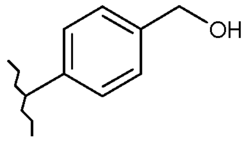

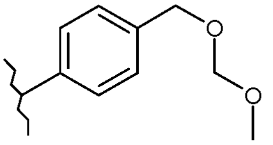

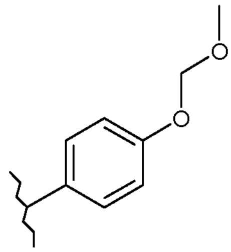

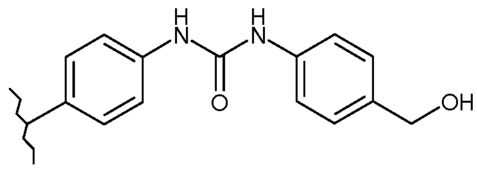

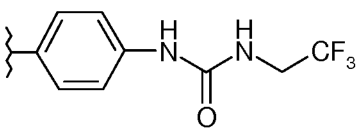

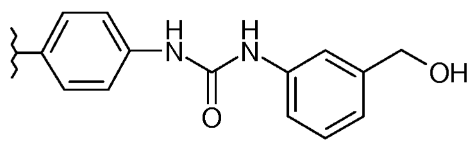

Selon un mode de réalisation, dans les composés de formules (I-3) et (I-3-1), $R_3$ représente un atome d'halogène, et notamment un chlore.

Selon un autre mode de réalisation, dans les composés de formule (I-3) et (I-3-1), $R_3$ représente un groupe alcényle comprenant de 1 à 20 atomes de carbone, éventuellement substitué. De préférence, $R_3$ représente un allyle.

Selon un autre mode de réalisation, dans les composés de formule (I-3) et (I-3-1), $R_3$ représente un groupe $-C(O)R_c$, $R_c$ représentant de préférence un atome d'hydrogène.

Selon un autre mode de réalisation, dans les composés de formule (I-3) et (I-3-1), $R_3$ représente un groupe $-C(O)OR'_c$, $R'_c$ représentant de préférence un atome d'hydrogène.

Selon un autre mode de réalisation, dans les composés de formule (I-3) et (I-3-1), $R_3$ représente un groupe $-C(R_e)=N-(OR_d)$, $R_d$ et $R_e$ étant tels que définis ci-dessus. De préférence, $R_e$ représente un atome d'hydrogène et $R_d$ représente un atome d'hydrogène ou un groupe méthyle.

Selon un autre mode de réalisation, dans les composés de formule (I-3) et (I-3-1), $R_3$ représente un groupe hétérocycloalkyle comprenant de 3 à 20 atomes de carbone, ledit groupe étant éventuellement substitué. En particulier, $R_3$ représente le groupe suivant :

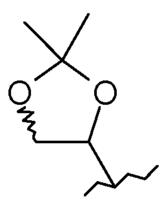

Selon un autre mode de réalisation, dans les composés de formule (I-3) et (I-3-1), $R_3$ représente un groupe alkyle comprenant de 1 à 20 atomes de carbone, ledit groupe alkyle étant éventuellement substitué par au moins un substituant choisi parmi le groupe constitué de :

- $OR_f$, $R_f$ étant tel que défini précédemment,
- $NHR_g$, $R_g$ étant tel que défini précédemment,
- $NR_hR_i$, avec $R_h$ et $R_i$ étant tels que définis précédemment,
- un halogène,

- $N_3$,
- CN, et
- un groupe (hétéro)aryle comprenant de 5 à 30 atomes, ledit groupe (hétéro)aryle étant éventuellement substitué éventuellement substitué par au moins un substituant choisi dans le groupe constitué de : $-CH_2OH$ , - $CH_2OMe$, $-CH_2NMe_2$, $-CH_2F$, $-CH_2OCH_2OMe$.

De préférence, dans les composés de formule (I-3) et (I-3-1), $R_3$ représente un groupe alkyle comprenant de 1 à 20 atomes de carbone, de préférence de 1 à 5 atomes de carbone, tel qu'un méthyle.

De préférence, dans les composés de formule (I-3) et (I-3-1), $R_3$ représente un groupe alkyle comprenant de 1 à 20 atomes de carbone, de préférence de 1 à 5 atomes de carbone, ledit groupe alkyle étant substitué par au moins un substituant choisi dans le groupe constitué de :

- OH ;
- NH-cyclopropyle ;
- NH-cyclohexyle ;
- N-morpholine ;
- N-pipérazine, éventuellement substitué par au moins un substituant choisi dans le groupe constitué de : méthyle, $-SO_2Me$, cyclohexyle et phényle ;
- OMe ;
- $N_3$ ;
- hetéroaryle, et notamment 1,2,3-triazole ou isoxazole, substitué par au moins un substituant choisi dans le groupe constitué de : $-CH_2OH$ , - $CH_2OMe$, $-CH_2NMe_2$, $-CH_2F$, $-CH_2OCH_2OMe$ ;
- CN ; et
- C(O)H.

De préférence, $R_3$ représente un groupe alkyle comprenant de 1 à 20 atomes de carbone, de préférence de 1 à 5 atomes de carbone, substitué par un des groupes suivants :

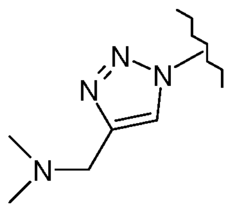
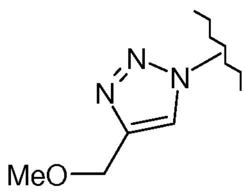
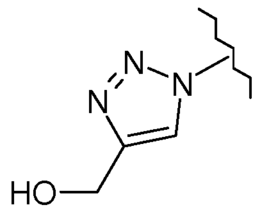
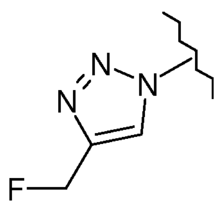
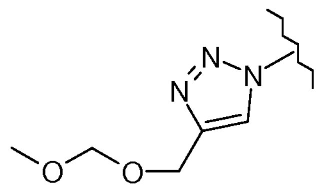
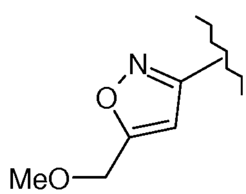

Une classe de composés selon l'invention est constituée de composés de formule (I-4) suivante :

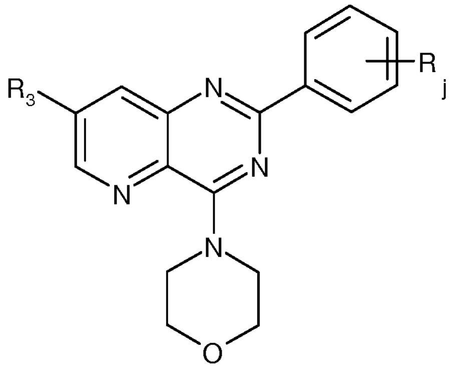

dans laquelle $R_3$ et $R_j$ sont tels que définis ci-dessus.

Une autre classe de composés selon l'invention est constituée de composés de formule (I-4-A) suivante :

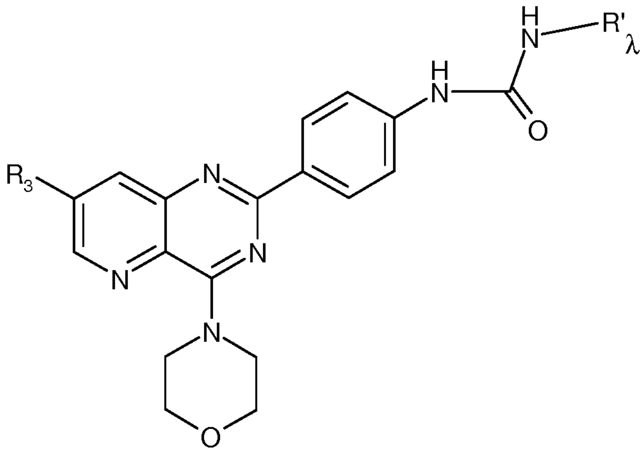

(I-4-A)

dans laquelle $R'_\lambda$ est tel que défini précédemment.

Selon un mode de réalisation, $R_3$ représente un groupe alkyle comprenant de 1 à 20 atomes de carbone, ledit groupe alkyle étant éventuellement substitué par au moins un substituant choisi parmi le groupe constitué de :

- $OR_f$, $R_f$ étant tel que défini précédemment,
- $NHR_g$, $R_g$ étant tel que défini précédemment,
- $NR_hR_i$, avec $R_h$ et $R_i$ étant tels que définis précédemment,
- un halogène, tel que F, Cl, Br ou I ;
- un groupe C(O)H,
- $-N_3$,
- -CN, et
- un groupe (hétéro)aryle comprenant de 5 à 30 atomes, tel qu'un isoxazole ou un triazole, ledit groupe (hétéro)aryle étant éventuellement substitué par au moins un substituant choisi dans le groupe constitué de : $-CH_2OH$, $-CH_2OMe$, $-CH_2NMe_2$, $-CH_2F$, $-CH_2OCH_2OMe$.

De préférence, $R_3$ représente un groupe alkyle comprenant de 1 à 20 atomes de carbone, de préférence de 1 à 5 atomes de carbone, ledit groupe alkyle étant substitué par au moins un substituant choisi dans le groupe constitué de :

- OH ;
- NH-cyclopropyle ;
- NH-cyclohexyle ;
- N-morpholine ;
- N-pipérazine, éventuellement substitué par au moins un substituant choisi dans le groupe constitué de méthyle, $-SO_2Me$, cyclohexyle, phényle ;
- OMe ;

- $N_3$ ;
- hetéroaryle, et notamment 1,2,3-triazole ou isoxazole, substitué par au moins un substituant choisi dans le groupe constitué de : $-CH_2OH$ , - $CH_2OMe$, $-CH_2NMe_2$, $-CH_2F$, $-CH_2OCH_2OMe$ ;
- CN ; et
- C(O)H.

Selon un mode de réalisation, R'$_\lambda$ représente un groupe aryle, et de préférence un phényle, éventuellement substitué.

Ainsi, la présente invention concerne les composés particuliers suivants :

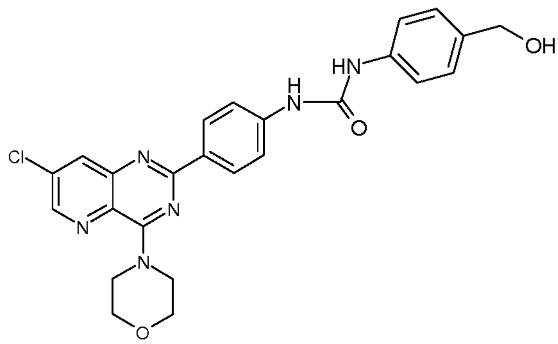

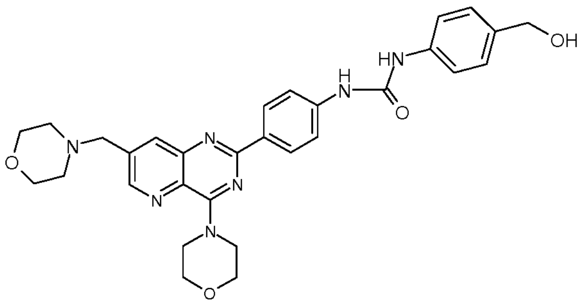

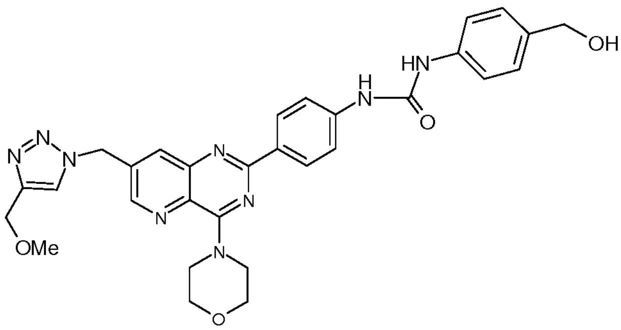

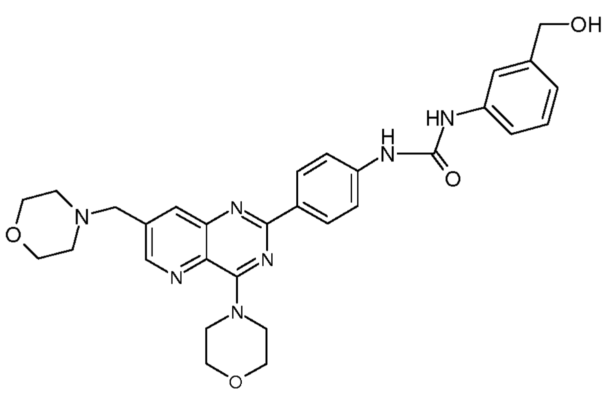

Selon un mode de réalisation, $R'_\lambda$ représente un groupe alkyle comprenant de 1 à 20 atomes de carbone, éventuellement substitué.

Ainsi, la présente invention concerne les composés particuliers suivants :

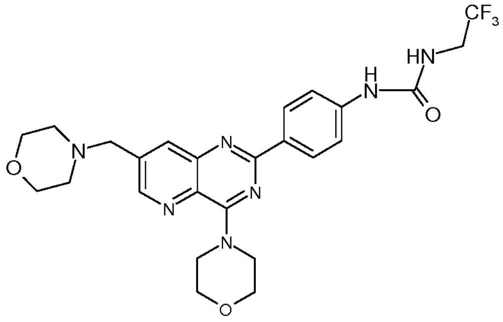

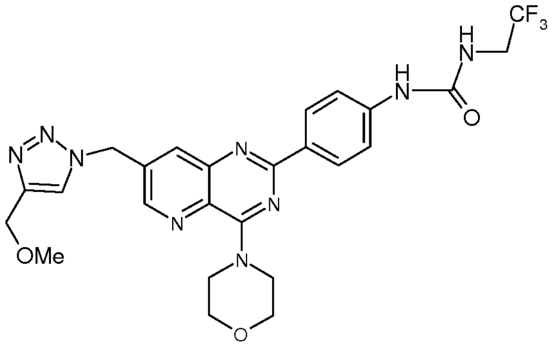

Selon un mode de réalisation, dans les composés de formule (I-4) et (I-4-A), $R_3$ représente un atome d'halogène choisi parmi F, Cl et I, et de préférence un atome de chlore.

Parmi les composés de formule (I-4), on peut notamment citer les composés de formule (I-4-a) suivante:

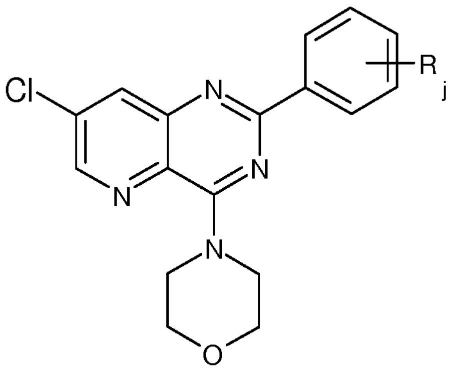

(I-4-a)

dans laquelle $R_j$ est tel que défini précédemment.

Ainsi, la présente invention concerne les composés particuliers suivants :

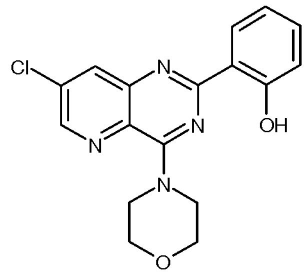

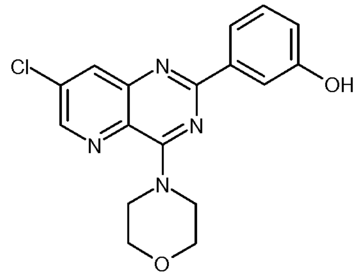

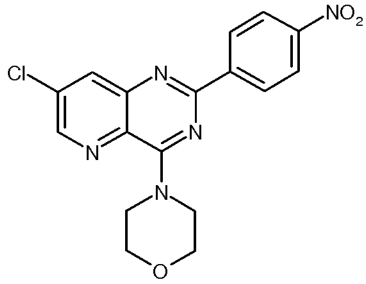

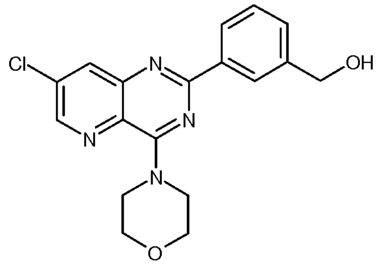

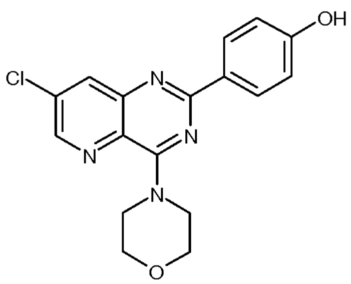

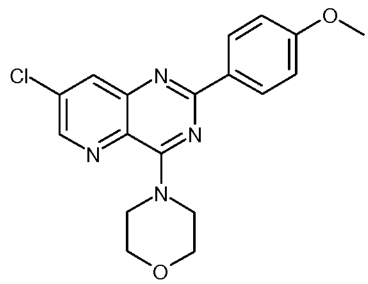

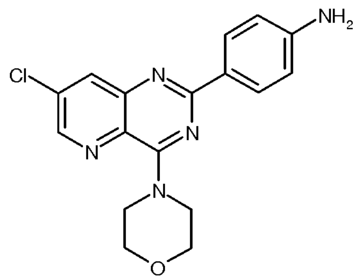

Selon un mode de réalisation, dans les composés de formule (I-4), $R_3$ représente un groupe alcényle comprenant de 1 à 20 atomes de carbone, éventuellement substitué. De préférence, $R_3$ représente un groupe alcényle non substitué comprenant de 1 à 5 atomes de carbone, et préférentiellement 2 atomes de carbone.

Parmi les composés de formule (I-4), on peut notamment citer les composés de formule (I-4-b) suivante:

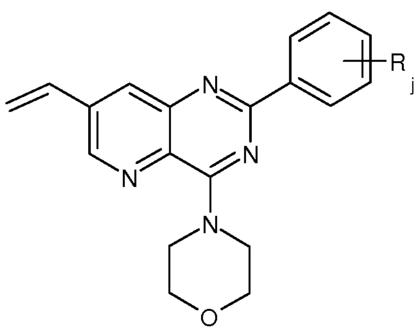

(I-4-b)

dans laquelle $R_j$ est tel que défini précédemment.

Ainsi, la présente invention concerne les composés particuliers suivants :

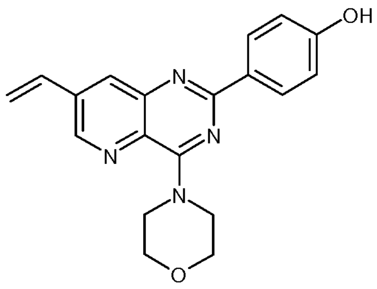

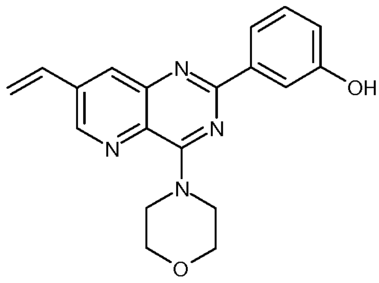

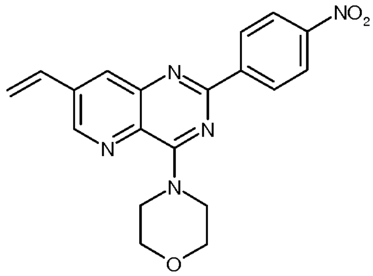

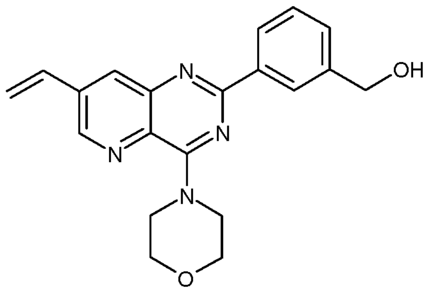

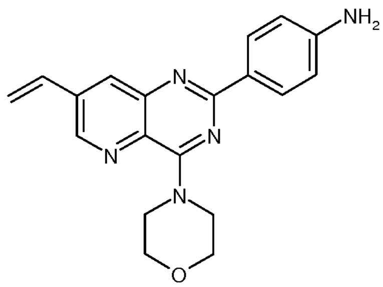

Une famille de composés selon l'invention est constituée de composés de formule (I-4) dans laquelle $R_3$ représente un groupe -C(O)$R_c$, $R_c$ étant tel que défini ci-dessus. De préférence, $R_c$ représente un atome d'hydrogène.

Parmi les composés de formule (I-4), on peut notamment citer les composés de formule (I-4-c) suivante :

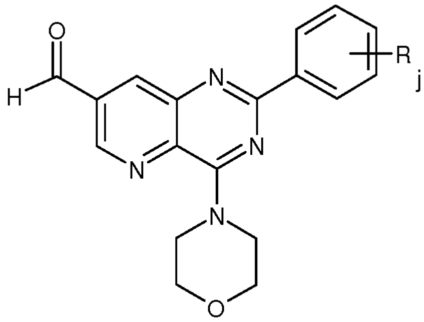

(I-4-c)

dans laquelle $R_j$ est tel que défini précédemment.

Ainsi, la présente invention concerne le composé particulier suivant :

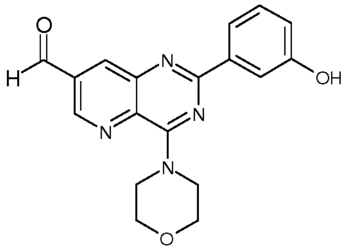

Une autre famille de composés selon l'invention est constituée de composés de formule (I-4) dans laquelle $R_3$ représente un groupe -C(O)OR'$_c$, R'$_c$ étant tel que défini précédemment. De préférence, R'$_c$ représente un atome d'hydrogène.

Parmi les composés de formule (I-4), on peut notamment citer les composés de formule (I-4-d) suivante:

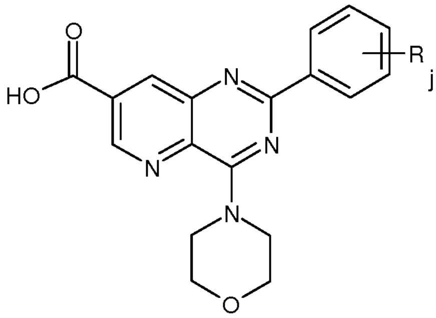

(I-4-d)

dans laquelle $R_j$ est tel que défini précédemment.

Ainsi, la présente invention concerne le composé particulier suivant :

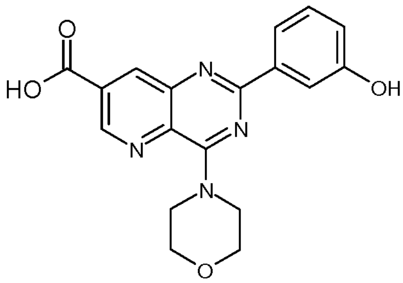

Une autre famille de composés selon l'invention est constituée de composés de formule (I-4) dans laquelle $R_3$ représente un groupe -C($R_e$)=N-(O$R_d$), $R_d$ et $R_e$ étant tels que définis ci-dessus. De préférence, $R_d$ représente un atome d'hydrogène ou un groupe méthyle.

Parmi les composés de formule (I-4), on peut notamment citer les composés de formule (I-4-e) suivante:

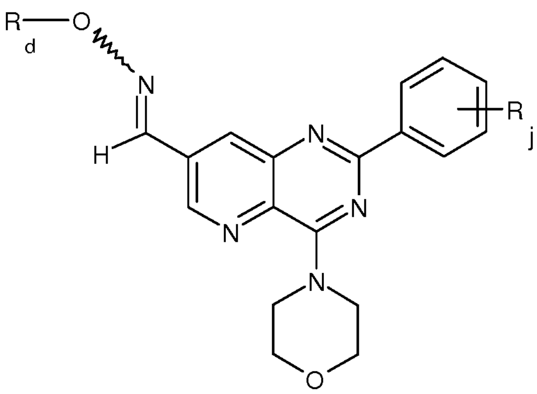

(I-4-e)

dans laquelle $R_d$ et $R_j$ sont tels que définis précédemment.

Ainsi, la présente invention concerne les composés particuliers suivants :

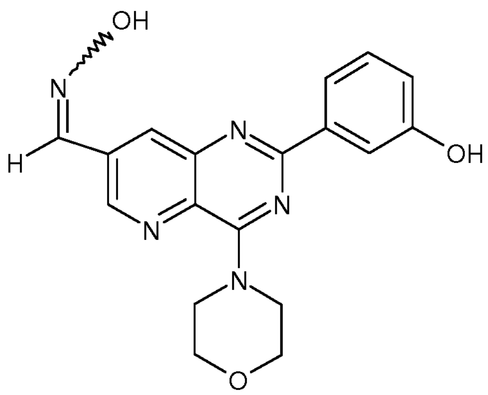

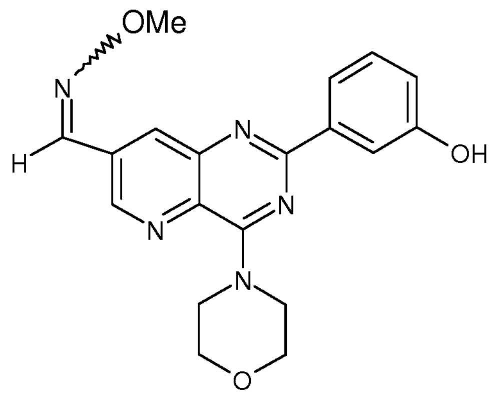

Une autre famille de composés selon l'invention est constituée de composés de formule (I-4) dans laquelle $R_3$ représente un groupe alkyle comprenant de 1 à 20 atomes de carbone, ledit groupe alkyle étant éventuellement substitué par au moins un substituant choisi parmi le groupe constitué de :

- $OR_f$, $R_f$ étant tel que défini ci-dessus,
- $NHR_g$, $R_g$ étant tel que défini ci-dessus,
- $NR_hR_i$, avec $R_h$ et $R_i$ étant tels que définis ci-dessus,
- un halogène,
- $-N_3$,
- -CN,
- un groupe (hétéro)aryle comprenant de 5 à 30 atomes, ledit groupe (hétéro)aryle étant éventuellement substitué.

Selon un mode de réalisation, parmi les composés de formule (I-4), on peut citer les composés pour lesquels $R_3$ représente un groupe alkyle comprenant de 1 à 20 atomes de carbone, étant substitué par au moins un groupe $OR_f$. De préférence, $R_3$ est un groupe éthyle substitué par deux groupements hydroxyles. De préférence, $R_3$ est un groupe éthyle substitué par deux groupements -O-méthyle.

Parmi les composés de formule (I-4), on peut notamment citer les composés de formule (I-4-f) suivante :

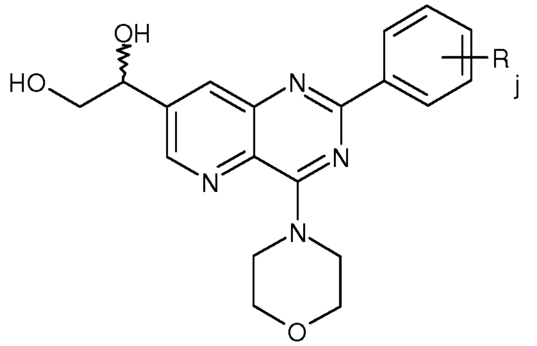

(I-4-f)

dans laquelle $R_j$ est tel que défini précédemment.

Ainsi, la présente invention concerne les composés particuliers suivants :

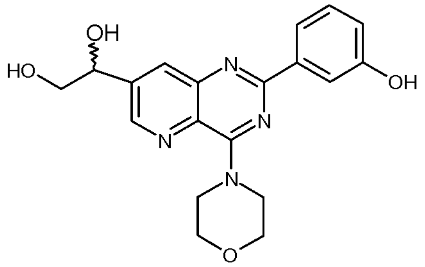 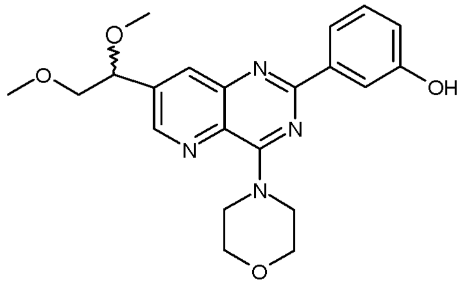

Dans le cadre de l'invention, et sauf mention contraire, le signe 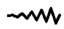 correspond à une liaison se trouvant en avant ou en arrière du plan de la molécule.

Parmi les composés de formule (I-4), on peut notamment citer les composés de formule (I-4-f') suivante :

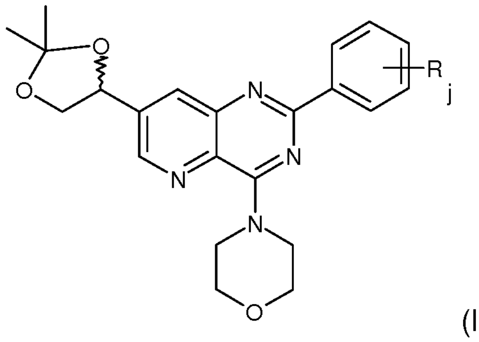

(I-4-f')

dans laquelle $R_j$ est tel que défini précédemment.

Ainsi, la présente invention concerne le composé particulier suivant :

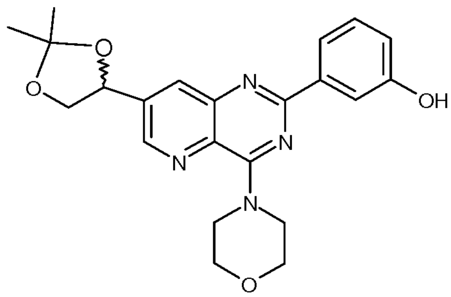

Dans le cadre de l'invention, et sauf mention contraire, le signe 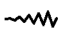 correspond à une liaison se trouvant en avant ou en arrière du plan de la molécule.

Parmi les composés de formule (I-4), on peut notamment citer les composés de formule (I-4-1) suivante :

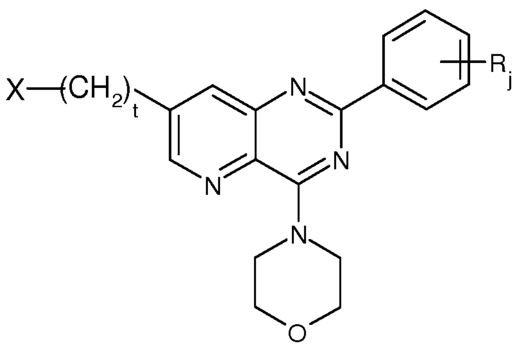

(I-4-1)

dans laquelle :

- $R_j$ est tel que défini précédemment ;
- t représente un entier compris de 1 à 12, de préférence de 1 à 5 ;
- X est choisi parmi le groupe constitué de :

  - $OR_f$, $R_f$ étant tel que défini ci-dessus ;
  - CN;
  - $N_3$ ;
  - halogène, notamment un atome d'iode ;
  - $NR_hR_i$, $R_h$ et $R_i$ étant tels que définis ci-dessus ;
  - $NHR_g$, $R_g$ étant tel que défini ci-dessus ;
  - C(O)H;
  - un groupe (hétéro)aryle comprenant de 5 à 30 atomes, ledit (hétéro)aryle étant éventuellement substitué par au moins un substituant pouvant représenter $-(CH_2)_uR''$, u représentant un entier choisi de 1 à 5, et R" représentant un groupe choisi parmi :

    - un atome d'halogène, notamment F,
    - un groupe $-OCH_2OMe$,
    - un groupe alkyle comprenant de 1 à10 atomes de carbone,
    - un groupe $-NR_4R_5$, $R_4$ et $R_5$ étant indépendamment choisis parmi un groupe alkyle comprenant de 1 à 10 atomes de carbone ou un atome d'hydrogène, tel que $NMe_2$,
    - $OR_6$, $R_6$ représentant un atome d'hydrogène ou un groupe alkyle comprenant de 1 à 10 atomes de carbone,
    - un groupe -C(O)H,
    - un groupe $-C(O)OR'_\alpha$, $R'_\alpha$ étant tel que défini précédemment,
    - un groupe $-OSO_2NHR''_\lambda$, $R''_\lambda$ étant tel que défini précédemment,
    - $NR_aR_b$, $R_a$ et $R_b$ étant tels que définis précédemment,
    - $NHCOOR'_\alpha$, $R'_\alpha$ étant tel que défini précédemment.

En particulier, u représente 1.

Parmi les composés selon l'invention, on peut citer les composés de formule (I-4-1) pour lesquels X représente OH.

Parmi les composés de formule (I-4), on peut notamment citer les composés de formule (I-4-1-a) suivante :

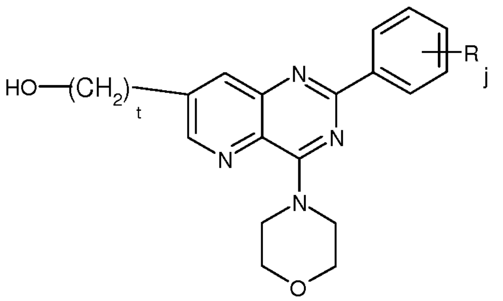

(I-4-1-a)

dans laquelle $R_j$ et t sont tels que définis précédemment.

Ainsi, la présente invention concerne le composé particulier suivant :

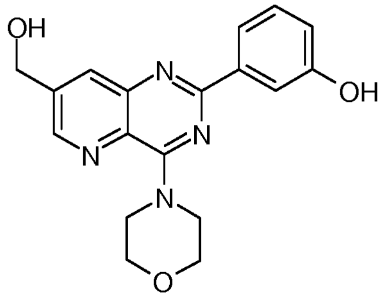

Parmi les composés selon l'invention, on peut citer les composés de formule (I-4-1) pour lesquels X représente OMe.

Parmi les composés de formule (I-4), on peut notamment citer les composés de formule (I-4-1-b) suivante:

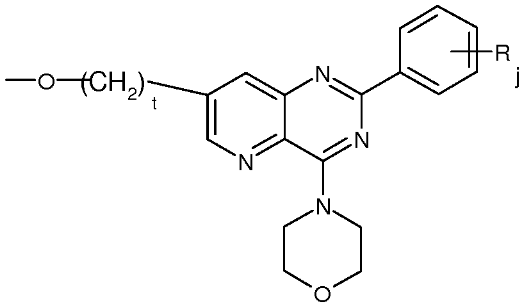

(I-4-1-b)

dans laquelle $R_j$ et t sont tels que définis précédemment.

Ainsi, parmi les composés selon l'invention, on peut citer le composé particulier suivant :

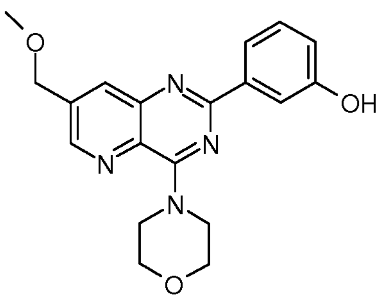

Parmi les composés selon l'invention, on peut citer les composés de formule (I-4-1) pour lesquels X représente un halogène, et notamment un atome d'iode.

Parmi les composés de formule (I-4), on peut notamment citer les composés de formule (I-4-1-c) suivante :

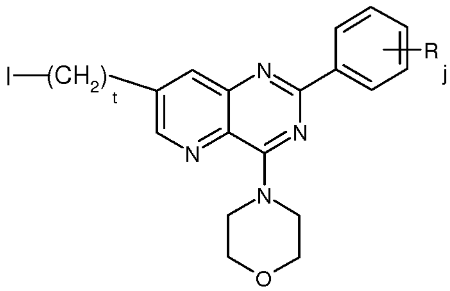

(I-4-1-c)

dans laquelle $R_j$ et t sont tels que définis précédemment.

Ainsi, la présente invention concerne le composé particulier suivant :

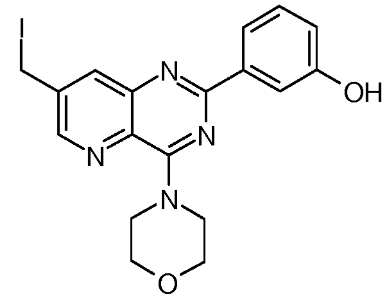

Parmi les composés selon l'invention, on peut citer les composés de formule (I-4-1) pour lesquels X représente $N_3$.

Parmi les composés de formule (I-4), on peut notamment citer les composés de formule (I-4-1-d) suivante :

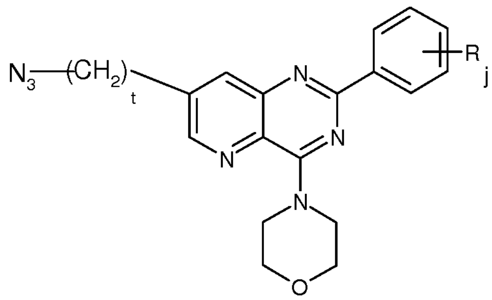

(I-4-1-d)

dans laquelle $R_j$ et t sont tels que définis précédemment.

Ainsi, la présente invention concerne le composé particulier suivant :

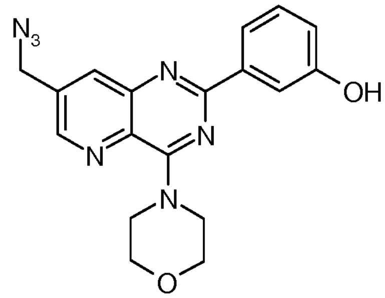

Parmi les composés selon l'invention, on peut citer les composés de formule (I-4-1) pour lesquels X représente CN.

Parmi les composés de formule (I-4), on peut notamment citer les composés de formule (I-4-1-d) suivante :

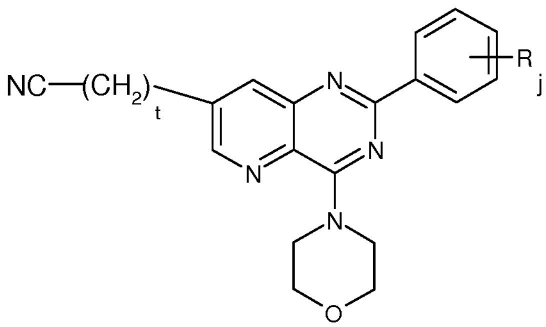

(I-4-1-e)

dans laquelle $R_j$ et t sont tels que défini précédemment.

Ainsi, la présente invention concerne le composé particulier suivant :

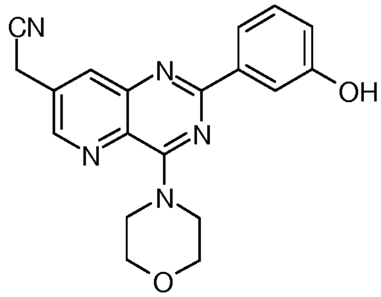

Parmi les composés selon l'invention, on peut citer les composés de formule (I-4-1) pour lesquels X représente :

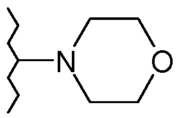

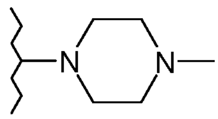

ou

Parmi les composés de formule (I-4), on peut notamment citer les composés de formule (I-4-1-f) suivante:

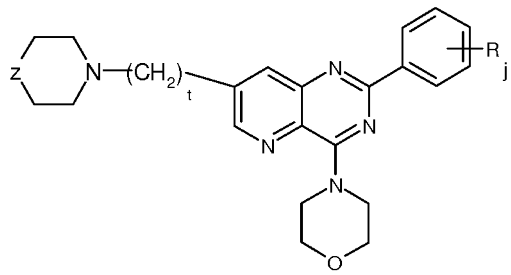

(I-4-1-f)

dans laquelle $R_j$ et t sont tels que défini précédemment et Z représente -O-, -S-, - $S(O_2)$-, -$C(F_2)$-, -$CH(CH_2OH)$, -NMe-, -$N(SO_2Me)$, -N(cyclohexyle), -N(phényle), - $CH(CH_3)$-, -N[C(O)$R'_\alpha$], $R'_\alpha$ étant tel que défini précédemment, ou -N[$S(O)_2NHR'_\lambda$]-, R\ étant tel que définit précédemment.

De préférence, $R_j$ représente -OH.

De préférence, Z représente O-, -$N(SO_2Me)$, -N(cyclohexyle), -N(phényle) ou - N(Me).

Ainsi, la présente invention concerne les composés particuliers suivants :

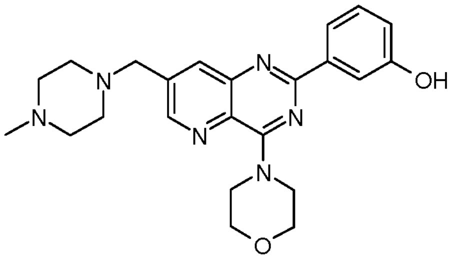

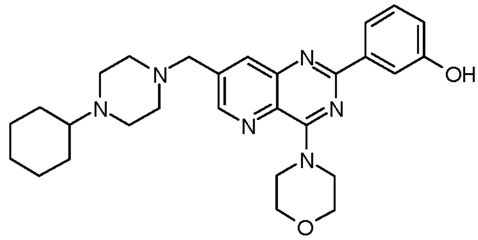

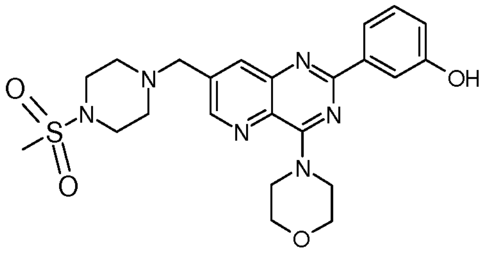
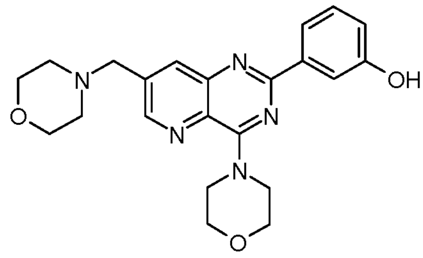

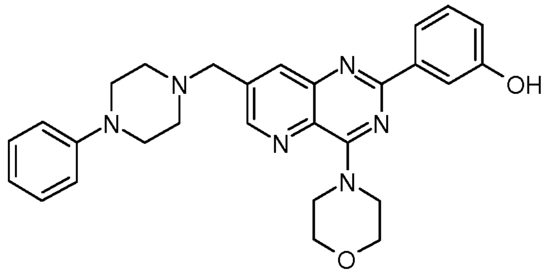

Parmi les composés selon l'invention, on peut citer les composés de formule (I-4-1) pour lesquels X représente un groupe choisi parmi :

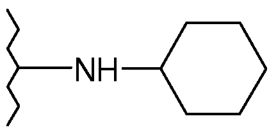
et
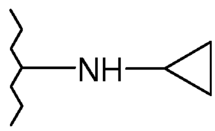

Parmi les composés de formule (I-4), on peut notamment citer les composés de formule (I-4-1-g) suivante :

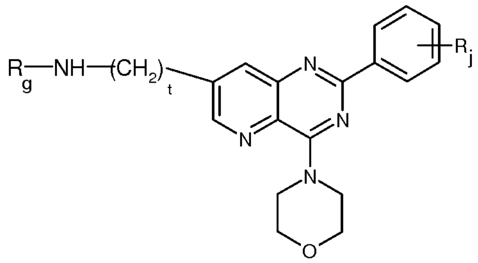
(I-4-1-g)

dans laquelle $R_g$, $R_j$ et t sont tels que définis précédemment.

Ainsi, la présente invention concerne les composés particuliers suivants :

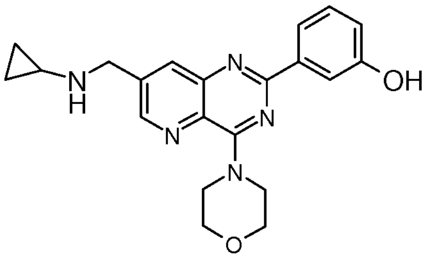
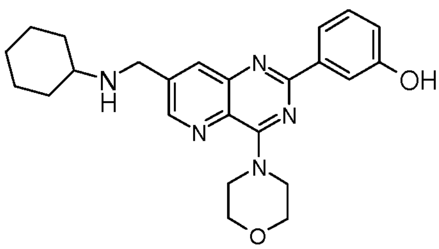

Parmi les composés selon l'invention, on peut citer les composés de formule (I-4-1) pour lesquels X représente -C(O)H.

Parmi les composés de formule (I-4), on peut notamment citer les composés de formule (I-4-1-h) suivante:

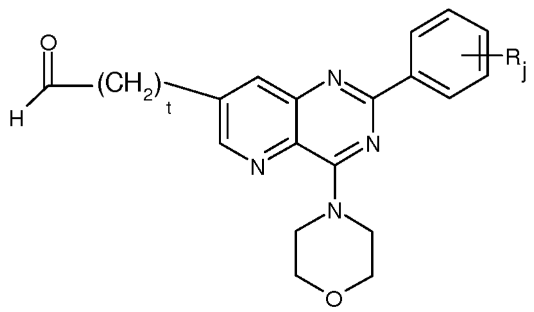

(I-4-1-h)

dans laquelle $R_j$ et t sont tels que définis précédemment.

Ainsi, la présente invention concerne le composé particulier suivant :

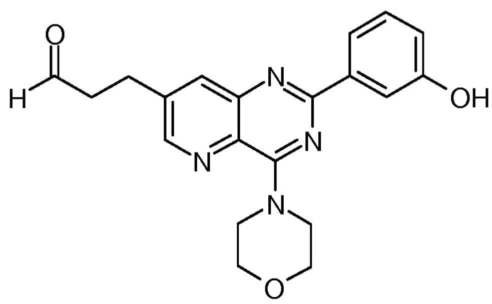

Parmi les composés selon l'invention, on peut également citer les composés de formule (I-4-1) pour lesquels X représente un groupe hétéroaryle comprenant de 5 à 30 atomes, de préférence de 5 à 10 atomes, choisi parmi le groupe constitué de :

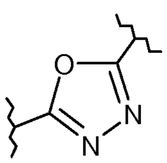
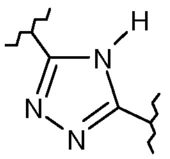
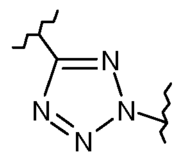
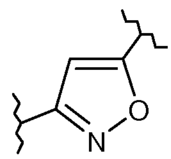
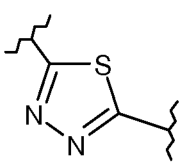
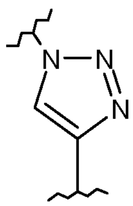
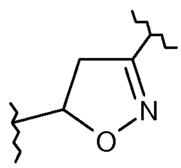

et ledit hétéroaryle étant de préférence substitué par un groupe $-(CH_2)_uR''$, u et R'' étant tels que définis précédemment.

Parmi les composés de formule (I-4), on peut notamment citer les composés de formule (I-4-1-i) suivante:

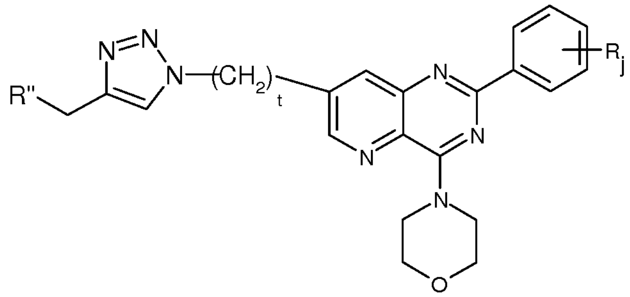

(I-4-1-i)

dans laquelle R", $R_j$ et t sont tels que définis précédemment.

Ainsi, la présente invention concerne les composés particuliers suivants :

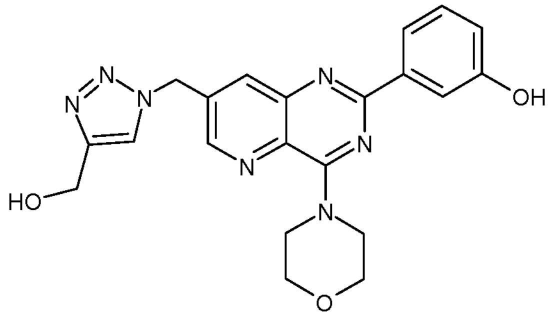

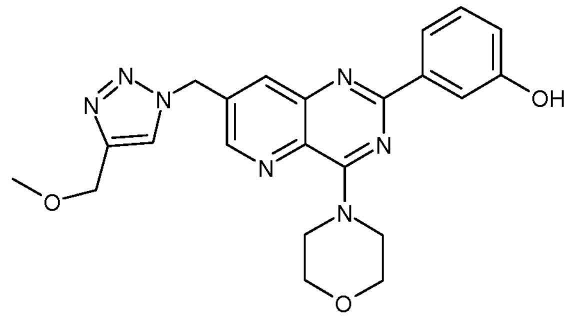

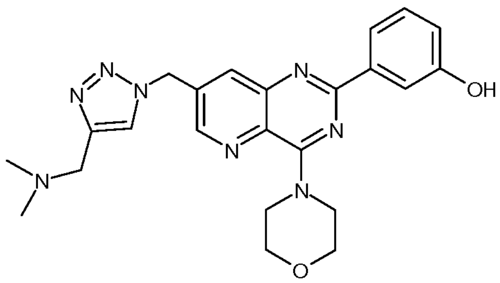

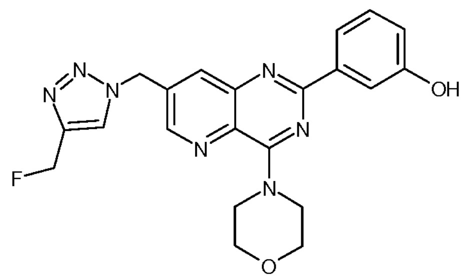

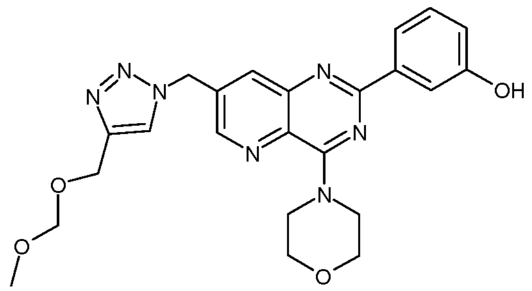

Parmi les composés de formule (I-4), on peut notamment citer les composés de formule (I-4-1-j) suivante:

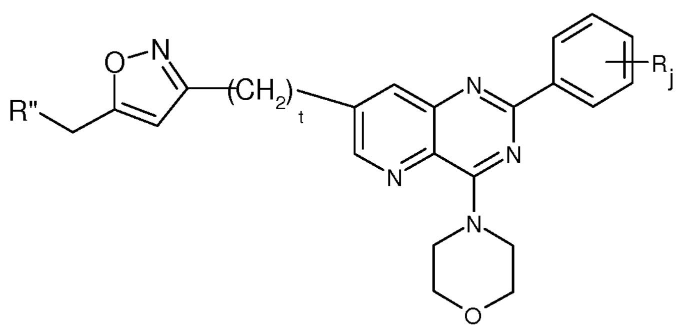

(I-4-1-j)

dans laquelle R", $R_j$ et t sont tels que définis précédemment.

Ainsi, la présente invention concerne le composé particulier suivant :

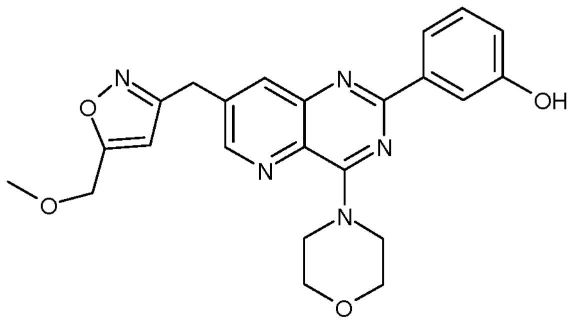

Une autre classe de composés selon l'invention est constituée de composés de formule (I-5) suivante :

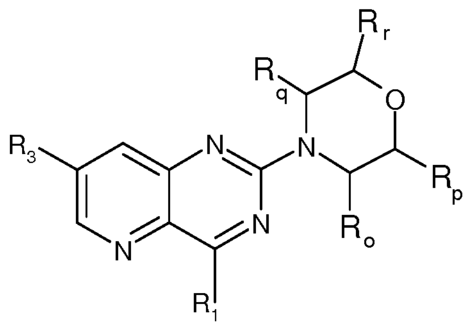

(I-5)

$R_1$, $R_3$, $R_o$, $R_p$, $R_q$ et $R_r$ étant tels que définis ci-dessus.

Parmi les composés de formule (I-5), la présente invention concerne les composés de formule (I-5-a) suivante :

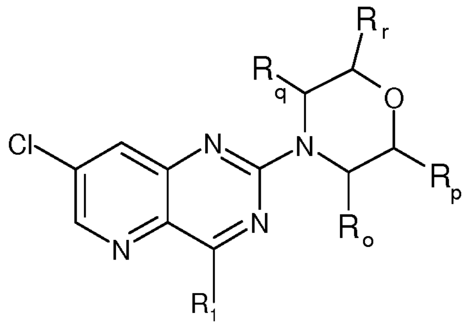

(I-5-a)

$R_1$, $R_3$, $R_o$, $R_p$, Rq et $R_r$ étant tels que définis ci-dessus.

Parmi les composés de formule (I-5), la présente invention concerne les composés de formule (I-5-1) suivante :

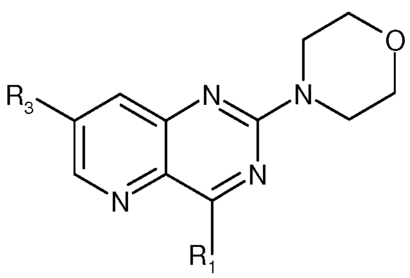

(I-5-1)

dans laquelle, $R_1$ et $R_3$ sont tels que définis ci-dessus.

Les composés de formule (I-5-1) selon l'invention, correspondent aux composés de formule (I-5) dans laquelle $R_o$, $R_p$, $R_q$ et $R_r$ représentent un atome d'hydrogène.

Selon un mode de réalisation, dans les composés de formules (I-5) et (I-5-1), $R_1$ représente un groupe hétéroaryle comprenant de 5 à 30 atomes, ledit groupe hétéroaryle étant éventuellement substitué.
De préférence, dans les composés de formules (I-5) et (I-5-1), $R_1$ représente un groupe hétéroaryle choisi parmi :

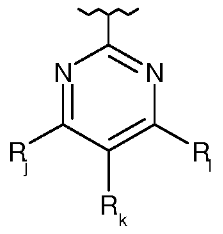
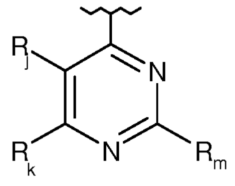
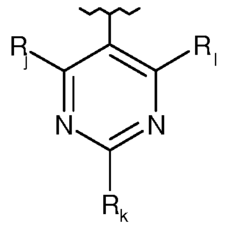
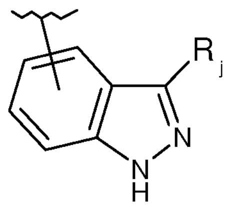
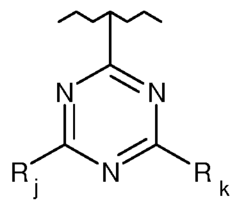
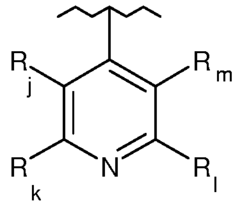
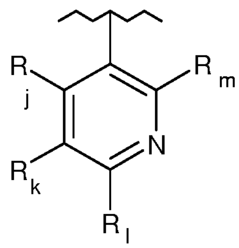

$R_j$, $R_k$, $R_l$ et $R_m$ étant tels que définis ci-dessus.

Selon un autre mode de réalisation, dans les composés de formules (I-5) et (I-5-1), $R_1$ représente un groupe aryle comprenant de 5 à 30 atomes de carbone, ledit groupe aryle étant éventuellement substitué.

De préférence, le groupe aryle est le groupe suivant :

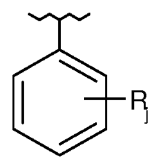

Rj étant tel que défini ci-dessus, et de préférence choisi parmi : H, $OCH_2OCH_3$, OH, OMe, $CH_2OH$ $NO_2$, $NH_2$ et NH-C(O)NHR'$_{\lambda}$, R'$_{\lambda}$ étant tel que défini ci-dessus. En particulier, R\ représente un groupe alkyle, aryle ou hétéroaryle, lesdits alkyle, aryle ou hétéroaryle étant éventuellement substitués par au moins un substituant choisi parmi un groupe $-NH_2$, -OH, alcoxy, $-O-(CH_2)_xOCH_3$, x étant un entier compris de 1 à 10, et -COOH.

Une classe de composés selon l'invention est constituée de composés de formule (I-6) suivante :

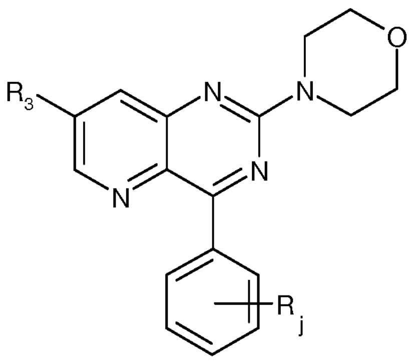

(I-6)

dans laquelle $R_3$ et $R_j$ sont tels que définis ci-dessus.

Selon un mode de réalisation, la présente invention concerne des composés de formule (I-6), dans laquelle $R_3$ représente un halogène, et notamment un atome de chlore.

Ainsi, la présente invention concerne le composé particulier suivant :

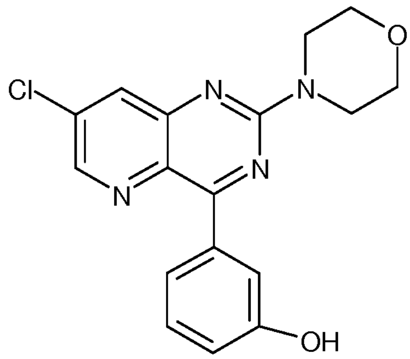

La présente invention concerne également une composition pharmaceutique comprenant un composé de formule (I) telle que définie ci-dessus ou l'un de ses sels pharmaceutiquement acceptables, ou tout composé tel que mentionné ci-dessus, en association avec un véhicule pharmaceutiquement acceptable.

La présente invention concerne donc un composé tel que défini ci-dessus de formule (I) pour son utilisation comme médicament.

Les compositions pharmaceutiques selon l'invention peuvent être présentées sous des formes destinées à l'administration par voie parentérale, orale, rectale, permuqueuse ou percutanée.

Les compositions pharmaceutiques incluant ces composés de formule générale (I) seront donc présentées sous forme de solutés ou de suspensions injectables ou flacons multi-doses, sous forme de comprimés nus ou enrobés, de dragées, de capsules, de gélules, de pilules, de cachets, de poudres, de suppositoires ou de capsules rectales, de solutions ou de suspensions, pour l'usage percutané dans un solvant polaire, pour l'usage permuqueux.

Les excipients qui conviennent pour de telles administrations sont les dérivés de la cellulose ou de la cellulose microcristalline, les carbonates alcalinoterreux, le phosphate de magnésium, les amidons, les amidons modifiés, le lactose pour les formes solides.

Pour l'usage rectal, le beurre de cacao ou les stéarates de polyéthylèneglycol sont les excipients préférés.

Pour l'usage parentéral, l'eau, les solutés aqueux, le sérum physiologique, les solutés isotoniques sont les véhicules les plus commodément utilisés.

La posologie peut varier dans les limites importantes (0,5 mg à 1 000 mg) en fonction de l'indication thérapeutique et de la voie d'administration, ainsi que de l'âge et du poids du sujet.

La présente invention concerne également un composé tel que défini ci-dessus de formule (I), ou tout composé tel que mentionné ci-dessus, pour son utilisation en tant qu'inhibiteur des enzymes PI3K et mTOR.

La présente invention concerne également un composé tel que défini ci-dessus de formule (I), ou tout composé tel que mentionné ci-dessus, pour son utilisation dans le cadre du traitement ou de la prévention de maladies liées à une dérégulation des enzymes PI3K et mTOR.

Plus particulièrement, lesdites maladies (pathologies) sont choisies dans le groupe constitué des cancers, tels que les cancers des poumons, des reins, des ovaires, du pancréas, de la peau, du colon, de la prostate, de leucémies,

des maladies non dégénératives, telles que l'arthrite, l'inflammation, les scléroses, les néphrites glomérulaires, le psoriasis, les allergies, l'asthme, les diabètes, les maladies thromboemboliques et les maladies auto-immunes.

La présente invention concerne également l'utilisation des composés de l'invention tels que définis ci-dessus, pour la préparation d'un médicament destiné au traitement ou à la prévention de maladies liées à une dérégulation des enzymes PI3K et mTOR, et plus particulièrement au traitement et à la prévention des maladies susmentionnées.

La présente invention concerne également un procédé de préparation d'un composé de formule (I-1) suivante :

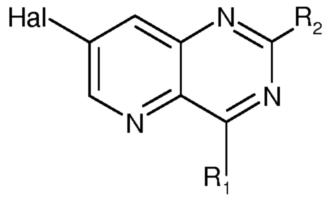

(I-1)

$R_1$, $R_2$, $R_o$, $R_p$, $R_q$ et $R_r$ étant tels que définis ci-dessus, ledit procédé comprenant les étapes suivantes :

a) une étape de substitution nucléophile aromatique (régiosélective en position 4) du composé (3) suivant :

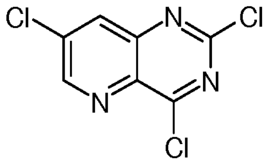

(3)

en présence du composé (3a) suivant :

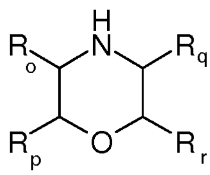

(3a)

$R_o$, $R_p$, $R_q$ et $R_r$ étant tels que définis ci-dessus, et d'une base, pour obtenir le composé intermédiaire de formule (I-2-1), tel que défini précédemment ; et
b) une étape de couplage de Suzuki (régiosélective en position 2) du composé de formule (I-2-1) susmentionnée, en présence du composé $R_2B(OH)_2$, $R_2$ étant tel que défini ci-dessus, pour obtenir le composé de formule (I-1) susmentionnée, dans laquelle Hal représente un chlore.

De préférence, l'étape a) susmentionnée est effectuée en présence de $Et_3N$. Elle est également effectuée dans un solvant tel que le THF, à température ambiante pendant 12 heures.

De préférence, l'étape b) susmentionnée est effectuée en présence de $K_2CO_3$. Elle est également effectuée en présence d'un catalyseur tel que $Pd(PPh_3)_4$, dans un solvant tel que le diméthyléther (DME), à 150°C. En particulier, la réaction est réalisée sous activation micro-ondes, pendant une heure.

La présente invention concerne également un procédé de préparation d'un composé de formule (I-5) telle que définie précédemment, ledit procédé comprenant les étapes suivantes :

a) une étape de couplage de Suzuki (régiosélective en position 4) du composé (3) tel que défini ci-dessus, en présence du composé $R_1BF_3K$, dans lequel $R_1$ représente un groupe (hétéro)aryle comprenant de 5 à 30 atomes, pour obtenir le composé intermédiaire de formule (X) suivante :

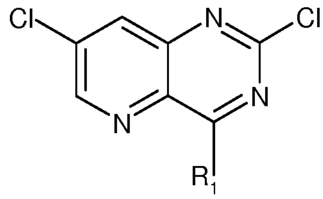

(X)

dans laquelle $R_1$ représente un groupe (hétéro)aryle éventuellement substitué tel que défini ci-dessus, et
b) une étape de couplage au palladium de type Suzuki-Myaura du composé de formule (X) susmentionnée, avec un composé de formule (3a) telle que définie ci-dessus, pour conduire à un composé de formule (I-5-a) telle que définie précédemment,

pour conduire à un composé de formule (I-5) susmentionnée.

Selon un mode de réalisation, le procédé de préparation d'un composé de formule (I-5) susmentionnée comprend une étape c) de déprotection, lorsque $R_1$ représentant un groupe (hétéro)aryle substitué, comprend un substituant hydroxyle doté d'un groupement protecteur, tel que le MOM (méthoxyméthyléther).

L'étape a) est de préférence effectuée en présence du catalyseur $Pd(PPh_3)_4$, dans un solvant tel que le toluène, à 100°C pendant environ deux heures. En particulier, la réaction est réalisée en présence de $K_2CO_3$.

L'étape b) est de préférence effectuée en présence du catalyseur $Pd(OAc)_2$, et du ligand Xantphos, dans un solvant tel que le dioxane. En particulier, la réaction est réalisée à 170°C, pendant environ une heure.

L'éventuelle étape c) est de préférence réalisée en présence d'un acide fort, notamment l'acide chlorhydrique, dans un solvant tel que le dioxane. En particulier, l'étape c) est réalisée à température ambiante.

La présente invention concerne également un procédé de préparation d'un composé de formule (I-4-b) telle que définie ci-dessus, ledit procédé comprenant une étape a) de couplage de Suzuki d'un composé de formule (I-4-a) telle que définie précédemment, avec un composé de formule ($X_1$) :

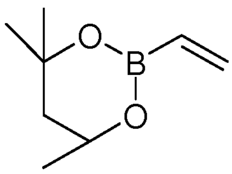

($X_1$)

pour conduire à un composé de formule (I-4-b).

L'étape a) est de préférence effectuée en présence du catalyseur $Pd(PPh_3)_4$, et de $K_2CO$, dans un solvant tel que le mélange toluène/éthanol (3/1). En particulier, la réaction est réalisée à 150°C, pendant environ une heure, sous activation micro-ondes.

Selon un mode de réalisation, le procédé de préparation d'un composé de formule (I-4-b) susmentionnée comprend une étape a') de protection de fonction hydroxyle ou amino avec un groupement protecteur tel que le MOM (méthoxyméthyléther), lorsque $R_j$ représente -OH ou $-NH_2$. En particulier, l'étape a') est réalisée en présence de MOMCl (chlorure de méthoxyméthyléther), et d'une base $K_2CO_3$. De préférence, la réaction de protection est réalisée dans un solvant, tel que l'acétone, à température ambiante, pendant environ 12 heures.

Selon un mode de réalisation, le procédé de préparation d'un composé de formule (I-4-b) susmentionnée comprend une étape a") de déprotection de la fonction hydroxyle ou amino protégée lors de l'étape a'). L'étape a") est de préférence réalisée en présence d'un acide fort, notamment l'acide chlorhydrique, dans un solvant tel que le dioxane. En particulier, l'étape a") est réalisée à température ambiante.

La présente invention concerne également un procédé de préparation d'un composé de formule (I-4-c) telle que définie ci-dessus, ledit procédé comprenant une étape d'oxydation d'un composé de formule (I-4-b) telle que définie ci-dessus, en présence de $OsO_4$.

De préférence, l'étape a) est réalisée en présence de $NaIO_4$, dans un solvant tel que le mélange $TH/H_2O$ (1/1), à température ambiante, pendant environ 3 heures.

Le procédé de préparation du composé de formule (I-4-c) peut comprendre une étape b) de déprotection, lorsque le groupe $R_j$ dans le composé (I-4-b) correspond à un groupe hydroxy protégé, notamment par un groupe $-CH_2OCH_3$ (-MOM). En particulier, l'étape b) de déprotection est réalisée en présence d'un acide fort, notamment l'acide chlorhydrique, dans un solvant tel que le dioxane. En particulier, l'étape c) est réalisée à température ambiante.

La présente invention concerne également un procédé de préparation d'un composé de formule (I-4-d) telle

que définie ci-dessus, ledit procédé comprenant une étape a) d'oxydation d'un composé de formule (I-4-c), dans lequel Rj peut représenter un groupe hydroxy éventuellement protégé par un groupement protecteur, en présence d'un oxydant.

De préférence, l'étape a) est réalisée en présence de $NaH_2PO_4$ et $NaClO_2$, et de t-BuOH, de l'eau et du 2-méthylbutène. En particulier, l'étape a) est réalisée à température ambiante, pendant environ 3 heures.

La présente invention concerne également un procédé de préparation d'un composé de formule (I-4-e) telle que définie ci-dessus, ledit procédé comprenant une étape d'amination d'un composé de formule (I-4-c) telle que définie précédemment, en présence d'un composé $R_dO-NH_2$.

De préférence, l'étape d'amination est réalisée en présence de $Et_3N$, dans un solvant tel que le dichlorométhane, au reflux, pendant environ 12 heures.

La présente invention concerne également un procédé de préparation d'un composé de formule (I-4-f) telle que définie ci-dessus, ledit procédé comprenant une étape d'oxydation d'un composé de formule (I-4-b) telle que définie ci-dessus, en présence de $OsO_4$.

De préférence, l'étape a) est réalisée en présence de NMO, dans un solvant tel que le mélange acétone/$H_2O$ (1/1), à température ambiante, pendant environ 3 heures.

Le procédé de préparation de composé de formule (I-4-d) peut éventuellement comprendre une étape b) de déprotection, lorsque le groupe $R_j$ dans le composé (I-4-b) correspond à un groupe hydroxy protégé, notamment par un groupe $-CH_2OCH_3$. En particulier, l'étape b) de déprotection est en présence d'un acide fort, notamment l'acide chlorhydrique, dans un solvant tel que le dioxane. En particulier, l'étape c) est réalisée à température ambiante.

La présente invention concerne également un procédé de préparation d'un composé de formule (I-4-1-a) telle que définie ci-dessus, ledit procédé comprenant une étape de réduction d'un composé de formule (I-4-c) susmentionnée.

De préférence, l'étape de réduction est réalisée en présence de $NaBH_4$, dans un solvant tel que le méthanol, à température ambiante, pendant environ deux heures.

Le procédé de préparation de composé de formule (I-4-1-a) peut comprendre une étape b) de déprotection, lorsque le groupe $R_j$ dans le composé (I-4-c) correspond à un groupe hydroxy protégé, notamment par un groupe $-CH_2OCH_3$ (-MOM). En particulier, l'étape b) de déprotection est réalisée en présence d'un acide fort, notamment l'acide chlorhydrique, dans un solvant tel que le dioxane.

La présente invention concerne également un procédé de préparation d'un composé de formule (I-4-1-b) telle que définie ci-dessus, ledit procédé comprenant les étapes suivantes :

- une étape a) d'alkylation d'un composé de formule (I-4-1-a) susmentionnée, et
- une éventuelle étape b) de déprotection, lorsque le groupe $R_j$ dans le composé (I-4-1-a) correspond à un groupe hydroxy protégé, notamment un groupe - $CH_2OCH_3$ (-MOM).

En particulier, l'étape b) de déprotection est réalisée en présence d'un acide fort, notamment l'acide chlorhydrique, dans un solvant tel que le dioxane.

De préférence, l'étape a) est réalisée en présence de NaH et d'un agent alkylant tel que MeI, dans un solvant tel que le méthanol, à température ambiante, pendant environ deux heures.

La présente invention concerne également un procédé de préparation d'un composé de formule (I-4-1-c) susmentionnée, ledit procédé comprenant une étape de substitution d'un composé de formule (I-4-1-a) susmentionnée.

De préférence, l'étape de substitution est réalisée en présence de $PPh_3$, de $I_2$ et d'imidazole, dans un solvant tel que le dichlorométhane, à 0°C, pendant environ sept heures.

La présente invention concerne également un procédé de préparation d'un composé de formule (I-4-1-d) telle que définie ci-dessus, ledit procédé comprenant une étape de substitution d'un composé de formule (I-4-1-a)susmentionnée, en présence notamment de $NaN_3$.

De préférence, la réaction est réalisée dans un solvant tel que le DMF, à une température de 65°C, pendant environ six heures.

Le procédé de préparation de composés de formule (I-4-1-d) peut comprendre une éventuelle étape b) de déprotection, lorsque le groupe $R_j$ dans le composé (I-4-1-a) correspond à un groupe hydroxy protégé, notamment un groupe $-CH_2OCH_3$ (-MOM). En particulier, l'étape b) de déprotection est réalisée en présence d'un acide fort, notamment l'acide chlorhydrique, dans un solvant tel que le dioxane.

La présente invention concerne également un procédé de préparation d'un composé de formule (I-4-1-e) susmentionnée, ledit procédé comprenant la mise en réaction d'un composé de formule (I-4-c) susmentionnée, avec le TosMIC (Isocyanate de p-toluenesulfonyleméthyle).

Selon un mode de réalisation, la réaction est réalisée en présence de t-BuOK, dans un solvant tel que le DME (diméthyléther), à -50°C pendant environ 40 minutes, puis dans le méthanol au reflux pendant environ une heure.

Le procédé de préparation de composés de formule (I-4-1-e) peut comprendre une éventuelle étape b) de déprotection, lorsque le groupe $R_j$ dans le composé (I-4-c) correspond à un groupe hydroxy protégé, notamment par un groupe $-CH_2OCH_3$ (-MOM). En particulier, l'étape b) de déprotection est réalisée en présence d'un acide fort, notamment l'acide chlorhydrique, dans un solvant tel que le dioxane.

La présente invention concerne également un procédé de préparation d'un composé de formule (I-4-1-f) telle que définie précédemment, ledit procédé comprenant une étape d'amination réductrice d'un composé de formule (I-4-c) susmentionnée, en présence de morpholine lorsque Z représente O ou de N-pipérazine, lorsque Z représente NMe.

Selon un mode de réalisation, l'étape d'amination est réalisée en présence de $NaB(OAc)_3$, d'acide acétique, dans un solvant tel que le dichlorométhane. De préférence, la réaction est effectuée à température ambiante pendant environ 6 heures.

Le procédé de préparation de composés de formule (I-4-1-f) peut comprendre une éventuelle étape b) de déprotection, lorsque le groupe $R_j$ dans le composé (I-4-c) correspond à un groupe hydroxy protégé, notamment par un groupe $-CH_2OCH_3$ (-MOM). En particulier, l'étape b) de déprotection est réalisée en présence d'un acide fort, notamment l'acide chlorhydrique, dans un solvant tel que le dioxane.

La présente invention concerne également un procédé de préparation d'un composé de formule (I-4-1-g) susmentionnée, ledit procédé comprenant une étape d'amination réductrice d'un composé de formule (I-4-c) susmentionnée, en présence d'un composé $R_gNH_2$.

Selon un mode de réalisation, l'étape d'amination est réalisée en présence de $NaBH_3CN$, dans un solvant tel que le dichlorométhane. De préférence, la réaction est effectuée à température ambiante pendant environ douze heures.

Le procédé de préparation de composés de formule (I-4-1-g) peut comprendre une éventuelle étape b) de déprotection, lorsque le groupe $R_j$ dans le composé (I-4-c) correspond à un groupe hydroxy protégé, notamment par un groupe $-CH_2OCH_3$ (-MOM). En particulier, l'étape b) de déprotection est réalisée en présence d'un acide fort, notamment l'acide chlorhydrique.

La présente invention concerne également un procédé de préparation d'un composé de formule (I-4-1-h) susmentionnée, ledit procédé comprenant une étape a) de couplage de Suzuki (en position 7 du noyau pyridopyrimidine) d'un composé de formule (I-4-a) susmentionnée, en présence du composé (W) suivant :

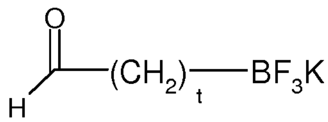

(W)

t étant tel que défini précédemment, pour conduire à un composé de formule (I-4-1-h) susmentionnée.

Selon un mode de réalisation, l'étape a) est réalisée en présence du catalyseur $Pd(PPh_3)_4$ et de la base $K_2CO_3$, dans un solvant tel que le mélange toluène/ethanol. De préférence, la réaction est effectuée à 150°C, pendant environ une heure, sous activation micro-ondes.

Le procédé de préparation de composés de formule (I-4-1-h) peut comprendre une éventuelle étape b) de déprotection, lorsque le groupe $R_j$ dans le composé (I-4-a) correspond à un groupe hydroxy protégé, notamment par un groupe $-CH_2OCH_3$ (-MOM). En particulier, l'étape b) de déprotection est réalisée en présence d'un acide fort, notamment l'acide chlorhydrique, dans un solvant tel que le dioxane.

La présente invention concerne également un procédé de préparation d'un composé de formule (I-4-1-i) susmentionnée, ledit procédé comprenant une étape a) de [3,2]-cycloaddition de type Huisgen d'un composé de formule (I-4-1-d) susmentionnée, réalisée en présence d'un composé (V) de formule suivante :

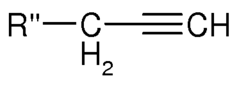

(V)

Selon un mode de réalisation, l'étape a) est réalisée en présence de CuI, dans un solvant tel que l'acétonitrile. De préférence, la réaction est effectuée à température ambiante, pendant environ douze heures.

La présente invention concerne également un procédé de préparation d'un composé de formule (I-4-A) telle que définie précédemment, ledit procédé comprenant une étape de couplage en position 2 du composés intermédiaires de formule ($X_2$) :

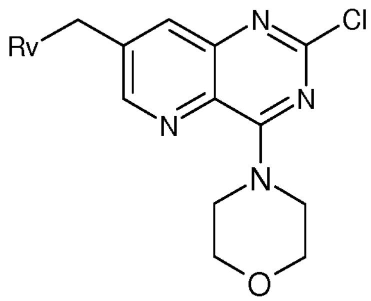

($X_2$)

dans laquelle Rv peut représenter un hétéroaryle éventuellement substitué, tel que défini précédemment, ou une morpholine,
en présence de composés borés de formule ($X_3$) suivante :

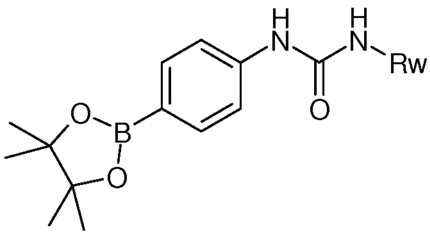

($X_3$)

dans laquelle Rw peut représenter l'un des groupes suivants :

$-CH_2CF_3$, 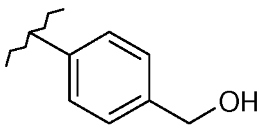 ou 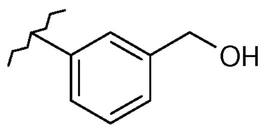 .

Selon un mode de réalisation, la réaction de couplage est réalisée en présence du catalyseur $Pd(PPh_3)_4$ et de la base $K_3PO_4$, dans un solvant tel que l'acétonitrile. De préférence, la réaction est effectuée à 120°C, pendant environ une heure, sous activation micro-ondes.

Selon un mode de réalisation, les composés de formule ($X_3$) peuvent être obtenus à partir du composé de formule ($X_4$) :

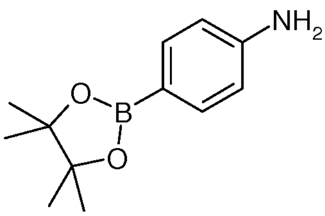

($X_4$)

en présence d'amine de formule Rw-$NH_2$.

Selon un mode de réalisation, la réaction est effectuée en présence de triphosgène et de triéthylamine, dans un solvant tel que le THF. En particulier, la réaction est réalisée pendant environ 20 heures à température ambiante.

Selon un mode de réalisation, les composés de formule ($X_2$) peuvent être obtenus à partir des composés de formule ($X_5$) :

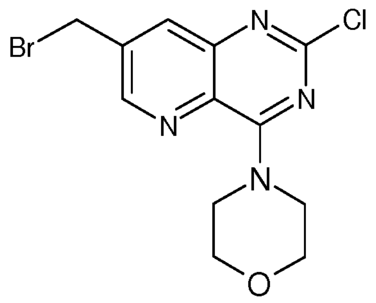

(X5)

Les exemples suivants permettent d'illustrer l'invention sans toutefois la limiter.

## Exemples

### A. Préparation des composés de l'invention

*A.1. Préparation de l'intermédiaire de synthèse (3)*

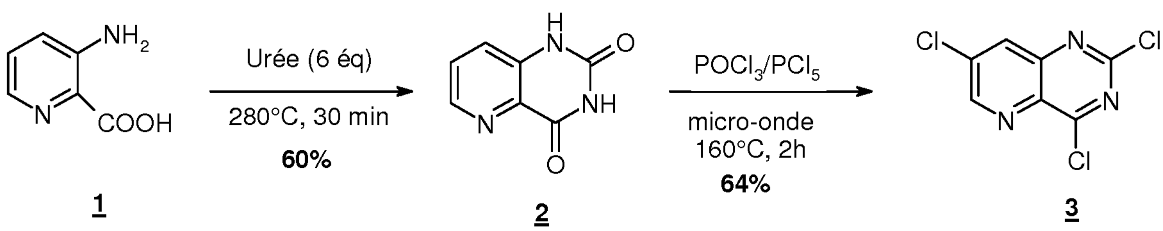


1 2 3

Le composé **(2)** a été synthétisé selon les conditions décrites dans Kad et al. (Synlett 2006, 12, 1938-1942).

**2,4,7-Trichloropyrido[3,2-*d*] pyrimidine (3).** Dans un vial de 20 mL, 1.0 g (6,13 mmol, 1 éq.) de 1*H*,3*H*-pyrido[3,2-*d*]pyrimidine-2,4-dione **(2)** est en suspension dans 10 mL d'oxychlorure de phosphore et 7.65 g (36,7 mmol , 6.0 éq.) de pentachlorure de phosphore ($PCl_5$). L'ensemble est chauffé sous irradiations micro-ondes à 160°C. Après 2 heures de réaction, l'excès de $POCl_3$ est évaporé sous pression réduite. Le résidu obtenu est amené à 0°C au moyen d'un bain de glace puis solubilisé dans le dichlorométhane, le mélange est versé dans un mélange eau/glace sans aucune basification. Après retour à température ambiante, la phase aqueuse est extraite au dichlorométhane. La phase organique est ensuite séchée sur $MgSO_4$, filtrée, puis concentrée sous pression réduite. Le résidu ainsi obtenu est chromatographié sur gel de silice (ether de pétrole/$CH_2Cl_2$, 40/60) pour donner un solide blanc avec un rendement de 64%. MP : 165-166°C ; IR (ATR, Diamond, $cm^{-1}$) $\nu$ : 3048, 2167, 1579, 1531, 1430, 1324, 1253, 1136, 1001, 872 ; RMN $^1H$ (400 MHz, $CDCl_3$) $\delta$ : 8.31 (d, 1H, $J$ = 2.2 Hz, $H_8$), 9.03 (d, 1H, $J$ = 2.2 Hz, $H_6$) ; RMN$^{13}C$ (100 MHz, $CDCl_3$) $\delta$ : 134.2 (CH), 135.1 (Cq), 138.5 (Cq), 148.8 (Cq), 152.7 (CH), 157.0 (Cq), 166.0 (Cq) ; HRMS (EI-MS) : $C_7H_2{}^{35}Cl_3N_3$, calculée m/z 232.9314, trouvée m/z 232.9323.

*A.2. Couplage de Suzuki en position 4 du composé (3)*

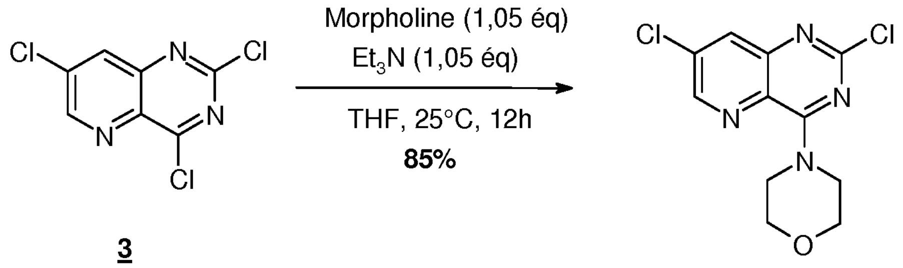


**3** **4**

Sous atmosphère d'argon, dans un ballon de 50 mL, 1,0 éq. de 2,4-trichloropyrido[3,2-d]pyrimidine (**3**) a été dissous dans 20 mL de tétrahydrofurane anhydre. 1,05 éq. de triéthylamine et 1,05 éq. de morpholine ont ensuite été ajoutées. Le mélange a été agité pendant 12 heures à température ambiante. Le solvant a alors été évaporé et le résidu a été repris dans du dichlorométhane (50 mL). La phase organique a été lavée avec une solution aqueuse saturée en $NaHCO_3$ (2 x 15 mL). La phase organique a été séchée sur $MgSO_4$, filtrée puis concentrée sous pression réduite. Le brut réactionnel a alors été purifié par colonne de chromatographie sur gel de silice sous pression (DCM/MeOH, 99/1) pour donner un solide blanc avec un rendement de 85 %. MP : 201 °C ; Infrarouge (Diamand ATR, $cm^{-1}$) $\upsilon$ : 3043, 2966, 1546, 1411, 1334, 1254, 1108, 927, 865, 686 ; RMN $^1H$ (250 MHz, $CDCl_3$) $\delta$ : 3,87 (t, 4H, $J$ = 5,0 Hz, $2xCH_2(O)$), 4,53 (m, 4H, $2xCH_2(N)$), 7,99 (dd, 1H, $J$ = 2,5 Hz, $H_8$), 8,58 (dd, 1H, $J$ = 2,5 Hz, $H_6$) ; RMN$^{13}C$ (101 MHz, $CDCl_3$) $\delta$: 47,8 ($2xCH_2$), 67,1 ($2xCH_2$), 130,7 (Cq), 133,7 (CH), 135,2 (Cq), 145,5 (CH), 149,4 (Cq), 158,3 (Cq), 159,2 (Cq) ; HRMS (EI-MS) : $C_{11}H_{10}Cl_2N_4O$ $[M+H]^+$, calculée m/z 286,0232, trouvée m/z 286,0302.

*A.3. Substitution nucléophile aromatique en position 2 du composé (4)*

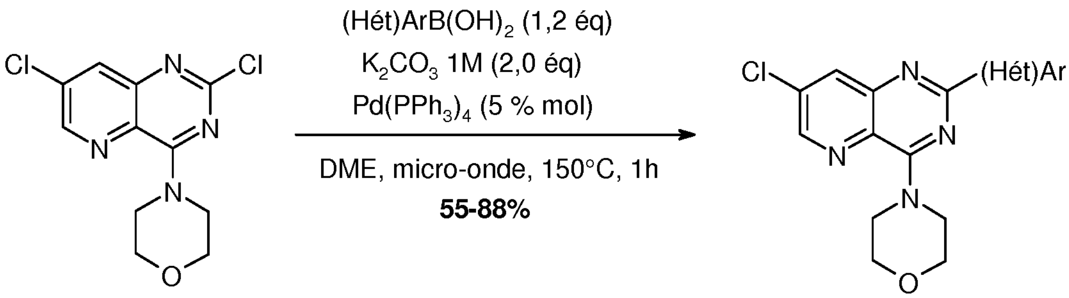


**4** **5-10**

Procédure générale A:

Sous une atmosphère d'argon, dans un vial de 20 mL, 1,0 éq. de **(4)** a été dissous dans du diméthoxyéthane (10 mL). Une solution aqueuse (1 mL) contenant 2,0 éq. de carbonate de potassium a alors été ajoutée au milieu. 1,2 éq. d'acide boronique ont été additionnés ainsi que 0,05 éq. de tetrakis(triphénylphosphino) palladium(0). Le mélange a été dégazé pendant 10 minutes avant d'être porté à 150 °C sous irradiation micro-ondes pendant 1 heure. Le milieu réactionnel résultant a été concentré sous pression réduite, puis repris dans du dichlorométhane (30 mL) et lavé avec de l'eau (2 × 10 mL). La phase organique a été séchée sur $MgSO_4$, filtrée puis concentrée sous pression réduite. Le brut réactionnel a alors été purifié par colonne de chromatographie.

| | |
|---|---|
| 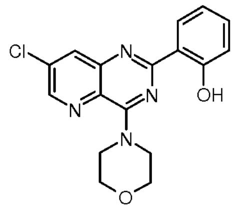 5 | 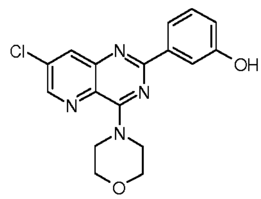 6 |
| 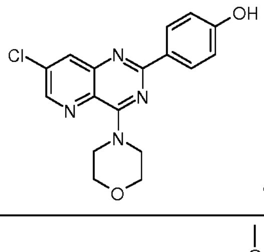 7 | 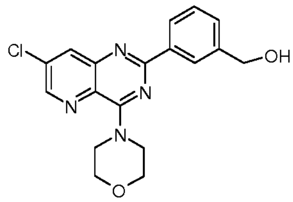 8 |
| 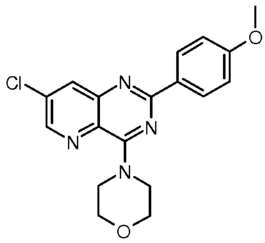 9 | 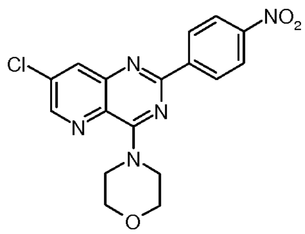 10 |

**2-(7-Chloro-4-morpholinopyrido[3,2-*d*]pyrimidin-2-yl)phénol (5)** : Le composé **(5)** a été synthétisé à partir de **(4)** (200 mg, 0,701 mmole) en suivant la procédure générale A puis purifié par colonne de chromatographie sur gel de silice sous pression (AcOEt/EP, 1/9) sous forme de solide jaune avec un rendement de 76 %. MP : 212 °C ; Infrarouge (Diamand ATR, $cm^{-1}$) υ : 2926, 1503, 1438, 1314, 1256, 1107, 868, 755 ; RMN $^1H$ (400 MHz, DMSO) δ : 3,93 (s, 4H, $2xCH_2(O)$), 4,59 (s, 4H, $2xCH_2(N)$), 6,85 (t, 1H, *J* = 8,0 Hz, $H_{arom}$), 7,08 (d, 1H, *J* = 8,0 Hz, $H_{arom}$), 7,38 (s, 1H, $H_{arom}$), 8,04 (s, 1H, $H_8$), 8,38 (d, 1H, *J* = 8,0 Hz, $H_{arom}$), 8,56 (s, 1H, $H_6$), 13,91 (s, 1H, OH) ; RMN$^{13}C$ (101 MHz, DMSO) δ : 47,6 ($2xCH_2$), 66,3 ($2xCH_2$), 116,7 (CH), 118,0 (CH), 118,8 (CH), 129,7 (CH), 130,7 (Cq), 133,2 (CH), 133,4 (Cq), 138,8 (Cq), 144,9 (CH), 148,3 (Cq), 157,4 (Cq), 158,3 (Cq), 159,8 (Cq) ; HRMS (EI-MS) : $C_{17}H_{15}ClN_4O_2$ $[M+H]^+$, calculée m/z 343,0884, trouvée m/z 343,0952.

**3-(7-Chloro-4-morpholinopyrido[3,2-*d*]pyrimidin-2-yl)phénol (6) :** Le composé (6) a été synthétisé à partir de **(4)** (200 mg, 0,701 mmole) en suivant la procédure générale A puis purifié par colonne de chromatographie sur gel de silice sous pression (AcOEt/EP, 3/7) sous forme de solide blanc avec un rendement de 73 %. MP : 230 °C ; Infrarouge (Diamand ATR, $cm^{-1}$) υ : 3301, 2853, 1527, 1425, 1370, 1270, 1229, 1107, 1022, 948, 876, 737 ; RMN $^1H$ (400 MHz, DMSO) δ : 3,85 (t, 4H, *J* = 2,5 Hz, $2xCH_2(O)$), 4,50 (m, 4H, $2xCH_2(N)$), 6,95 (d, 1H, *J* = 5,0 Hz, $H_{arom}$), 7,32 (dd, 1H, *J* = 2,5 Hz, *J* = 5,0 Hz, $H_{arom}$), 7,90 (d, 1H, *J* = 2,5 Hz, $H_{arom}$), 7,91 (s, 1H, $H_{arom}$), 8,29 (s, 1H, $H_8$), 8,75 (s, 1H, $H_6$), 9,61 (s, 1H, OH) ; RMN $^{13}C$ (101 MHz, DMSO) δ : 47,6 ($2xCH_2$), 66,3 ($2xCH_2$), 115,0 (CH), 117,8 (CH), 119,1 (CH), 129,3 (CH), 130,7 (Cq), 133,6 (Cq), 134,2 (CH), 138,8 (Cq), 144,9 (CH), 148,3 (Cq), 157,4 (Cq), 158,3 (Cq), 159,8 (Cq) ; HRMS (EI-MS) : $C_{17}H_{15}ClN_4O_2[M+H]^+$, calculée m/z 343,0884, trouvée m/z 343,0956.

**4-(7-Chloro-4-morpholinopyrido[3,2-*d*]pyrimidin-2-yl)phénol (7) :** Le composé **(7)** a été synthétisé à partir de **(4)** (200 mg, 0,701 mmole) en suivant la procédure générale A puis purifié par colonne de chromatographie sur gel de silice sous pression (AcOEt/EP, 2/8) sous forme de solide jaune avec un rendement de 76 %. MP : 253-254 °C ; Infrarouge (Diamand ATR, $cm^{-1}$) υ: 2852, 1503, 1413, 1347, 1266, 1151, 1110, 1021, 923, 804, 751 ; RMN $^1H$ (400 MHz, DMSO) δ : 3,80 (d, 4H, *J* = 3,9 Hz, $2xCH_2(O)$), 4,44 (s, 4H, $2xCH_2(N)$), 6,86 (m, 2H, $2xH_{arom}$), 8,20 (s, 1H, $H_8$), 8,27 (d, 2H, *J* = 8,4 Hz, $2xH_{arom}$), 8,66 (s, 1H, $H_6$), 9,98 (s, 1H, OH) ; RMN $^{13}C$ (101 MHz, DMSO) δ : 47,7 ($2xCH_2$), 66,4 ($2xCH_2$), 115,6 (2xCH), 128,4 (Cq), 130,2 (2xCH), 130,5 (Cq), 133,5 (Cq), 134,0 (CH), 144,4 (CH), 148,5 (Cq), 158,3 (Cq), 160,0 (Cq), 160,2 (Cq) ; HRMS (EI-MS) : $C_{17}H_{15}ClN_4O_2$ $[M+H]^+$, calculée m/z 343,0884, trouvée m/z 343,0968.

**(3-(7-Chloro-4-morpholinopyrido[3,2-d*]*pyrimidin-2-yl)phényl)méthanol (8)** : Le composé **(8)** a été synthétisé à partir de **(4)** (200 mg, 0,701 mmole) en suivant la procédure générale A puis purifié par colonne de chromatographie sur gel de silice sous pression (AcOEt/EP, 1/9) sous forme de solide jaune avec un rendement de 88 %. MP : 157 °C ;

Infrarouge (Diamand ATR, cm-$^{1}$) υ : 3174, 3044, 2855, 1507, 1417, 1278, 1115, 1025, 874, 726 ; RMN $^{1}$H (400 MHz, $CDCl_3$) δ : 3,91 (d, 4H, 2x$CH_2$(O)), 4,56 (bs, 4H, 2x$CH_2$(N)), 4,79 (s, 2H, $CH_2OH$), 7,47 (m, 2H, 2x$H_{arom}$), 8,14 (d, 1H, *J* = 2,4 Hz, $H_8$), 8,37 (d, 1H, *J* = 6,8 Hz, $H_{arom}$), 8,43 (s, 1H, $H_{arom}$), 8,55 (d, 1H, *J* = 2,4 Hz, $H_6$) ; RMN $^{13}$C (101 MHz, $CDCl_3$) δ : 47,7 (2x$CH_2$), 60,6 ($CH_2$), 66,4 (2x$CH_2$), 127,3 (CH), 128,2 (CH), 128,9 (CH), 129,5 (CH), 131,3 (Cq), 134,6 (Cq), 134,8 (CH), 138,5 (Cq), 141,3 (Cq), 145,2 (CH), 149,0 (Cq), 159,2 (Cq), 161,0 (Cq) ; HRMS (EI-MS) : $C_{18}H_{17}ClN_4O_2$ $[M+H]^+$, calculée m/z 357,1113, trouvée m/z 357,1113.

**4-(7-chloro-2-(4-méthoxyphényl)pyrido[3,2-*d*]pyrimidin-4-yl)morpholine (9)** : Le composé **(9)** a été synthétisé à partir de **(4)** (200 mg, 0,701 mmole) en suivant la procédure générale A puis purifié par colonne de chromatographie sur gel de silice sous pression (AcOEt/EP, 1/9) sous forme d'un solide jaune avec un rendement de 87 %. MP : 176 °C ; Infrarouge (Diamand ATR, cm-$^{1}$) υ : 2970, 1502, 1426, 1366, 1299, 1249, 1166, 1107, 1027, 925, 796 ; RMN $^{1}$H (400 MHz, $CDCl_3$) δ: 3,88 (s, 3H, $OCH_3$), 3,92 (d, 4H, 2x$CH_2$(O)), 4,55 (s, 4H, 2x$CH_2$(N)), 6,77 (s, 1H, $H_{arom}$), 6,99 (d, 2H, *J* = 8,8 Hz, 2x$H_{arom}$), 8,12 (d, 1H, *J* = 2,3 Hz, $H_8$), 8,43 (d, 2H, *J* = 8,8 Hz, 2x$H_{arom}$), 8,52 (d, 1H, *J* = 2,3 Hz, $H_6$) ; RMN $^{13}$C (101 MHz, $CDCl_3$) δ : 48,3 (2x$CH_2$), 55,4 ($CH_3$), 67,2 (2x$CH_2$), 113,9 (2xCH), 130,2 (2xCH), 130,8 (Cq), 131,2 (Cq), 134,4 (CH), 134,6 (Cq), 144,7 (CH), 149,2 (Cq), 159,2 (Cq), 161,0 (Cq), 162,1 (Cq) ; HRMS (EI-MS) : $C_{18}H_{17}ClN_4O_2$ $[M+H]^+$, calculée m/z 357,1040, trouvée m/z 357,1131.

4-**(7-chloro-2-(4-nitrophényl)pyrido[3,2-*d*]pyrimidin-4-yl)morpholine (10) :** Le composé **(10)** a été synthétisé à partir de **(4)** (200 mg, 0,701 mmole) en suivant la procédure générale A puis purifié par colonne de chromatographie sur gel de silice sous pression (AcOEt/EP, 1/9) sous forme de solide jaune avec un rendement de 55 %. MP : 200 °C ; Infrarouge (Diamand ATR, cm-$^{1}$) υ : 3084, 2983, 2921, 2872, 1594, 1519, 1507, 1342, 1119, 1109, 867 ; RMN $^{1}$H (250 MHz, $CDCl_3$) δ : 3,92-3,95 (m, 4H, 2x$CH_2$(O)), 4,60 (bs, 4H, 2x$CH_2$(N)), 8,18 (d, 1H, *J* = 1,5 Hz, $H_8$), 8,32 (d, 2H, *J* = 5,6 Hz, 2x$H_{arom}$), 8,62 (s, 1H, $H_6$), 8,63 (d, 2H, *J* = 5,6 Hz, 2x$H_{arom}$) ; RMN$^{13}$C (101 MHz, $CDCl_3$) δ: ; HRMS (EI-MS) : $C_{17}H_{16}ClN_5O_3[M+H]^+$, calculée m/z 372,0858, trouvée m/z 372,0859.

*A.4. Protection des fonctions hydroxyles par un groupement protecteur*

- *Voie A :*

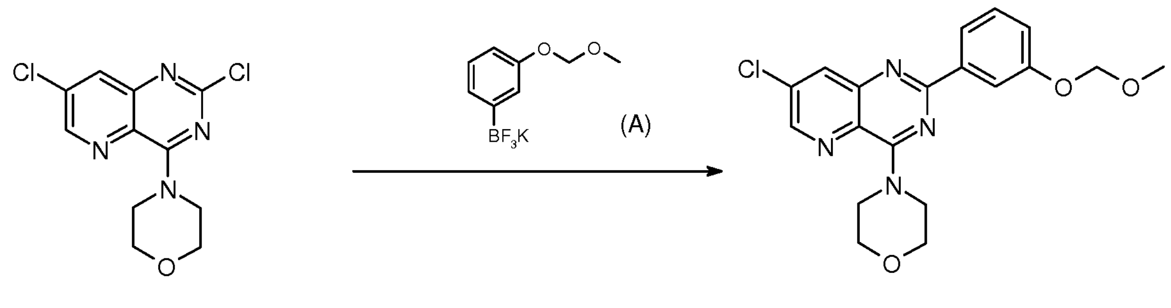


4 — 11

- *Voie B :*

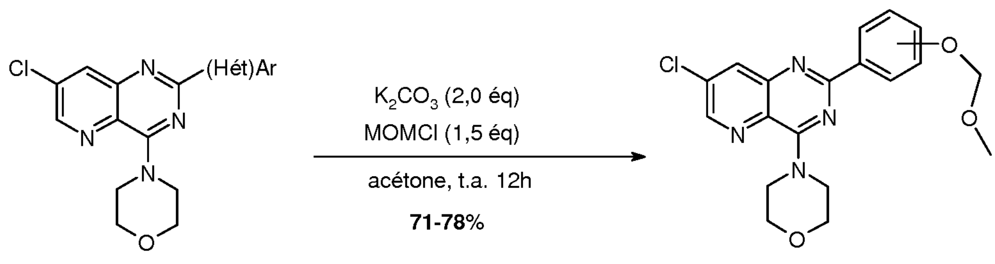


6 et 7 — 11 et 12

**4-(7-chloro-2-(3-(méthoxymethoxy)phényl)pyrido[3,2-*d*]pyrimidin-4-yl)morpholine (11)** :

- Voie A :

Le composé (11) a été synthétisé à partir de (4) (200 mg, 0,701 mmole) en suivant la procédure générale A puis purifié par colonne de chromatographie sur gel de silice sous pression (AcOEt/EP, 2/8) sous forme de solide jaune avec un rendement de 80 %.

- Voie B :

Dans un ballon de 50 mL, 200 mg (0,701 mmol, 1,0 éq.) de **(6)** ont été dissous dans de l'acétone (30 mL), 291 mg (2,1 mmol; 3,0 éq.) de carbonate de potassium et 80 μL (1,05 mmol; 1,5 éq.) de méthyle chlorométhyle éther ont été successivement additionnés dans le milieu. Le mélange a été laissé sous agitation à température ambiante pendant 12 heures. Après concentration sous pression réduite, le résidu a été repris dans 30 mL d'acétate d'éthyle. La phase organique a été lavée avec une solution saturée en bicarbonate de sodium (2 x 10 mL), séchée sur $MgSO_4$, filtrée puis concentrée sous pression réduite. Le produit **(11)** a été obtenu après purification par colonne de chromatographie sur gel de silice sous pression (AcOEt/EP, 2/8) sous forme de solide jaune avec un rendement de 71 %. MP : 196 °C ; Infrarouge (Diamand ATR, $cm^{-1}$) υ : 2950, 1516, 1454, 1344, 1307, 1266, 1148, 1074, 1009, 874, 731 ; RMN $^1H$ (400 MHz, $CDCl_3$) δ : 3,53 (s, 3H, $CH_3$), 3,92 (d, 4H, *J* = 4,9 Hz, $2xCH_2(O)$), 4,57 (bs, 4H, $2xCH_2(N)$), 5,28 (s, 2H, $CH_2OH$) 7,17 (ddd, 1H, *J* = 1,1 Hz, *J* = 2,4 Hz, *J* = 8,1 Hz, $H_{arom}$), 7,40 (t, 1H, *J* = 8,1 Hz, $H_{arom}$), 8,14 (m, 3H, $2xH_{arom}$ and $H_8$), 8,59 (d, 1H, *J* = 2,4 Hz, $H_6$) ; RMN $^{13}C$ (101 MHz, $CDCl_3$) δ : 48,3 ($2xCH_2$), 56,3 ($CH_3$), 66,6 ($CH_2$), 67,5 ($CH_2$), 94,8 ($CH_2$), 116,6 (CH), 118,7 (CH), 120,2 (Cq), 122,4 (CH), 129,6 (CH), 131,3 (Cq), 135,0 (CH), 139,8 (Cq), 145,2 (CH), 149,1 (Cq), 157,6 (Cq), 159,2 (Cq), 160,9 (Cq) ; HRMS (EI-MS) : $C_{19}H_{19}ClN_4O_3$ $[M+H]^+$, calculée m/z 387,1146, trouvée m/z 387,1151.

**4-(7-chloro-2-(4-(méthoxymethoxy)phényl)pyrido[3,2-*d*]pyrimidin-4-yl)morpholine (12) :** Dans un ballon de 50 mL, 200 mg (0,701 mmol, 1,0 éq.) de **(6)** ont été dissous dans de l'acétone (30 mL), 291 mg (2,1 mmol; 3,0 éq.) de carbonate de potassium et 80 μL (1,05 mmol; 1,5 éq.) de méthyle chlorométhyle éther ont été additionnés dans le milieu. Le mélange a été laissé sous agitation à température ambiante pendant 12 heures. Après concentration sous pression réduite, le résidu a été repris dans 30 mL d'acétate d'éthyle. La phase organique a été lavée avec une solution saturée en bicarbonate de sodium (2 x10 mL), séchée sur $MgSO_4$, filtrée puis concentrée sous pression réduite. Le composé **(12)** a été isolé après purification par colonne de chromatographie sur gel de silice sous pression (AcOEt/EP, 2/8) sous forme de solide jaune avec un rendement de 78 %. MP : 138 °C ; Infrarouge (Diamand ATR, $cm^{-1}$) υ : 3084, 3025, 2970, 2917, 1594, 1582, 1494, 1164, 941 ; RMN $^1H$ (400 MHz, $CDCl_3$) δ : 3,51 (s, 3H, $CH_3$), 3,90-3,93 (m, 4H, $2xCH_2(O)$), 4,55 (bs, 4H, $2xCH_2(N)$), 5,25 (s, 2H, $CH_2$), 7,13 (d, 2H, *J* = 8,8 Hz, $2xH_{arom}$), 8,28 (d, 1H, *J* = 2,4 Hz, $H_8$), 8,38 (d, 2H, *J* = 8,8 Hz, $2xH_{arom}$), 8,72 (d, 1H, *J* = 2,4 Hz, $H_6$) ; RMN $^{13}C$ (101 MHz, $CDCl_3$) δ: 48,1 ($2xCH_2$), 56,1 ($CH_3$), 67,2 ($2xCH_2$), 94,3 ($CH_2$), 115,8 (2xCH), 130,2 (2xCH), 131,0 (Cq), 131,7 (Cq), 134,2 (Cq), 134,5 (CH), 144,5 (CH), 149,0 (Cq), 159,0 (Cq), 159,5 (Cq), 160,7 (Cq) ; HRMS (EI-MS) : $C_{19}H_{19}ClN_4O_3$ $[M+H]^+$, calculée m/z 387,1218, trouvée m/z 387,1221.

| | |
|---|---|
| 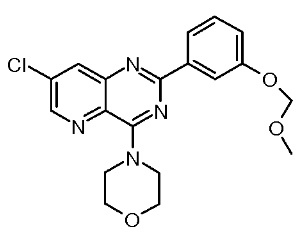 **11** | 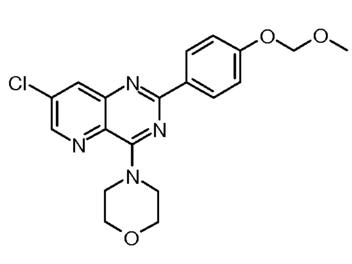 **12** |

Le composé **11** a également pu être synthétisé à partir du composé **4**, par le biais d'un couplage de Suzuki, en présence du dérivé boré suivant : 3 méthoxyéthyléther phényl trifluoroborate de potassium **(A) :**

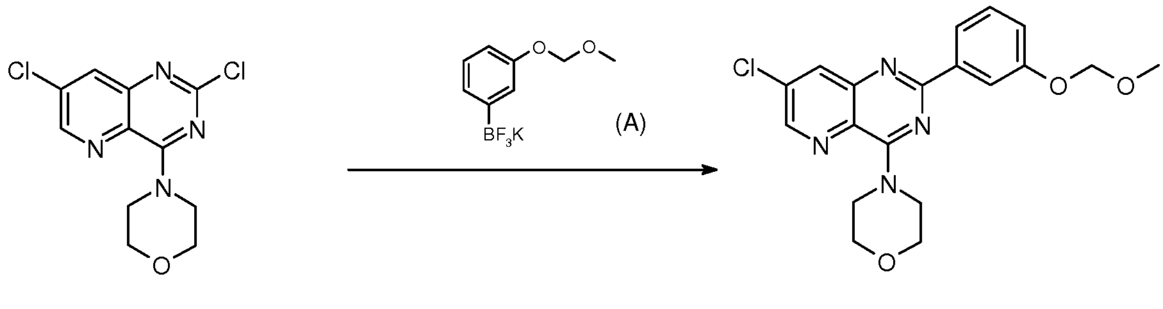


4 11

*A.5. Fonctionnalisation de la position C-7*

A.5.1. Insertion de la fonction vinyle

- ***Procédure B***

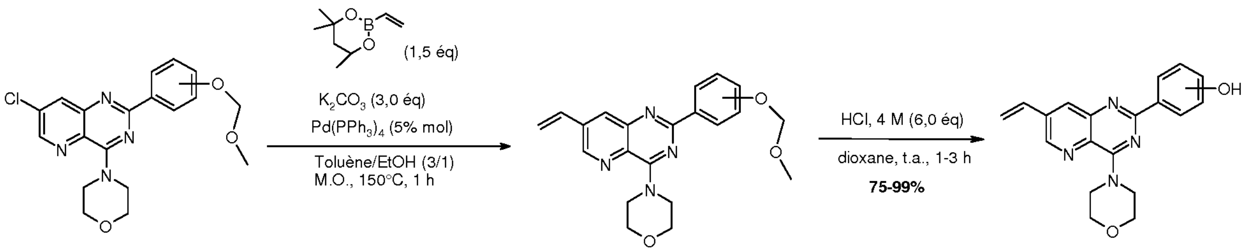


11 et 12 13 et 15 14 et16

**Procédure générale B :** Sous une atmosphère d'argon, dans un vial de 5 mL, 1,0 éq. de 2-Chloro-4-(*N*-$R_1R_2$)pyrido[3,2-*d*]pyrimidine (**11** ou **12**) a été dissous dans un mélange (toluène/éthanol, 2/1). 2,0 éq. de carbonate de potassium, 1,2 éq. d'acide boronique ont été ajoutés dans le milieu ainsi que 0,05 éq. de tetrakis(triphénylphosphino) palladium(0). Le mélange a été irradié sous micro-ondes à 150 °C pendant une heure. Après concentration sous pression réduite, le résidu a été repris dans de l'acétate d'éthyle (25 mL) et lavé avec de l'eau (10 mL). Les extraits organiques ont été séchés sur $MgSO_4$, filtrés puis concentrés sous pression réduite. Le brut réactionnel a alors été purifié par colonne de chromatographie.

Etape de déprotection : Sous un ballon de 25 mL, 1,0 éq. d'un composé protégé par un groupement méthoxyméthoxy (**13** et **15**) a été dissous dans du dioxane (10 mL). 6 éq. d'une solution d'acide chlorhydrique gazeux (4 M dans du dioxane) ont été injectés dans le milieu. Le mélange a été laissé sous agitation à température ambiante pendant une à trois heures. Le précipité a été lavé à l'éther de pétrole puis récupéré par filtration pour donner le produit final sans purification supplémentaire.

- ***Procédure C***

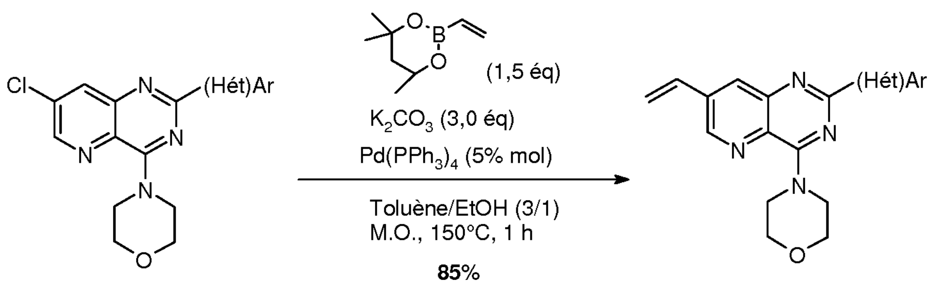


<u>**8 et 10**</u> <u>**17 et 18**</u>

**Procédure générale C :**

Sous une atmosphère d'argon, dans un vial de 5 mL, 1,0 éq. de 2-Chloro-4-(*N*-$R_1R_2$)pyrido[3,2-*d*]pyrimidine (**8** ou **10**) a été dissous dans un mélange (toluène/éthanol, 2/1). 2,0 éq. de carbonate de potassium, 1,2 éq. d'acide boronique ont été ajoutés dans le milieu ainsi que 0,05 éq. de tetrakis(triphénylphosphino) palladium(0). Le mélange a été irradié sous micro-ondes à 150 °C pendant une heure. Après concentration sous pression réduite, le résidu a été repris dans de l'acétate d'éthyle (25 mL) et lavé avec de l'eau (10 mL). Les extraits organiques ont été séchés sur $MgSO_4$, filtrés puis concentrés sous pression réduite. Le brut réactionnel a alors été purifié par colonne de chromatographie.

| | |
|---|---|
| 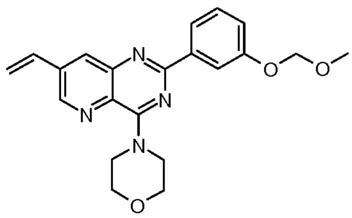 **13** | 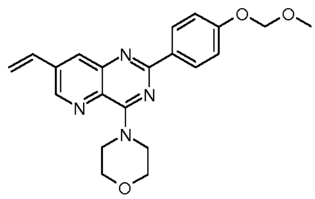 **15** |
| 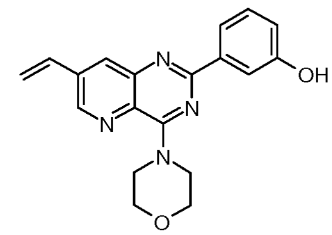 **14** | 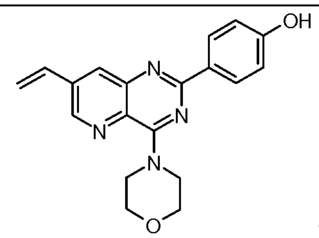 **16** |

| | |
|---|---|
| 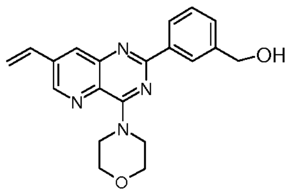 **17** | 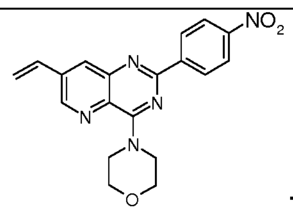 **18** |

**4-(2-(3-(méthoxyméthoxy)phényl)-7-vinylpyrido[3,2-*d*]pyrimidin-4-yl)morpholine (13)** : Le composé (13) a été synthétisé à partir de **(11)** (250 mg, 0,65 mmole) en suivant la procédure générale B puis purifié par colonne de chromatographie sur gel de silice sous pression (AcOEt/EP, 2/8) pour donner un solide jaune avec un rendement de 91 %. MP : 105 °C ; Infrarouge (Diamand ATR, $cm^{-1}$) $\upsilon$ : 2856, 1527, 1487, 1454, 1343, 1275, 1111, 1070, 1021, 956, 910, 739 ; RMN $^1H$ (400 MHz, $CDCl_3$) $\delta$ : 3,52 (s, 3H, $CH_3$), 3,96-3,89 (m, 4H, 2x$CH_2$(O)), 4,58 (bs, 4H, 2x$CH_2$(N)), 5,28 (s, 2H, $CH_2$), 5,56 (d, 1H, *J* = 11,0 Hz, $CH_{2alkene}$), 6,05 (d, 1H, *J* = 17,7 Hz, $CH_{2alkene}$), 6,84 (dd, 1H, *J* = 11,0 Hz, *J* =

17,7 Hz, $CH_{alkene}$), 7,20-7,13 (m, 1H, $H_{arom}$), 7,40 (t, 1H, $J$ = 7,9 Hz, $H_{arom}$), 8,19-8,09 (m, 3H, 2x$H_{arom}$ and $H_8$), 8,72 (d, 1H, $J$ = 1,9 Hz, $H_6$); RMN $^{13}C$ (101 MHz, $CDCl_3$) δ : 48,3 (2x$CH_2$), 56,3 ($CH_3$), 67,5 (2x$CH_2$), 94,7 ($CH_2$), 116,5 (CH), 118,4 (CH), 119,0 ($CH_2$), 122,3 (CH), 129,5 (CH), 132,5 (Cq), 132,6 (CH), 133,2 (CH), 136,2 (Cq), 140,2 (Cq), 144,9 (CH), 148,4 (Cq), 157,6 (Cq), 159,4 (Cq), 160,2 (Cq) ; HRMS (EI-MS) : $C_{21}H_{22}N_4O_3$ $[M+H]^+$, calculée m/z 379,1765, trouvée m/z 379,1766.

**3-(4-morpholino-7-vinylpyrido[3,2-*d*]pyrimidin-2-yl)phénol (14) :** Le composé **(14)** a été synthétisé à partir du composé (13) (100 mg, 0,264 mmole) en suivant la fin de la procédure générale B pour donner un solide blanc avec un rendement de 98 %. MP : 183 °C ; Infrarouge (Diamand ATR, $cm^{-1}$) υ : 3338, 2856, 1597, 1531, 1483, 1438, 1230, 1107, 968, 858, 739 ; RMN $^1H$ (400 MHz, $CDCl_3$) δ: 3,89 (m, 4H, 2x$CH_2$(O)), 4,54 (s, 4H, 2x$CH_2$(N)), 5,51 (d, 1H, $J$ = 11,0 Hz, $H_{alcene}$), 5,99(d, 1H, $J$ = 17,6 Hz, $H_{alcene}$), 6,77 (dd, 1H, $J$ = 11,0 Hz, $J$ = 17,6 Hz, $H_{alcene}$), 6,92 (d, 1H, $J$ = 7,5 Hz, $H_{arom}$), 7,29 (t, 1H, $J$ = 7,5 Hz, $H_{arom}$), 7,98 (s, 2H, 2x$H_{arom}$), 8,08 (s, 1H, $H_8$), 8,70 (d, 1H, $H_6$) ; RMN $^{13}C$ (101 MHz, $CDCl_3$) δ : 48,3 (2x$CH_2$), 66,1 ($CH_2$), 67,5 ($CH_2$), 115,6 (CH), 118,0 (CH), 119,2 ($CH_2$), 121,0 (CH), 129,8 (CH), 132,2 (CH), 132,3 (Cq), 133,1 (CH), 136,4 (Cq), 140,0 (Cq), 144,9 (CH), 148,2 (Cq), 156,4 (Cq), 159,3 (Cq), 160,5 (Cq) ; HRMS (EI-MS) : $C_{19}H_{18}N_4O_2$ $[M+H]^+$, calculée m/z 335,1430, trouvée m/z 335,1504.

**4-(2-(4-(méthoxyméthoxy)phényl)-7-vinylpyrido[3,2-d]pyrimidin-4-yl)morpholine (15)** : Le composé **(15)** a été synthétisé à partir de **(12)** (250 mg, 0,65 mmole) en suivant la procédure générale B puis purifié par colonne de chromatographie sur gel de silice sous pression (AcOEt/EP, 1/9) pour donner un solide blanc avec un rendement de 87 %. MP : 95-96 °C ; Infrarouge (Diamand ATR, $cm^{-1}$) υ: 2856, 1527, 1487, 1454, 1343, 1275, 1111, 1070, 1021, 956, 910, 739 ; RMN $^1H$ (400 MHz, $CDCl_3$) δ: 3,52 (s, 3H, $CH_3$), 3,96-3,89 (m, 4H, 2x$CH_2$(O)), 4,58 (bs, 4H, 2x$CH_2$(N)), 5,28 (s, 2H, $CH_2$), 5,56 (d, 1H, $J$ = 11,0 Hz, $CH_{2alkene}$), 6,05 (d, 1H, $J$ = 17,7 Hz, $CH_{2alkene}$), 6,84 (dd, 1H, $J$ = 11,0 Hz, $J$ = 17,7 Hz, $CH_{alkene}$), 7,20-7,13 (m, 1H, $H_{arom}$), 7,40 (t, 1H, $J$ = 7,9 Hz, $H_{arom}$), 8,19-8,09 (m, 3H, 2x$H_{arom}$ and $H_8$), 8,72 (d, 1H, $J$ = 1,9 Hz, $H_6$) ; RMN $^{13}C$ (101 MHz, $CDCl_3$) δ: ; HRMS (EI-MS) : $C_{21}H_{22}N_4O_3[M+H]^+$, calculée m/z 379,1765, trouvée m/z 379,1768.

**4-(4-Morpholino-7-vinylpyrido[3,2-*d*]pyrimidin-2-yl)phénol (16) :** Le composé **(16)** a été synthétisé à partir du composé (15) (100 mg, 0,264 mmole) en suivant la fin de la procédure générale B pour donner un solide jaune avec un rendement de 86 %. MP : >260 °C ; Infrarouge (Diamand ATR, $cm^{-1}$) υ : 3292, 2918, 2861, 1591, 1550, 1520, 1503, 1376, 1277, 1130, 1029 ; RMN $^1H$ (400 MHz, $CDCl_3$) δ : 3,83 (bs, 4H, 2x$CH_2$(O)), 4,47 (bs, 4H, 2x$CH_2$(N)), 5,61 (d, 1H, $J$ = 10,8 Hz, $CH_{2alkene}$), 6,28 (d, 1H, $J$ = 17,5 Hz, $CH_{2alkene}$), 6,80 (d, 2H, $J$ = 8,4 Hz, 2x$H_{arom}$), 6,96 (dd, 1H, $J$ = 10,8 Hz, $J$ = 17,5 Hz, $CH_{alkene}$), 8,14 (s, 1H, $H_8$), 8,26 (d, 2H, $J$ = 8,4 Hz, 2x$H_{arom}$), 8,88 (s, 1H, $H_6$); HRMS (EI-MS) : $C_{19}H_{18}N_4O_2[M+H]^+$, calculée m/z 335,1503, trouvée m/z 335,1504.

(**3**-(**4-Morpholino**-**7**-**vinylpyrido**[**3**,**2**-***d***]**pyrimidin**-**2**-**yl**)**phényl**)**méthanol (17) :** Le composé **(17)** a été synthétisé à partir du composé **(8)** (210 mg, 0,59 mmole) en suivant la procédure générale C puis purifié par colonne de chromatographie sur gel de silice sous pression (AcOEt/EP, 2/8) pour donner un solide jaune avec un rendement de 85 %. MP : 160 °C ; Infrarouge (Diamand ATR, $cm^{-1}$) υ: 3293, 2922, 1488, 1440, 1308, 1109, 1069, 973 ; RMN $^1H$ (400 MHz, $CDCl_3$) δ : 3,92-3,95 (m, 4H, 2x$CH_2$(O)), 4,60 (bs, 4H, 2x$CH_2$(N)), 4,81 (s, 2H, $CH_2$), 5,58 (d, 1H, $J$ = 11,0 Hz, $H_{alkene}$), 6,06 (d, 1H, $J$ = 17,6 Hz, $H_{alkene}$), 6,86 (dd, 1H, $J$ = 11,0 Hz, $J$ = 17,6 Hz, $H_{alkene}$), 7,48-7,50 (m, 2H, 2x$H_{arom}$), 8,12 (d, 1H, $J$ = 2,1 Hz, $H_8$), 8,41-8,43 (m, 1H, $H_{arom}$), 8,48 (s, 1H, $H_{arom}$), 8,74 (d, 1H, $J$ = 2,1 Hz, $H_6$) ; RMN $^{13}C$ (101 MHz, $CDCl_3$) δ : 48,1 (2x$CH_2$), 65,4 ($CH_2$), 67,3 (2x$CH_2$), 119,0 ($CH_2$), 127,0 (CH), 127,9 (CH), 128,6 (CH), 129,2 (CH), 132,1 (CH), 132,9 (CH), 136,2 (Cq), 138,4 (Cq), 141,1 (Cq), 144,8 (CH), 148,0 (Cq), 159,1 (Cq), 160,1 (Cq), 167,5 (Cq); HRMS (EI-MS) : $C_{20}H_{20}N_4O_2$ $[M+H]^+$, calculée m/z 349,1659, trouvée m/z 349,1661.

**4-(2-(4-nitrophényl)-7-vinylpyrido[3,2-*d*]pyrimidin-4-yl)morpholine (18) :** Le composé (18) a été synthétisé à partir du composé **(10)** (210 mg, 0,59 mmole) en suivant la procédure générale C puis purifié par colonne de chromatographie sur gel de silice sous pression (AcOEt/EP, 1/9) pour donner un solide jaune avec un rendement de 85 %. MP : >260 °C ; Infrarouge (Diamand ATR, $cm^{-1}$) υ: 3028, 2972, 2920, 1602, 1553, 1519, 1437, 1345, 1109, 867 ; RMN $^1H$ (400 MHz, $CDCl_3$) δ: 3,90-3,94 (m, 4H, 2x$CH_2$(O)), 4,56-4,53 (bs, 4H, 2x$CH_2$(N)), 5,54 (d, 1H, $J$ = 11,0 Hz, $CH_{2alkene}$), 6,04 (d, 1H, $J$ = 17,7 Hz, $CH_{2alkene}$), 6,84 (dd, 1H, $J$ = 11,0 Hz, $J$ = 17,7 Hz, $CH_{alkene}$), 8,06 (d, 1H, $J$ = 2,2 Hz, $H_8$), 8,32 (d, 2H, $J$ = 8,7 Hz, 2x$H_{arom}$), 8,63 (d, 2H, $J$ = 8,6 Hz, 2x$H_{arom}$), 8,71 (d, 1H, $J$ = 2,2 Hz, $H_6$) ; RMN $^{13}C$ (101 MHz, $CDCl_3$) δ : 48,2 (2x$CH_2$), 67,4 (2x$CH_2$), 114,7 (2xCH), 118,6 ($CH_2$), 128,7 (Cq), 130,2 (2xCH), 132,2 (Cq), 132,3 (CH), 133,3 (CH), 135,9 (Cq), 144,1 (CH), 147,5 (Cq), 148,9 (Cq), 155,4 (Cq), 160,6 (Cq) ; HRMS (EI-MS) : $C_{19}H_{17}N_5O_3$ $[M+H]^+$, calculée m/z 364,1404, trouvée m/z 364,1407.

A.5.2. Oxydation de la fonction vinyle

- ***Procédure D1 et D2***

*voie D1*

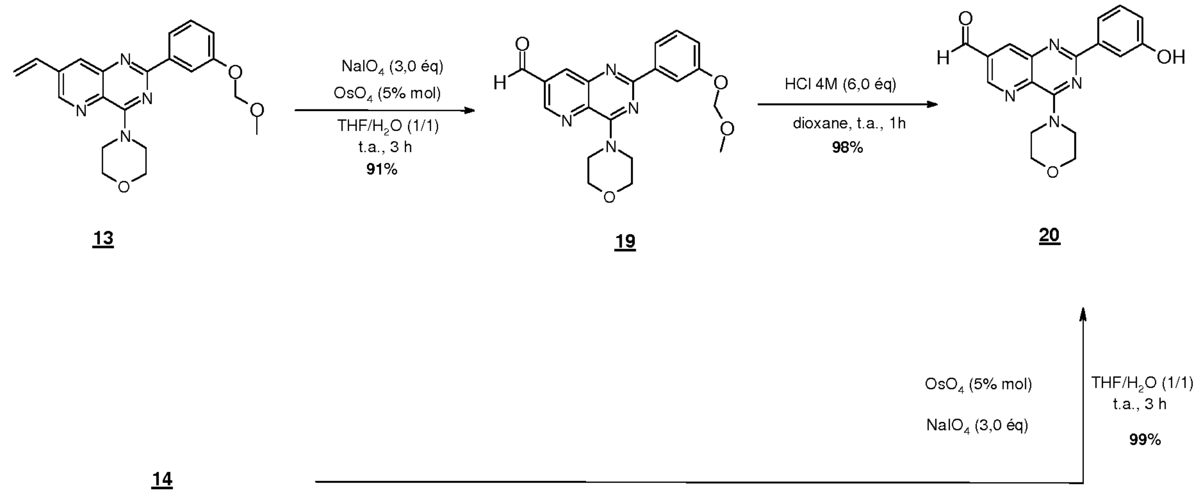


13 19 20

14

*voie D2*

- ***Procédure E1 et E2***

*voie E1*

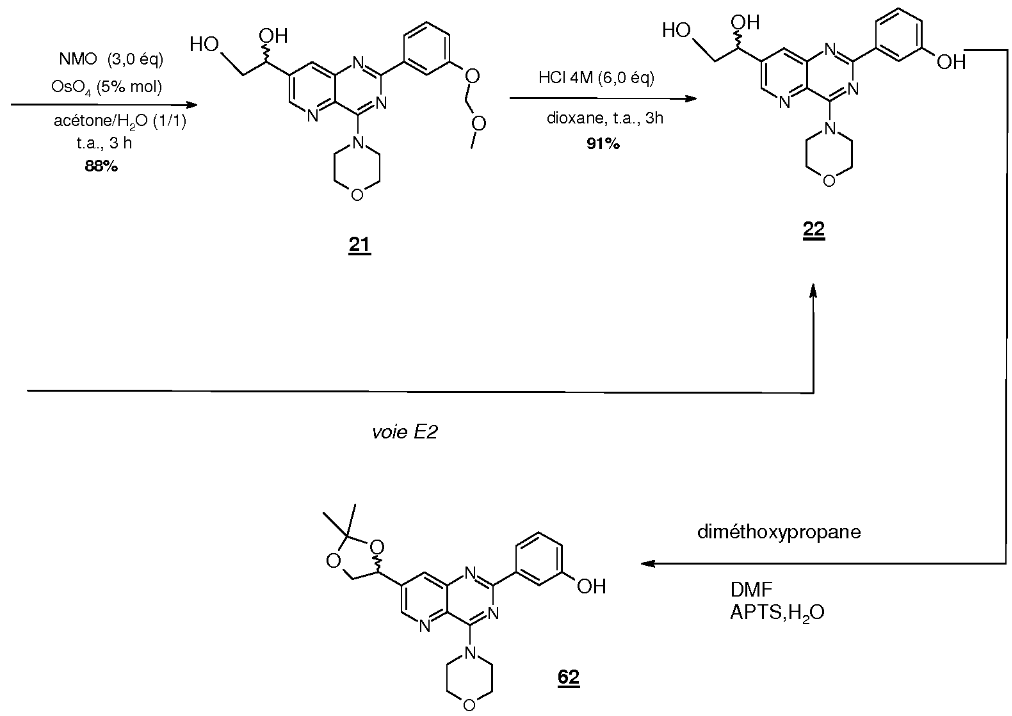


13 21 22

14

*voie E2*

62

**2-(3-(méthoxyméthoxy)phényl)-4-morpholinopyrido[3,2-*d*]pyrimidine-7-carbaldéhyde (19) :** Le composé **(19)** a été synthétisé selon la première étape de la procédure D1. Dans un ballon de 25 mL, 100 mg (0,3 mmol, 1,0 éq.) de composé **(13)** a été dissous dans un mélange (THF/$H_20$, 1/1, 8 mL). 0,2 mL (0,015 mmol, 0,05 éq.) de trétroxide d'osmium ont été injectés dans le milieu. Une fois la solution devenue noire, 192 mg (0,9 mmol, 3,0 éq.) de périodate de sodium ont été ajoutés en trois fois à 5 minutes d'intervalle. La solution a été agitée pendant trois heures. Une solution

aqueuse de thiosulfate de sodium à 7,5 % a ajoutée dans le milieu (10 mL), puis la solution a été agitée pendant 5-10 min, puis filtrée sur célite. Le filtrat obtenu a alors été extrait avec de l'acétate d'éthyle (40 mL) tandis que la phase organique résultante a été lavée avec de l'eau (1 x 10 mL), puis séchée sur $MgSO_4$, filtrée et concentrée sous pression réduite. Le composé **(19)** a été isolé sous forme de solide jaune par colonne de chromatographie sur gel de silice sous pression (AcOEt/EP, 2/8) avec un rendement de 97 %. MP : 141 °C ; Infrarouge (Diamand ATR, $cm^{-1}$) υ : 2911, 1701, 1508, 1461, 1426, 1268, 1154, 1116, 1071, 1008, 957, 739 ; RMN $^1H$ (400 MHz, $CDCl_3$) δ : 3,43 (s, 3H, $CH_3$), 3,83 (s, 4H, $2xCH_2(O)$), 4,52 (bs, 4H, $2xCH_2(N)$), 5,28 (s, 2H, $CH_2$), 7,25-7,13 (m, 1H, $H_{arom}$), 7,45 (t, 1H, *J* = 7,5 Hz, $H_{arom}$), 8,10 (d, 2H, *J* = 7,4 Hz, $2xH_{arom}$), 8,67 (s, 1H, $H_8$), 9,09 (s, 1H, $H_6$), 10,28 (s, 1H, CHO) ; RMN $^{13}C$ (62,5 MHz, $CDCl_3$) δ : 47,8 ($2xCH_2$), 55,6 ($CH_3$), 66,3 ($2xCH_2$), 94,0 ($CH_2$), 115,7 (CH), 118,5 (CH), 121,7 (CH), 129,6 (CH), 133,3 (Cq), 135,1 (Cq), 138,9 (CH), 139,2 (Cq), 144,4 (CH), 147,4 (Cq), 157,0 (Cq), 158,5 (Cq), 159,7 (Cq), 192,4 (CH) ; HRMS (EI-MS) : $C_{20}H_{20}N_4O_4$ $[M+H]^+$, calculée m/z 381,1557, trouvée m/z 381,1560.

**2-(3-hydroxyphényl)-4-morpholinopyrido[3,2-*d*]pyrimidine-7-carbaldéhyde (20) :**

- Voie D2 :

Dans un ballon de 25 mL, 100 mg (0,30 mmole, 1,0 éq.) du composé (14) ont été dissous dans un mélange ($THF/H_20$, 1/1, 8 mL). 0,2 mL (0,015 mmol, 0,05 éq.) de trétroxide d'osmium ont été injectés dans le milieu. Une fois la solution devenue noire, 192 mg (0,9 mmol, 3,0 éq.) de périodate de sodium ont été ajoutés en trois fois à 5 minutes d'intervalle. La solution a été agitée pendant deux heures. Une solution aqueuse de thiosulfate de sodium à 7,5 % a été ajoutée dans le milieu (10 mL). La solution a été agitée pendant 5-10 min, puis filtrée sur célite. Le filtrat obtenu a alors été extrait avec de l'acétate d'éthyle (40 mL). La phase organique résultante a été lavée avec de l'eau (1 x 10 mL). L'extrait organique a été séché sur $MgSO_4$, filtré puis concentré sous pression réduite puis purifié par colonne de chromatographie sur gel de silice sous pression (AcOEt/EP, 2/8) pour donner un solide jaune avec un rendement de 99 %.

- Voie D1:

Le composé **(20)** a été synthétisé à partir du composé **(19)** (100 mg, 0,30 mmole) en suivant l'étape de déprotection de la procédure générale D1 (similaire à l'étape de déprotection de la procédure B) pour donner un solide jaune avec un rendement de 90 %. MP : 182 °C ; Infrarouge (Diamand ATR, $cm^{-1}$) υ : 2852, 1695, 1556, 1516, 1426, 1377, 1283, 1107, 1025, 882, 743 ; RMN $^1H$ (400 MHz, DMSO) δ : 3,84 (m, 4H, $2xCH_2(O)$), 4,52 (s, 4H, $2xCH_2(N)$), 4,79 (s, 2H, $CH_2OH$), 6,92 (m, 1H, $H_{arom}$), 7,30 (t, 1H, *J* = 8,1 Hz, $H_{arom}$), 7,90 (m, 2H, $2xH_{arom}$), 8,64 (d, 1H, *J* = 1,9 Hz, $H_8$), 9,09 (d, 1H, *J* = 1,9 Hz, $H_6$), 9,58 (s, 1H, OH), 10,28 (s, 1H, CHO); RMN $^{13}C$ (101 MHz, DMSO) δ : 48,1 ($2xCH_2$), 66,3 ($2xCH_2$), 114,9 (CH), 117,78 (CH), 119,1 (CH), 129,4 (CH), 133,3 (Cq), 136,1 (Cq), 138,8 (CH), 139,0(Cq), 144,3 (CH) 147,43 (Cq), 157,5 (Cq), 158,4 (Cq), 159,7 (Cq), 192,4 (CH) ; HRMS (EI-MS) : $C_{18}H_{16}N_4O_3$ $[M+H]^+$, calculée m/z 337,1563, trouvée m/z 337,1546.

**1-(2-(3-(méthoxyméthoxy)phényl)-4-morpholinopyrido[3,2-*d*]pyrimidin-7-yl)éthane-1,2-diol (21) :** Le composé **(21)** a été synthétisé selon la première étape de la procédure suivante E1. Dans un ballon de 25 mL, 80 mg (0,24 mmol, 1,0 éq.) de composé **(13)** ont été dissous dans un mélange ($Acétone/H_20$, 3/1, 8 mL). 0,2 mL (0,012 mmol, 0,05 éq.) de trétroxide d'osmium ont été injectés dans le milieu. Une fois la solution devenue noire, 42 mg (0,36 mmol, 1,5 éq.) de *N*-méthylmorpholine-*N*-oxide (NMO) ont été ajoutés en deux fois à 5 minutes d'intervalle. La solution a été laissée sous agitation pendant deux heures. Une solution aqueuse de thiosulfate de sodium à 10 % (10 mL) a été ajoutée dans le milieu. La solution a été agitée pendant 5-10 min, puis filtrée sur célite. Le filtrat obtenu a alors été extrait avec de l'acétate d'éthyle (40 mL). La phase organique résultante a été lavée avec de l'eau (10 mL). L'extrait organique a été séché sur $MgSO_4$, filtré puis concentré sous pression réduite puis purifié par colonne de chromatographie sur gel de silice sous pression (AcOEt/EP, 2/8) pour donner un solide blanc avec un rendement de 88 %. MP : 167 °C ; Infrarouge (Diamand ATR, $cm^{-1}$) υ: 3444, 2921, 1495, 1442, 1356, 1266, 1152, 1078, 1013, 743 ; RMN $^1H$ (400 MHz, DMSO) δ: 3,42 (s, 3H, $CH_3$), 3,61 (m, 2H, $CH_2OH$), 3,82 (m, 4H, $2xCH_2(O)$), 4,52 (bs, 4H, $2xCH_2(N)$), 4,80 (q, 1H, *J* = 5,4 Hz, OH), 4,88 (t, 1H, *J*= 5,8 Hz, CHOH), 5,28 (s, 2H, $CH_2$), 5,67 (d, 1H, *J*= 4,7 Hz, OH), 7,17 (m, 1H, $H_{arom}$), 7,43 (t, 1H, *J* = 8,2 Hz, $H_{arom}$), 8,09 (m, 3H, $2xH_{arom}$ and $H_8$), 8,75 (d, 1H, *J* = 2,0 Hz, $H_6$) ; RMN $^{13}C$ (101 MHz, DMSO) δ: 48,1 ($2xCH_2$), 55,6 ($CH_3$), 66,4 ($2xCH_2$), 66,5 ($CH_2$), 71,3 (CH), 94,0 ($CH_2$), 115,5 (CH), 118,2 (CH), 121,5 (CH), 129,4 (CH), 131,2 (Cq), 132,9 (CH), 139,4 (Cq), 143,3 (Cq), 146,1 (CH), 147,4 (Cq), 156,9 (Cq), 158,3 (Cq), 158,5 (Cq) ; HRMS (EI-MS) : $C_{21}H_{24}N_4O_5$ $[M+H]^+$, calculée m/z 413,1747, trouvée m/z 413,1825.

**1-(2-(3-hydroxyphényl)-4-morpholinopyrido[3,2-*d*]pyrimidin-7-yl)éthane-1,2-diol (24):**

- Voie E2 :

Dans un ballon de 25 mL, 80 mg (0,24 mmol, 1,0 éq.) du composé **(14)** ont été dissous dans un mélange (Acétone/$H_20$, 3/1, 8 mL). 0,2 mL (0,012 mmol, 0,05 éq.) de trétroxide d'osmium ont été injectés dans le milieu. Une fois la solution devenue noire, 42 mg (0,36 mmol, 1,5 éq.) de *N*-méthylmorpholine-*N*-oxide (NMO) ont été ajoutés en deux fois à 5 minutes d'intervalle. La solution a été laissée sous agitation pendant deux heures. Une solution aqueuse de thiosulfate de sodium à 10 % (10 mL) a été ajoutée dans le milieu. La solution a été agitée pendant 5-10 min, puis filtrée sur célite. Le filtrat obtenu a alors été extrait avec de l'acétate d'éthyle (40 mL). La phase organique résultante a été lavée avec de l'eau (10 mL). L'extrait organique a été séché sur $MgSO_4$, filtré puis concentré sous pression réduite puis purifié par colonne de chromatographie sur gel de silice sous pression (DCM/MeOH, 98/2) pour donner un solide blanc avec un rendement de 88 %.

- Voie E1 :

Le composé **(22)** a été synthétisé à partir du composé **(14)** (80 mg, 0,24 mmol) en suivant l'étape de déprotection de la procédure générale E1 (similaire à l'étape de déprotection de la procédure B) pour donner un solide blanc avec un rendement de 91 %.
MP : 206 °C ; Infrarouge (Diamand ATR, $cm^{-1}$) $\upsilon$: 3293, 2856, 1528, 1495, 1442, 1352, 1111, 1021, 861, 739 ; RMN $^1H$ (400 MHz, DMSO) $\delta$: 3,32 (m, 2H, $CH_2OH$), 3,82 (m, 4H, $2xCH_2(O)$), 4,52 (s, 4H, $2xCH_2(N)$), 4,80 (d, 1H, OH), 4,88 (t, 1H, CHOH), 5,66 (d, 1H, OH), 6,90 (t, 1H, *J* = 8,1 Hz, $H_{arom}$), 7,29 (m, 2H, $2xH_{arom}$), 7,90 (d, 1H, *J* = 1,9 Hz, $H_8$), 8,07 (d, 1H, $H_6$), 9,53 (s, 1H, OH) ; RMN $^{13}C$ (101 MHz, DMSO) $\delta$: 48,1 ($2xCH_2$), 66,4 ($2xCH_2$), 66,5 ($CH_2$), 71,3 (CH), 114,1 (CH), 117,4 (CH), 118,9 (CH), 129,3 (CH), 131,2 (Cq), 132,8 (CH), 139,3 (Cq), 143,2 (Cq), 145,9 (CH) 147,4 (Cq), 157,3 (Cq), 158,5 (Cq), 158,7 (Cq) ; HRMS (EI-MS) : $C_{19}H_{20}N_4O_4$ $[M+H]^+$, calculée m/z 369,1485, trouvée m/z 369,1554.

**4-[7-(2,2-diméthyl-1,3-dioxolan-4-yl)-2-[3-(méthoxyméthoxy)phenyl]pyrido[3,2-d]pyrimidin-4-yl]morpholine (62) :** Dans un ballon de 10 mL, 83 mg (0,23 mmol; 1 éq.) de (22) a été mis en suspension dans 2 mL de diméthylformamide (DMF). 142,6 $\mu$L (1,15 mmol, 5 eq) de 2,2 diméthoxypropane et 4 mg d'acide para-toluènesulfonique monohydrate (APTS, $H_2O$) ont été ajoutés. Au bout d'une heure à température ambiante, une solution aqueuse saturée en $NaHCO_3$ (10mL) a été ajoutée. La phase organique a été extraite 3 fois avec de l'acétate d'éthyle (10mL), lavée avec une solution saline saturée, séchée sur $MgSO_4$, filtrée puis concentrée sous pression réduite. Le résidu brut a alors été purifié par colonne de chromatographie sur gel de silice sous pression (AE/ EP 30/70) permettant d'isoler un solide blanc avec un rendement de 53 %.; MP : 210 °C ; Infrarouge (Diamand ATR, $cm^{-1}$) $\upsilon$ : 3293, 2856, 1557, 1513, 1495, 1429, 1352, 1111, 1021, 861, 739 ; RMN $^1H$ (400 MHz, DMSO) $\delta$: 1.46 et 153 (2 s, 6H, $2xCH_3$), 3,82 (m, 4H, $2xCH_2(O)$), 4,46 (m, 2H, $CH_2$), 4,48 (s, 4H, $2xCH_2(N)$),5,34 (t, 1H, *J* = 6,7 Hz, CH), 6,90 (t, 1H, *J* = 8,1 Hz, $H_{arom}$), 7,29 (m, 2H, $2xH_{arom}$), 7,90 (d, 1H, *J*= 1,9 Hz, $H_8$), 8,07 (d, 1H, $H_6$), 9,53 (s, 1H, OH) ; RMN $^{13}C$ (101 MHz, DMSO) $\delta$: 26,1 ($CH_3$), 26,8 ($CH_3$), 48,4 ($2xCH_2$), 66,9 ($2xCH_2$), 70,58 ($CH_2$), 75,1 (CH), 110,22 (Cq), 115,5 (CH), 117,3 (CH), 119,8 (CH), 129,9 (CH), 132,28 (Cq), 133,6 (CH),139,6 (Cq) 140,0 (Cq), 145,6 (CH) 147,4 (Cq), 157,9 (Cq), 158,9 (Cq), 159,5 (Cq) ; HRMS (EI-MS) : $C_{22}H_{25}N_4O_4$ $[M+H]^+$, calculée m/z 409,1876, trouvée m/z 409,1876.

A.5.3. Préparation de dérivés aminés par amination réductrice

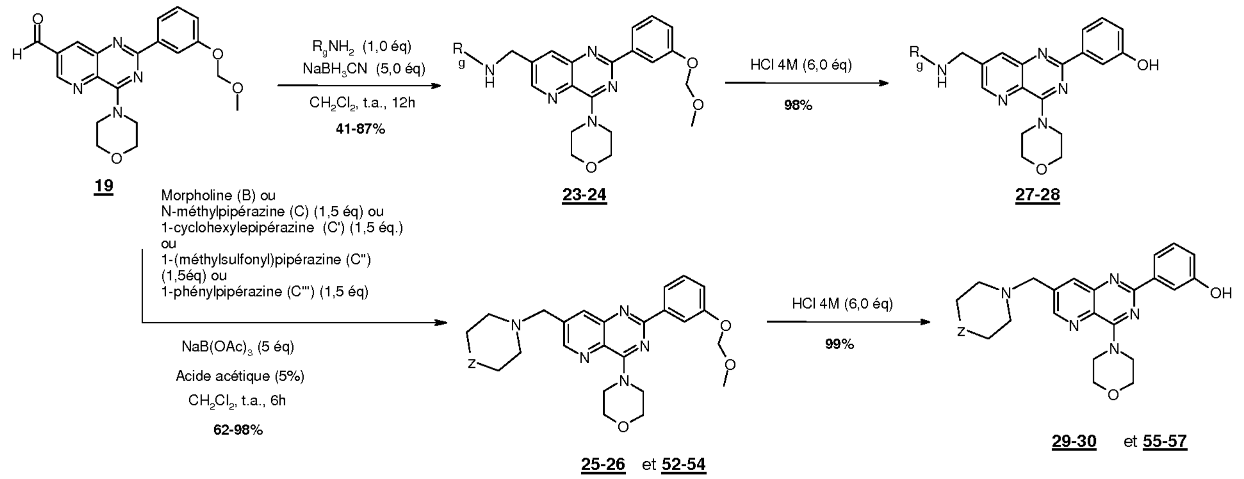


**Chlorhydrate du 3-(7-((cyclopropylamino)méthyl)-4-morpholinopyrido[3,2-*d*]pyrimidin-2-yl)phénol (27) :** Dans un ballon de 25 mL, sous une atmosphère inerte, 70 mg (0,184 mmol; 1 éq.) de **(19)** ont été dissous dans 5 mL de dichlorométhane anhydre. Une pointe de spatule de $MgSO_4$ a été ajouté dans le milieu ainsi que 12 μL (0,184 mmol; 1 éq.) de cyclopropylamine. Le mélange a été agité à température ambiante pendant une nuit. Le $MgSO_4$ a été éliminé par filtration sur fritté et le filtrat a été concentré sous pression réduite. Le résidu a été repris dans 5 mL de méthanol auquel 60 mg (0,92 mmol; 5 éq.) de cyanohydroborure de sodium ont été ajoutés. Après 20 minutes d'agitation à température ambiante, le milieu réactionnel a été concentré sous pression réduite.

Etape de déprotection : Dans un ballon de 25 mL, 1,0 éq. du composé **(23)** a été dissous dans du dioxane (10 mL). 6 éq. d'une solution d'acide chlorhydrique gazeux (4 M dans du dioxane) ont été injectés dans le milieu. Le mélange a été laissé sous agitation à température ambiante pendant une à trois heures. Le précipité a été lavé à l'éther de pétrole puis récupéré par filtration pour obtenir le composé **(27)** avec un rendement de 40 % pour donner un solide blanc. MP : 244 °C ; Infrarouge (Diamand ATR, $cm^{-1}$) υ : 3351, 3047, 1613, 1552, 1517, 1428, 1385, 1310, 1114, 1024, 864, 732 ; RMN $^1H$ (400 MHz, DMSO) δ: 0,81 (s, 2H, $2xH_{cyclopropyl}$), 1,09 (s, 2H $2xH_{cyclopropyl}$), 2,72 (s, 1H, $H_{cyclopropyl}$), 3,87 (s, 4H, $2xCH_2(O)$), 4,48 (s, 2H, $CH_2$), 4,64 (bs, 4H, $2xCH_2(N)$), 7,06 (s, 1H, $H_{arom}$), 7,38 (s, 1H, $H_{arom}$), 7,91 (d, 2H, *J* = 11,7 Hz, $2xH_{arom}$), 8,70 (s, 1H, $H_8$), 9,10 (s, 1H, $H_6$), 10,39 (s, 1H, NH) ; RMN $^{13}C$ (101 MHz, DMSO) δ: ; HRMS (EI-MS) : $C_{21}H_{23}N_5O_2$ $[M+H]^+$, calculée m/z 378,1852, trouvée m/z 378,1927.

**Chlorhydrate du 3-(7-((cyclohexylamino)méthyl)-4-morpholinopyrido[3,2-*d*] pyrimidin-2-yl)phénol (28)** Dans un ballon de 25 mL, sous une atmosphère inerte, 80 mg (0,21 mmol; 1 éq.) de **(19)** ont été dissous dans 5 mL de dichlorométhane anhydre. Une pointe de spatule de $MgSO_4$ a été ajouté dans le milieu ainsi que 24 μL (0,21 mmol; 1 éq.) de cyclohexylamine. Le mélange a été agité à température ambiante pendant une nuit. Le $MgSO_4$ a été éliminé par filtration sur fritté et le filtrat a été concentré sous pression réduite. Le résidu a été repris dans 5 mL de méthanol auquel 69 mg (1,05 mmol; 5 éq.) de cyanohydroborure de sodium ont été ajoutés. Après 20 minutes d'agitation à température ambiante, le milieu a été concentré sous pression réduite.

Etape de déprotection : Dans un ballon de 25 mL, 1,0 éq. du composé **(24)** a été dissous dans du dioxane (10 mL). 6 éq. d'une solution d'acide chlorhydrique gazeux (4 M dans du dioxane) ont été injectés dans le milieu. Le mélange a été laissé sous agitation à température ambiante pendant une à trois heures. Le précipité a été lavé à l'éther de pétrole puis récupéré par filtration pour obtenir un solide blanc avec un rendement de 85 %. MP : 254 °C ; Infrarouge (Diamand ATR, $cm^{-1}$) υ : 3364, 2933, 1617, 1556, 1510, 1428, 1388, 1110, 732 ; RMN $^1H$ (400 MHz, DMSO) δ: 1,17 (m, 6H, $6xH_{cyclohexane}$), 1,56 (m, 2H, $2xH_{cyclohexane}$), 1,76 (m, 2H, $2xH_{cyclohexane}$), 2,18 (m, 1H, $H_{cyclohexane}$), 3,83 (S, 4H, $2xCH_2(O)$), 4,38 (s, 2H, $CH_2$), 4,54 (s, 4H, $2xCH_2(N)$), 6,96 (s, 1H, $H_{arom}$), 7,30 (s, 1H, $H_{arom}$), 7,92 (s, 2H, $2xH_{arom}$), 8,55 (s, 1H, $H_8$), 9,03 (s, 1H, $H_6$), 9,80 (bs, 1H, OH), 10,05 (bs, 1H, NH) ; RMN $^{13}C$ (101 MHz, DMSO) δ: ; HRMS (EI-MS) : $C_{24}H_{29}N_5O_2$ $[M+H]^+$, calculée m/z 420,2394, trouvée m/z 420,2390.

**Chlorhydrate du 3-(4-morpholino-7-(morpholinométhyl)pyrido[3,2-*d*]pyrimidin-2-yl) phénol (29) :** Dans un ballon de 25 mL, sous une atmosphère inerte, 80 mg (0,21 mmol; 1 éq.) de **(19)** ont été dissous dans 6 mL d'un mélange (DCM/DMF, 5/1). Après avoir injecté 28 μL (0,315 mmol; 1,5 éq.) de morpholine, le mélange a été refroidi à 0 °C au moyen d'un bain de glace et 223 mg (1,051 mmol; 5 éq.) de triacétatehydroborure de sodium y ont été ajoutés. Après dix minutes d'agitation à 0 °C, 4 gouttes d'acide acétique ont été additionnées, la solution a alors été laissée sous agitation à température ambiante pendant six heures. 5 mL d'eau et 40 mL de dichlorométhane ont ensuite été ajoutés. La phase organique résultante a été lavée avec une solution aqueuse saturée en $NaHCO_3$ (10 mL), séchée sur $MgSO_4$, filtrée puis concentrée sous pression réduite.

L'étape de déprotection du MOM a été effectuée directement avec le résidu (25), qui a été dissous dans du dioxane (10

mL). 6 éq. d'une solution d'acide chlorhydrique gazeux (4 M dans du dioxane) ont été injectés dans le milieu. Le mélange a été laissé sous agitation à température ambiante pendant une à trois heures. Le précipité a été lavé à l'éther de pétrole puis récupéré par filtration pour obtenir un solide blanc (29) avec un rendement de 61 %. MP : 239 °C ; Infrarouge (Diamand ATR, $cm^{-1}$) υ : 3344, 3037, 1552, 1510, 1417, 1292, 1114, 1028, 864, 736 ; RMN $^{1}H$ (400 MHz, DMSO) δ: 3,26 (bs, 4H, $2xCH_2(N)$), 3,86 (bs, 8H, $4xCH_2(O)$), 4,63 (bs, 6H, $CH_2$ and $2xCH_2(N)$), 7,03 (s, 1H, $H_{arom}$), 7,37 (s, 1H, $H_{arom}$), 7,96 (m, 2H, $2xH_{arom}$), 8,76 (s, 1H, $H_8$), 9,13 (s, 1H, $H_6$), 12,35 (s, 1H, NH) ; RMN $^{13}C$ (101 MHz, DMSO) δ: ; HRMS (EI-MS) : $C_{22}H_{25}N_5O_3$ $[M+H]^+$, calculée m/z 408,1957, trouvée m/z 408,2020.

**Chlorhydrate du 3-(7-((4-méthylpipérazin-1-yl)méthyl)-4-morpholinopyrido[3,2-*d*] pyrimidin-2-yl)phénol (30) :** Dans un ballon de 25 mL, sous une atmosphère inerte, 100 mg (0,263 mmol; 1 éq.) de **(19)** ont été dissous dans 6 mL d'un mélange (dichlorométhane/DMF, 5/1). Après avoir injecté 44 μL (0,394 mmol; 1,5 éq.) de N-méthylpipérazine, le mélange a été refroidi à 0 °C au moyen d'un bain de glace et 279 mg (1,315 mmol; 5 éq.) de triacétatehydroborure de sodium y ont été ajoutés. Après dix minutes d'agitation à 0 °C, 4 gouttes d'acide acétique ont été additionnées et la solution a été laissée sous agitation à température ambiante pendant six heures. 5 mL d'eau et 40 mL de dichlorométhane ont alors été ajoutés. La phase organique résultante a été lavée avec une solution aqueuse saturée en $NaHCO_3$ (10 mL), séchée sur $MgSO_4$, filtrée puis concentrée sous pression réduite.

L'étape de déprotection du MOM a été effectuée directement avec l'intermédiaire réactionnel **(26),** par dilution dans du dioxane et ajout de 6 éq. d'une solution d'acide chlorhydrique gazeux (4 M dans du dioxane). Le mélange a été laissé sous agitation à température ambiante pendant une à trois heures. Le précipité a été lavé à l'éther de pétrole puis récupéré par filtration pour obtenir un solide blanc avec un rendement de 92 %. MP : 243 °C ; Infrarouge (Diamand ATR, $cm^{-1}$) υ : 2927, 1616, 1556, 1508, 1420, 1388, 1312, 1112, 881, 729 ; RMN $^{1}H$ (400 MHz, DMSO) δ: 2,81 (s, 3H, $NCH_3$), 3,46 (s, 2H, $CH_2(N)$), 3,88 (s, 5H, H and $2xCH_2(O)$), 4,86-4,45 (m, 6H, $3xCH_2(N)$), 7,10 (d, 1H, $J$= 7,2 Hz, $H_{arom}$), 7,40 (t, 1H, $J$ = 8,2 Hz, $H_{arom}$), 7,99-7,76 (m, 2H, $2xH_{arom}$), 8,81 (s, 1H, $H_8$), 9,10 (s, 1H, $H_6$), 11,91 (s, 1H, NH) ; RMN $^{13}C$ (101 MHz, DMSO) δ: ; HRMS (EI-MS) : $C_{22}H_{25}N_5O_3$ $[M+H]^+$, calculée m/z 421,2274, trouvée m/z 421,2361.

**3-{7-[(4-méthanesulfonylpipérazin-1-yl)methyl]-4-(morpholin-4-yl)pyrido[3,2-d]pyrimidin-2-yl}phénol (55):** Dans un ballon de 25 mL, sous une atmosphère inerte, 100 mg (0,26 mmol; 1 éq.) de **(19)** ont été dissous dans 6 mL d'un mélange (DCM/DMF, 5/1). Après avoir additionné 64 mg (0,39 mmol; 1,5 éq.) de 1-(méthylsulfonyl)pipérazine, le mélange a été refroidi à 0 °C au moyen d'un bain de glace et 165 mg (0.78 mmol; 5 éq.) de triacétatehydroborure de sodium y ont été ajoutés. Après dix minutes d'agitation à 0 °C, 4 gouttes d'acide acétique ont été additionnées, la solution a alors été laissée sous agitation à température ambiante pendant cinq heures. 10 mL d'eau et 40 mL de dichlorométhane ont ensuite été ajoutés. La phase organique résultante a été lavée avec une solution aqueuse saturée en $NaHCO_3$ (10 mL), séchée sur $MgSO_4$, filtrée puis concentrée sous pression réduite.

Etape de déprotection : Dans un ballon de 25 mL, 1,0 éq. du résidu obtenu a été dissous dans du dioxane (10 mL). 6 éq. d'une solution d'acide chlorhydrique gazeux (4 M dans du dioxane) ont été injectés dans le milieu. Le mélange a été laissé sous agitation à température ambiante pendant une à trois heures. Le précipité a été lavé à l'éther de pétrole puis récupéré par filtration pour obtenir un solide grisâtre (55) avec un rendement de 98 %. MP : 208 °C ; Infrarouge (Diamand ATR, $cm^{-1}$) υ : 3344, 3037, 1552, 1510, 1417, 1292, 1114, 1028, 864, 736 ; RMN $^{1}H$ (400 MHz, DMSO+$D_2O$ δ: 2,96 (s, 3H, $CH_3$), 3,26 (bs, 4H, $2xCH_2(N)$), 3,30 (bs, 4H, $2xCH_2(N)$), 3,40 (bs, 4H, $2xCH_2(N)$), 3,83 (bs, 4H, $2xCH_2(O)$), 4,54 (s, 2H, $CH_2$), 4,58 (bs, 4H, $2xCH_2(N)$), 6.99 (dd, $J$ = 8.0, 2.5 Hz, 1H, $H_{arom}$), 7.36 (t, $J$ = 7.9 Hz, 1H, $H_{arom}$), 7.88 - 7.77 (m, 2H, $H_{arom}$), 8.43 (d, $J$ = 2.1 Hz, 1H, $H_{arom}$), 8.90 (d, $J$ = 2.1 Hz, 1H, $H_{arom}$) ; RMN $^{13}C$ (101 MHz, DMSO) δ: ; HRMS (EI-MS) : $C_{23}H_{29}N_6O_4S[M+H]^+$, calculée m/z 485,1966, trouvée m/z 485,1961.

**3-{7-[(4-cyclohexylpipérazin-1-yl)méthyl]-4-(morpholin-4-yl)pyrido[3,2-d]pyrimidin-2-yl}phénol (56):** Sous une atmosphère inerte, Dans un ballon de 25 mL, 50 mg (0,13 mmol; 1 éq.) de **(19)** ont été dissous dans 5 mL d'un mélange (DCM/DMF, 5/1). Après avoir additionné 33 mg (0,19 mmol; 1,5 éq.) de 1-cyclohexylpipérazine, le mélange a été refroidi à 0 °C au moyen d'un bain de glace et 138 mg (0.66 mmol; 5 éq.) de triacétatehydroborure de sodium y ont été ajoutés. Après dix minutes d'agitation à 0 °C, 4 gouttes d'acide acétique ont été additionnées, la solution a alors été laissée sous agitation à température ambiante pendant cinq heures. 10 mL d'eau et 40 mL de dichlorométhane ont ensuite été ajoutés. La phase organique résultante a été lavée avec une solution aqueuse saturée en $NaHCO_3$ (10 mL), séchée sur $MgSO_4$, filtrée puis concentrée sous pression réduite.

L'étape de déprotection du MOM a été effectuée directement avec l'intermédiaire réactionnel (53), par dilution dans du dioxane et ajout de 6 éq. d'une solution d'acide chlorhydrique gazeux (4 M dans du dioxane). Le mélange a été laissé sous agitation à température ambiante pendant une à trois heures. Le précipité a été lavé à l'éther de pétrole puis récupéré par filtration pour obtenir un solide marron **(56)** avec un rendement de 58 %. MP : 250 °C ; Infrarouge (Diamand ATR, $cm^{-1}$) υ : 3344, 3037, 1552, 1510, 1417, 1292, 1114, 1028, 864, 736 ; RMN $^{1}H$ (400 MHz, DMSO) δ: 1.18-150 (m, 6H, $CH_2$), 1.6 (m, 1H, CH), 1.82 (m, 2H, $CH_2$), 2.18 (m, 2H, $CH_2$), 3,45 (bs, 4H, $2xCH_2(N)$), 3,60 (bs, 4H, $2xCH_2(N)$), 3,40 (bs, 4H, $2xCH_2(N)$), 3,90 (bs, 4H, $2xCH_2(O)$), 4,46 (bs, 4H, $2xCH_2(N)$), 4,70 (s, 2H, $CH_2$), 6.99 (dd, $J$ = 8.0, 2.5 Hz, 1H, $H_{arom}$), 7.36 (t, $J$ = 7.9 Hz, 1H, $H_{arom}$), 7.88 - 7.77 (m, 2H, $H_{arom}$), 8.43 (d, $J$ = 2.1 Hz, 1H, $H_{arom}$), 8.90 (d, $J$ = 2.1 Hz, 1H, $H_{arom}$), 11,51 (s, 1H, NH) ; RMN $^{13}C$ (101 MHz, DMSO) δ: ; HRMS (EI-MS) : $C_{28}H_{37}N_6O_2$ $[M+H]^+$, calculée m/z 489,2978, trouvée m/z 489,2973.

**3-[4-(morpholin-4-yl)-7-[(4-phénylpipérazin-1-yl)méthyl]pyrido[3,2-d]pyrimidin-2-yl]phénol (57):** Sous une atmosphère inerte, dans un ballon de 25 mL, 50 mg (0,13 mmol; 1 éq.) de **(19)** ont été dissous dans 5 mL d'un mélange (DCM/DMF, 5/1). Après avoir additionné 30 μL (0,19 mmol; 1,5 éq.) de 1-cyclohexylpipérazine, le mélange a été refroidi à 0°C au moyen d'un bain de glace et 139 mg (0.66 mmol ; 5 éq.) de triacétatehydroborure de sodium y ont été ajoutés. Après dix minutes d'agitation à 0°C, 4 gouttes d'acide acétique ont été additionnées, la solution a alors été laissée sous agitation à température ambiante pendant cinq heures. 10 mL d'eau et 40 mL de dichlorométhane ont ensuite été ajoutés. La phase organique résultante a été lavée avec une solution aqueuse saturée en $NaHCO_3$ (10 mL), séchée sur $MgSO_4$, filtrée puis concentrée sous pression réduite.

L'étape de déprotection du MOM a été effectuée directement avec l'intermédiaire réactionnel **(54),** par dilution dans du dioxane et ajout de 6 éq. d'une solution d'acide chlorhydrique gazeux (4 M dans du dioxane). Le mélange a été laissé sous agitation à température ambiante pendant une à trois heures. Le précipité a été lavé à l'éther de pétrole puis récupéré par filtration pour obtenir un solide marron **(57)** avec un rendement de 58 %. MP >250 °C ; Infrarouge (Diamand ATR, $cm^{-1}$) υ : 3271, 1552, 1510, 1417, 1292, 1114, 1028, 864, 736 ; RMN $^1H$ (400 MHz, DMSO) δ: 3,26 (bs, 4H, $2xCH_2(N)$), 3,47 (bs, 4H, $2xCH_2(N)$), 3,89 (bs, 4H, $2xCH_2(O)$), 4,20 (bs, 4H, $2xCH_2(N)$), 4,70 (s, 2H, $CH_2$), 6,87 (t, *J* = 7.3 Hz, 1H, $H_{arom}$), 7,0 (m, 2H, $H_{arom}$), 7,08 (d, *J* = 2.3 Hz, 1H, $H_{arom}$), 7,27 (m, 2H, $H_{arom}$) 7,41 (t, *J* = 7.9 Hz, 1H, $H_{arom}$), 7,86 (t, *J* = 2.0 Hz, 1H, $H_{arom}$), 7,91 (m, 1H, $H_{arom}$), 8,71 (d, *J* = 3.4 Hz, 1H, $H_{arom}$) 9,17 (d, *J* = 2.8 Hz, 1H, $H_{arom}$), 12,08 (s, 1H, NH) ; RMN $^{13}C$ (101 MHz, DMSO) δ: ; HRMS (EI-MS) : $C_{28}H_{31}N_6O_2$ $[M+H]^+$, calculée m/z 483,2508, trouvée m/z 489,2903.

| | |
|---|---|
| 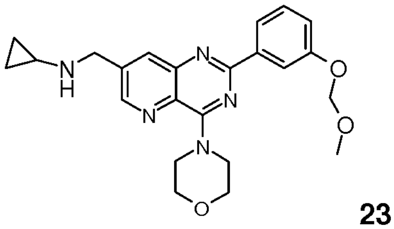 23 | 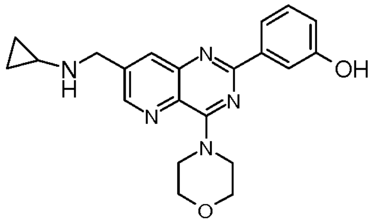 HCl 27 |
| 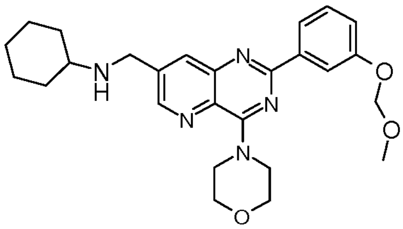 24 | 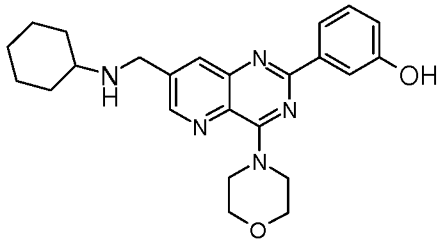 HCl 28 |
| 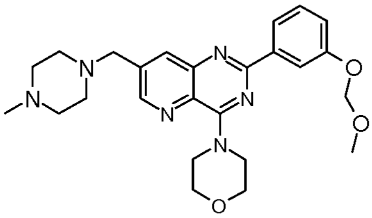 26 | 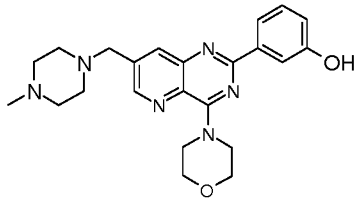 HCl 30 |
| 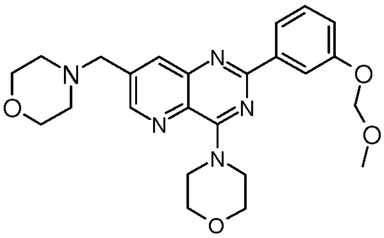 25 | 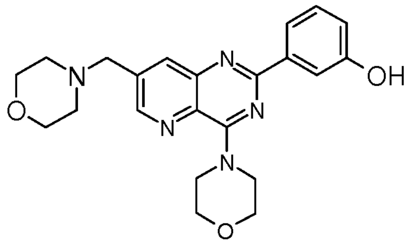 HCl 29 |
| 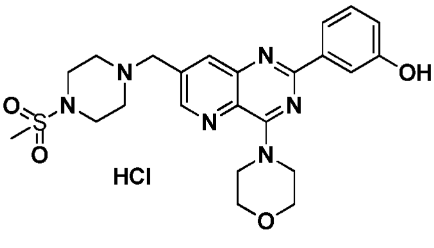 HCl 55 | 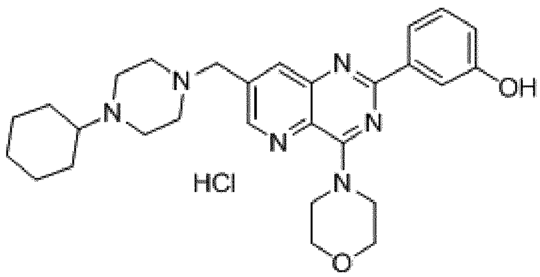 HCl 56 |

| | |
|---|---|
| 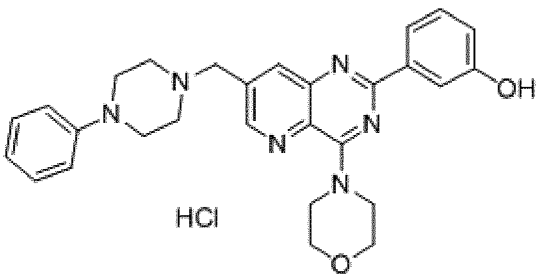 57 | |

A.5.4. Préparation de dérivés oxymes et autres

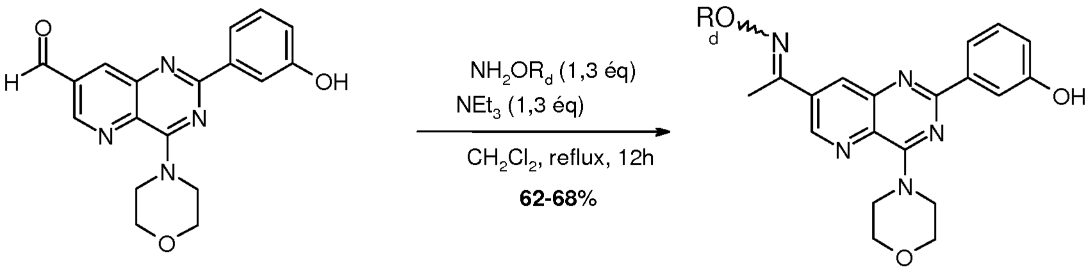


20

31-32

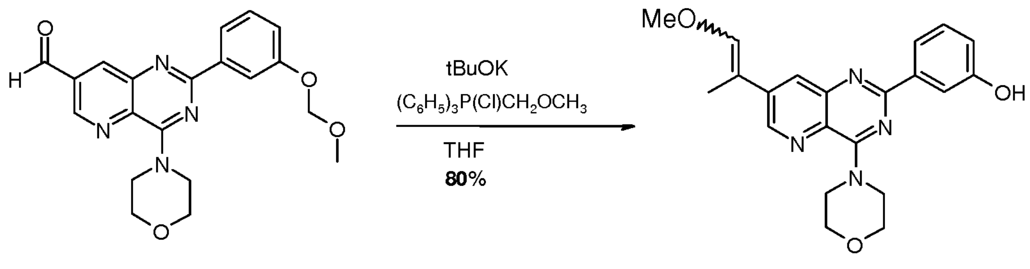


19

63

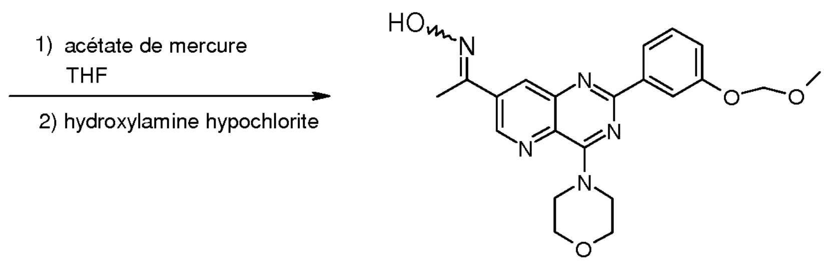


64

**2-(3-hydroxyphényl)-4-morpholinopyrido[3,2-*d*]pyrimidine-7-carbaldéhyde oxime** (**31**):

Dans un ballon de 25 mL, 66 mg (0,196 mmol; 1,0 éq.) de **(20)** ont été dissous dans 6 mL de dichlorométhane anhydre. 16 mg (0,235 mmol; 1,2 éq.) d'hydrochlorure d'hydroxylamine ont été ajoutés ainsi que 32 µL (0,235 mmol; 1,2 éq.) de triéthylamine. La solution a été laissée sous agitation pendant douze heures avant l'ajout supplémentaire

de dichlorométhane (40 mL). Le milieu réactionnel a été lavé avec une solution aqueuse saturée en bicarbonate de sodium (10 mL), séché sur $MgSO_4$, filtré puis concentré sous pression réduite pour obtenir un solide blanc pur sous forme d'un mélange d'isomère (*Z*/*E*, 85/15) avec un rendement de 62 %. Ces isomères peuvent être séparés par colonne de chromatographie sur gel de silice sous pression (DCM/MeOH, 99/1). MP : 231 °C ; Infrarouge (Diamand ATR, $cm^{-1}$) $\upsilon$ : 3277, 2864, 1736, 1561, 1520, 1434, 1356, 1311, 1234, 1107, 972, 739, 678, RMN $^1H$ (400 MHz, DMSO) $\delta$: 3,91-3,69 (m, 4H, $2xCH_2(O)$), 4,50 (bs, 4H, $2xCH_2(N)$), 6,90 (dd, 1H, *J* = 1,8 Hz, *J* = 7,7 Hz, $H_{arom}$), 7,30 (t, 1H, *J* = 8,0 Hz, $H_{arom}$), 7,81-7,99 (m, 2H, $2xH_{arom}$), 8,23 (d, 1H, *J* = 2,0 Hz, $H_8$), 8,40 (s, 1H, $H_{arom}$), 9,00 (d, 1H, *J* = 2,0 Hz, $H_6$), 9,54 (d, 1H, *J* = 5,7 Hz, OH), 11,92 (s, 1H, CNH) ; RMN $^{13}C$ (101 MHz, DMSO) $\delta$: 47,3 ($2xCH_2$), 66,4 ($2xCH_2$), 114,9 (CH), 117,6 (CH), 119,0 (CH), 129,3 (CH), 132,1 (Cq), 132,3 (Cq), 133,5 (CH), 139,1 (Cq), 143,5 (CH), 145,7 (CH), 147,6 (Cq), 157,4 (Cq), 158,4 (Cq), 159,3 (Cq) ; HRMS (EI-MS) : $C_{18}H_{17}N_5O_3$ $[M+H]^+$, calculée m/z 352,1331, trouvée m/z 352,1407.

**2-(3-hydroxyphényl)-4-morpholinopyrido[3,2-*d*]pyrimidine-7-carbaldéhyde O-méthyl oxime (32) :** Dans un ballon de 25 mL, 60 mg (0,178 mmol; 1,0 éq.) de **(20)** ont été dissous dans 6 mL de dichlorométhane anhydre. 20 mg (0,232 mmol; 1,3 éq.) d'hydrochlorure de méthoxyamine a été ajouté ainsi que 31 $\mu$L (0,232 mmol; 1,3 éq.) de triéthylamine. La solution a été laissée sous agitation pendant douze heures à reflux, avant l'ajout supplémentaire de dichlorométhane (40 mL). Le milieu réactionnel a été lavé avec une solution aqueuse saturée en bicarbonate de sodium (10 mL), séché sur $MgSO_4$, filtré puis concentré sous pression réduite pour obtenir un solide blanc pur sous forme d'un mélange d'isomère (*Z*/*E,* 90/10) avec un rendement de 68 %. Ces isomères peuvent être séparés par colonne de chromatographie sur gel de silice sous pression (DCM/MeOH, 99/1). MP : 244 °C ; Infrarouge (Diamand ATR, $cm^{-1}$) $\upsilon$ : 3040, 2962, 1517, 1442, 1348, 1148, 1115, 1054, 927, 743, 674, RMN $^1H$ (400 MHz, DMSO) $\delta$ : 3,83 (d, 4H, *J* = 4,1 Hz, $2xCH_2(O)$), 4,00 (s, 3H, $CH_3$), 4,50 (s, 4H, $2xCH_2(N)$), 6,90 (d, 1H, *J* = 8,1 Hz, $H_{arom}$), 7,30 (t, 1H, *J* = 8,1 Hz, $H_{arom}$), 7,88 (s, 2H, $2xH_{arom}$), 8,28 (s, 1H, $H_8$), 8,49 (s, 1H, $H_{oxime}$), 8,97 (s, 1H, $H_6$), 9,55 (s, 1H, OH) ; RMN $^{13}C$ (101 MHz, DMSO) $\delta$: 48,7 ($2xCH_2$), 62,3 ($CH_3$), 66,3 ($2xCH_2$), 114,9 (CH), 117,7 (CH), 119,0 (CH), 121,1 (Cq), 129,3 (CH), 131,1 (Cq), 132,4 (Cq), 134,2 (Cq), 143,6 (CH), 146,2 (CH), 157,4 (Cq), 158,4 (Cq), 159,3 (Cq), 164,8 (CH) ; HRMS (EI-MS) : $C_{19}H_{19}N_5O_3$ $[M+H]^+$, calculée m/z 366,1561, trouvée m/z 366,1564.

**4-[7-(2-méthoxyéthényl)-2-[3-(méthoxyméthoxy)phényl]pyrido[3,2-d]pyrimidin-4 yl]morpholine (63) :** Sous une atmosphère inerte, dans un ballon de 100 mL, 665.0 mg (1,94 mmol; 1,5 éq.) de méthoxyméthyltriphénylphosphonium de chlorure a été mis en suspension dans 15 mL de tétrahydrofurane (THF). A 0°C, 1,94 mL (C = 1 M, 1,94 mmol, 1,5 eq) de ter-butoxide de potassium a été ajouté. Au bout 1 h à 0°C, l'aldéhyde (19) (1,29 mmol, 1 eq) solubilisé dans 15 mL de THF a été ajouté goutte à goutte. De l'eau (30mL) a également été ajouté au bout de 48h à température ambiante. La phase organique a été extraite 3 fois avec de l'acétate d'éthyle (10mL), lavée avec une solution saline saturée, séchée sur $MgSO_4$, filtrée puis concentrée sous pression réduite. Le résidu brut a alors été purifié par colonne de chromatographie sur gel de silice sous pression (AE/ EP 2/8) permettant d'isoler un mélange de configuration E/Z (1/1) sous forme d'une huile incolore avec un rendement de 80%.; Infrarouge (Diamand ATR, $cm^{-1}$) $\upsilon$: 3293, 2856, 1528, 1495, 1442, 1352, 1111, 1021, 861, 739 ; RMN $^1H$ (400 MHz, $CDCl_3$) $\delta$: 3,55 (s, 3H, $CH_3$), 3,80 (s, 3H, $CH_3$ de E), 3,92 (s, 2H, $CH_3$ de Z), 3,94 (m, 4H, $2xCH_2(O)$), 4,48 (s, 4H, $2xCH_2(N)$), 5,30 (s, 2H, $CH_2$), 5,37 (d, 1H, *J* = 6,8 Hz, CH de Z), 5,90 (d, 1H, *J* = 13 Hz, CH de E), 6,45 (d, 1H, *J* = 6,8 Hz, CH de Z), 7,35 (d, 1H, *J* = 13 Hz, CH de E), 7,41 (t, 1H, *J* = 8,1 Hz, $H_{arom}$), 7,95 (d, 1H, *J* = 1,9 Hz, $H_8$ de E) 8,17 (m, 2H, $2xH_{arom}$), 8,42 (d, 1H, *J* = 1,9 Hz, $H_8$ de Z), 8,58 (d, 1H, *J* = 1,9 Hz, $H_6$ de E), 8,77 (d, 1H, $H_6$ de Z); RMN $^{13}C$ (101 MHz, $CDCl_3$) $\delta$: 48,0 ($2xCH_2$), 56,08 ($CH_3$), 57,22 ($OCH_3$), 61,29 ($OCH_3$), 67,3 ($2xCH_2$), 94,5 ($2xCH_2$), 100,9 (CH de E), 101 ,1 (CH de Z) et 110,4 (Cq), 116,2 et 116,3(CH), 117,9 et 118,0 (CH), 119,8 (CH), 129,9 (CH), 130,4 et 130,8 (Cq), 133,6 (CH), 135,4 et 135,9 (Cq) 147,8 et 148,4 (Cq), 148,2 et 146,3 (CH) 152,1 (CH de Z),152,2 (CH de E) 157,3 et 157,4 (Cq), 158,1 et 159,2 (Cq), 159,6 et 159,9 (Cq) ; HRMS (EI-MS) : $C_{22}H_{25}N_4O_4$ $[M+H]^+$, calculée m/z 409,1876, trouvée m/z 409,1870.

**N-(2-{2-[3-(méthoxyméthoxy)phényl]-4-(morpholin-4-yl)pyrido[3,2-d]pyrimidin-7-yl}éthylidène)hydroxylamine (64).** Dans un ballon de 100 mL, 342 mg (0.838 mmol; 1 éq.) de (19) a été solubilisé dans 15 mL de tétrahydrofurane (THF) et 5 mL d'eau. A 0°C, 800 mg (2,51 mmol, 3 eq) d'acétate de mercure ont été ajoutés. Au bout 2h à 0°C, une solution saline (30 mL) a été ajoutée. La phase organique a été extraite 3 fois avec de l'acétate d'éthyle (10mL), séchée sur $MgSO_4$, filtrée puis concentrée sous pression réduite. Le résidu brut a été solubilisé dans 40 mL de DCM anhydre sous une atmosphère inerte. 112 mg (1.62 mmol, 2 éq) d'hydroxylamine hypochlorite et 337,7 $\mu$L (2,43 mmol, 4 eq) de triéthylamine ont ensuite été ajoutés. Au bout d'une nuit à température ambiante, une solution aqueuse saturée en $NaHCO_3$ (10mL) a été ajoutée. La phase organique a été extraite 3 fois avec du DCM (30mL), séchée sur $MgSO_4$, filtrée puis concentrée sous pression réduite. Le résidu a été purifié par colonne de chromatographie sur gel de silice sous pression (AE/ EP 1/1) permettant d'isoler un solide jaunâtre avec un rendement de 45% correspondant à un mélange d'isomères (*E*/*Z* 1/1). MP : 154 °C ; Infrarouge (Diamand ATR, $cm^{-1}$) $\upsilon$ : 3200, 2911, 1701, 1640, 1508, 1461, 1426, 1268, 1154, 1116, 1071, 1008, 957, 739 ; RMN $^1H$ (400 MHz, $CDCl_3$) $\delta$ : 3,52 (s, 3H, $CH_3$), 3, 72 (d, *J* = 6,1 Hz, $CH_2$ de *E),* 3,92 (m, 6H, $CH_2$ de *Z* et $2xCH_2(O)$), 4,60 (bs, 4H, $2xCH_2(N)$), 5,28 (s, 2H, $CH_2$), 6,88 (t, *J* = 5,5 Hz, 1H, $CH_{oxime}$), 7,18 (m, 1H, $H_{arom}$), 7,40 (t, 1H, *J* = 7,5 Hz, $H_{arom}$), 7,62 (t, *J* = 6,1 Hz, 1H, $CH_{oxime}$), 8,12 (m, 4H, $H_8$ de *E* et *Z* et $2xH_{arom}$), 8,56 (d, *J* = 2 Hz, 1H, $H_6$ de *Z*), 8,58 (d, *J* = 2 Hz, 1H, $H_6$ de *E*), 8,85 (bs, 1*H*, OH); RMN $^{13}C$ (101 MHz, $CDCl_3$) $\delta$: 28,8 ($CH_2$ de *Z*), 33,3 ($CH_2$ de *E*), 48,4 ($2xCH_2$), 55,9 ($CH_3$), 66,6 ($2xCH_2$), 94,6 ($CH_2$), 115,7 (CH),

118,5 (CH), 122,8 (CH), 129,6 (CH), 131,9 (Cq), 135,1 (CH), 136,2 (Cq), 139,8 (Cq), 146,9 (CH), 147,0 (Cq), 148,3 (CH de *E*), 148,7 (CH de *Z*), 157,4 (Cq), 159,2 (Cq), 160,2 (Cq); HRMS (EI-MS) : $C_{21}H_{24}N_5O_4$ [M+H]$^+$, calculée m/z 410,1828, trouvée m/z 410,1823.

| | |
|---|---|
| 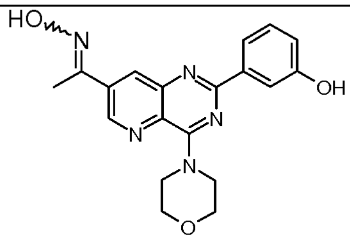 **31** | 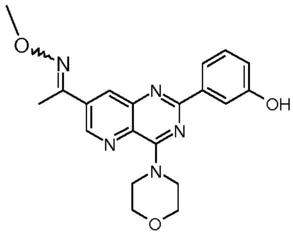 **32** |

A.5.5. <u>Réduction et fonctionnalisation en position C-7</u>

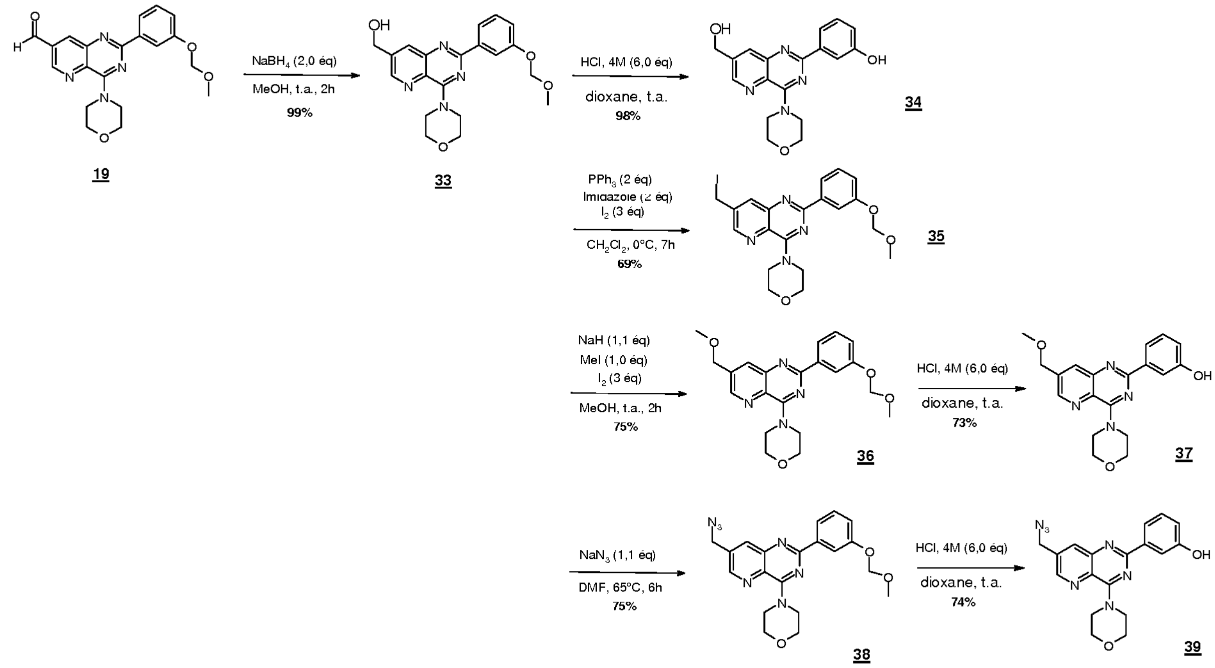

19 33 34 35 36 37 38 39

**(2-(3-(méthoxyméthoxy)phényl)-4-morpholinopyrido[3,2-*d*]pyrimidin-7-yl)méthanol (33) :** Dans un ballon de 25 mL, 25 mg (0,652 mmol, 1,0 éq.) de tétrahydruroborate de sodium ont été dissous dans 7 mL de méthanol. 180 mg (0,652 mmol, 1,0 éq.) de composé **(19)** ont été ajoutés dans le milieu. La solution a été laissée sous agitation pendant 3 heures avant d'être concentrée sous pression réduite. Le résidu obtenu a alors repris dans de l'acétate d'éthyle (40 mL). Cette phase organique a ensuite été lavée avec de l'eau (2 x 10 mL) puis séchée sur $MgSO_4$, filtrée et concentrée sous pression réduite pour obtenir le composé **(33)** sous forme d'un solide blanc avec un rendement de 98 %. MP : 134 °C. Infrarouge (Diamand ATR, cm$^{-1}$) υ : 3265, 2913, 1532, 1491, 1438, 1356, 1262, 1152, 1115, 1066, 1009, 915, 874, 739, RMN $^1$H (400 MHz, $CDCl_3$) δ: 3,54 (s, 3H, $CH_3$), 3,90 (m, 4H, 2x$CH_2$(O)), 4,55 (m, 4H, 2x$CH_2$(N)), 4,82 (s, 2H, $CH_2$), 5,29 (s, 2H, $CH_2$), 7,18 (dd, 1H, *J* = 2,3 Hz, *J* = 8,1 Hz, $H_{arom}$), 7,41 (t, 1H, *J* = 7,9 Hz, $H_{arom}$), 8,12 (m, 3H, $H_{arom}$, et $H_8$), 8,55 (d, *J* = 1,7 Hz, $H_6$). RMN $^{13}$C (101 MHz, DMSO) δ: 48,3 (2x$CH_2$), 56,3 ($CH_3$), 62,3 (2x$CH_2$), 67,4 ($CH_2$), 94,7 ($CH_2$), 116,5 (CH), 118,5 (CH), 122,3 (CH), 129,6 (CH), 132,2 (Cq),133,4 (CH), 139,9 (Cq), 140,5 (Cq), 145,5 (CH), 148,0 (Cq), 157,6 (Cq), 159,3 (Cq), 160,0 (Cq). HRMS (EI-MS) : $C_{20}H_{22}N_4O_4$ [M+H]$^+$, calculée m/z 383,1641 trouvée m/z 383,1715.

**3-(7-(hydroxyméthyl)-4-morpholinopyrido[3,2-d]pyrimidin-2-yl)phénol (34):** Le composé **(34)** a été synthétisé à partir du composé **(33)** (200 mg, 0,53 mmole) par dilution dans du dioxane et ajout de 6 éq. d'une solution

d'acide chlorhydrique gazeux (4 M dans du dioxane). Le mélange a été laissé sous agitation à température ambiante pendant une à trois heures. Le précipité a été lavé à l'éther de pétrole puis récupéré par filtration pour donner un solide jaune avec un rendement de 87 %. MP : 229 °C ; Infrarouge (Diamand ATR, $cm^{-1}$) υ : 3248, 2851, 1511, 1458, 1356, 1238, 1111, 1054, 886, 735 ; RMN $^1H$ (400 MHz, DMSO) δ: 3,82 (m, 4H, $2xCH_2(O)$), 4,51 (m, 4H, $2xCH_2(N)$), 4,73 (d, 2H, *J* = 5,6 Hz, $CH_2OH$) 5,59 (t, 1H, OH), 6,89 (dd, 1H, *J* = 1,6 Hz, *J* = 7,9 Hz, $H_{arom}$), 7,29 (t, *J* = 7,9 Hz, 1H, $H_{arom}$), 7,89 (m, 2H, $2xH_{arom}$), 8,04 (s, 1H, $H_8$), 8,70 (s, 1H, $H_6$), 9,54 (s, 1H, OH) ; RMN $^{13}C$ (101 MHz, DMSO) δ: 47,6 ($2xCH_2$), 60,3 ($CH_2$), 66,4 ($2xCH_2$), 114,8 (CH), 117,5 (CH), 119,0 (CH), 129,3 (CH), 131,1 (Cq), 132,5 (CH), 139,3 (Cq), 142,2 (Cq), 145,8 (CH), 147,6 (Cq), 157,4 (Cq), 158,5 (Cq), 158,8 (Cq) ; HRMS (EI-MS) : $C_{18}H_{18}N_4O_3$ $[M+H]^+$, calculée m/z 339,1379, trouvée m/z 339,1441.

**4-(7-(iodométhyl)-2-(3-(méthoxyméthoxy)phényl)pyrido[3,2-d]pyrimidin-4-yl)morpholine (35):** Dans un ballon de 25 mL, 110 mg (0,418 mmol; 2 éq.) de triphénylphosphine ont été dissous dans 5 mL de dichlorométhane anhydre. 159 mg (0,628 mmol; 3 éq.) de diiode et 29 mg (0,418 mmol; 2 éq.) d'imidazole ont été ajoutés au mélange. La solution a alors été refroidie à 0 °C au moyen d'un bain de glace et 80 mg (0,209 mmol; 1 éq.) de **(33)** ont été ajoutés. Le milieu réactionnel a été agité à 0 °C pendant 7 heures. 8 mL d'une solution aqueuse de $Na_2S_2O_3$ (10 %) ont été additionnés et après 15 minutes d'agitation, le mélange a été dilué dans 30 mL de dichlorométhane. La phase organique a été lavée avec de l'eau (2x10 mL), séchée sur $MgSO_4$, filtrée puis concentrée sous pression réduite. Le résidu a alors été purifié par chromatographie sur gel de silice sous pression (AcOEt/EP, 1/9) pour obtenir un solide jaune avec un rendement de 76 %. MP : 144 °C ; Infrarouge (Diamand ATR, $cm^{-1}$) υ : 2921, 1732, 1663, 1532, 1491, 1430, 1270, 1234, 1148, 1107, 1021, 964, 874, 739 ; RMN $^1H$ (400 MHz, $CDCl_3$) δ : 3,53 (s, 3H, $CH_3$), 3,92 (m, 4H, $2xCH_2(O)$), 4,53 (s, 2H, $CH_2I$), 4,58 (s, 4H, $2xCH_2(N)$), 5,28 (s, 2H, $CH_2$), 7,17 (m, 1H, $H_{arom}$), 7,40 (t, 1H, *J* = 7,9 Hz, $H_{arom}$), 8,17-8,10 (m, 3H, $2xH_{arom}$ et $H_8$), 8,66 (d, 1H, *J* = 2,3 Hz, $H_6$) ; RMN $^{13}C$ (101 MHz, $CDCl_3$) δ: 48,3 ($2xCH_2$), 56,3 (CH3), 62,4 ($CH_2$), 67,4 ($2xCH_2$), 94,8 ($CH_2$), 116,5 (CH), 118,5 (CH), 122,4 (CH), 129,6 (CH), 132,2 (Cq), 133,4 (CH), 135,40 (Cq), 140,0 (Cq), 145,5 (CH), 148,0 (Cq), 157,6 (Cq), 159,3 (Cq), 160,0 (Cq) ; HRMS (EI-MS) : $C_{20}H_{21}IN_4O_3$ $[M+H]^+$, calculée m/z 493,0658 trouvée m/z 493,0738.

**3-(7-méthoxy-4-morpholinopyrido[3,2-d]pyrimidin-2-yl)phénol (37)** Dans un ballon de 25 mL, 80 mg (0,209 mmol; 1 éq.) de **(33)** ont été dissous dans 7 mL de tétrahydrofurane anhydre. La solution a été refroidie à 0 °C au moyen d'un bain de glace, et 8 mg (0,23 mmol; 1,1 éq.) de NaH (60 % massique dans l'huile) ont été ajoutés au milieu. 13 μL (0,209 mmol; 1,0 éq.) de iodométhane ont alors été additionnés. Après dix minutes d'agitation à 0 °C, le bain de glace a été retiré et le mélange réactionnel a alors été laissé sous agitation pendant 2 heures 30 minutes à température ambiante. Après concentration sous pression réduite, le brut résiduel a été repris dans du dichlorométhane (40 mL), la phase organique a été lavée avec une solution aqueuse saturée en NaCl (2 x 8 mL), séchée sur $MgSO_4$, filtrée puis concentrée sous pression réduite.

L'étape de déprotection du MOM a été effectuée directement avec l'intermédiaire réactionnel **(36)** par dilution dans du dioxane et ajout de 6 éq. d'une solution d'acide chlorhydrique gazeux (4 M dans du dioxane). Le mélange a été laissé sous agitation à température ambiante pendant une à trois heures. Le précipité a été lavé à l'éther de pétrole puis récupéré par filtration pour obtenir le composé pur avec un rendement de 74 % sous la forme d'un solide jaune. MP : 165-166 °C ; Infrarouge (Diamand ATR, $cm^{-1}$) υ : 3273, 2852, 1540, 1491, 1438, 1352, 1270, 1103, 1021, 968, 878, 739, 678, RMN $^1H$ (400 MHz, $CDCl_3$) δ : 3,46 (s, 3H, $CH_3$), 3,92 (m, 4H, $2xCH_2(O)$), 4,58 (m, 4H, $2xCH_2(N)$), 4,63 (s, 2H, $CH_2$), 6,94 (dd, 1H, *J* = 2,2 Hz, *J* = 7,7 Hz, $H_{arom}$), 7,38 (t, 1H, *J* = 7,9 Hz, $H_{arom}$), 7,98 (m, 1H, $H_{arom}$), 8,02 (d, 1H, *J* = 7,9 Hz, $H_{arom}$), 8,10 (m, 1H, $H_8$), 8,65 (d, *J* = 2,1 Hz, $H_6$) ; RMN $^{13}C$ (101 MHz, $CDCl_3$) δ : 48,3 (2xCH2), 58,9 ($CH_3$), 67,5 ($2xCH_2$), 72,0 ($CH_2$), 115,5 (CH), 117,9 (CH), 121,1 (CH), 129,8 (CH), 132,5 (Cq), 134,5 (CH), 137,7(Cq), 140,1 (Cq), 146,1 (CH), 148,1(Cq), 156,2 (Cq), 159,4 (Cq), 160,3 (Cq) ; HRMS (EI-MS) : $C_{19}H_{20}N_4O_3$$[M+H]^+$, calculée m/z 353,1535 trouvée m/z 353,1609.

**4-(7-(azidométhyl)-2-(3-(méthoxyméthoxy)phényl)pyrido[3,2-d]pyrimidin-4-yl)morpholine (38)** : Dans un ballon de 25 mL, 78 mg (0,158 mmol; 1 éq.) de **(33)** ont été dilués dans 6 mL de diméthylformamide séché sur tamis 4À, ainsi que 15 mg (0,238 mmol; 1,5 éq.) d'azoture de sodium. Le mélange a été chauffé à 65 °C pendant 6 heures. Après un retour à température ambiante, 60 mL de dichlorométhane a été additionné. La phase organique résultante a été lavée avec une solution aqueuse d'acide citrique 10 % (10mL), avec une solution aqueuse saturée en $NaHCO_3$ (10mL) et à l'eau (2 x 10 mL). La phase organique a alors été séchée sur $MgSO_4$, filtrée puis concentrée sous pression réduite. Le composé **(38)** a été obtenu après purification par colonne de chromatographie sur gel de silice sous pression (DCM/MeOH, 98/2) avec un rendement de 75 % sous la forme d'un solide jaune. MP : 143 °C ; Infrarouge (Diamand ATR, $cm^{-1}$) υ : 2872, 2086, 1612, 1523, 1428, 1307, 1109, 1021, 862, 731 ; RMN $^1H$ (400 MHz, $CDCl_3$) δ : 3,53 (s, 3H, $CH_3$), 3,88 (m, 4H, $2xCH_2(O)$), 4,68 (bs, 4H, $2xCH_2(N)$), 4,90 (s, 2H, $CH_2N_3$), 5,28 (s, 2H, $CH_2$), 7,08 (m, 1H, $H_{arom}$), 7,41 (m, 1H, $H_{arom}$), 7,87 (m, 2H, $2xH_{arom}$), 8,46 (s, 1H, $H_8$), 8,91 (s, 1H, $H_6$) ; RMN $^{13}C$ (101 MHz, $CDCl_3$) δ : 48,4 ($2xCH_2$), 50,4 ($CH_2$), 56,3 ($CH_3$), 66,3 ($2xCH_2$), 94,7 ($CH_2$), 115,5 (CH), 119,6 (2xCH), 130,0 (CH), 132,2 (Cq), 134,8 (Cq), 137,8 (CH), 140,2 (Cq), 147,5 (CH), 149,2 (Cq), 157,8 (Cq), 159,5 (Cq), 160,3 (Cq) ; HRMS (EI-MS) : $C_{20}H_{21}N_7O_3$ $[M+H]^+$, calculée m/z 408,1706 trouvée m/z 408,1803.

**3-(7-(azidométhyl)-4-morpholinopyrido[3,2-*d*]pyrimidin-2-yl)phénol (39)** : Le composé **(9)** a été synthétisé

à partir du composé **(38)** (50 mg, 0,123 mmol) par dilution dans du dioxane et ajout de 6 éq. d'une solution d'acide chlorhydrique gazeux (4 M dans du dioxane). Le mélange a été laissé sous agitation à température ambiante pendant une à trois heures. Le précipité a été lavé à l'éther de pétrole puis récupéré par filtration pour donner un solide jaune avec un rendement de 98 %. MP : 169 °C ; Infrarouge (Diamand ATR, $cm^{-1}$) υ : 3383, 3043, 2868, 2096, 1613, 1552, 1511, 1434, 1307, 1111, 1021, 862, 731, 670; RMN $^1H$ (400 MHz, $CDCl_3$) δ : 3,89 (m, 4H, $2xCH_2(O)$), 4,70 (bs, 4H, $2xCH_2(N)$), 4,89 (s, 2H, $CH_2N_3$), 7,12 (m, 1H, $H_{arom}$), 7,43 (m, 1H, $H_{arom}$), 7,87 (m, 2H, $2xH_{arom}$), 8,49 (s, 1H, $H_8$), 8,87 (s, 1H, $H_6$), 10,06 (bs, 1H, OH) ; RMN $^{13}C$ (101 MHz, $CDCl_3$) δ : 48,4 ($2xCH_2$), 50,4 ($CH_2$), 66,3 ($2xCH_2$), 115,4 (CH), 119,6 (2xCH), 130,0 (CH), 132,7 (Cq), 133,8 (Cq), 137,4 (CH), 139,9 (Cq), 146,9 (CH), 148,8 (Cq), 157,8 (Cq), 159,1 (Cq), 160,0 (Cq) ; HRMS (EI-MS) : $C_{18}H_{17}N_7O_2$ $[M+H]^+$, calculée m/z 363,1444 trouvée m/z 364,1517.

A.5.6. Préparation de dérivés nitrile en position C7

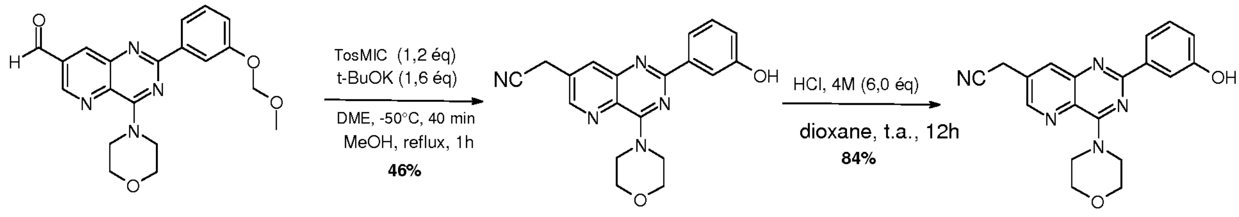


19 40 41

**2-(2-(3-(méthoxyméthoxy)phényl)-4-morpholinopyrido[3,2-d]pyrimidin-7-yl)acétonitrile (40)** : Sous une atmosphère inerte, dans un ballon de 25 mL, 108 mg (0,962 mmol; 1,6 éq.) de *t*-BuOK ont été dissous dans 4 mL de diméthoxyéthane anhydre, à -50 °C. Une solution contenant 141 mg (0,722 mmol; 1,2 éq.) de Toluènesulfonylméthyl isonitrile dans du diméthoxyéthane anhydre (4 mL) a été additionnée au goutte-à-goutte. Après 10 min d'agitation à -50 °C, une solution de 230 mg (0,602 mmol; 1,0 éq.) de **2(19)** dans 4 mL de diméthoxyéthane anhydre, a été ajoutée au goutte-à-goutte. Le mélange réactionnel a ensuite été laissé sous agitation pendant 40 minutes. Du méthanol (5 mL) a alors été additionné au mélange réactionnel avant de le porter à reflux pendant 1 heure. Après concentration sous pression réduite, le résidu a été repris dans de l'acétate d'éthyle (30 mL). La phase organique a été lavée avec de l'eau (2 x 10 mL), séchée sur $MgSO_4$, filtrée puis concentrée sous pression réduite. Le composé attendu **(40)** a été obtenu après purification par colonne de chromatographie sur gel de silice sous pression (DCM/MeOH, 99,4/0,6) avec un rendement de 46 % sous la forme d'un solide blanc. MP : 158 °C ; Infrarouge (Diamand ATR, $cm^{-1}$) υ : 2952, 2259, 1600, 1553, 1524, 1502, 1435, 1350, 1271, 1150, 1112, 1078, 1017, 967, 916, 875, 736, 685 ; RMN $^1H$ (400 MHz, $CDCl_3$) δ: 3,56 (s, 3H, $CH_3$), 3,95 (t, 6H, *J* = 4,8 Hz, $CH_2CN$ and $2xCH_2(O)$), 4,61 (s, 4H, $2xCH_2(N)$), 5,31 (s, 2H, $CH_2$), 7,20 (ddd, 1H, *J* = 1,1 Hz, *J* = 2,5 Hz, *J* = 8,1 Hz, $H_{arom}$), 7,43 (t, 1H, *J* = 7,9 Hz, $H_{arom}$), 8,16 (dd, 3H, *J* = 1,2 Hz, *J* = 7,8 Hz, $H_{arom}$ and $H_8$), 8,62 (d, 1H, *J* = 2,3 Hz, $H_6$) ; RMN $^{13}C$ (101 MHz, $CDCl_3$) δ : 21,6 ($CH_2$), 48,3 ($2xCH_2$), 56,3 ($CH_3$), 67,4 ($2xCH_2$), 94,7 ($CH_2$), 116,6 (CH), 118,6 (CH), 122,4 (CH), 129,5 (CH), 129,6 (Cq), 132,9 (Cq), 135,6 (CH), 139,8 (Cq), 145,0 (CH), 148,1 (Cq), 157,6 (Cq), 159,2 (Cq), 160,6 (Cq) ; HRMS (EI-MS) : $C_{21}H_{21}N_5O_3$ $[M+H]^+$, calculée m/z 392,1644 trouvée m/z 392,1718.

**2-(2-(3-hydroxyphényl)-4-morpholinopyrido[3,2-d]pyrimidin-7-yl)acétonitrile (41)** : Le composé **(41)** a été synthétisé à partir du composé **(40)** (70 mg, 0,179 mmol) par dilution dans du dioxane et ajout de 6 éq. d'une solution d'acide chlorhydrique gazeux (4 M dans du dioxane). Le mélange a été laissé sous agitation à température ambiante pendant une à trois heures. Le précipité a été lavé à l'éther de pétrole puis récupéré par filtration pour donner un solide blanc avec un rendement de 84 %. MP : 201 °C ; Infrarouge (Diamand ATR, $cm^{-1}$) υ : 3402, 2930, 2246, 1616, 1556, 1505, 1439, 1385, 1318, 1245, 1116, 1024, 865, 732 ; RMN $^1H$ (400 MHz, $CDCl_3$) δ: 3,87 (s, 4H, $2xCH_2(O)$), 4,44 (s, 2H, $CH_2$), 4,66 (bs, 4H, $2xCH_2(N)$), 7,08 (d, 1H, *J* = 8,0 Hz, $H_{arom}$), 7,41 (t, 1H, *J* = 7,9 Hz, $H_{arom}$), 8,04-7,76 (m, 2H, $H_{arom}$ and $H_8$), 8,46 (s, 1H, $H_{arom}$), 8,82 (d, 1H, *J* = 2,0 Hz, $H_6$), 9,85 (s, 1H, OH) ; RMN $^{13}C$ (101 MHz, $CDCl_3$) δ : ; HRMS (EI-MS) : $C_{19}H_{17}N_5O_2$ $[M+H]^+$, calculée m/z 348,1382 trouvée m/z 348,1455.

A.5.7. Préparation de dérivés acide carboxylique en position C7

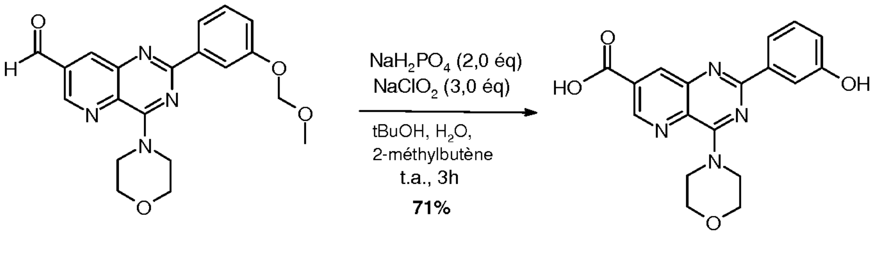


**19** **42**

**Acide 2-(3-hydroxyphényl)-4-morpholinopyrido[3,2-d]pyrimidine-7-carboxylique (42) :**

Dans un ballon de 50 mL, 150 mg (0,39 mmol; 1,0 éq.) du composé **(19)** ont été dissous dans un mélange THF/*t*-BuOH/2-méthylbutene, (10/11/3 mL). Une solution aqueuse (4mL) contenant 123 mg (0,79 mmol; 2,0 éq.) de $NaH_2PO_4$ et 107 mg (1,12 mmol; 3,0 éq.) de $NaCl_2O$ a été ajoutée au milieu réactionnel. Le mélange a été agité pendant 3 heures à température ambiante. 5 mL d'une solution d'acide citrique (10 %) a été additionné et après une agitation pendant 15 minutes le mélange a été dilué dans 30 mL d'acétate d'éthyle. La phase organique a été lavée avec une solution saturée en bicarbonate de sodium (10 mL) puis une solution saturée en chlorure de sodium (10 mL), l'extrait organique a été séché sur $MgSO_4$, filtré puis concentré sous pression réduite pour obtenir le produit sous forme de solide jaune avec un rendement de 71 %. MP : 158 °C ; Infrarouge (Diamand ATR, $cm^{-1}$) $\upsilon$ : 3177, 3048, 2965, 1728, 1619, 1559, 1509, 1193, 882 ; RMN $^1H$ (400 MHz, DMSO) $\delta$ : 3,80-3,87 (m, 4H, $2xCH_2(O)$), 4,69 (bs, 4H, $2xCH_2(N)$), 7,09 (d, 1H, $J$ = 4,9 Hz, $H_{arom}$), 7,41 (dd, 1H, $J$ = 4,9 Hz, $J$ = 5,0 Hz, $H_{arom}$), 7,85 (s, 1H, $H_8$), 7,93 (d, 1H, $J$ = 4,9 Hz, $H_{arom}$), 8,96 (s, 1H, $H_6$), 9,21 (s, 1H, OH), 9,95 (s, 1H, COOH) ; RMN $^{13}C$ (101 MHz, DMSO) $\delta$ : 49,2 ($2xCH_2$), 66,8 ($2xCH_2$), 116,2 (2xCH), 120,3 (CH), 120,6 (CH), 130,4 (2xCH), 130,9 (Cq), 133,1 (Cq), 147,5 (CH), 157,1 (Cq), 157,9 (Cq), 158,3 (2xCq), 165,1 (2xCq) ; HRMS (EI-MS) : $C_{18}H_{16}N_4O_4$ $[M+H]^+$, calculée m/z 353,1244, trouvée m/z 353,1246.

A.5.8. Préparation de dérivés triazoles en position C7

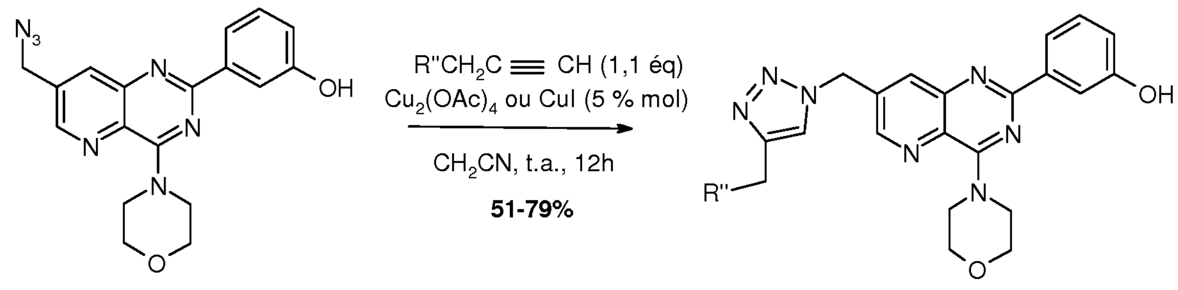


39

43 et 44
58 et 59

ou

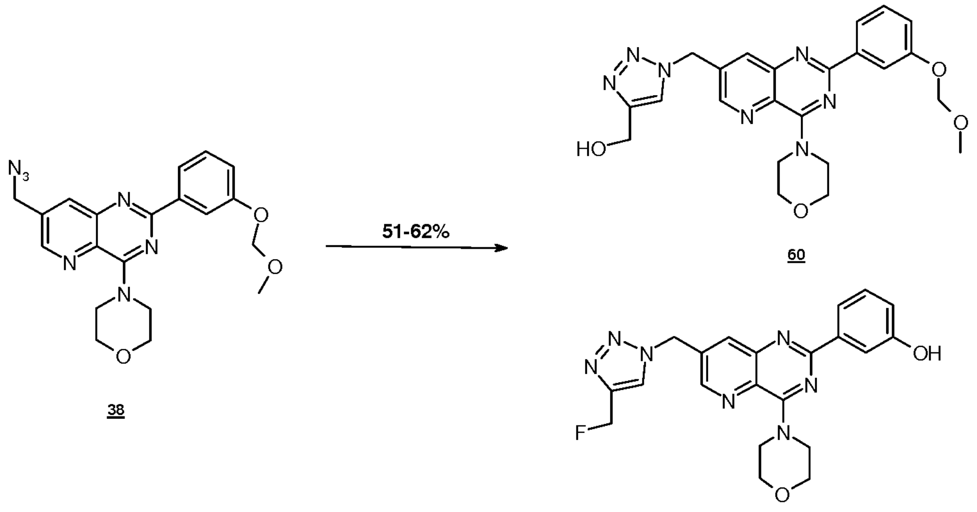


60

38

61

| | |
|---|---|
| 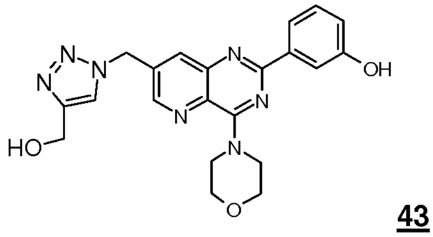 43 | 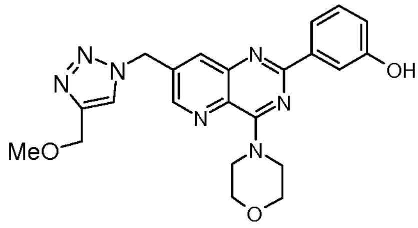 44 |
| 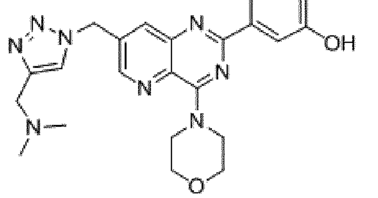 58 | 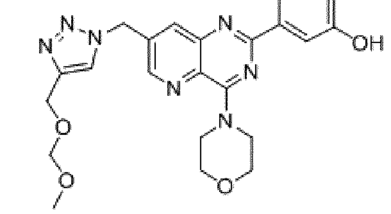 59 |
| 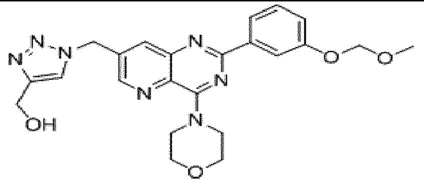 60 | 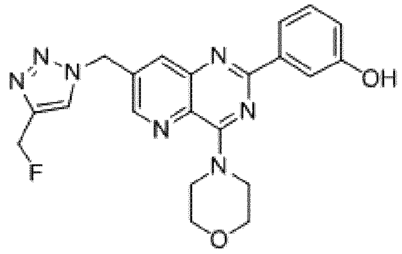 61 |

**3-(7-((4-(méthoxyméthyl)-1*H*-1,2,3-triazol-1-yl)méthyl)-4-morpholinopyrido[3,2-*d*]pyrimidin-2-yl)phénol (43)** : Dans un ballon de 10 mL, 80 mg (0,22 mmol; 1,0 éq.) de **(39)** ont été mis en suspension dans 3 mL d'acétonitrile. De la triéthylamine a été ajoutée goutte-à-goutte jusqu'à une parfaite dissolution du composé dans la solution. Ont alors été ajoutés, 3 mg (0,011 mmol; 0,05 éq.) de iodure de cuivre et 19 μL (0,22 mmol, 1 éq.) de méthoxy propargylique éther. Le mélange a été agité à température ambiante pendant 12 heures. La solution a été diluée dans de l'acétate d'éthyle (30 mL). La phase organique a été lavée avec une aqueuse saturée en $NaHCO_3$ (10mL), séchée sur $MgSO_4$, filtrée puis concentrée sous pression réduite. Le composé **(44)** a été isolé après purification par colonne de chromatographie sur gel de silice sous pression (DCM/MeOH, 99/1) avec un rendement de 79 %, sous la forme d'un solide blanc. MP : 241 °C ; Infrarouge (Diamand ATR, $cm^{-1}$) υ : 3130, 2856, 1589, 1552, 1516, 1430, 1315, 1275, 1107, 1062, 1029, 968, 792, 739, 674, RMN $^1H$ (400 MHz, $CDCl_3$) δ:3,28 (s, 3H, $CH_3$), 3,81 (m, 4H, $2xCH_2(O)$), 4,47 (m, 6H, $CH_2$ and $2xCH_2(N)$), 5,88 (s, 2H, $CH_2OCH_3$), 6,89 (d, 1H, $J$ = 7,8 Hz, $H_{arom}$), 7,28 (t, 1H, $J$ = 8,0 Hz, $H_{arom}$), 7,87 (d, 2H, $J$ = 6,6 Hz, $2xH_{arom}$), 7,94 (s, 1H, $H_8$), 8,32 (s, 1H, CH), 8,72 (s, 1H, $H_6$), 9,53 (s, 1H, OH) ; RMN $^{13}C$ (101 MHz, $CDCl_3$) δ : 47,6 ($2xCH_2$), 49,9 ($CH_2$), 57,4 (CH), 64,9 ($CH_2$), 66,4 ($2xCH_2$), 114,8 (CH), 117,6 (CH), 119,0 (CH), 124,5 (CH), 129,3 (CH), 131,8 (Cq), 134,5 (CH), 135,8 (Cq), 139,0 (Cq), 144,4 (Cq), 145,9 (CH), 147,3 (Cq), 157,4 (Cq), 158,4 (Cq), 159,2 (Cq) ; HRMS (EI-MS) : $C_{22}H_{23}N_7O_3$ $[M+H]^+$, calculée m/z 433,1862 trouvée m/z 434,1939.

**3-(7-((4-(hydroxyméthyl)-1*H*-1,2,3-triazol-1-yl)méthyl)-4-morpholinopyrido[3,2-*d*]pyrimidin-2-yl)phénol (44)** Dans un ballon de 10 mL, 82 mg (0,226 mmol; 1 éq.) de **(39)** ont été mis en suspension dans 3 mL d'acétonitrile. De la triéthylamine a été ajoutée goutte-à-goutte jusqu'à une parfaite dissolution du composé dans la solution. Ont alors été ajoutés, 3 mg (0,011 mmol; 0,05 éq.) de iodure de cuivre et 16 μL (0,249 mmol, 1,1 éq.) d'alcool propargylique. Le mélange a été agité à température ambiante pendant 12 heures. La solution a été diluée dans de l'acétate d'éthyle (30 mL). La phase organique a été lavée avec une aqueuse saturée en $NaHCO_3$ (10mL), séchée sur $MgSO_4$, filtrée puis concentrée sous pression réduite. Le résidu brut a alors été purifié par colonne de chromatographie sur gel de silice sous pression (DCM/MeOH, 98/2) permettant d'isoler un solide blanc avec un rendement de 51 %. MP : 238 °C ;

Infrarouge (Diamand ATR, $cm^{-1}$) υ : 3154, 2962, 2848, 1605, 1556, 1495, 1458, 1348, 1266, 1115, 1025, 886, 739, 678, RMN $^1H$ (400 MHz, $CDCl_3$) δ : 3,81 (m, 4H, $2xCH_2(O)$), 4,50 (s, 4H, $2xCH_22(N)$), 4,54 (d, 2H, *J* = 5,6 Hz, $CH_2$), 5,20 (t, 1H, *J* = 5,6 Hz, OH), 5,87 (s, 1H, CH), 6,89 (d, 1H, *J* = 7,8 Hz, $H_{arom}$), 7,28 (t, 1H, *J* = 8,0 Hz, $H_{arom}$), 7,84 (m, 2H, $2xH_{arom}$), 7,94 (d, 1H, *J* = 2,0 Hz, $H_8$), 8,19 (s, 1H, CH), 8,72 (d, 1H, *J* = 2,0 Hz, $H_6$), 9,53 (s, 1H, OH) ; RMN $^{13}C$ (101 MHz, $CDCl_3$) δ : 47,6 ($2xCH_2$), 49,9 ($CH_2$), 55,0 ($CH_2$), 66,4 ($2xCH_2$), 114,8 (CH), 117,6 (CH), 119,0 (CH), 123,4 (Cq), 129,3 (CH), 131,8 (CH), 134,4 (Cq), 136,0 (Cq), 139,0 (CH), 145,9 (CH), 147,3 (Cq), 148,6 (Cq), 157,4 (Cq), 158,4 (Cq), 159,2 (Cq) ; HRMS (EI-MS) : $C_{21}H_{21}N_7O_3[M+H]^+$, calculée m/z 420,1706 trouvée m/z 420,1784.

**3-[7-({4-[(diméthylamino)méthyl]-1H-1,2,3-triazol-1-yl}méthyl)-4-(morpholin-4-yl)pyrido[3,2-d]pyrimidin-2-yl]phénol (58)** : Sous une atmosphère inerte, dans un ballon de 10 mL, 80 mg (0,22 mmol; 1,0 éq.) de **(39)** ont été mis en suspension dans 3 mL d'acétonitrile. De la triéthylamine a été ajoutée goutte-à-goutte jusqu'à une parfaite dissolution du composé dans la solution. Ont alors été ajoutés, 25 μL (C = 0.4 M; 0,011 mmol; 0,05 éq.) de l'acétate de cuivre hydraté et 26 μL (0,22 mmol, 1 éq.) de 3-diméthylamino-1-propyne. Le mélange a été agité à température ambiante pendant 12 heures. La solution a été diluée dans de l'acétate d'éthyle (30 mL). La phase organique a été lavée avec une aqueuse saturée en $NaHCO_3$ (10mL), séchée sur $MgSO_4$, filtrée puis concentrée sous pression réduite. Le composé **(58)** a été trituré avec le dichlorométhane, puis filtré sous vide, pour conduire au composé avec un rendement de 31 %, sous la forme d'un solide jaunâtre. MP : 224 °C ; Infrarouge (Diamand ATR, $cm^{-1}$) υ : 3271, 2856, 1595, 1557, 1508, 1437, 1308, 1269, 1166, 1113, 1062, 1029, 968, 792, 739, 674, RMN $^1H$ (400 MHz, DMSO-*d₆*) δ: 2,18 (bs, 6H, $2xCH_3$) ; 3,55 (bs, 2H, $CH_2$), 3,83 (m, 4H, $2xCH_2(O)$), 4,51 (m, 6H, $2xCH_2(N)$), 5,89 (s, 2H, $CH_2OCH_3$), 6,90 (d, 1H, *J* = 7,8 Hz, $H_{arom}$), 7,30 (t, 1H, *J* = 8,0 Hz, $H_{arom}$), 7,87 (d, 2H, *J* = 6,6 Hz, $2xH_{arom}$), 7,94 (s, 1H, $H_8$), 8,24 (s, 1H, CH), 8,73 (s, 1H, $H_6$), 9,55 (s, 1H, OH) ; RMN $^{13}C$ (101 MHz, DMSO-$d_6$) δ : 45,0 ($2xCH_3$), 48,1 ($2xCH_2$), 50,4 ($CH_2$), 55,9 (CH), 55,4 ($CH_2$), 66,8 ($2xCH_2$), 115,3 (CH), 118,1 (CH), 119,5 (CH), 124,8 (CH), 129,8 (CH), 132,3 (Cq), 135,0 (CH), 136,3 (Cq), 139,5 (Cq), 144,4 (Cq), 146,4 (CH), 147,8 (Cq), 157,9 (Cq), 158,8 (Cq), 159,6 (Cq) ; HRMS (EI-MS) : $C_{23}H_{26}N_8O_2$ $[M+H]^+$, calculée m/z 447,2257 trouvée m/z 447,2251.

**3-[7-({4-[(méthoxyméthoxy)méthyl]-1H-1,2,3-triazol-1-yl}méthyl)-4-(morpholin-4-yl)pyrido[3,2-d]pyrimidin-2-yl]phénol (59)** : Sous une atmosphère inerte, dans un ballon de 10 mL, 45 mg (0,13 mmol; 1,0 éq.) de **(39)** ont été mis en suspension dans 3 mL d'acétonitrile. De la triéthylamine a été ajoutée goutte-à-goutte jusqu'à une parfaite dissolution du composé dans la solution. Ont alors été ajoutés, 1.2 mg (0,006 mmol; 0,05 éq.) de iodure de cuivre et 14 mg (0,14 mmol, 1.1 éq.) de méthoxy(prop-2-yn-1-yloxy)méthane. Le mélange a été agité à température ambiante pendant 12 heures. La solution a été diluée dans de l'acétate d'éthyle (30 mL). La phase organique a été lavée avec une aqueuse saturée en $NaHCO_3$ (10 mL), séchée sur $MgSO_4$, filtrée puis concentrée sous pression réduite. Le brut a été trituré avec de l'éther diéthylique suivi d'une filtration sous vide qui a conduit au composé avec un rendement de 25 %, sous la forme d'un solide brun. MP : 218 °C ; Infrarouge (Diamand ATR, $cm^{-1}$) υ : 3204, 2937, 1618, 1589, 1552, 1516, 1430, 1315, 1275, 1107, 1062, 1029, 968, 792, 739, 674, RMN $^1H$ (400 MHz, $CDCl_3$) δ: 3,29 (s, 3H, $CH_3$), 3,82 (m, 4H, $2xCH_2(O)$), 4,47 (bs, $2xCH_2(N)$), 4,61 (s, 2H, $CH_2$), 4,65 (s, 2H, CH2) ; 5,90 (s, 2H, $CH_2$), 6,89 (d, 1H, *J* = 7,8 Hz, $H_{arom}$), 7,30 (t, 1H, *J* = 8,0 Hz, $H_{arom}$), 7,88 (d, 2H, *J* = 6,6 Hz, $2xH_{arom}$), 7,96 (s, 1H, $H_8$), 8,35 (s, 1H, CH), 8,74 (s, 1H, $H_6$), 9,55 (s, 1H, OH) ; RMN $^{13}C$ ($101_{MHz}$, $CDCl_3$) δ : 47,6 ($2xCH_2$), 49,9 ($CH_2$), 56,6 ($CH_2$), 61,74 ($CH_2$), 68,2 ($2xCH_2$), 95,5 ($CH_2$), 115,3 (CH), 118,0 (CH), 119,5 (CH), 125,1 (CH), 129,8 (CH), 135,0 (Cq), 136,26 (Cq), 139,5 (Cq), 144,8 (Cq), 146,5 (CH), 147,8 (Cq), 157,9 (Cq), 158,9 (Cq), 159,6 (Cq) ; HRMS (EI-MS) : $C_{23}H_{26}N_7O_4$ $[M+H]^+$, calculée m/z 464,2046 trouvée m/z 464,2041.

**[1-({2-[3-(méthoxyméthoxy)phényl]-4-(morpholin-4-yl)pyrido[3,2-d]pyrimidin-7-yl}méthyl)-1H-1,2,3-triazol-4-yl]méthanol (60):** Sous une atmosphère inerte, dans un ballon de 10 mL, 70 mg (0,17 mmol; 1 éq.) de **(38)** ont été mis en suspension dans 3 mL d'acétonitrile. De la triéthylamine a été ajoutée goutte-à-goutte jusqu'à une parfaite dissolution du composé dans la solution. Ont alors été ajoutés, 2 mg (0,008 mmol; 0,05 éq.) de iodure de cuivre et 11 μL (0,187 mmol, 1,1 éq.) d'alcool propargylique. Le mélange a été agité à température ambiante pendant 12 heures. La solution a été diluée dans de l'acétate d'éthyle (30 mL). La phase organique a été lavée avec une solution aqueuse saturée en $NaHCO_3$ (10mL), séchée sur $MgSO_4$, filtrée puis concentrée sous pression réduite. Le résidu brut a alors été purifié par colonne de chromatographie sur gel de silice sous pression (Acétate d'éthyle 100%) permettant d'isoler un solide blanc amorphe avec un rendement de 51 %.; Infrarouge (Diamand ATR, $cm^{-1}$) υ : 3154, 2962, 2848, 1605, 1556, 1495, 1458, 1348, 1266, 1115, 1025, 886, 739, 678, RMN $^1H$ (400 MHz, $CDCl_3$) δ : 3,55 (s, 3H, CH3), 3,94 (m, 4H, $2xCH_2(O)$), 4,61 (bs, 4H, $2xCH_2(N)$), 4,84 (s, 2H, $CH_2$), 5,30 (s, 2H,$CH_2$), 5,75 (s, 2H, $CH_2$), 7,18 (m, 1H, $H_{arom}$), 7,40 (t, 1H, *J* = 8,0 Hz, $H_{arom}$), 7.58 (s, 1H, CH), 8,02 (m, 2H, $H_8$ et $H_{arom}$), 8,10 (m, 1H, $H_{arom}$), 8,60 (d, 1H, *J* = 2,0 Hz, $H_6$); RMN $^{13}C$ (101 MHz, $CDCl_3$) δ: 48,1 ($2xCH_2$), 51,6 ($CH_2$), 56,1 ($CH_3$), 57,4 ($CH_2$), 67,5 ($2xCH_2$), 94.53 ($CH_2$), 116,3 (CH), 118,5 (CH), 122,2 (CH), 123,4 (Cq), 129,4 (CH), 135,3 (CH), 134,4 (Cq), 135,0 (Cq), 139,0 (CH), 145,9 (CH), 152,7 (Cq), 157,8 (Cq), 158,4 (Cq), 160,5 (Cq), 167.0 (Cq) ; HRMS (EI-MS) : $C_{21}H_{21}N_7O_3$ $[M+H]^+$, calculée m/z 464,2046 trouvée m/z 464,2041.

**3-(7-{[4-(fluorométhyl)-1H-1,2,3-triazol-1-yl]méthyl}-4-(morpholin-4-yl)pyrido[3,2-d]pyrimidin-2-yl)phénol** (61) : Sous une atmosphère inerte, dans un ballon de 10 mL, 35 mg (0,17 mmol; 1 éq.) de **(38)** ont été mis en solution dans 4 mL de dichlorométhane. A 0°C, on ajoute 11 μL (0.083 mmol, 1.1 eq) de Diéthylaminosulfure trifluoride

(DAST) goutte à goutte. Au bout d'une heure, on rajoute 22 μL (0.34 mmol, 2 eq) de Diéthylaminosulfure trifluoride. Le mélange a été agité toujours à 0°C pendant 1 heure. La réaction a été arrêtée en ajoutant une solution aqueuse saturée en $NaHCO_3$ (10mL). La phase organique a été extraite 3 fois avec de l'acétate d'éthyle (10mL), séchée sur $MgSO_4$, filtrée puis concentrée sous pression réduite. L'étape de déprotection du MOM a été effectuée directement avec le résidu pour obtenir un solide jaunâtre **(61)** avec un rendement de 62%. MP : 168 °C ; Infrarouge (Diamand ATR, $cm^{-1}$) υ : 3354, 3046, 1620, 1562, 1506, 1425, 1314, 1266, 1115, 1025, 886, 739, 678, RMN $^1H$ (400 MHz, $CDCl_3$) δ : 3,86 (m, 4H, $2xCH_2(O)$), 4,56 (s, 4H, $2xCH_2$ (N)), 5,55 (d, 2H, J= 48 Hz, $CH_2$ F), 6.01 (s, 2H, $CH_2$), 7,03 (m, 1H, $H_{arom}$), 7,39 (t, 1H, *J* = 8,0 Hz, $H_{arom}$), 7,77 (m, 1H, $H_{arom}$), 7,83 (d, 1H, J = 2,0 Hz, $H_8$), 8,09 (s, 1H, CH), 8,56 (d, 1H, *J* = 2,0 Hz, $H_6$), 8.86 (s, 1H, OH) ; RMN $^{13}C$ (101 MHz, $CDCl_3$) δ : 47,6 ($2xCH_2$), 49,9 ($CH_2$), 55,0 ($CH_2$), 66,4 ($2xCH_2$), 76,4 (d, *J* = 159 Hz, $CH_2$), 114,8 (CH), 117,6 (CH), 119,0 (CH), 123,4 (Cq), 129,3 (CH), 131,8 (CH), 134,4 (Cq), 136,0 (Cq), 139,0 (CH), 145,9 (CH), 147,3 (Cq), 148,6 (Cq), 157,4 (Cq), 158,4 (Cq), 159,2 (Cq) ; RMN $^{19}F$ (376 MHz, DMSO-$d_6$) δ : -202.6 ($CH_2F$) ; HRMS (EI-MS) : $C_{21}H_{21}FN_7O_2$ $[M+H]^+$, calculée m/z 422,1741 trouvée m/z 422,1735.

A.5.9. Préparation de dérivés aldéhydes en position C7

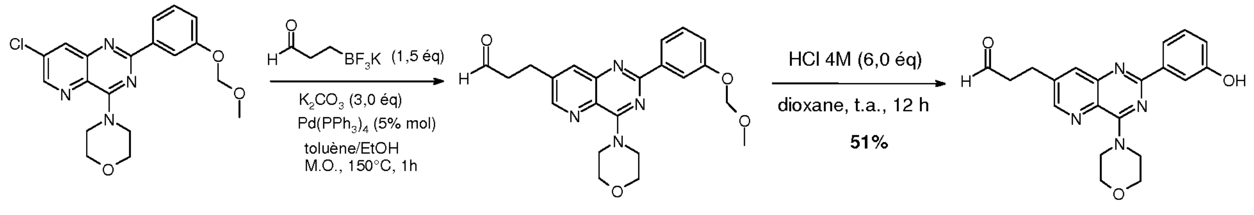


11 45 46

**3-(2-(3-(méthoxyméthoxy)phényl)-4-morpholinopyrido[3,2-d]pyrimidin-7-yl)propanal (46)** : Le composé **(45)** a été synthétisé à partir du composé **(11)** (110 mg, 0,284 mmole) en suivant la procédure générale B décrite précédemment.

L'intermédiaire réactionnel **(45)** a été directement dilué dans du dioxane et 6 éq. d'une solution d'acide chlorhydrique gazeux a été ajouté au milieu (4 M dans du dioxane). Le mélange a été laissé sous agitation à température ambiante pendant une à trois heures. Le précipité a été lavé à l'éther de pétrole puis récupéré par filtration pour donner un solide blanc avec un rendement de 51 %. MP : 213 °C. Infrarouge (Diamand ATR, $cm^{-1}$) υ : 2866, 1723, 1542, 1515, 1423, 1368, 1109, 1023, 882, 743 ; RMN $^1H$ (400 MHz, $CDCl_3$) δ: 2,90 (dd, 2H, *J* = 7,1 Hz, *J* = 7,2 Hz, $CH_2$), 3,10 (dd, 2H, *J* = 7,2 Hz, *J* = 7,3 Hz, $CH_2$), 3,89-3,93 (m, 4H, $2xCH_2(O)$), 4,55-4,58 (bs, 4H, $2xCH_2(N)$), 6,98 (ddd, 1H, *J* = 0,9 Hz, *J* = 2,7 Hz, *J* = 8,0 Hz, $H_{arom}$), 7,32 (dd, 1 H, *J* = 7,8 Hz, *J* = 7,9 Hz, $H_{arom}$), 7,94-7,97 (m, 3H, $2xH_{arom}$, $H_8$), 8,54 (d, 1H, *J* = 2,2 Hz, $H_6$), 9,83 (s, 1H, CHO) ; RMN $^{13}C$ (101 MHz, $CDCl_3$) δ: 25,1 ($CH_2$), 44,1 ($CH_2$), 48,0 ($2xCH_2$), 67,3 ($2xCH_2$), 115,3 (CH), 117,7 (CH), 120,9 (CH), 129,6 (CH), 131,5 (Cq), 134,3 (CH), 139,8 (Cq), 139,9 (Cq), 147,2 (CH), 147,9 (Cq), 156,1 (Cq), 159,1 (Cq), 160,1 (Cq), 200,1 (CH) ; HRMS (EI-MS) : $C_{20}H_{20}N_4O_3$ $[M+H]^+$, calculée m/z 365,1659; trouvée m/z 365,1661.

A.5.10. Préparation de dérivés isoxazole en position 7

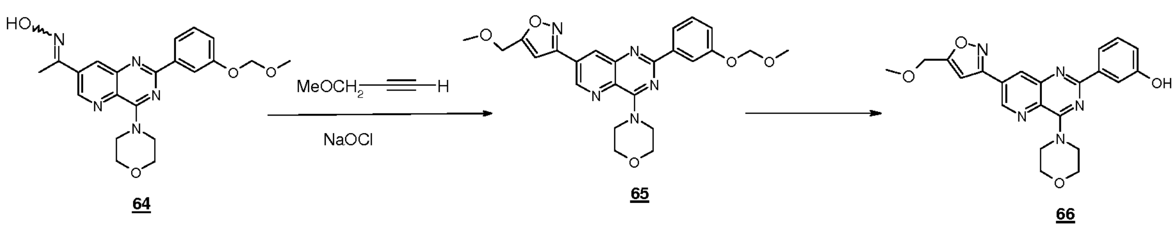


64 65 66

**4-{2-[3-(méthoxymethoxy)phényl]-7-{[5-(méthoxyméthyl)-1,2-oxazol-3-yl]méthyl}pyrido[3,2-d]pyrimidin-4-yl}morpholine (65):** Dans un ballon de 10 mL, 88mg (0,22 mmol; 1 éq.) de **(64)** a été solubilisé dans 4 mL de tétrahydrofurane (THF). 20 μL (0,22 mmol, 1,1 éq.) de méthoxy propargylique éther et 246 μL (15% dans l'eau, 0.43

mmol, 2eq) de l'hypochlorite de sodium ont été ajoutés. Au bout d'une nuit à température ambiante, de l'eau (10 mL) a été ajoutée. La phase organique a été extraite 3 fois avec de l'acétate d'éthyle (10mL), séchée sur $MgSO_4$, filtrée puis concentrée sous pression réduite. Le résidu brut a alors été purifié par colonne de chromatographie sur gel de silice sous pression (AE/ EP 1/1) permettant d'isoler une huile jaunâtre avec un rendement de 50%. Infrarouge (Diamand ATR, $cm^{-1}$) υ : 2923, 2852, 1701, 1640, 1508, 1461, 1426, 1268, 1154, 1116, 1071, 1008, 957, 739 ; RMN $^1H$ (400 MHz, $CDCl_3$) δ : 3,41 (s, 3H, $CH_3$), 3,52 (s, 3H, $CH_3$), 3,92 (m, 4H, $2xCH_2(O)$), 4,20 (s, 2H, $CH_2$), 4,50 (s, 2H, $CH_2$), 4,60 (bs, 4H, $2xCH_2(N)$), 5,28 (s, 2H, $CH_2$), 6,88 (t, *J* = 5,5 Hz, 1H, CH), 7,18 (m, 1H, $H_{arom}$), 7,40 (t, 1H, *J* = 7,5 Hz, $H_{arom}$), 6,11 (s, 1H, CH), 7,17 (m, 1H, $H_{arom}$), 7,40 (m, 1H, $H_{arom}$), 8,15 (m, 3H, $H_8$ et $2xH_{arom}$), 8,58 (d, *J* = 2 Hz, 1H, $H_6$), RMN $^{13}C$ (101 MHz, $CDCl_3$) δ : 30,0 ($CH_2$), 47,7 ($2xCH_2$), 56,2 ($CH_3$), 59,16 ($CH_3$), 65,7 ($CH_2$), 67,4 ($2xCH_2$), 94,8 ($CH_2$), 100,1 (Cq), 102,5 (CH), 116,5 (CH), 118,3 (CH), 122,1 (CH), 129,4 (CH), 135,4 (Cq), 136,5 (CH), 136,2 (Cq), 146,9 (CH), 157,2 (Cq), 157,7 (Cq), 159,2 (Cq), 161,2 (Cq), 170,13 (Cq); HRMS (EI-MS) : $C_{25}H_{28}N_5O_5$ $[M+H]^+$, calculée m/z 478,2090, trouvée m/z 478,2085.

**3-(7-{[5-(méthoxyméthyl)-1,2-oxazol-3-yl]méthyl}-4-(morpholin-4-yl)pyrido[3,2-d]pyrimidin-2-yl)phénol (66)** : Le composé **(66)** a été synthétisé à partir du composé **(65)** (50 mg, 0,123 mmol) par déprotection du MOM selon les procédures décrites précédemment. Un solide jaune a été obtenu avec un rendement de 98 %. MP : 169 °C ; Infrarouge (Diamand ATR, $cm^{-1}$) υ : 3200, 2911, 1701, 1640, 1508, 1461, 1426, 1268, 1154, 1116, 1071, 1008, 957, 739 ; RMN $^1H$ (400 MHz, DMSO-$d_6$) δ : 3,52 (s, 3H, $CH_3$), 3,90 (m, 4H, $2xCH_2(O)$), 4,37 (s, 2H, $CH_2$), 4,54 (s, 2H, $CH_2$), 4,69 (bs, 4H, $2xCH_2(N)$), 6,88 (s, 1H, CH), 7,18 (m, 1H, $H_{arom}$), 7,40 (t, 1H, *J* = 7,5 Hz, $H_{arom}$), 7,12 (m, 1H, $H_{arom}$), 7,80 (m, 2H, $H_{arom}$), 8,38 (m, 1H, $H_8$), 8,88 (d, *J* = 2 Hz, 1H, $H_6$), 10.0 (bs, 1H, OH) ; RMN $^{13}C$ (101 MHz, DMSO-$d_6$) δ : 30,0 ($CH_2$), 47,7 ($2xCH_2$), 58,2 ($CH_3$), 66,0 ($CH_2$), 68,4 ($2xCH_2$), 104,5 (CH), 116,5 (CH), 118,3 (CH), 120,1 (CH), 130,6(CH), 135,4 (Cq), 136,5 (CH), 136,2 (Cq), 139,8 (Cq), 146,9 (CH), 147,0 (Cq), 157,7 (Cq), 159,2 (Cq), 163,1 (Cq), 170,13 (Cq); HRMS (EI-MS) : $C_2gH_{24}N_5O_4$ $[M+H]^+$, calculée m/z 434,1828, trouvée m/z 434,1823.

*A.6. Réduction de groupement nitro*

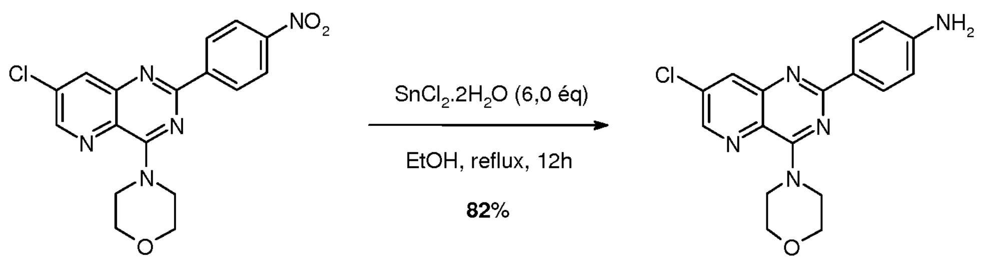


**10** **47**

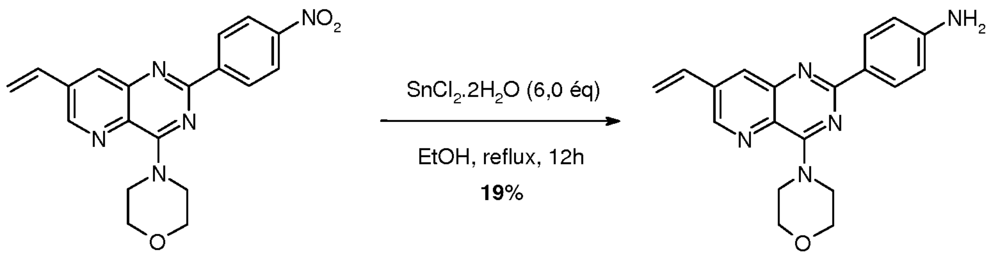


**18** **48**

**4-(7-chloro-4-morpholinopyrido[3,2-d]pyrimidin-2-yl)aniline (47)** : Dans un ballon de 50 mL, 120 mg (0,323 mmol; 1,0 éq.) de **(10)** ont été dissous dans de l'éthanol (20 mL), 437 mg (1,94 mmol; 6 éq.) de dihydrate de chlorure d'étain (II) ont été ajoutés au milieu réactionnel. Le mélange a été porté à reflux pendant 12 heures. L'éthanol a ensuite été évaporé et le résidu a été repris dans une solution aqueuse de NaOH 1 M (100mL). La phase aqueuse a été extraite à l'acétate d'éthyle (2 x 50 mL). La phase organique a été lavée avec de l'eau (2 x 20 mL), séchée sur $MgSO_4$, filtrée puis concentrée sous pression réduite. Le produit attendu a été isolé sans purification supplémentaire sous forme de solide jaune avec un rendement de 82 %. MP : 220 °C ; Infrarouge (Diamand ATR, $cm^{-1}$) υ: 3419, 3323, 3205, 2966, 2865, 1609, 1587, 1518, 1426, 1306, 1109, 1025, 925 ; RMN $^1H$ (250 MHz, $CDCl_3$) δ: 3,80-3,83 (m, 4H, $2xCH_2(O)$),

4,44 (bs, 4H, $2xCH_2(N)$), 5,71 (s, 2H, $NH_2$), 6,64 (d, 2H, *J* = 8,8 Hz, $2xH_{arom}$), 8,16 (d, 2H, *J* = 8,8 Hz, $2xH_{arom}$), 8,19 (d, 1H, *J* = 2,4 Hz, $H_8$), 8,65 (d, 1H, *J* = 2,4 Hz, $H_6$) ; RMN $^{13}C$ (101 MHz, $CDCl_3$) δ: ; HRMS (EI-MS) : $C_{17}H_{16}ClN_5O$ $[M+H]^+$, calculée m/z 342,1116, trouvée m/z 342,1117.

**4-(4-morpholino-7-vinylpyrido[3,2-d]pyrimidin-2-yl)aniline (48)** : Dans un ballon de 25 mL, 60 mg (0,162 mmol; 1,0 éq.) de **(18)** ont été dissous dans de l'éthanol (15 mL), 219 mg (0,97 mmol, 6,0 éq.) de dihydrate de chlorure d'étain (II) a été ajouté au milieu. Le mélange a été porté à reflux pendant 12 heures. Après concentration sous pression réduite, le résidu a été repris dans une solution aqueuse de NaOH 1 M (80 mL) qui est extraite à l'acétate d'éthyle (40 mL). La phase organique résultante a été lavée avec de l'eau (2 x 15 mL), séchée sur $MgSO_4$, filtrée puis concentrée sous pression réduite. Le composé **(48)** a été isolé sous forme de solide jaune avec un rendement de 19 % sans purification supplémentaire. MP : >260 °C ; Infrarouge (Diamand ATR, $cm^{-1}$) υ: 3028, 2972, 2920, 1602, 1553, 1519, 1437, 1345, 1109, 867 ; RMN $^1H$ (400 MHz, $CDCl_3$) δ: 3,90-3,94 (m, 4H, $2xCH_2(O)$), 4,56-4,53 (bs, 4H, $2xCH_2(N)$), 5,54 (d, 1H, *J* = 11,0 Hz, $CH_{2alkene}$), 6,04 (d, 1H, *J* = 17,7 Hz, $CH_{2alkene}$), 6,75 (d, 2H, *J* = 8,7 Hz, $2xH_{arom}$), 6,84 (dd, 1H, *J* = 11,0 Hz, *J* = 17,7 Hz, $CH_{alkene}$), 8,06 (d, 1H, *J* = 2,2 Hz, $H_8$), 8,32 (d, 2H, *J* = 8,7 Hz, $2xH_{arom}$), 8,67 (d, 1H, *J* = 2,2 Hz, $H_6$) ; RMN $^{13}C$ (101 MHz, $CDCl_3$) δ: 48,2 ($2xCH_2$), 67,4 ($2xCH_2$), 114,7 (2xCH), 118,6 ($CH_2$), 128,7 (Cq), 130,2 (2xCH), 132,2 (Cq), 132,3 (CH), 133,3 (CH), 135,9 (Cq), 144,1 (CH), 148,5 (Cq), 148,9 (Cq), 159,3 (Cq), 160,6 (Cq) ; HRMS (EI-MS) : $C_{19}H_{19}N_5O$ $[M+H]^+$, calculée m/z 335,1662, trouvée m/z 335,1666.

*A.8. Formation d'urée*

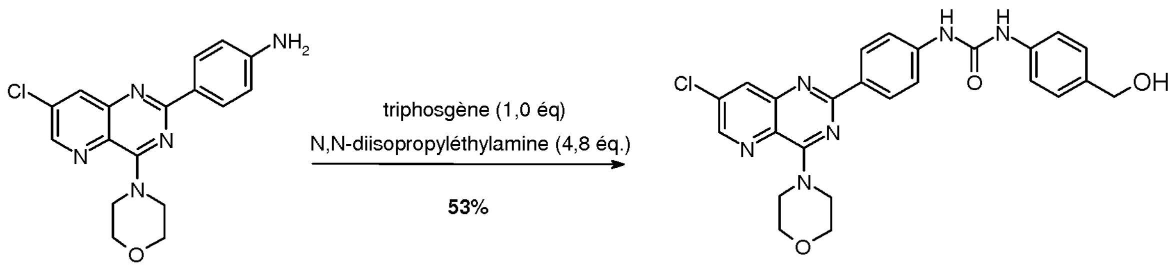


47 52

**1-(4-(7-chloro-4-morpholinopyrido[3,2-d]pyrimidin-2-yl)phényl)-3-(4-méthoxyphenyl)urée (52)** : Sous une atmosphère d'argon, 26 mg (0,09 mmol; 1 éq.) de trisphosgène ont été dissous dans 10 mL de tétrahydrofurane anhydre à -78 °C. Une solution contenant 90 mg (0,26 mmol; 3 éq.) de **(47)** ainsi que 71 µL (0,42 mmol, 4,8 éq.) de *N,N*-di*i*sopropyléthylamine, dissous dans 5 mL de tétrahydrofurane, a été additionnée goutte-à-goutte à la solution refroidie de triphosgène. Le mélange a été agité à -78 °C pendant 5 minutes puis à température ambiante. 26 mg (0,26 mmol, 3 éq.) de 4-hydroxyméthylphénylamine et 35 µL (0,21 mmol, 2,4 éq.) de *N,N*-di*i*sopropyléthylamine, dissous dans 5 mL de tétrahydrofurane ont alors été ajoutés au goutte-à-goutte. Le milieu réactionnel a ensuite été laissé sous agitation pendant 24 heures. Ce dernier a été hydrolysé avec une solution aqueuse saturée de $NaHCO_3$ (10 mL). La phase aqueuse a été extraite avec de l'acétate d'éthyle (50 mL). Les phases organiques regroupées ont été lavées à l'eau (1 x 10 mL), séchées sur $MgSO_4$, filtrées puis concentrées sous pression réduite. Le produit **(52)** a été obtenu sans purification supplémentaire avec un rendement de 53 % sous forme d'un solide jaune. MP : > 260 °C ; Infrarouge (Diamand ATR, $cm^{-1}$) υ: 3291, 2912, 2857, 1640, 1588, 1509, 1426, 1309, 1208, 1111 ; RMN $^1H$ (400 MHz, $CDCl_3$) δ: 3,80 (s, 4H, $2xCH_2(O)$), 4,42 (s, 2H, $CH_2$), 4,46 (bs, 4H, $2xCH_2(N)$), 7,21 (d, 2H, *J* = 8,3 Hz, $2xH_{arom}$), 7,43 (d, 2H, *J* = 8,3 Hz, $2xH_{arom}$), 7,59 (d, 2H, *J* = 8,6 Hz, $2xH_{arom}$), 8,25 (d, 1H, *J* = 2,3 Hz, $H_8$), 8,34 (d, 2H, *J* = 8,6 Hz, $2xH_{arom}$), 8,69 (d, 1H, *J* = 2,3 Hz, $H_6$), 9,24 (s, 1H, NH), 9,24 (s, 1H, NH) ; RMN $^{13}C$ (101 MHz, $CDCl_3$) δ: 48,1 ($2xCH_2$), 63,1 ($CH_2$), 66,8 ($2xCH_2$), 117,7 (2xCH), 118,3 (2xCH), 127,6 (2xCH), 129,6 (2xCH), 130,9 (Cq), 131,0 (Cq), 134,0 (CH), 136,4 (Cq), 138,7 (Cq), 143,1 (Cq), 144,9 (CH), 148,9 (Cq), 152,9 (Cq), 152,9 (Cq), 158,8 (Cq), 160,2 (Cq) ; HRMS (EI-MS) : $C_{25}H_{23}ClN_6O_3$ $[M+H]^+$, calculée m/z 491,1593, trouvée m/z 491,1594.

*A.8. Couplage de Suzuki en position 4 du composé (3)*

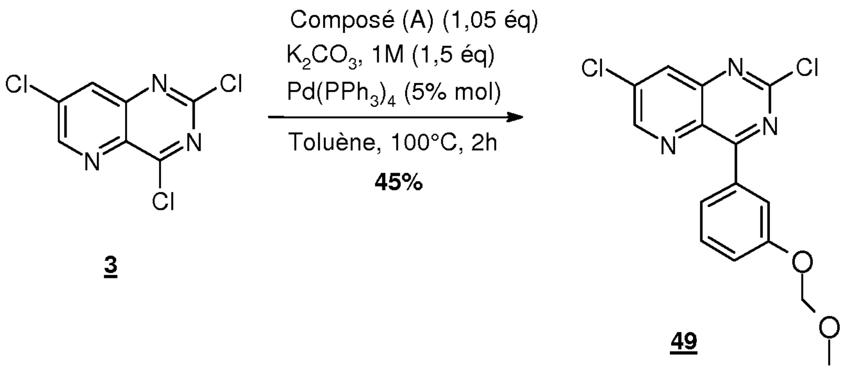


3

49

**2,7-dichloro-4-(3-(méthoxyméthoxy)phényl)pyrido[3,2-d]pyrimidine (49)** : Sous une atmosphère inerte, dans un ballon de 25 mL, 200 mg (0,85 mmol, 1,0 éq.) de **(3)** ont été dissous dans du toluène (10 mL). 145 mg (0,90 mmol, 1,05 éq.) de 3-méthoxyméthoxyphénylboronic acide, une solution aqueuse (1 mL) contenant 176 mg (1,28 mmol, 1,5 éq.) de carbonate de potassium, puis 49 mg (0,05 mmol, 0,05 éq.) de tetrakis triphénylphosphine ont été additionnés au le milieu. Le mélange a alors été porté à 100 °C pendant 2 heures. Après concentration sous pression réduite, le résiduel a été repris dans du dichlorométhane (30 mL). La phase organique a étélavée avec de l'eau (2 x 10 mL), séchée sur $MgSO_4$, filtrée puis concentrée sous pression réduite. Le résidu brut a alors été directement purifié par chromatographie sur gel de silice avec (DCM/MeOH, 99/1) pour donner le produit sous forme de solide jaune avec un rendement de 43 %. MP : 214 °C. Infrarouge (Diamand ATR, $cm^{-1}$) $\upsilon$ : 2972, 1592, 1556, 1492, 1460, 1381, 1335, 1242, 1142, 746 ; RMN $^1H$ (400 MHz, $CDCl_3$) $\delta$: 3,82 (s, 3H, $CH_3$), 5,30 (s, 2H, $CH_2$), 6,96 (s, 1H, $H_{arom}$), 7,35 (d, 1H, $J$ = 5,5 Hz, $H_{arom}$), 7,94 (s, 2H, $2xH_{arom}$), 8,48 (d, 1H, $J$ = 3,2 Hz, $H_8$), 8,92 (d, 1H, $J$ = 3,4 Hz, $H_6$) ; RMN $^{13}C$ (101 MHz, $CDCl_3$) $\delta$: 56,3 ($CH_3$), 94,5 ($CH_2$), 115,0 (CH), 118,5 (CH), 119,3 (CH), 129,7 (CH), 134,0 (CH), 138,0 (Cq), 142,1 (Cq), 149,1 (CH), 157,6 (Cq), 160,5 (Cq), 162,9 (Cq), 163,8 (Cq), 165,6 (Cq) ; HRMS (EI-MS) : $C_{15}H_{11}Cl_2N_3O_2$ $[M+H]^+$, calculée m/z 337,0228 ; trouvée m/z 337,0316.

*A.9. Fonctionnalisation en position C2 du composé (49)*

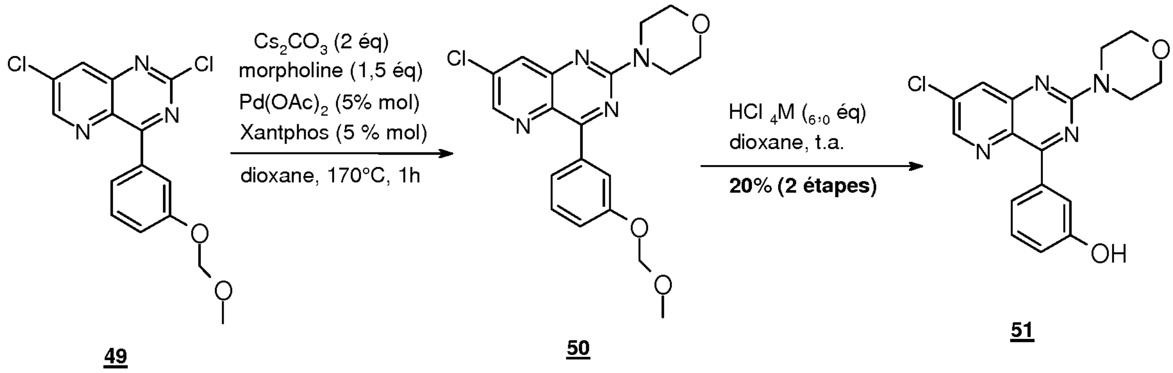


49

50

51

**3-(7-chloro-2-morpholinopyrido[3,2-d]pyrimidin-4-yl)phénol (51) :** Dans un vial de 5 mL, 150 mg (0,45 mmol, 1,0 éq.) de **(49)** ont été dissous dans du dioxane (10 mL), 60 $\mu$L (0,67 mmol, 1,5 éq.) de morpholine, 291 mg (0,9 mmol, 2,0 éq.) de carbonate de césium, puis 5 mg (0,05 mmol, 0,1 éq.) de palladium acétate et 13 mg (0,2 mmol, 0,05 éq.) de xantphos ont été additionnés dans le milieu. Le mélange a alors été porté à 170 °C pendant 1 heure sous irradiation micro-ondes. Après concentration sous pression réduite, le résidu a été repris dans du dichlorométhane (30 mL). La phase organique a été lavée avec une solution saturée en chlorure de sodium (2 x 10mL), séchée sur $MgSO_4$, filtrée puis concentrée sous pression réduite. Le résidu a subit une première étape de purification par colonne de chromatographie sur gel de silice sous pression (DCM/MeOH, 99/1).

Le produit intermédiaire obtenu **(50)** présentant des impuretés, il subit une deuxième réaction de déprotection pour séparer le produit désiré des impuretés par filtration. **(50)** a été directement dilué dans du dioxane et 6 éq. d'une solution d'acide chlorhydrique gazeux a été ajouté au milieu (4 M dans du dioxane). Le mélange a été laissé sous agitation à température ambiante pendant une à trois heures. Le précipité a été lavé à l'éther de pétrole puis récupéré par filtration pour donner un produit final sous forme d'un solide jaune, avec un rendement de 20 %. MP : 231 °C ;

Infrarouge (Diamand ATR, $cm^{-1}$) υ : 3183, 1595, 1563, 1438, 1338, 1231, 1114, 996, 728 ; RMN $^1H$ (400 MHz, $CDCl_3$) δ: 3,44-3,36 (m, 4H, $2xCH_2(O)$), 3,98-3,84 (m, 4H, $2xCH_2(N)$), 7,18 (ddd, 1H, *J* = 1,0 Hz, *J* = 2,5 Hz, *J* = 8,1 Hz, $H_{arom}$) 7,48-7,35 (m, 2H, $2xH_{arom}$), 8,20 (m, 2H, $H_{arom}$ and $H_8$), 8,70 (d, 1H, *J* = 2,8 Hz, $H_6$) ; RMN $^{13}C$ (101 MHz, $CDCl_3$) δ: 44,1 ($2xCH_2$), 65,3 ($2xCH_2$), 116,2 (CH), 118,1 (CH), 118,7 (CH), 129,6 (CH), 133,2 (CH), 138,1 (Cq), 141,8 (Cq), 149,7 (CH), 158,2 (Cq), 160,1 (Cq), 162,3 (Cq), 163,1 (Cq), 165,6 (Cq) ; HRMS (EI-MS) : $C_{17}H_{15}ClN_4O_2[M+H]^+$, calculée m/z 343,0884 ; trouvée m/z 343,0921.

**B. Préparation des composés urées de l'invention**

B.1. Préparation des intermédiaires substitués en position 7

*B.1.1. Préparation des intermédiaires de synthèse*

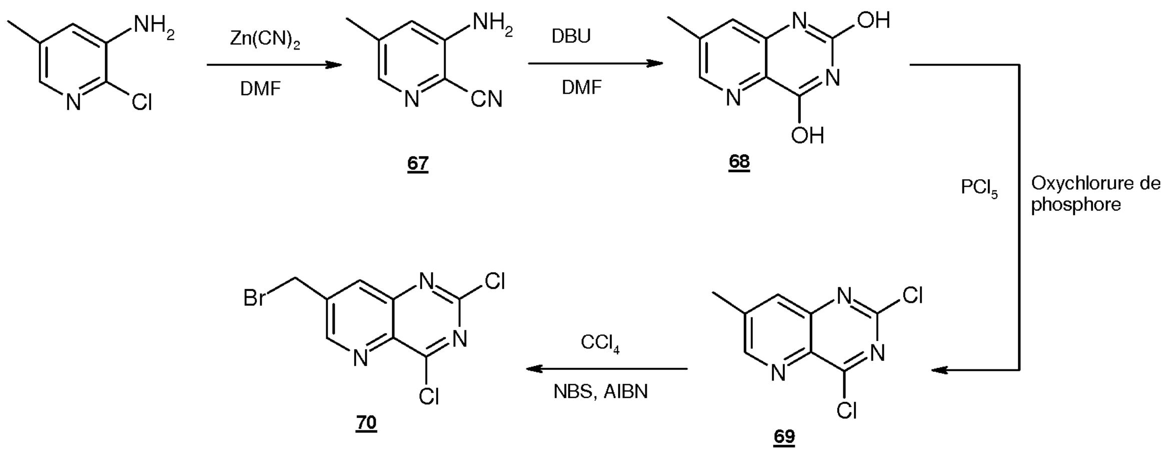


**67** **68** **69** **70**

**3-amino-5-méthylpyridine-2-carbonitrile (67)** : Sous une atmosphère d'argon, dans un vial de 20 mL, 1,0 g (7 mmol, 1éq) de 2-Chloro-5-méthylpyridin-3-amine a été dissous dans 15 mL de DMF anhydre. 821 mg (7 mmol, 1eq) du $Zn(CN)_2$ ont été ajoutés. Ensuite, la solution a été dégazée pendant 10 minute et 405 mg (0.35 mmol, 0,05 éq) de tetrakis(triphénylphosphino) palladium(0) ont été ajoutés. Le mélange a été chauffé à 105 °C pendant 20 heures. Le mélange réactionnel a été filtré sur célite et évaporé sous vide. Le résidu brut a alors été purifié par colonne de chromatographie sur gel de silice sous pression (AE/ EP 2/8) permettant d'isoler un solide blanc avec un rendement de 65%. MP : 154 °C Infrarouge (Diamand ATR, $cm^{-1}$) υ: 3404, 2216, 1600, 1465, 1339, 1230, 858, 739; RMN $^1H$ (400 MHz, $CDCl_3$) δ : 2,34 (m, 3H, $CH_3$), 4,37 (bs, 2H, $NH_2$), 6,93 (m, 1H, $H_{arom}$), 7,93 (m, 1H, $H_{arom}$); RMN $^{13}C$ (101 MHz, $CDCl_3$) δ: 28,7 ($CH_3$), 114,9 (CH), 116,3 (CN), 122,6 (CH), 138,6 (Cq), 142,2 (CH), 146,3 (Cq).

**7-méthylpyrido[3,2-d]pyrimidine-2,4-diol (68)** : Sous une atmosphère de dioxyde de carbone, dans un vial de 20 mL, 400 mg (3,0 mmol, 1 éq) de 3-amino-5-méthylpyridine-2-carbonitrile ont été dissous dans 8 mL de DMF anhydre. 448 μL (3,0 mmol, 1eq) du 1,8-diazabicyclo[5.4.0]undéc-7-ène (DBU) ont été ajoutés. Ensuite, la solution a été dégazée pendant 15 minute et le vial a été scellé. Le mélange a été chauffé à 105 °C pendant 6 heures (précipitation du produit). A 0°C, 2 mL HCl à 1 M ont été ajoutés. Le précipité a été filtré sous vide permettant d'isoler un solide beige avec un rendement de 90%. MP >260°C ; Infrarouge (Diamand ATR, $cm^{-1}$) υ 3052, 1673, 1410, 1127, 846, 820, 686 ; RMN $^1H$ (400 MHz, DMSO-$d_6$) δ : 2,34 (s, 3H, $CH_3$), 7,34 (s, 1H, $H_{arom}$), 7,93 (s, 1H, $H_{arom}$) 11,07 (bs, 2H, 2xOH); RMN $^{13}C$ (101 MHz, DMSO-$d_6$) δ: 18,6 ($CH_3$), 123,46 (CH), 129,74 (Cq), 138,5 (Cq), 139,7 (Cq), 146,2 (CH), 150,5 (Cq), 161,7 (Cq). HRMS (EI-MS) : $C_8H_7N_3O_2$ $[M+H]^+$, calculée m/z 178,0617, trouvée m/z 178,0611.

**2,4-dichloro-7-méthylpyrido[3,2-d]pyrimidine (69)** : Dans un ballon de 50 mL, 1 g (6,65 mmol; 1,0 éq.) de 7-méthylpyrido[3,2-d]pyrimidine-2,4-diol (68) a été mis en suspension dans 10 mL d'oxychlorure de phosphore et 4,7 g (22,60 mmol; 4,0 éq.) de $PCl_5$. L'ensemble a été chauffé à 130 °C. Après 12 heures de réaction, l'excès de $POCl_3$ a été évaporé sous pression réduite. Le résidu obtenu a été amené à 0 °C au moyen d'un bain de glace puis solubilisé dans le dichlorométhane (150 mL), le mélange a été versé dans un mélange eau/glace (200 mL) sans aucune basification. Après retour à température ambiante, la phase aqueuse a été extraite au dichlorométhane (1 x 100 mL). Les phases organiques ont été séchées sur $MgSO_4$, filtrées, puis concentrées sous pression réduite. Le résidu ainsi obtenu a été purifié par colonne de chromatographie sur gel de silice sous pression (AE/EP, 2/8) pour donner un solide blanc avec un rendement de 70 %. MP : 146 °C ; Infrarouge (Diamand ATR, $cm^{-1}$) υ 1539, 1439, 1398, 1255, 1137, 1004, 869,

698, 690; RMN $^1H$ (400 MHz, $CDCl_3$) δ :2,68 (m, 3H, $CH_3$), 8,08 (m, 1H, $H_8$), 8,99 (d, 1H, *J* = 2,0 Hz, $H_6$) ; RMN $^{13}C$ (101 MHz, $CDCl_3$) δ: 20,7 ($CH_3$),135,8 (CH), 136,4 (Cq), 143,2 (Cq), 150,4 (Cq), 156,6 (CH), 157,0 (Cq), 166,7 (Cq) ; HRMS (EI-MS) : $C_7H_2Cl_3N_3$ $[M+H]^+$, calculée m/z 213,9939, trouvée m/z 213,9933.

**7-(bromométhyl)-2,4-dichloropyrido[3,2-d]pyrimidine (70)** : Sous une atmosphère d'argon, dans un ballon de 20 mL, 206 mg (0,99 mmol, 1éq) de 2,4-dichloro-7-methylpyrido[3,2-d]pyrimidine (69) a été mis en suspension dans 15 mL de tétrachlométhane ($CCl_4$) anhydre. 193 mg (1,09 mmol, 1,1eq) du n-bromosuccimide (NBS) et 20 mg (0,12 mmol, 0,12 eq) d'azobisisobutyronitrile (AIBN) ont ensuite été ajoutés. La solution a été dégazée pendant 15 minute et le mélange a été chauffé à reflux pendant 12 heures. Le mélange réactionnel a été filtré sur coton et évaporé sous vide. Le résidu brut a alors été purifié par colonne de chromatographie sur gel de silice sous pression (AE/ EP 2/8) permettant d'isoler un solide blanc avec un rendement de 27%. Infrarouge ($cm^{-1}$) u : 1538, 1440, 1380, 1331, 1266, 1209, 927, 868, 698 ; RMN $^1H$ (400 MHz, $CDCl_3$) δ :4,60 (s, 2H, $CH_2$), 8,26 (m, 1H, $H_8$), 9,12 (d, 1H, *J* = 2,0 Hz, $H_6$) ; RMN $^{13}C$ (101 MHz, $CDCl_3$) δ: 27,4 ($CH_3$),135,8 (CH), 136,4 (Cq), 143,2 (Cq), 154,4 (Cq), 156,6 (CH), 157,0 (Cq), 166,7 (Cq) ; HRMS (EI-MS) : $C_8H_4BrCl_2N_3$ $[M+H]^+$, calculée m/z 213,9939, trouvée m/z 213,9933.

*B. 1.2. Couplage de suzuki en position 4*

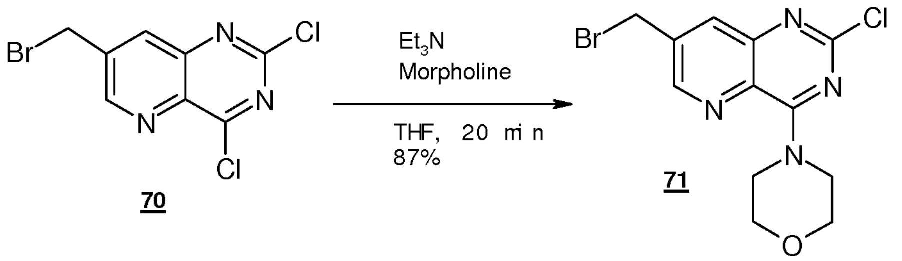


**4-[7-(bromométhyl)-2-chloropyrido[3,2-d]pyrimidin-4-yl]morpholine (71) :** Sous atmosphère d'argon, dans un ballon de 10 mL, 49 mg (0,16 mmol, 1éq) de 7-(bromomethyl)-2,4-dichloropyrido[3,2-d]pyrimidine (70) a été dissous dans 5 mL de tétrahydrofurane anhydre. A 0°C, 14,7μL (0,16 mmol, 1éq) de morpholine dilué dans 1 mL de THF anhydre et 23,3 μL (0,16 mmol, 1eq) de triéthylamine ont ensuite été ajoutés. Le mélange a été agité pendant 20 min à 0°C. Ensuite, une solution aqueuse saturée en $NaHCO_3$ (10 mL) a été introduite. La phase organique a été extraite 3 fois avec de l'acétate d'éthyle (10mL), séchée sur $MgSO_4$, filtrée puis concentrée sous pression réduite. Le résidu brut a alors été purifié par colonne de chromatographie sur gel de silice sous pression (AE/ EP 2/9) permettant d'isoler une solide blanc avec un rendement de 87%. MP : 192°C ; MP Infrarouge ($cm^{-1}$) u : 3033, 2978, 2861, 1614, 1557, 1430, 1324, 1292, 1136, 1001, 872 ; RMN $^1H$ (400 MHz, $CDCl_3$) δ :3,87 (t, 4H, *J*= 2,0 Hz, $2xCH_2(O)$), 4,56 (bs, 6H, $CH_2$ et $2xCH_2(N)$), 7,97 (d, 1H, *J* = 2,0 Hz, $H_8$), 8,69(d, 1H, *J* = 2,0 Hz, $H_6$) ; RMN $^{13}C$ (101 MHz, $CDCl_3$) δ: 28,2 ($CH_2$), 48,5 ($2xCH_2$), 67,2 ($2xCH_2$), 132,2 (Cq), 134,8 (CH), 137,7 (Cq), 147,8 (CH), 148,8 (Cq), 157,6 (Cq), 159,3 (Cq) ; HRMS (EI-MS) : $C_{12}H_{12}BrClN_4O$ $[M+H]^+$, calculée m/z 342,9961, trouvée m/z 342,9956.

*B. 1.3. Fonctionnalisation en position 7*

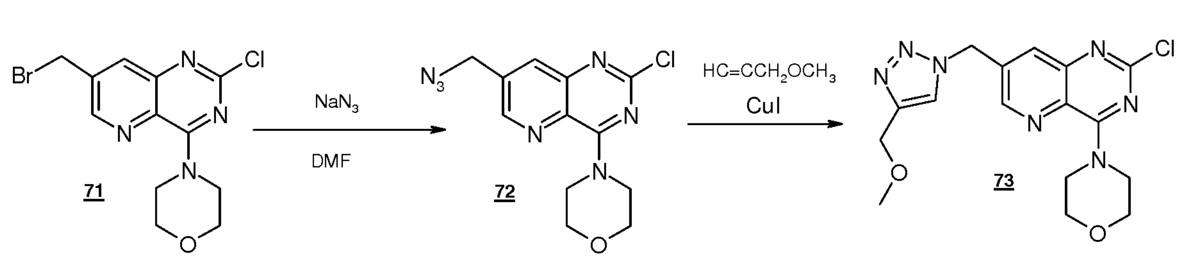


**4-[7-(azidométhyl)-2-chloropyrido[3,2-d]pyrimidin-4-yl]morpholine (72) :** Dans un ballon de 20 mL, 141 mg (0,409 mmol; 1 éq.) de **(71)** ont été dilués dans 10 mL de diméthylformamide séché sur tamis 4À, ainsi que 40 mg (0,615 mmol; 1,5 éq.) d'azoture de sodium. Le mélange a été agité pendant 15h à température ambiante. 20 mL d'eau et 20 mL de dichlorométhane (DCM) ont été additionnés. La phase organique a été extraite 3 fois avec DCM (10mL),

lavée avec une solution saline (2x 20 mL), séchée sur $MgSO_4$, filtrée puis concentrée sous pression réduite. Un solide beige a été obtenu avec un rendement de 97%. MP : 118°C ; MP Infrarouge ($cm^{-1}$) u : 3033, 2978, 2861, 1614, 1557, 1430, 1324, 1292, 1136, 1001, 872 ; RMN $^1H$ (400 MHz, $CDCl_3$) δ :3,87 (t, 4H, $J$ = 2,0 Hz, $2xCH_2(O)$), 4,56 (bs, 6H, $CH_2$ et $2xCH_2(N)$), 7,97 (d, 1H, $J$ = 2,0 Hz, $H_8$), 8,69(d, 1H, $J$ = 2,0 Hz, $H_6$) ; RMN $^{13}C$ (101 MHz, $CDCl_3$) δ: 48,5 ($2xCH_2$), 51,9 ($CH_2$), 67,2 ($2xCH_2$), 132,2 (Cq), 134,8 (CH), 137,7 (Cq), 147,8 (CH), 148,8 (Cq), 157,6 (Cq), 159,3 (Cq) ; HRMS (EI-MS) : $C_{12}H_{13}ClN_7O[M+H]^+$, calculée m/z 306,0870, trouvée m/z 306,0865.

**4-(2-chloro-7-{[4-(méthoxymethyl)-1H-1,2,3-triazol-1-yl]méthyl}pyrido[3,2-d]pyrimidin-4-yl)morpholine (73) :** Dans un ballon de 10 mL, 21 mg (0,395 mmol; 1,0 éq.) de **(72)** ont été mis en suspension dans 3 mL d'acétonitrile. 4 mg (0,02 mmol; 0,05 éq.) de iodure de cuivre et 37 μL (0,434 mmol, 1,1 éq.) de méthoxypropargylique éther ont été ajoutés. De la triéthylamine a été ajoutée goutte-à-goutte jusqu'à une parfaite dissolution du composé dans la solution. Le mélange a été agité à température ambiante pendant 12 heures. La solution a été diluée dans de l'acétate d'éthyle (30 mL). La phase organique a été lavée avec une aqueuse saturée en $NaHCO_3$ (10mL), séchée sur $MgSO_4$, filtrée, puis concentrée sous pression réduite. Le composé **(73)** a été isolé après purification par colonne de chromatographie sur gel de silice sous pression (AE, 100%) avec un rendement de 67%, sous la forme d'un solide blanc. MP : 166 °C ; Infrarouge (Diamand ATR, $cm^{-1}$) υ : 2913, 2856, 1558, 1516, 1430, 1315, 1275, 1107, 1062, 1029, 968, 792, 739, 674, RMN $^1H$ (400 MHz, $CDCl_3$) δ: 3,41 (s, 3H, $CH_3$), 3,85 (m, 4H, $2xCH_2(O)$), 4,58 (m, 6H, $CH_2$ and $2xCH_2(N)$), 5,70 (s, 2H, $CH_2$), 7,56 (s, 1H, $H_{triazol}$), 7,82 (d, 1H, $J$ = 2,0 Hz, $H_8$), 8,57 (d, $J$ = 2,0 Hz, 1H, $H_6$),; RMN $^{13}C$ (101 MHz, $CDCl_3$) δ : 47,6 ($2xCH_2$), 50,7 ($CH_2$), 58,26 ($CH_3$), 66,0 ($CH_2$), 67,3 ($2xCH_2$), 122,7 (CH), 132,3 (Cq) 133,9 (CH), 134,5 (Cq), 145,9 (CH), 148,0 (Cq), 158,0 (Cq), 159,6 (Cq) ; HRMS (EI-MS) : $C_{16}H_{19}ClN_7O_2[M+H]^+$, calculée m/z 376,1289 trouvée m/z 376,1283.

*B. 1.4. Fonctionnalisation en positions 4 et 7*

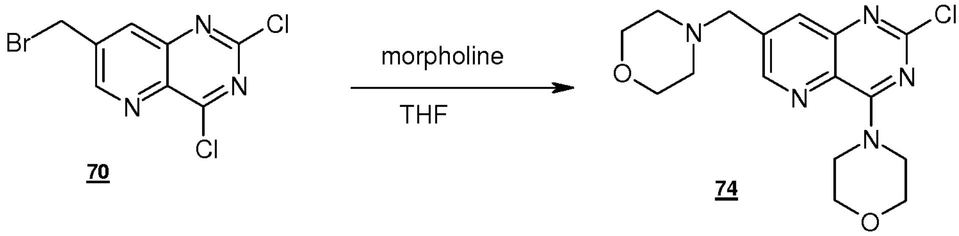


70

74

**4-[2-chloro-7-(morpholin-4-ylméthyl)pyrido[3,2-d]pyrimidin-4-yl]morpholine (74) :**

Sous atmosphère d'argon, dans un ballon de 10 mL, 75 mg (0,253 mmol, 1 éq) de 7-(bromomethyl)-2,4-dichloropyrido[3,2-d]pyrimidine (73) a été dissous dans 2 mL de tétrahydrofurane anhydre. A 0°C, 29,4 μL (0,506 mmol, 2 éq) de morpholine dilué dans 1 mL de THF anhydre et 46,6 μL (0,506 mmol, 2 eq) de triéthylamine ont ensuite été ajoutés. Le mélange a été agité pendant 1 heure à température ambiante. Ensuite, une solution aqueuse saturée en $NaHCO_3$ (10 mL) a été ajoutée. La phase organique a été extraite 3 fois avec de l'acétate d'éthyle (10mL), séchée sur $MgSO_4$, filtrée puis concentrée sous pression réduite. Le résidu brut a alors été purifié par colonne de chromatographie sur gel de silice sous pression (AE/ EP 1/1) permettant d'isoler une solide blanc avec un rendement de 83%. MP : 162°C ; MP Infrarouge ($cm^{-1}$) u : 3148, 3048, 2840, 1555, 1531, 1430, 1324, 1253, 1136, 950, 872, 640; RMN $^1H$ (400 MHz, $CDCl_3$) δ :2,47 (t, 4H, $J$ = 2,0 Hz, $2xCH_2(O)$), 3,64 (s, 2H, $CH_2$), 3,70 (t, 4H, $J$ = 2,0 $2xCH_2(N)$), 3,85 (t, 4H, $J$ = 2,0 Hz, $2xCH_2(O)$), 4,57 (bs, 4H, $2xCH_2(N)$), 7,92 (m, 1H, $H_8$), 8,66 (d, 1H, $J$ = 2,0 Hz, $H_6$) ; RMN $^{13}C$ (101 MHz, $CDCl_3$) δ: 49,6 ($2xCH_2$), 54,9 ($2xCH_2$), 61,6 ($CH_2$), 68,3 ($2xCH_2$), 68,6 ($2xCH_2$), 133,2 (Cq), 136,1 (CH), 139,7 (Cq), 149,1 (CH), 150,2 (Cq), 158,6 (Cq), 160,7 (Cq) ; HRMS (EI-MS) : $C_{16}H_{21}ClN_5O_2$ $[M+H]^+$, calculée m/z 350,1384, trouvée m/z 350,1378.

B.2. Préparation des intermédiaires pinalcolesters

Procédure générale :

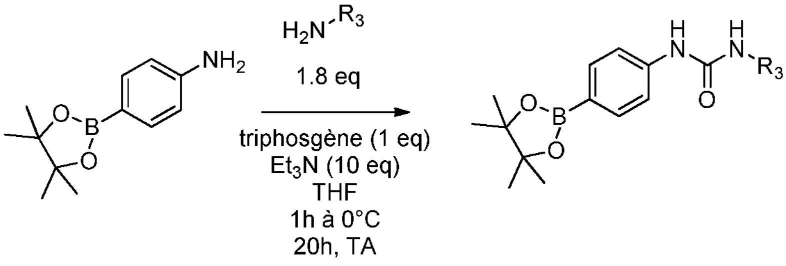


**75-77**

Sous une atmosphère inerte, dans un ballon de 10 mL, 1,2 éq de 4-(4,4,5,5-Tetramethyl-1,3,2-dioxaborolan-2-y)aniline a été solubilisé dans 5 mL de THF. A 0°C, 1 éq de triphosgène et 10 éq de triéthylamine ont été ajoutés. Au bout d'une heure à 0°C, 1,87 éq d'amine a été ajoutée. Le mélange a été agité à température ambiante pendant 20 heures. La solution a été diluée dans de l'acétate d'éthyle (30 mL) et dans de l'eau (20 mL). La phase organique a été extraite avec de l'acétate d'éthyle (3x10 mL), séchée sur $MgSO_4$, filtrée puis concentrée sous pression réduite. Les composés ont été isolés après purification par colonne de chromatographie sur gel de silice sous pression.

**1-[4-(hydroxyméthyl)phényl]-3-[4-(tetraméthyl-1,3,2-dioxaborolan-2-yl)phényl]urée (75) :** Le composé **(75)** a été synthétisé à partir 4-(aminophényl)méthanol (87 mg, 0,71 mmol) en suivant la procédure générale pour donner un solide jaunâtre avec un rendement de 60 %. MP : 184 °C ; Infrarouge (Diamand ATR, $cm^{-1}$) υ : 3261, 3114, 2980, 1597, 1531, 1483, 1438, 1230, 1107, 968, 858, 739 ; RMN $^1H$ (400 MHz, DMSO-$d_6$) δ: 1,28 (s, 12H, $4xCH_3$), 4,42(d, 2H, $J$ = 5,6 Hz, $CH_2$), 5,05 (t, 1H, $J$ = 5,6 Hz, OH), 7,23 (m,2H, $H_{arom}$), 7,43 (m, 4H, $H_{arom}$), 7,60 (m,2H, $H_{arom}$), 8,65 (s, 1H, NH), 8,75 (s, 1H, NH) ; RMN $^{13}C$ (101 MHz, DMSO-$d_6$) δ: 25,2 ($4xCH_3$), 63,1 ($CH_2$), 83,8 (Cq), 117,47 ($2xCH_{arom}$), 118,5 ($2xCH_{arom}$), 127,6 ($2xCH_{arom}$), 135,6 ($2xCH_{arom}$), 136,6 (Cq), 138,6 (Cq), 143,2 (Cq), 152,8 (Cq); RMN "B (128 MHz, DMSO-$d_6$): δ: 20,0 (s, B); HRMS (EI-MS) : $C_{20}H_{26}BN_2O_4$[M+H]$^+$, calculée m/z 369,1986, trouvée m/z 369,1984.

**1-[3-(hydroxyméthyl)phényl]-3-[4-(tétraméthyl-1,3,2-dioxaborolan-2-yl)phényl]urée (76):** Le composé **(76)** a été synthétisé à partir du 3-(aminophényl)méthanol (87 mg, 0,71 mmol) en suivant la procédure générale pour donner un solide blanc avec un rendement de 43 %. MP : 190°C ; Infrarouge (Diamand ATR, $cm^{-1}$) υ : 3261, 3114, 2980, 1597, 1531, 1483, 1438, 1230, 1107, 968, 858, 739 ; RMN $^1H$ (400 MHz, DMSO-$d_6$) δ: 1,28 (s, 12H, $4xCH_3$), 4,42 (d, 2H, J= 5,6 Hz, $CH_2$), 5,20 (t, 1H, $J$ = 5,6 Hz, OH), 6,90 (m, 1H, $H_{arom}$), 7,22 (m,1H, $H_{arom}$), 7,43 (m, 4H, $H_{arom}$), 7,60 (m,2H, $H_{arom}$), 8,65 (s, 1H, NH), 8,75 (s, 1H, NH) ; RMN $^{13}C$ (101 MHz, DMSO-$d_6$) δ: 25,2 ($4xCH_3$), 63,1 ($CH_2$), 83,4 (Cq), 116,7 (CH), 117,0 (CH), 117,47 ($2xCH_{arom}$), 120,0 ($CH_{arom}$), 128,6 ($CH_{arom}$), 135,9 ($2xCH_{arom}$), 139,8 (Cq), 143,2 (Cq), 143,7 (Cq), 152,8 (Cq) ; RMN $^{11}B$ (128 MHz, DMSO-$d_6$): δ: 20,0 (s, B) ; HRMS (EI-MS) : $C_{20}H_{26}BN_2O_4$[M+H]$^+$, calculée m/z 369,1986, trouvée m/z 369,1984.

**1-[4-(tétraméthyl-1,3,2-dioxaborolan-2-yl)phényl]-3-(2,2,2-trifluoroéthyl)urée (77) :** Le composé **(77)** a été synthétisé à partir 2,2,2-trifluoroéthanamine (266 μL, 1,53 mmol) en suivant la **procédure générale** pour donner un solide blanc avec un rendement de 60 %. MP : 134 °C ; Infrarouge (Diamand ATR, $cm^{-1}$) υ : 3337, 1646, 1596, 1560, 1399, 1360, 1515, 1240, 1597, 1230, 1107, 968, 858, 739 ; RMN $^1H$ (400 MHz, DMSO-$d_6$) δ: 1,28 (s, 12H, $4xCH_3$), 4,42(d, 2H, $J$ = 5,6 Hz, $CH_2$), 5,05 (t, 1H, $J$ = 5,6 Hz, OH), 7,23 (m,2H, $H_{arom}$), 7,43 (m, 4H, $H_{arom}$), 7,60 (m,2H, $H_{arom}$), 8,65 (s, 1H, NH), 8,75 (s, 1H, NH) ; RMN $^{13}C$ (101 MHz, DMSO-$d_6$) δ: 25,2 ($4xCH_3$), 41,8 (m, $CH_2$), 83,7 (Cq), 117,47 ($2xCH_{arom}$), 125,2 (d, $J$ = 277 Hz, Cq), 135,6 ($2xCH_{arom}$), 143,2 (2xCq), 154,9 (Cq), 157,4 (Cq) ; RMN $^{11}B$ (128 MHz, DMSO-$d_6$) : δ: 20,0 (s, B), ; RMN $^{31}F$ (376 MHz, DMSO-$d_6$): δ: -69,5 (t, 3F, $CF_3$), HRMS (EI-MS) : $C_{30}H_{34}N_7O_4$[M+H]$^+$, calculée m/z 345,1597, trouvée m/z 345,1596.

| | |
|---|---|
| 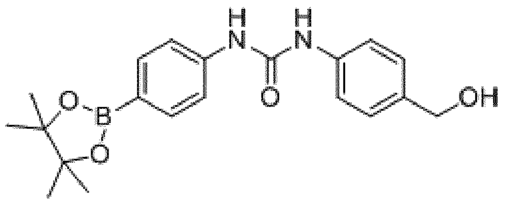 **75** | 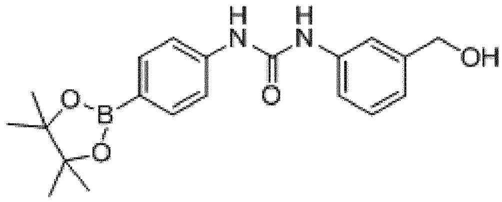 **76** |
| 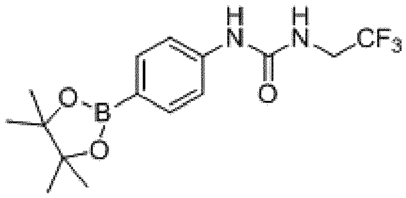 **<u>77</u>** | |

B.3. Insertion de la fonction urée en position 2

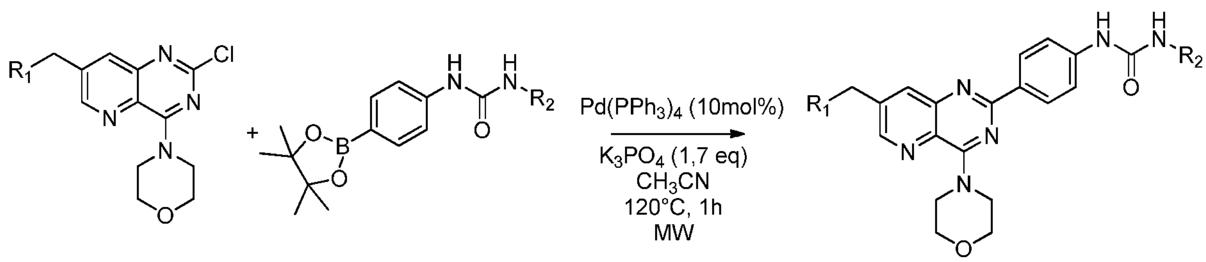


**73-74** **75-77** **78-82**

**Procédure générale :** Sous une atmosphère d'argon, dans un vial de 5 mL, 1,0 éq. de 4-[2-chloro-7-(morpholin-4-ylmethyl)pyrido[3,2-d]pyrimidin-4-yl]morpholine (74) ou 4-(2-chloro-7-{[4-(methoxymethyl)-1H-1,2,3-triazol-1-yl]methyl}pyrido[3,2-d]pyrimidin-4 yl)morpholine (73) a été mis en suspension dans l'acétonitrile ($CH_3CN$), 1,7 éq. phosphate de potassium (C = 1.27 M), 1,2 éq. de pinacolester ont été ajoutés dans le milieu ainsi que 0,10 éq. de tetrakis(triphénylphosphino) palladium(0). Le mélange a été irradié sous micro-ondes à 120 °C pendant 1h. Ensuite, le milieu réactionnel a été agité à température ambiante pendant 1h (précipitation du produit) et filtré sous vide. Le précipité a été lavé avec du DCM, de l'acétate d'éthyle et du MeOH.

**1-[4-(hydroxymethyl)phenyl]-3-{4-[4-(morpholin-4-yl)-7-(morpholin-4 ylmethyl)pyrido[3,2-d]pyrimidin-2-yl]phenyl}urée (78) :** Le composé **(78)** a été synthétisé à partir du 4-[2-chloro-7-(morpholin-4-ylmethyl)pyrido[3,2-d]pyrimidin-4-yl]morpholine (74) (66 mg, 0,19 mmol) en suivant la procédure générale susmentionnée pour donner un solide jaunâtre avec un rendement de 56 %. MP >260 °C ; Infrarouge (Diamand ATR, $cm^{-1}$) υ : 3338, 2856, 1597, 1531, 1483, 1438, 1230, 1107, 968, 858, 739 ; RMN $^1H$ (400 MHz, DMSO-$d_6$) δ: 2,44 (t, 4H, *J* = 2,0 Hz, $2xCH_2(O)$), 3,61 (t, 4H, *J* = 2,0 $2xCH_2(N)$), 3,70 (s, 2H, $CH_2$), 3,82 (t, 4H, *J* = 2,0 Hz, $2xCH_2(O)$), 4,43 (s, 2H, $CH_2$) 4,51 (bs, 4H, $2xCH_2(N)$), 7,23 (m,2H, $H_{arom}$), 7,43 (m,2H, $H_{arom}$), 7,60 (m,2H, $H_{arom}$), 8,03 (m, 1H, $H_8$), 8,36 (m,2H, $H_{arom}$), 8,61 (d, 1H, *J* = 2,0 Hz, $H_6$) ; 9,00 (bs, 2H, 2xNH) ; RMN $^{13}C$ (DEPT) (101 MHz, DMSO-$d_6$) δ: 49,1 ($2xCH_2$), 53,6 ($2xCH_2$), 58,9 ($CH_2$), 63,2 ($CH_2$), 67,2 ($2xCH_2$), 67,3 ($2xCH_2$), 117,28 ($2xCH_{arom}$), 118,2 ($2xCH_{arom}$), 127,12 ($2xCH_{arom}$), 129,3 ($2xCH_{arom}$), 135,4 (CH), 147,8 (CH), HRMS (EI-MS) : $C_{30}H_{34}N_7O_4$ $[M+H]^+$, calculée m/z 556,2672, trouvée m/z 556,2667.

**1-{4-[4-(morpholin-4-yl)-7-(morpholin-4-ylméthyl)pyrido[3,2-djpyrimidin-2-yl]phényl}-3-(2,2,2-trifluoro-**

**éthyl)urée (79) :** Le composé **(79)** a été synthétisé à partir du 4-[2-chloro-7-(morpholin-4-ylmethyl)pyrido[3,2-d]pyrimidin-4-yl]morpholine (74) (45 mg, 0,13 mmol) en suivant la procédure générale susmentionnée pour donner un solide jaune pale avec un rendement de 56 %. MP : 186-188 °C ; Infrarouge (Diamand ATR, $cm^{-1}$) υ : 3338, 3295, 1648, 1599, 1571, 1452, 1433, 1230, 1107, 1016, 968, 858, 739 ; RMN $^1H$ (400 MHz, DMSO-$d_6$) δ: 2,44 (t, 4H, $J$ = 2,0 Hz, $2xCH_2(O)$), 3,61 (t, 4H, $J$ = 2,0 $2xCH_2(N)$), 3,70 (s, 2H, $CH_2$), 3,82 (t, 4H, $J$ = 2,0 Hz, $2xCH_2(O)$), 4,43 (s, 2H, $CH_2$) 4,51 (bs, 4H, $2xCH_2(N)$), 7,23 (m,2H, $H_{arom}$), 7,43 (m,2H, $H_{arom}$), 7,60 (m,2H, $H_{arom}$), 8,03 (m, 1H, $H_8$), 8,36 (m,2H, $H_{arom}$), 8,61 (d, 1H, $J$ = 2,0 Hz, $H_6$) ; 9,00 (bs, 2H, 2xNH) ; RMN $^{13}C$ (DEPT) (101 MHz, DMSO-$d_6$) δ: 49,1 ($2xCH_2$), 53,6 ($2xCH_2$), 58,9 ($CH_2$), 63,2 ($CH_2$), 67,2 ($2xCH_2$), 67,3 ($2xCH_2$), 117,28 ($2xCH_{arom}$), 118,2 ($2xCH_{arom}$), 127,12 ($2xCH_{arom}$), 129,3 ($2xCH_{arom}$), 135,4 (CH), 147,8 (CH), HRMS (EI-MS) : $C_{30}H_{34}N_7O_4$ $[M+H]^+$, calculée m/z 556,2672, trouvée m/z 556,2667.

**1-[3-(hydroxyméthyl)phényl]-3-{4-[4-(morpholin-4-yl)-7-(morpholin-4 ylméthyl)pyrido[3,2-d]pyrim idin-2-yl]phényl}urée (80) :** Le composé **(80)** a été synthétisé à partir du 4-[2-chloro-7-(morpholin-4-ylmethyl)pyrido[3,2-d]pyrimidin-4-yl]morpholine (74) (45 mg, 0,13 mmol) en suivant la procédure générale susmentionnée pour donner un solide jaune pâle avec un rendement de 34 %. MP : 200-202 °C ; Infrarouge (Diamand ATR, $cm^{-1}$) υ : 3338, 3281, 2857, 1699, 1596, 1483, 1438, 1230, 1107, 1029, 968, 858, 739 ; RMN $^1H$ (400 MHz, DMSO-$d_6$) δ: 2,44 (t, 4H, $J$ = 2,0 Hz, $2xCH_2(O)$), 3,61 (t, 4H, $J$ = 2,0 $2xCH_2(N)$), 3,70 (s, 2H, $CH_2$), 3,82 (t, 4H, $J$ = 2,0 Hz, $2xCH_2(O)$), 4,43 (s, 2H, $CH_2$) 4,51 (bs, 6H, $CH_2$ et $2xCH_2(N)$), 6,92 (m, 1H, $H_{arom}$), 7,23 (m,1H, $H_{arom}$), 7,37 (m, 1H, $H_{arom}$), 7,47 (m,1H, $H_{arom}$), 7,60 (m,2H, $H_{arom}$), 8,04 (m, 1H, $H_8$), 8,36 (m,2H, $H_{arom}$), 8,61 (d, 1H, $J$ = 2,0 Hz, $H_6$) ; 9,00 (bs, 2H, 2xNH) ; RMN $^{13}C$ (DEPT) (101 MHz, DMSO-$d_6$) δ: 48,4 ($2xCH_2$), 53,96 ($2xCH_2$), 59,6 ($CH_2$), 65,6 ($CH_2$), 66,6 ($2xCH_2$), 116,4 ($2xCH_{arom}$), 117,6 ($2xCH_{arom}$), 120,7 ($CH_{arom}$), 124,3 ($CH_{arom}$), 129, 3 ($2xCH_{arom}$), 135,4 (CH), 147,8 (CH), HRMS (EI-MS) : $C_{30}H_{34}N_7O_4$ $[M+H]^+$, calculée m/z 556,2672, trouvée m/z 556,2667.

**1-[4-(hydroxyméthyl)phényl]-3-[4-(7-{[4-(méthoxyméthyl)-1H-1,2,3-triazol-1-yl]méthyl}-4-(morpholin-4-yl)pyrido[3,2-d]pyrimidin-2-yl)phényl]urée (81)** : Le composé **(81)** a été synthétisé à partir du 4-(2-chloro-7-{[4-(methoxymethyl)-1H-1,2,3-triazol-1-yl]methyl}pyrido[3,2-d]pyrimidin-4 yl)morpholine (73) (53 mg, 0,14 mmol) en suivant la procédure générale susmentionnée pour donner un solide jaunâtre avec un rendement de 25 %. MP>260 °C ; Infrarouge (Diamand ATR, $cm^{-1}$) υ : 3193, 2856, 1696, 1596, 1506, 1435, 1438, 1230, 1107, 968, 858, 739 ; RMN $^1H$ (400 MHz, DMSO-$d_6$) δ: 3,29 (s, 3H, $CH_3$), 3,82 (m, 4H, $2xCH_2(O)$), 4,48 (m, 8H, $2xCH_2$ and $2xCH_2(N)$), 5,89 (s, 2H, $CH_2$), 7,23 (m,2H, $H_{arom}$), 7,43 (m,2H, $H_{arom}$), 7,60 (m,2H, $H_{arom}$), 8,03 (m, 1H, $H_8$), 8,33 (s, 1H, $H_{triazole}$), 8,36 (m,2H, $H_{arom}$), 8,61 (d, 1H, $J$ = 2,0 Hz, $H_6$) ;9,00 (bs, 2H, 2xNH) ; RMN $^{13}C$ (101 MHz, DMSO-$d_6$) δ : 47,6 ($2xCH_2$), 57,8 ($CH_3$), 63,1 ($CH_2$), 65,4 ($2xCH_2$), 50,7 ($CH_2$), 117,9 (2xCH), 118,9 (2xCH), 125,0 (CH), 127,6 (2xCH), 129,5 (2xCH), 131,3 (Cq), 132,2 (Cq), 134,5 (Cq), 136,2 (CH), 136,6 (Cq), 138,6 (2xCq), 142,7 (Cq), 144,8 (Cq) 148,0 (CH), 152,8 (Cq), 158,9 (Cq), 159,5 (Cq) ; HRMS (EI-MS) : $C_{30}H_{32}N_9O_4$ $[M+H]^+$, calculée m/z 582,2577, trouvée m/z 582,2577.

**1-[4-(7-{[4-(méthoxyméthyl)-1H-1,2,3-triazol-1-yl]méthyl}-4-(morpholin-4-yl)pyrido[3,2-d]pyrimidin-2-yl)phényl]-3-(2,2,2-trifluoroéthyl)urée (82):** Le composé **(82)** a été synthétisé à partir du 4-(2-chloro-7-{[4-(methoxymethyl)-1H-1,2,3-triazol-1-yl]methyl}pyrido[3,2-d]pyrimidin-4 yl)morpholine (73) (53 mg, 0,14 mmol) en suivant la procédure générale susmentionnée pour donner un solide jaunâtre avec un rendement de 25 %. MP>260 °C Infrarouge (Diamand ATR, $cm^{-1}$) υ : 3361, 3021, 2820, 1600, 1556, 1513, 1483, 1438, 1230, 1107, 968, 858, 811, 739 ; RMN $^1H$ (400 MHz, DMSO-$d_6$) δ: 3,29 (s, 3H, $CH_3$), 3,82 (m, 4H, $2xCH_2(O)$), 3,93 (m, 2H, $CH_2CF_3$), 4,48 (bs, 6H, $CH_2$ and $2xCH_2(N)$), 5,88 (s, 2H, $CH_2$), 6,87 (t, 1H, $J$ = 7 Hz, NH), 7,23 (m,2H, $H_{arom}$), 7,43 (m,2H, $H_{arom}$) 7,95 (m,1H, $H_8$), 8,33 (s, 2H, $H_{arom}$), 8,36 (s, 1H, $H_{triazole}$), 8,71 (d, 1H, $J$ = 2,0 Hz, $H_6$) ; 9,05 (s, 1H, NH) ; RMN $^{13}C$ (101 MHz, DMSO-$d_6$) δ : 47,6 ($2xCH_2$), 57,8 ($CH_3$), 63,1 ($CH_2$), 65,4 ($2xCH_2$), 50,7 ($CH_2$), 117,9 (2xCH), 118,9 (2xCH), 125,0 (CH), 127,6 (2xCH), 129,5 (2xCH), 131,3 (Cq), 132,2 (Cq), 134,5 (Cq), 136,2 (CH), 136,6 (Cq), 138,6 (2xCq), 142,7 (Cq), 144,8 (Cq) 148,0 (CH), 152,8 (Cq), 158,9 (Cq), 159,5 (Cq) ; HRMS (EI-MS) : $C_{25}H_{27}F_3N_9O_3$ $[M+H]^+$, calculée m/z 558,2189, trouvée m/z 582,2183.

| | |
|---|---|
| 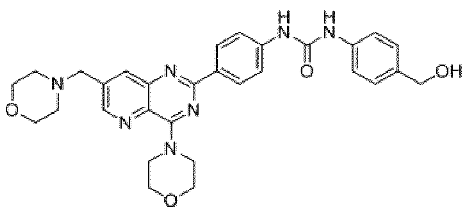 78 | 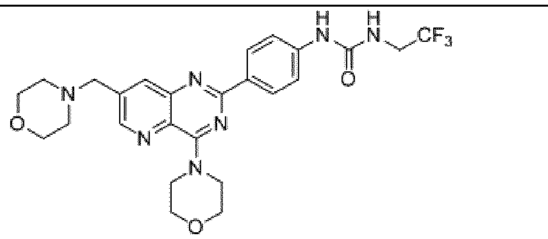 79 |

| | |
|---|---|
| 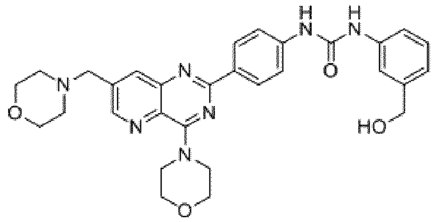 **80** | 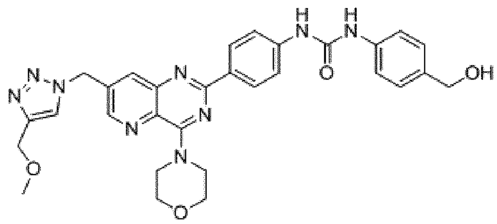 **81** |
| 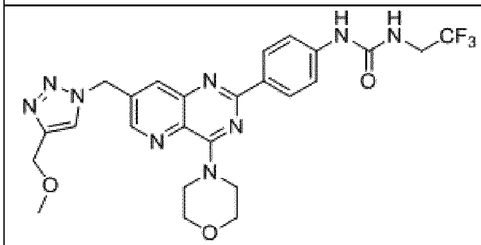 **82** | |

## C. Résultats biologiques

L'activité de la kinase PI3K$\alpha$ a été évaluée en utilisant un hétérodimère purifié (référence : PV4788 chez Invitrogen) composé de la sous-unité p110 catalytique $\alpha$ (PIK3CA) et la sous-unité p85 de régulation $\alpha$ (PIK3R1). Le dosage de la kinase par le kit Adapta™ (référence : PV5099 chez Invitrogen) est un test enzymatique basée sur la détection de l'ADP, produit lors de la réaction, par la technique de fluorescence TR-FRET. Il s'est déroulé en plaque 384 puits et a pu être divisé en deux phases: une phase de réaction enzymatique, et une phase de détection du niveau d'ADP.

Lors de la phase de réaction enzymatique, les composants suivants ont été mélangés dans 10 $\mu$L d'un même puit: 2,5 $\mu$L de chaque dilution en série d'inhibiteurs repris dans du DMSO puis dilués au 1/25$^{\text{ème}}$ dans le tampon réaction (250 mM HEPES pH 7,5, 500 mM NaCl, 0,15 % CHAPS, 5 mM EGTA, 15 mM de $MgCl_2$ et 1 mM de DTT, réf : PV5101 chez Invitrogen), 2,5 $\mu$L d'une solution contenant PI3K à la concentration optimale (concentration définie selon l'étape d'optimisation décrite dans le mode opératoire du kit) dilué dans le même tampon réaction, et enfin, 5 $\mu$L d'une solution contenant 20 $\mu$M ATP et 100 $\mu$M PIP2 dilués dans le tampon réaction. Pour chaque concentration en inhibiteur, la réaction a été exécutée en trois exemplaires (triplicat). La réaction a ensuite été incubée 60 min à température ambiante sans agitation dans l'obscurité.

Lors de la phase de détection d'ADP, 5 $\mu$L d'une solution de détection contenant un anticorps anti-ADP étiqueté à l'europium (6 nM), un traceur de type Alexa Fluor ® 647 couplé à l'ADP (30 nM), et de l'EDTA (30 mM, pour arrêter la réaction kinase) ont été dilués dans le tampon de dilution (référence : PV3574 chez Invitrogen). Après 30 min d'incubation à température ambiante avec agitation (40 rpm) dans l'obscurité, les plaques 384 puits ont été lues dans un lecteur de plaque Victor V configuré pour HTRF (Perkin Elmer). L'excitation a été réalisée à 340 nm et l'émission a été mesurée à 665 nm et 615 nm. La courbe d'inhibition a ensuite été tracée selon le rapport d'émission 665nm/615nm en fonction de la concentration en inhibiteur.

Afin d'obtenir une réponse linéaire du signal, une courbe de titration ATP-ADP, correspondant au % de conversion d'ATP en ADP, a été réalisée en faisant varier ces deux espèces tout en gardant [ADP]+ [ATP]=10 $\mu$M Le rapport d'émission 665nm/615nm résultant (Y) a été tracé en fonction du % de conversion de l'ATP en ADP (X). Les données de cette courbe ont été formatées suivant un modèle à 3 paramètres hyperboliques issu de l'équation suivante :

$$Y = C + A * (1 - (X / (B + X))).$$

Le logiciel GraphPad™ Prism® permet ainsi le calcul des paramètres A, B et C. Un % de conversion correspond à un rapport d'émission 665nm/615nm, ce pourcentage est calculé en utilisant l'équation suivante :

$$\text{Conversion \%} = B * (C + A\text{-Rapport})/(\text{Rapport-}C)$$

Les 3 paramètres A, B et C ayant été défini par l'équation précédente, le % de conversion a donc pu être tracé en fonction de la concentration en inhibiteur. La quantité d'inhibiteur nécessaire pour provoquer une variation de 50 % dans la conversion du % d'ATP en ADP correspond à la valeur $IC_{50}$ de l'inhibiteur.

L'activité de la kinase PI3Kγγ (PV4786 chez Invitrogen) a été évaluée sur la sous-unité catalytique p110γγ (PI3KCG) et celle de la kinase PI3Kδδ sur l'hétérodimère purifié (PV5273) composé respectivement de la sous-unité p110 catalytique δδ (PIK3CD) et la sous-unité p85 de régulation α (PIK3R1) en suivant le même protocole.

*C.2. Activité kinase mTOR*

L'activité de la kinase mTOR a été évaluée en utilisant une protéine tronquée des acides aminés 1-1359, purifiée (référence : PV4753 chez Invitrogen). Le dosage de l'activité de l'enzyme a été effectué par le kit LANCE® Ultra qui est un test enzymatique basé sur la détection d'un peptide phosphorylé produit au cours de la réaction, en utilisant la technique de fluorescence TR-FRET. Il s'est déroulé en plaque 384 puits et a pu être divisé en deux phases: une phase de réaction enzymatique, et une phase de détection du peptide phosphorylé.

Lors de la phase de réaction enzymatique, les composants suivants ont été mélangés dans 10 µL d'un même puit: 5 µL de chaque dilution en série d'inhibiteurs repris dans du DMSO puis dilués au 1/25ème dans le tampon réaction (50 mM HEPES pH 7,5, 0,1 % Tween-20, 1 mM EGTA, 10 mM de $MnCl_2$, 3 mM $MgCl_2$ et 2 mM de DTT), 2,5 µL d'une solution contenant mTOR à la concentration optimale (concentration définie selon l'étape d'optimisation décrite dans le mode opératoire du kit) dilué dans le même tampon réaction, et enfin 2,5 µL d'une solution contenant 40 µM d'ATP et 200 µM de peptide non-phosphorylé dilués dans le tampon de réaction. Pour chaque concentration en inhibiteur, la réaction a été exécutée en trois exemplaires (triplicat). La réaction a ensuite été incubée 120 min à température ambiante sans agitation dans l'obscurité.

Lors de la phase de détection du peptide phosphorylé, 5 µL d'une solution d'EDTA (32 mM) dilué dans le tampon de dilution (réf : CR97-100 chez Perkin Elmer) ont été injectés dans chaque puit. Après 5 min d'incubation sous agitation, 5 µL d'une solution d'anticorps anti-peptide phosphorylé étiquetés à l'europium (8 nM) et dilués dans le même tampon de dilution a été ajouté dans chaque puit. Après 60 min d'incubation à température ambiante avec agitation (40 rpm) dans l'obscurité, les plaques 384 puits ont été lues dans un lecteur de plaque Victor V configuré pour HTRF (Perkin Elmer). L'excitation a été réalisée à 340 nm et l'émission a été mesurée à 665 nm. La courbe d'inhibition a ensuite été tracée selon la valeur d'intensité de fluorescence à 665nm en fonction de la concentration en inhibiteur.

La quantité d'inhibiteur nécessaire pour provoquer une variation de 50% dans l'intensité du signal correspond à la valeur $IC_{50}$ de l'inhibiteur.

*C.3. Résultats*

Les résultats obtenus sont indiqués dans les tableaux ci-après.

| | | Kinases (IC 50 en µM) | | | |
|---|---|---|---|---|---|
| N° | Espèce | PI3Kα | PI3Kγ | PI3Kδ | mTOR |
| 6 | 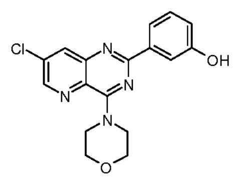 | 0.012 | 0.273 | 0.010 | 0.183 |

(suite)

| | | Kinases (IC 50 en μM) | | | |
|---|---|---|---|---|---|
| N° | Espèce | PI3Kα | PI3Kγ | PI3Kδ | mTOR |
| 7 | 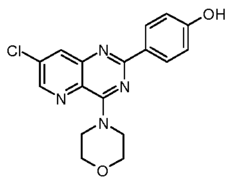 | 15.2 | >50 | 1.5 | 0.184 |
| 8 | 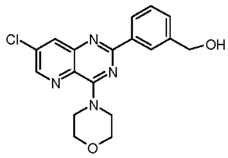 | 0.032 | 0.431 | 0.016 | >50 |
| 9 | 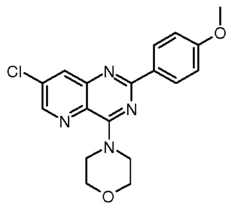 | 6.2 | 3.1 | 0.363 | > 50 |
| 14 | 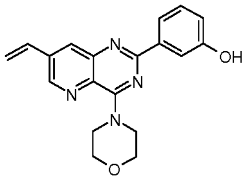 | 0.098 | 0.196 | 0.011 | 0.109 |
| 17 | 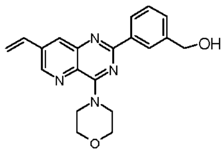 | 0.021 | 0.248 | 0.008 | 0.37 |
| 16 | 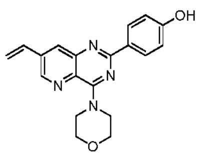 | 1.6 | 1.7 | 2.1 | 0.37 |
| 22 | 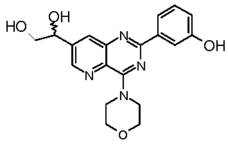 | 0.082 | 0.344 | 0.041 | 0.46 |
| 20 | 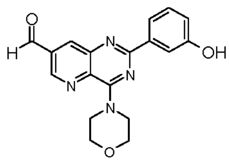 | 0.047 | >50 | 0.010 | 0.071 |

(suite)

| | | Kinases (IC 50 en µM) | | | |
|---|---|---|---|---|---|
| N° | Espèce | PI3Kα | PI3Kγ | PI3Kδ | mTOR |
| 27 | 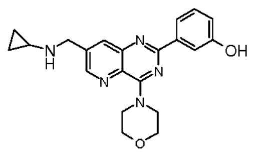 HCl | 0.109 | 0.428 | 0.011 | 0.249 |
| 28 | 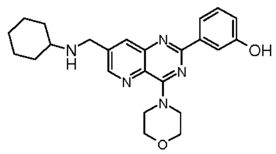 HCl | 0.358 | 0.769 | 0.213 | 1.2 |
| 29 | 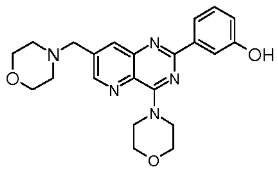 HCl | 0.043 | 1.3 | 0.041 | 0.286 |
| 30 | 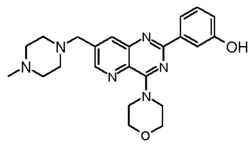 HCl | 0.391 | 0.244 | 0.157 | 2.2 |
| 31 | 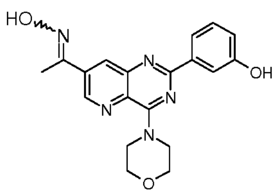 | 0.050 | 0.039 | 0.047 | 0.064 |
| 32 | 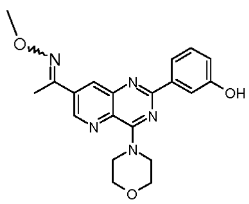 | 0.0026 | 0.260 | 0.012 | 0.005 |
| 34 | 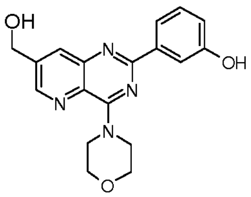 | 0.366 | 0.022 | 0.126 | 0.35 |

(suite)

| | | Kinases (IC 50 en μM) | | | |
|---|---|---|---|---|---|
| N° | Espèce | PI3Kα | PI3Kγ | PI3Kδ | mTOR |
| 37 | 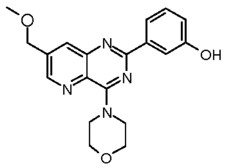 | 0.103 | 0.179 | 0.083 | 0.070 |
| 39 | 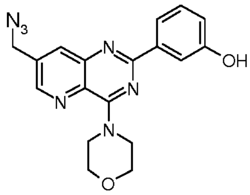 | 0.010 | 0.043 | 0.022 | 0.055 |
| 43 | 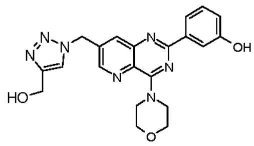 | 0.808 | 0.018 | 0.231 | 0.404 |
| 44 | 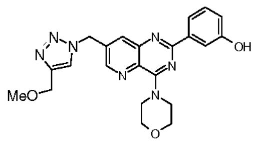 | 0.0098 | 0.081 | 0.033 | 0.256 |
| 41 | 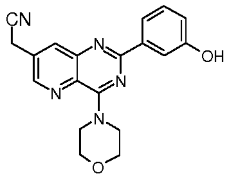 | 0.051 | 0.127 | 0.34 | 0.82 |
| 42 | 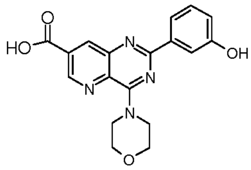 | 0.010 | 0.399 | 0.063 | 0.135 |
| 46 | 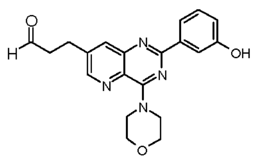 | 0.057 | 0.563 | 0.081 | 1.9 |
| 51 | 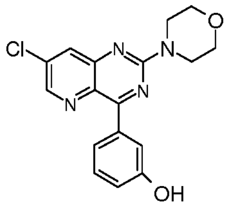 | 0.546 | 5.0 | 3.9 | 5.2 |

(suite)

| | | Kinases (IC 50 en µM) | | | |
|---|---|---|---|---|---|
| N° | Espèce | PI3Kα | PI3Kγ | PI3Kδ | mTOR |
| 55 | 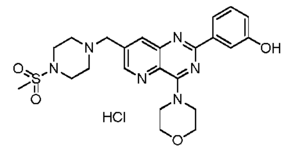 | 0.056 | - | - | - |
| 56 | 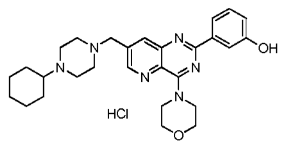 | 0.011 | - | - | - |
| 58 | 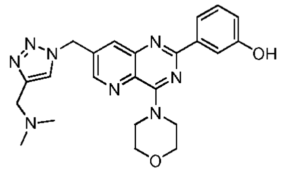 | 0.038 | - | - | - |
| 59 | 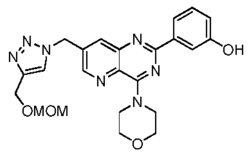 | 0.116 | - | - | - |
| 61 | 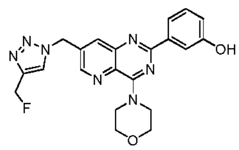 | 0.391 | - | - | - |
| 62 | 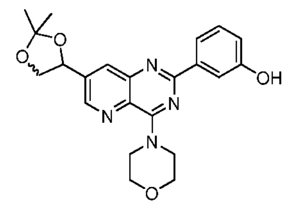 | 0.102 | - | - | - |
| 78 | 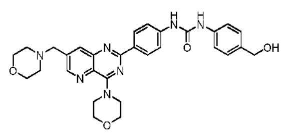 | 0.023 | - | - | - |
| 81 | 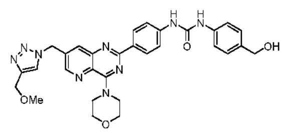 | 0.003 | - | - | - |
| 82 | 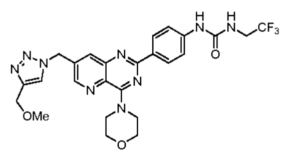 | 0.018 | - | - | - |

Les composés selon l'invention ont également été testés sur les lignées cellulaires HuH7, CaCo-2, HCT116,

PC3, NCI, HacaT et Fibroblastes. Sur lignées cancereuses, des $IC_{50}$ pouvant aller jusqu'à 0.1 μM ont été évaluées dans les conditions décrites (sur 48 h).

Mode opératoire : Les cellules ont été cultivées selon les recommendations ECACC. Le test de toxicité des composés sur ces cellules a été réalisé comme suit: $4x10^3$ cellules/puit ont été ensemencées dans 96 puits. 24 heures après l'ensemencement des cellules, les cellules ont été exposées à des concentrations accrues des composés (0.1μM-0.3μM-0.9μM-2.7μM-8.3μM-25μM). Après 48 heures de traitement, les cellules ont été lavées dans PBS et fixées dans un mélange froid éthanol/acide acétique (90/5) pendant 20 minutes. Ensuite, les noyaux ont été colorés avec Hoechst 3342 (Sigma). L'acquisition d'images et d'analyse a été effectuée à l'aide d'un lecteur Cellomics ArrayScan VTI/ HCS (Thermo Scientific).

Les résultats obtenus sont rassemblés dans le tableau suivant :

| Composés | IC 50 μM | | | | | | | |
|---|---|---|---|---|---|---|---|---|
| | HUH7 | CaC o2 | MD A | HCT116 | PC3 | NCI | HacaT | Fibrobl astes |
| 5 | >25 | >25 | >25 | >25 | >25 | >25 | - | >25 |
| 6 | 0.8 | 8 | >25 | 9 | >25 | >25 | 4 | >25 |
| 7 | 25 | 25 | 25 | >25 | 25 | >25 | - | >25 |
| 8 | 25 | 3 | 20 | 5 | 20 | >25 | - | 2 |
| 9 | >25 | >25 | >25 | >25 | >25 | >25 | >25 | >25 |
| 51 | >25 | 20 | >25 | >25 | >25 | >25 | >25 | >25 |
| 14 | 20 | 1.5 | 20 | 5 | 20 | 20 | - | >25 |
| 16 | >25 | >25 | >25 | >25 | >25 | >25 | >25 | >25 |
| 17 | 15 | 5 | >25 | 6 | 25 | >25 | 4 | >25 |
| 20 | 20 | 4 | 20 | 6 | 25 | >25 | - | >25 |
| 22 | >25 | 4 | 10 | 5 | 4 | >25 | - | >25 |
| 34 | 1 | 1.5 | 25 | 3 | 5 | >25 | - | 25 |
| 37 | 1.5 | 1.5 | 20 | 4 | 4 | 10 | 3 | >25 |
| 31 | 3 | 1.2 | >25 | 2 | 2 | >25 | 2 | >25 |
| 32 | 4 | 3 | 20 | 5 | 5 | 6 | 4 | 0.8 |
| 30 | 5 | 2 | 20 | 10 | 7 | 15 | 8 | >25 |
| 27 | >25 | 3 | >25 | 5 | 2 | >25 | >25 | >25 |
| 28 | 15 | 10 | >25 | 20 | 25 | 25 | 20 | >25 |
| 29 | >25 | 3 | >25 | 5 | 2 | >25 | 8 | >25 |
| 39 | 4 | 2 | >25 | 5 | 3 | 3 | 4 | >25 |
| 44 | 4 | 1 | 15 | 3 | 4 | 4 | 2 | >25 |
| 43 | 4 | 1 | 15 | 3 | 4 | 4 | 2 | >25 |
| 56 | 6 | 6 | 12 | 7 | 8 | 11 | 8 | 10 |
| 78 | 2 | 12 | 3 | 1 | 2 | 3 | 2 | >25 |
| 81 | 7 | 2 | 0.5 | 0.1 | 0.1 | 0.3 | 0.3 | >25 |

## Revendications

1. Composé de formule générale (I) suivante :

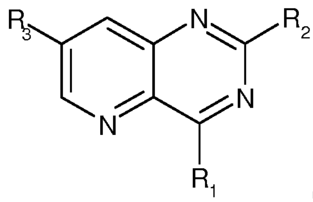

(I)

dans laquelle :

- $R_1$ est choisi dans le groupe constitué :

. des (hétéro)aryles comprenant de 5 à 30 atomes, éventuellement substitués, et
. des groupes $-NR_aR_b$, $R_a$ et $R_b$ formant ensemble avec l'atome d'azote sur lequel ils sont reliés, un hétérocycle comprenant de 5 à 30 atomes , comprenant éventuellement un radical $SO_2$ ou un autre hétéroatome choisi parmi N, O ou S, ledit hétérocycle étant éventuellement substitué ;

- $R_2$ est choisi dans le groupe constitué :

. des atomes d'halogène choisis parmi F, Br et I,
. des aryles comprenant de 5 à 30 atomes de carbone, éventuellement substitués,
. des groupes $-NR'_aR'_b$, $R'_a$ et $R'_b$ formant ensemble avec l'atome d'azote sur lequel ils sont reliés, un hétérocycle comprenant de 5 à 30 atomes, comprenant éventuellement un radical $SO_2$ ou un autre hétéroatome choisi parmi N, O ou S, ledit hétérocycle étant éventuellement substitué; et

- $R_3$ est choisi dans le groupe constitué :

. des atomes d'halogène choisis parmi F, Cl et I,
. des alcényles comprenant de 1 à 20 atomes de carbone, éventuellement substitués,
. des groupes $-C(O)R_c$, $R_c$ étant choisi dans le groupe constitué d'un atome d'hydrogène ou d'un groupe alkyle comprenant de 1 à 10 atomes de carbone, ledit groupe alkyle étant éventuellement substitué,
. des groupes $-C(O)OR'_c$, $R'_c$ étant choisi dans le groupe constitué d'un atome d'hydrogène ou d'un groupe alkyle comprenant de 1 à 10 atomes de carbone, ledit groupe alkyle étant éventuellement substitué,
. des groupes $-C(R_e)=N-(OR_d)$, $R_d$ et $R_e$ étant indépendamment choisi parmi le groupe constitué d'un atome d'hydrogène ou d'un groupe alkyle comprenant de 1 à 10 atomes de carbone,
. des hétérocycloalkyles comprenant de 3 à 20 atomes, éventuellement substitués,
. des alkyles, comprenant de 1 à 20 atomes de carbone, éventuellement substitués par au moins un substituant ;

à condition qu'au moins un de $R_1$ et $R_2$ représente respectivement un groupe $-NR_aR_b$ ou un groupe $-NR'_aR'_b$,
ainsi que ses sels pharmaceutiquement acceptables, ses hydrates, ses racémates, diastéréoisomères ou énantiomères,
les radicaux "alkyle", "aryle", "hétéroaryle", "alcényle", "hétérocycle", "hétérocycloalkyle" et "cycloalkyle" étant éventuellement substitués par un ou plusieurs substituants choisis parmi les groupes suivants : $-N_3$, CHO, amino, amine, hydroxy, halogène, carboxyle, alkyle (éventuellement substitué tel que par un halogène), $CH_2OH$, $CH_2F$, $CF_3$, $SO_2$alkyle, $CH_2OMe$, alkaryle, alkoxy, alkylcarbonyle, aryle (éventuellement substitué), aminocarbonyle, alkylcarboxyle, alkylamino, -NH-hétérocycloalkyle, hétérocycloalkyle (éventuellement substitué par un groupe Me, $SO_2Me$, cyclohexyle ou phényle), hétéroaryle (éventuellement substitué par un groupe $-CH_2NMe_2$, $CH_2F$, $CH_2OCH_2OMe$ ou $CH_2OMe$), aryloxy, arylalkoxy, cyano, trifluorométhyle, -NHC(O)NH-aryle (avec aryle éventuellement substitué par $-CH_2OH$), -NHC(O)NH-alkyle (avec alkyle éventuellement substitué par halogène), carboxyalkyle, alkoxyalkoxy et nitro.

**2.** Composé selon la revendication 1, dans lequel $R_2$ est choisi dans le groupe constitué :

. des aryles comprenant de 5 à 30 atomes de carbone, éventuellement substitués, et
. des groupes $-NR'_aR'_b$, $R'_a$ et $R'_b$ étant tels que définis dans la revendication 1.

**3.** Composé selon la revendication 1 ou 2, dans lequel $R_1$ est choisi dans le groupe constitué :

. des (hétéro)aryles comprenant de 5 à 30 atomes, éventuellement substitués, et
. des groupes $-NR_aR_b$, $R_a$ et $R_b$ étant tels que définis dans la revendication 1.

**4.** Composé selon l'une quelconque des revendications 1 à 3, de formule (I-3-1) suivante :

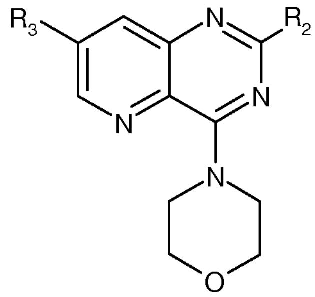

dans laquelle $R_2$ et $R_3$ sont tels que définis dans la revendication 1.

**5.** Composé selon l'une quelconque des revendications 1 à 3, de formule (I-5-1) suivante :

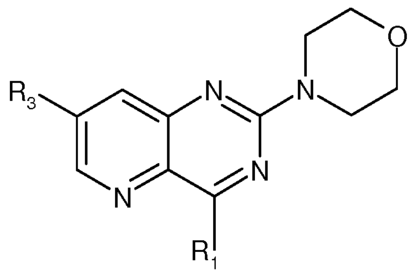

(I-5-1)

dans laquelle $R_1$ et $R_3$ sont tels que définis dans la revendication 1.

**6.** Composé selon l'une quelconque des revendications 1 à 3, de formule (I-1) suivante :

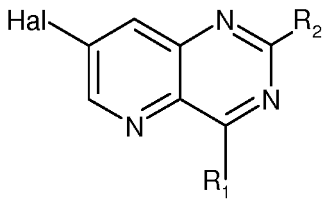

(I-1)

dans laquelle Hal représente un halogène choisi parmi F, Cl et I, et $R_1$ et $R_2$ sont tels que définis selon la revendication 1.

**7.** Composé selon l'une quelconque des revendications 1 à 6, choisi dans le groupe constitué des composés suivants :

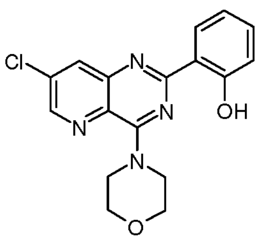

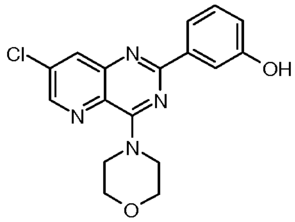

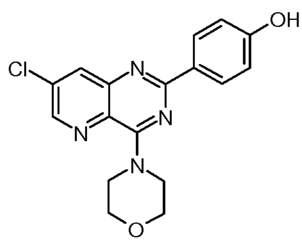

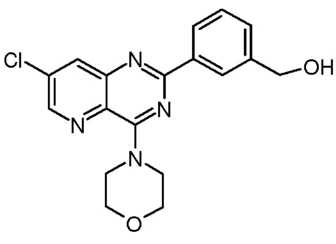
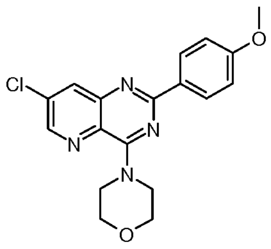
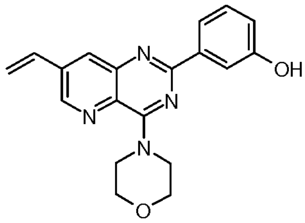

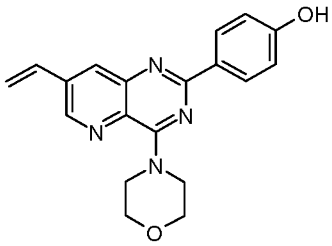
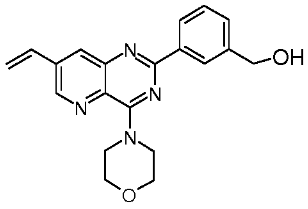
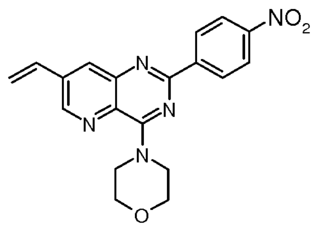

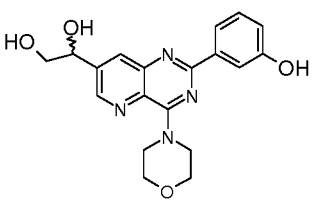
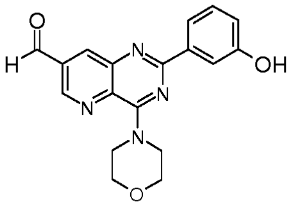
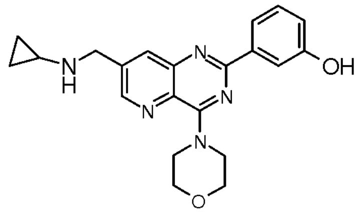

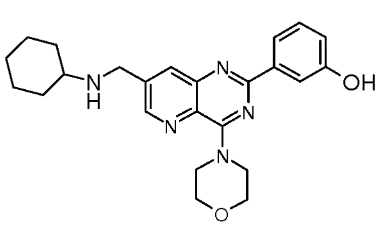
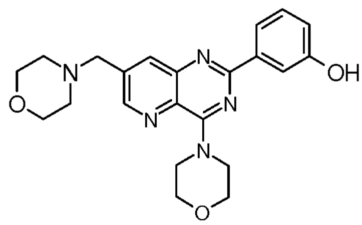

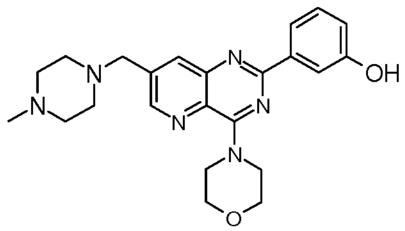
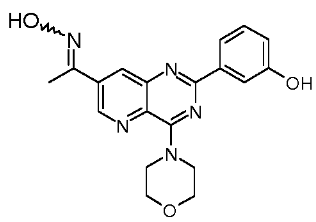
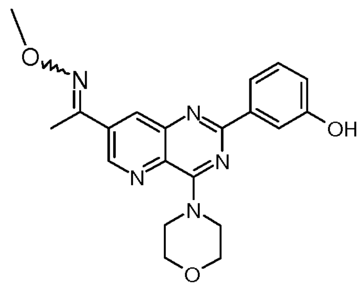

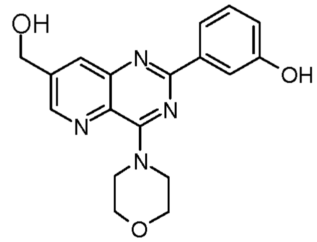
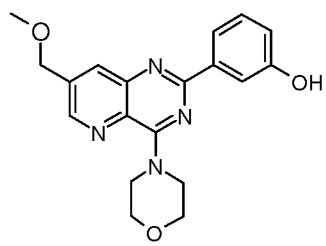
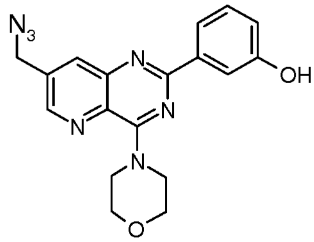

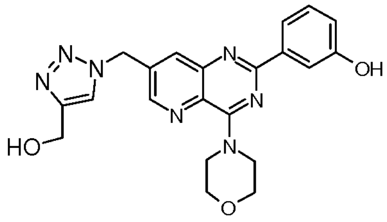

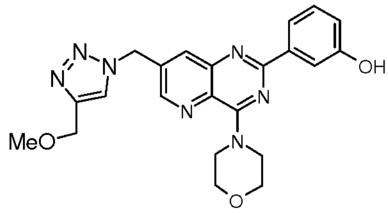

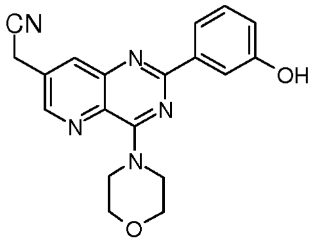

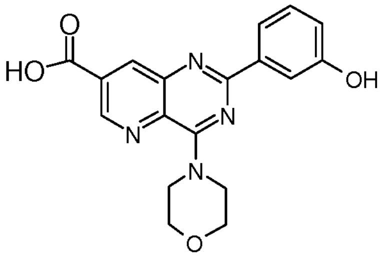

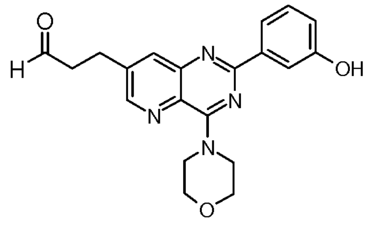

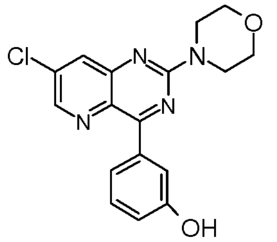

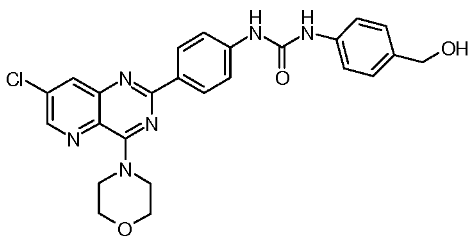

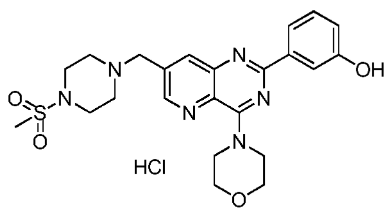

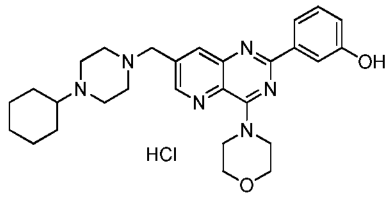

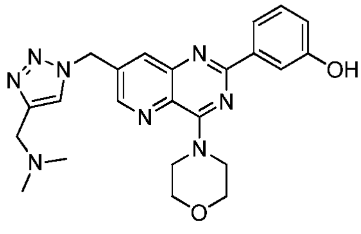

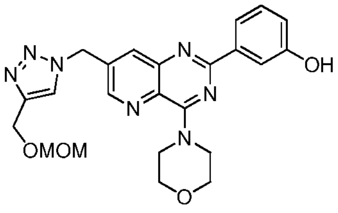

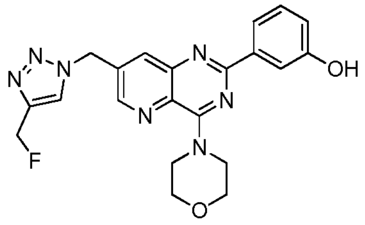

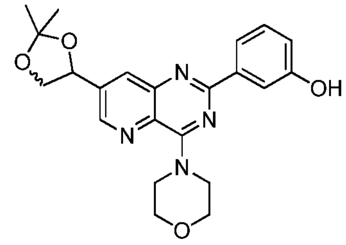

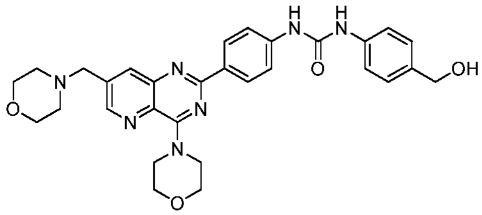

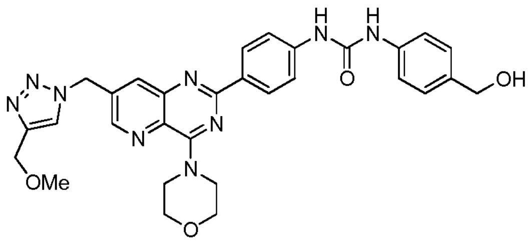

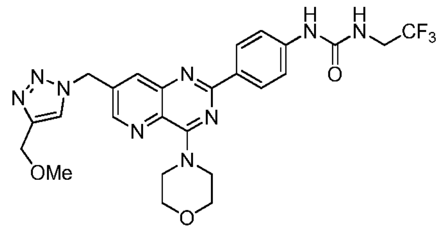

sous forme libre ou salifiée, notamment chlorhydrate.

8. Composé selon l'une quelconque des revendications 1 à 7, pour son utilisation comme médicament.

9. Composition pharmaceutique comprenant un composé de formule (I) tel que défini dans l'une quelconque des revendications 1 à 7, ou l'un de ses sels pharmaceutiquement acceptables, en association avec un véhicule pharmaceutiquement acceptable.

10. Composé selon l'une quelconque des revendications 1 à 7, pour son utilisation en tant qu'inhibiteur des enzymes PI3K et mTOR.

11. Composé selon l'une quelconque des revendications 1 à 7, pour son utilisation dans le cadre du traitement ou de la prévention de maladies liées à une dérégulation des enzymes PI3K et mTOR.

12. Composé, pour son utilisation selon la revendication 11, dans le cadre du traitement ou de la prévention de pathologies choisies dans le groupe constitué des cancers, tels que les cancers des poumons, des reins, des ovaires, du pancréas, de la peau, du colon, de la prostate, de leucémies, des maladies non dégénératives, telles que l'arthrite, l'inflammation, les scléroses, les néphrites glomérulaires, le psoriasis, les allergies, l'asthme, les diabètes, les maladies thrombo-emboliques et les maladies auto-immunes.

**Patentansprüche**

1. Verbindung der folgenden allgemeinen Formel (I):

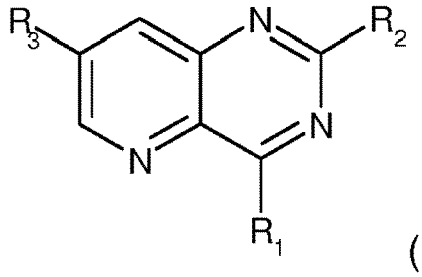

(I)

wobei:

- $R_1$ ausgewählt ist aus der Gruppe bestehend aus:

  . (Hetero-)Aryl umfassend 5 bis 30 Atome, optional substituiert, und
  . den Gruppen $-NR_aR_b$, wobei $R_a$ und $R_b$ zusammen mit dem Stickstoffatom an das sie gebunden sind einen Heterozyklus umfassend 5 bis 30 Atome bilden, optional umfassend ein $SO_2$-Radikal oder ein anderes Heteroatom ausgewählt aus N, O oder S, wobei der Heterozyklus optional substituiert ist;

- $R_2$ ausgewählt ist aus der Gruppe bestehend aus:

  . Halogenatomen ausgewählt aus F, Br und I,
  . Aryl umfassend 5 bis 30 Kohlenstoffatome, optional substituiert,
  . den Gruppen $-NR'_aR'_b$, wobei $R'_a$ und $R'_b$ zusammen mit dem Stickstoffatom an das sie gebunden sind einen Heterozyklus umfassend 5 bis 30 Atome bilden, optional umfassend ein $SO_2$-Radikal oder ein anderes Heteroatom ausgewählt aus N, O oder S, wobei der Heterozyklus optional substituiert ist; und

- $R_3$ ausgewählt ist aus der Gruppe bestehend aus:

  . Halogenatomen ausgewählt aus F, Cl und I,
  . Alkenyl umfassend 1 bis 20 Kohlenstoffatome, optional substituiert,
  . den Gruppen $-C(O)R_c$, wobei $R_c$ ausgewählt ist aus der Gruppe bestehend aus Wasserstoffatomen oder einer Alkylgruppe umfassend 1 bis 10 Kohlenstoffatomen, wobei die Alkylgruppe optional substituiert ist,
  . den Gruppen $-C(O)R'_c$, wobei $R'_c$ ausgewählt ist aus der Gruppe bestehend aus Wasserstoffatomen oder einer Alkylgruppe umfassend 1 bis 10 Kohlenstoffatomen, wobei die Alkylgruppe optional substituiert ist,
  . den Gruppen $-C(R_e)=N-(OR_d)$, wobei $R_d$ und $R_e$ unabhängig ausgewählt sind aus der Gruppe bestehend aus Wasserstoffatomen oder einer Alkylgruppe umfassend 1 bis 10 Kohlenstoffatomen,
  . Heterocycloalkyl umfassend 3 bis 20 Atome, optional substituiert,
  . Alkyl, umfassend 1 bis 20 Kohlenstoffatome, optional substituiert mit mindestens einem Substituenten;

unter der Bedingung dass mindestens einer von $R_1$ und $R_2$ jeweils eine Gruppe $-NR_aR_b$ oder eine Gruppe $-NR'_aR'_b$ darstellt,
sowie pharmazeutisch akzeptable Salze, Hydrate, Racemate, Diastereomere oder Enantiomere,
wobei die Radikale "Alkyl", "Aryl", "Heteroaryl", "Alkenyl", Heterocyclus", "Hetercycloalkyl" und "Cycloalkyl" optional substituiert sind mit einem oder mehreren Substituenten ausgewählt aus den folgenden Gruppen: $-N_3$, CHO, Amino, Amin, Hydroxy, Halogen, Carboxyl, Alkyl (optional substituiert mit Halogen), $CH_2OH$, $CH_2F$, $CF_3$, $SO_2$Alkyl, $CH_2OMe$, Alkaryl, Alkoxy, Alkylcarbonyl, Aryl (optional substituiert), Aminocarbonyl, Alkylcarboxyl, Alkylamino, -NH-Heterocycloalkyl, Heterocycloalkyl (optional substituiert mit einer Gruppe Me, $SO_2Me$, Cyclohexyl oder Phenyl), Heteroaryl (optional substituiert mit einer Gruppe $-CH_2NMe_2$, $CH_2F$, $CH_2OCH_2OMe$ oder $CH_2OMe$), Aryloxy, Arylalkoxy, Cyano, Trifluoromethyl, -NHC(O)NH-Aryl (mit Aryl optional substituiert mit $-CH_2OH$), -NHC(O)NH-Alkyl (mit Alkyl optional substituiert mit einem Halogen), Carboxylalkyl, Alkoxyalkoxy und Nitro.

**2.** Verbindung gemäß Anspruch 1, wobei $R_2$ ausgewählt ist aus der Gruppe bestehend aus:

  . Aryl umfassend 5 bis 30 Kohlenstoffatome, optional substituiert, und
  . den Gruppen $-NR'_aR'_b$, wobei $R'_a$ und $R'_b$ so definiert sind wie in Anspruch 1.

**3.** Verbindung gemäß Anspruch 1 oder 2, wobei $R_1$ ausgewählt ist aus der Gruppe bestehend aus:

. (Hetero-)Aryl umfassend 5 bis 30 Atome, optional substituiert, und
. den Gruppen $-NR_aR_b$, wobei $R_a$ und $R_b$ so definiert sind wie in Anspruch 1.

**4.** Verbindung gemäß einem der vorhergehenden Ansprüche 1 bis 3, der folgenden Formel (I-3-1):

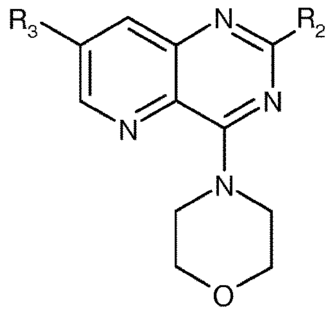

wobei $R_2$ und $R_3$ so definiert sind wie in Anspruch 1.

**5.** Verbindung gemäß einem der vorhergehenden Ansprüche 1 bis 3, der folgenden Formel (I-5-1):

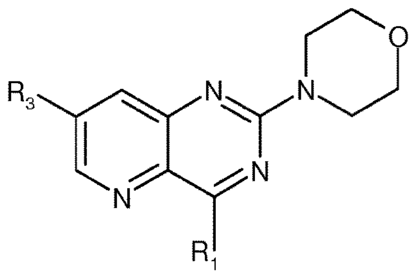

(I-5-1)

wobei $R_1$ und $R_3$ so definiert sind wie in Anspruch 1.

**6.** Verbindung gemäß einem der vorhergehenden Ansprüche 1 bis 3, der folgenden Formel (I-1):

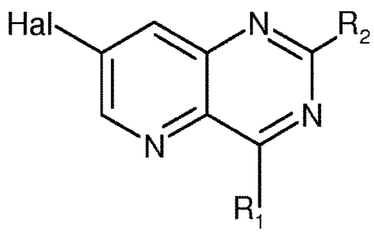

(I-1)

wobei Hal ein Halogen ausgewählt aus der Gruppe bestehend aus F, Cl und I darstellt und $R_1$ und $R_2$ so definiert sind wie in Anspruch 1.

**7.** Verbindung gemäß einem der vorhergehenden Ansprüche 1 bis 6, ausgewählt aus der Gruppe bestehend aus den folgenden Verbindungen:

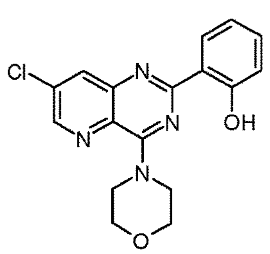 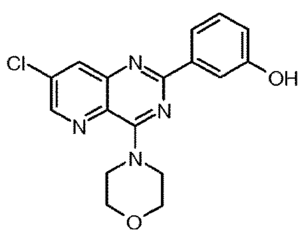 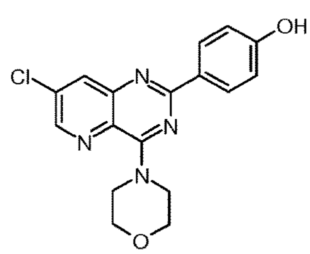

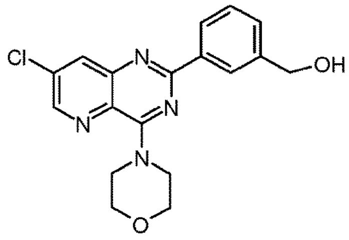
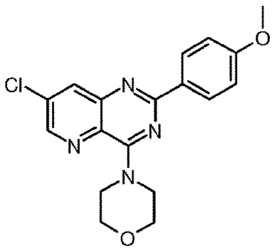
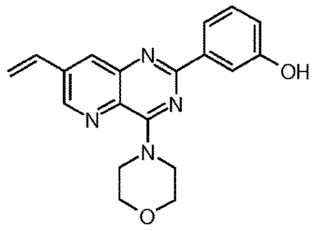

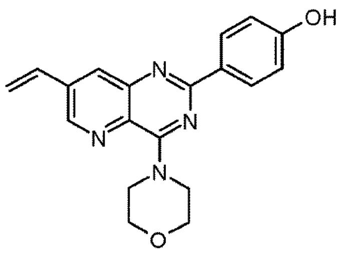
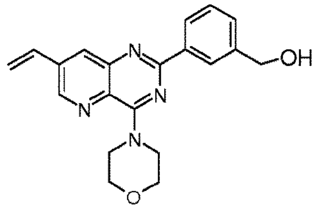
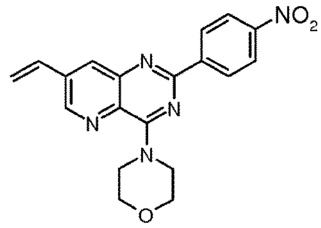

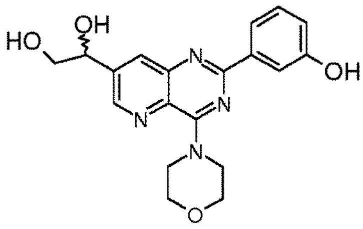
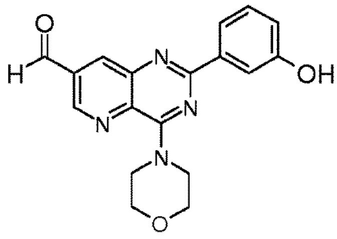
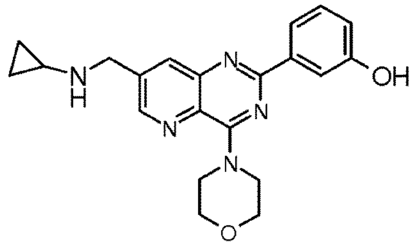

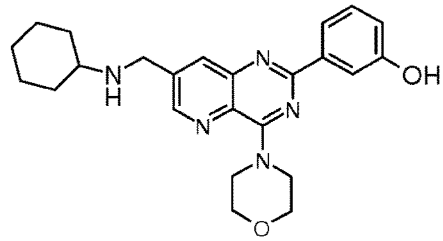
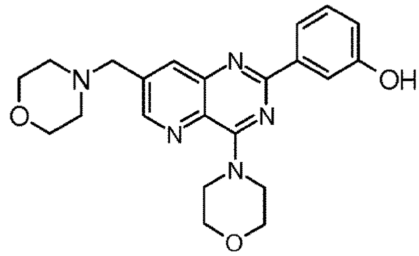

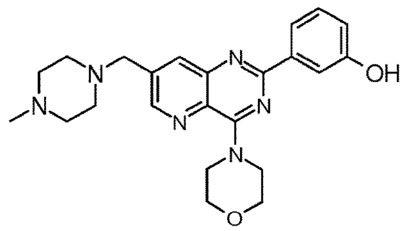
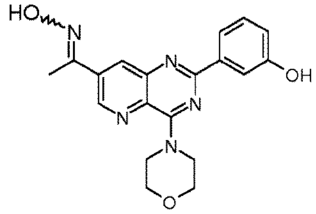
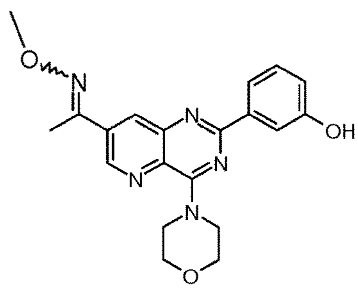

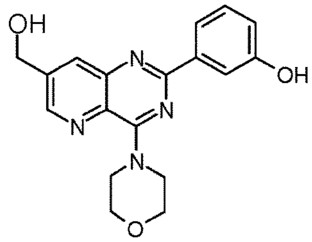
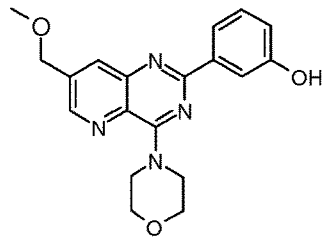
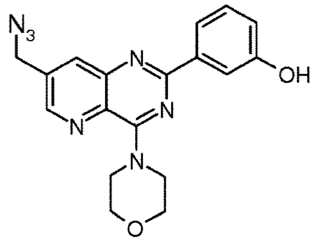

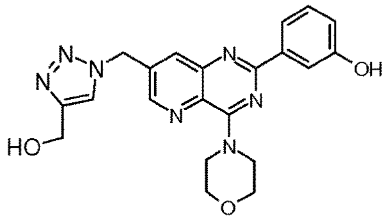

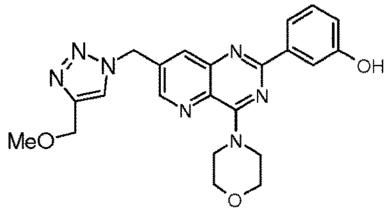

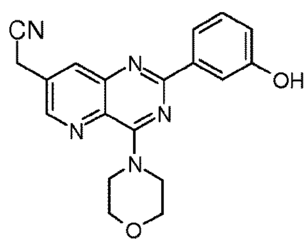

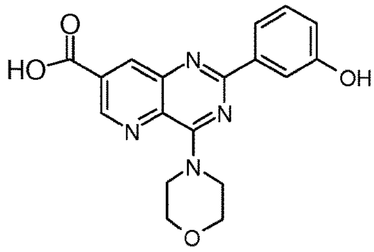

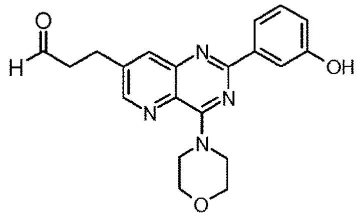

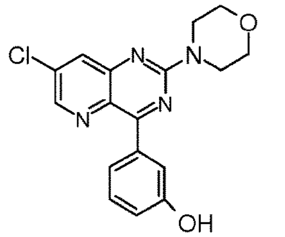

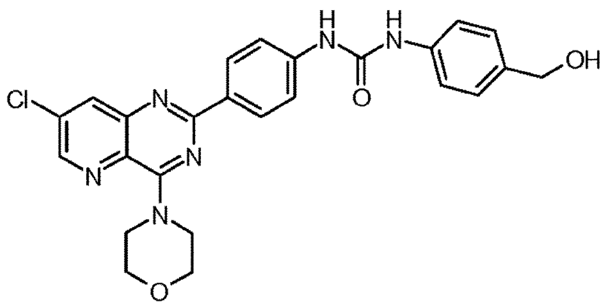

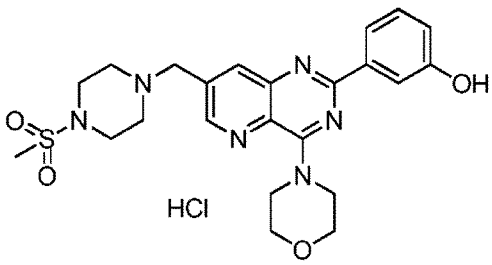

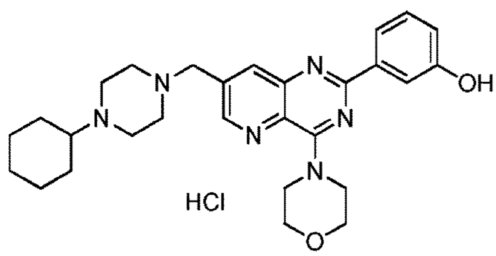

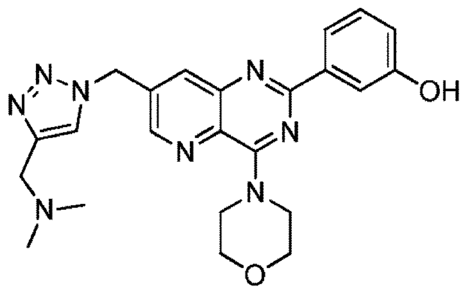

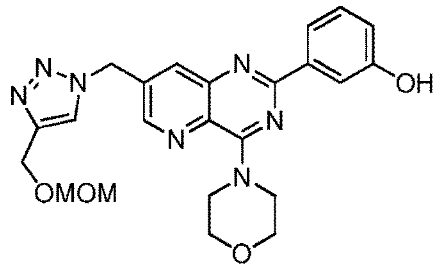

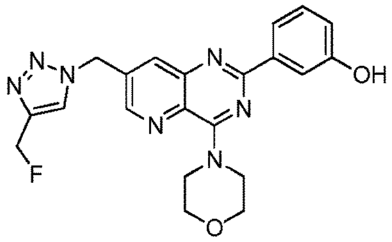

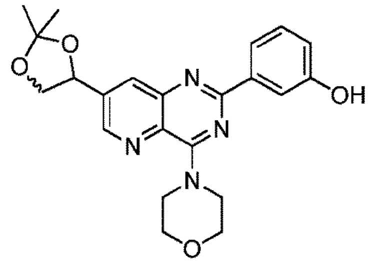

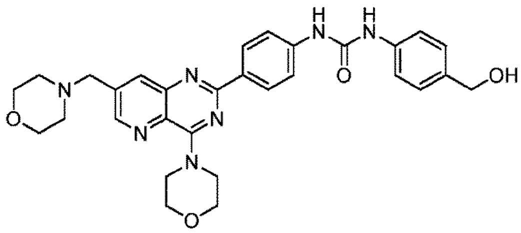

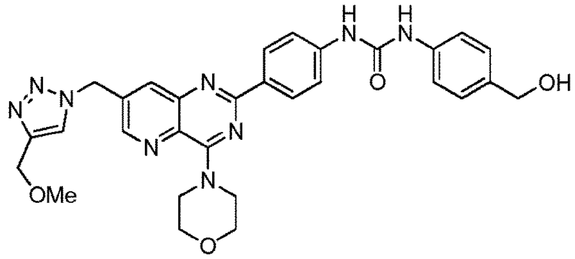

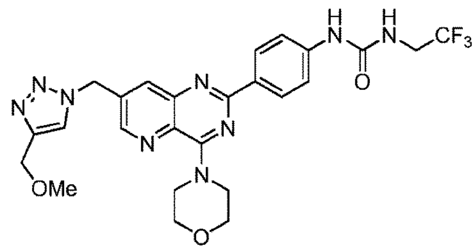

in freier oder Salzform, im Besonderen als Hydrochlorid.

**8.** Verbindung gemäß einem der vorhergehenden Ansprüche 1 bis 7, zur Verwendung als Medikament.

**9.** Pharmazeutische Zusammensetzung umfassend eine Verbindung der Formel (I) definiert wie in einem der Ansprüche 1 bis 7 oder eine ihrer pharmazeutisch akzeptablen Salze, in Verbindung mit einem pharmazeutisch akzeptablem Träger.

**10.** Verbindung gemäß einem der vorhergehenden Ansprüche 1 bis 7, zur Verwendung als Inhibitor der Enzyme PI3K und mTOR.

**11.** Verbindung gemäß einem der vorhergehenden Ansprüche 1 bis 7, zur Verwendung im Rahmen einer Behandlung oder Vorbeugung von Krankheiten verbunden mit einer Deregulierung der Enzyme PI3K und mTOR.

**12.** Verbindung zur Verwendung gemäß Anspruch 11, im Rahmen der Behandlung oder Vorbeugung von Erkrankungen ausgewählt aus der Gruppe bestehend aus Krebs, als da sind Lungenkrebs, Nierenkrebs, Eileiterkrebs, Bauchspeicheldrüsenkrebs, Hautkrebs, Darmkrebs, Prostatakrebs, Leukämie, nicht-degenerativen Krankheiten, als da sind Arthritis, Entzündungen, Sklerosen, Glomerulonephritis, Schuppenflechten, Allergien, Asthma, Diabetes, thromboembolische Erkrankungen und Autoimmunerkrankungen.

## Claims

**1.** A compound of the following general formula (I):

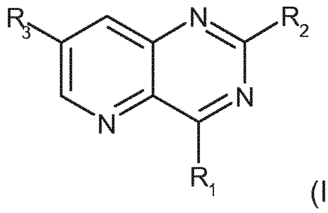

(I)

wherein:

- $R_1$ is selected from the group consisting of:

. (hetero)aryls comprising from 5 to 30 atoms, optionally substituted, and
. groups $-NR_aR_b$, $R_a$ and $R_b$ forming together with the nitrogen atom to which they are bound, a heterocycle comprising from 5 to 30 atoms, optionally comprising an $SO_2$ radical or another heteroatom selected from N, O or S, said heterocycle being optionally substituted;

- $R_2$ is selected from the group consisting of:

. halogen atoms selected from F, Br and I,
. aryls comprising from 5 to 30 carbon atoms, optionally substituted,
. groups $-NR'_aR'_b$, $R'_a$ and $R'_b$ forming together with the nitrogen atom to which they are bound, a heterocycle comprising from 5 to 30 atoms, optionally comprising an $SO_2$ radical or another heteroatom selected from N, O or S, said heterocycle being optionally substituted; and

- $R_3$ is selected from the group consisting of:

. halogen atoms selected from F, Cl and I,
. alkenyls comprising from 1 to 20 carbon atoms, optionally substituted,
. groups $-C(O)R_c$, $R_c$ being selected from the group consisting of a hydrogen atom or an alkyl group comprising from 1 to 10 carbon atoms, said alkyl group being optionally substituted,
. groups $-C(O)OR'_c$, $R'_c$ being selected from the group consisting of a hydrogen atom or an alkyl group comprising from 1 to 10 carbon atoms, said alkyl group being optionally substituted,
. groups $-C(R_e)=N-(OR_d)$, $R_d$ and $R_e$ being selected independently from the group consisting of a hydrogen atom or an alkyl group comprising from 1 to 10 carbon atoms,
. heterocycloalkyls comprising from 3 to 20 atoms, optionally substituted,
. alkyls, comprising from 1 to 20 carbon atoms, optionally substituted with at least one substituent;

provided that at least one of $R_1$ and $R_2$ represents a group $-NR_aR_b$ or a group $-NR'_aR'_b$, respectively as well as its pharmaceutically acceptable salts, its hydrates, its racemates, diastereoisomers or enantiomers, the "alkyl", "aryl", "heteroaryl", "alkenyl", "heterocycle", "heterocycloalkyl" and "cycloalkyl" radicals being possibly substituted with one or several substituents, chosen from the following groups: $-N_3$, CHO, amino, amine, hydroxy, halogen, carboxyl, alkyl (optionally substituted such as with a halogen), $CH_2OH$, $CH_2F$, $CF_3$, $SO_2$alkyl, $CH_2OMe$, alkaryl, alkoxy, alkylcarbonyl, aryl (optionally substituted), aminocarbonyl, alkylcarboxyl, alkylamino, -NH-heterocycloalkyl, heterocycloalkyl (optionally substituted with a group Me, $SO_2Me$, cyclohexyl or phenyl), heteroaryl (optionally substituted with a group $-CH_2NMe_2$, $CH_2F$, $CH_2OCH_2OMe$ or $CH_2OMe$), aryloxy, arylalkoxy, cyano, trifluoromethyl, - NHC(O)NH-aryl (with aryl optionally substituted with $-CH_2OH$), -NHC(O)NH-alkyl (with alkyl optionally substituted with a halogen), carboxyalkyl, alkoxyalkoxy and nitro.

**2.** The compound according to claim 1, wherein $R_2$ is selected from the group consisting of:

. aryls comprising from 5 to 30 carbon atoms, optionally substituted, and
. groups $-NR'_aR'_b$, $R'_a$ and $R'_b$ being as defined in claim 1.

**3.** The compound according to claim 1 or 2, wherein $R_1$ is selected from the group consisting of:

. (hetero)aryls comprising from 5 to 30 atoms, optionally substituted, and
. groups $-NR_aR_b$, $R_a$ and $R_b$ being as defined in claim 1.

**4.** The compound according to any of claims 1 to 3, of the following formula (I-3-1):

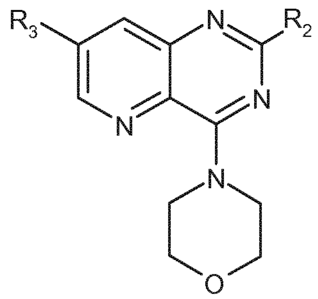

wherein $R_2$ and $R_3$ are as defined in claim 1.

**5.** The compound according to any of claims 1 to 3, of the following formula (I-5-1):

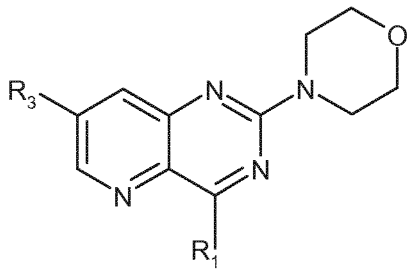

(I-5-1)

wherein and $R_3$ are as defined in claim 1.

**6.** The compound according to any of claims 1 to 3, of the following formula (I-1):

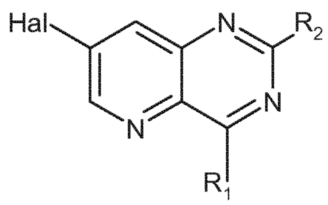

(I-1)

wherein Hal represents a halogen selected from F, Cl and I, and $R_1$ and $R_2$ are defined in claim 1.

**7.** The compound according to any of claims 1 to 6, selected from the group consisting of the following compounds:

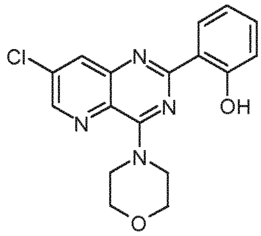

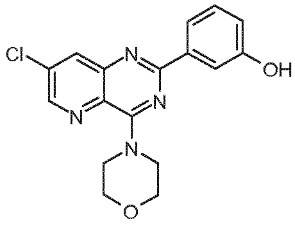

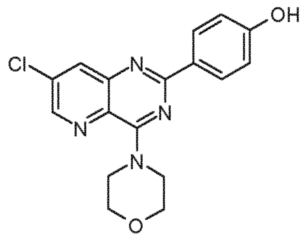

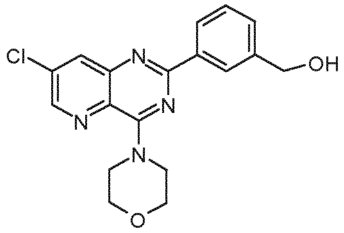

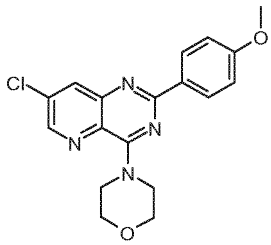

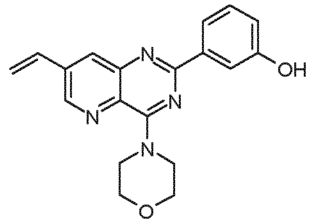

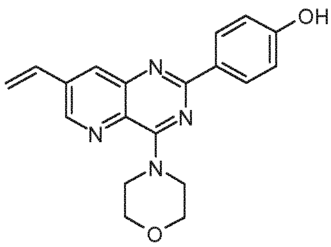

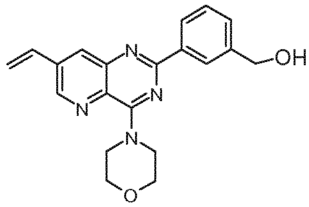

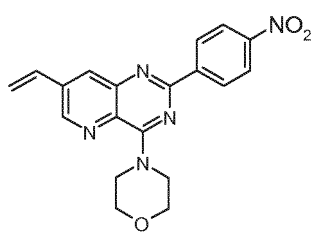

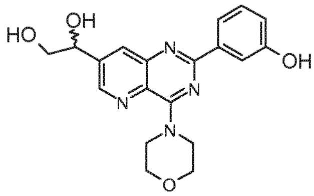

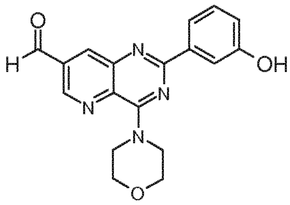

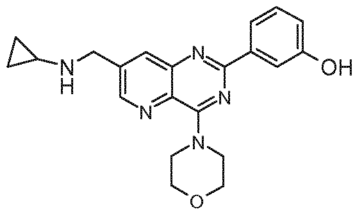

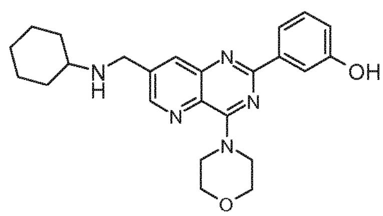

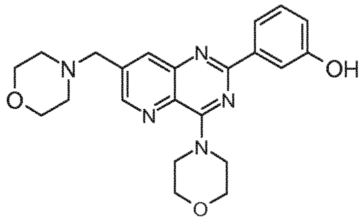

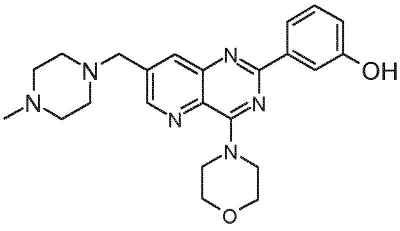 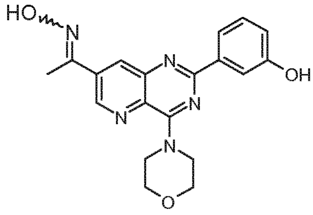 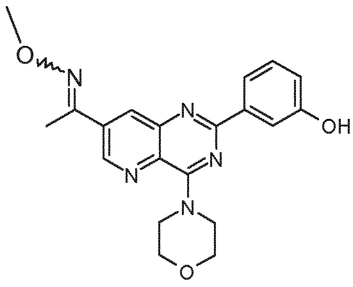

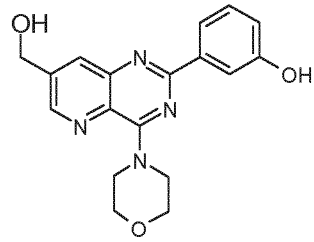 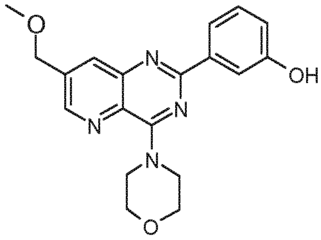 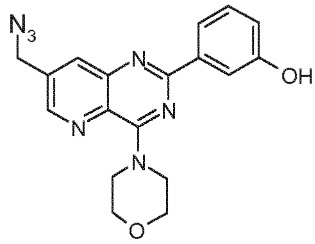

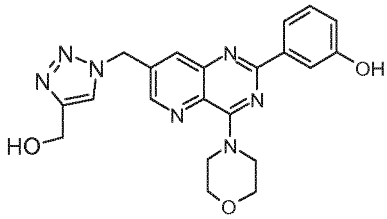 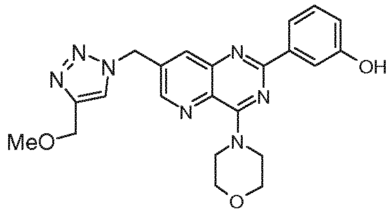

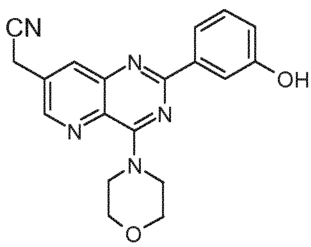 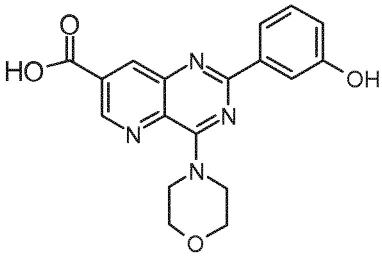 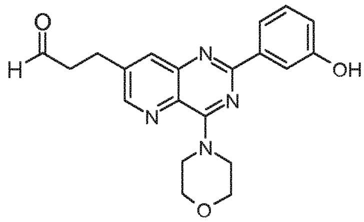

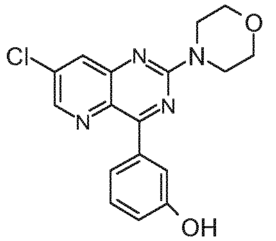

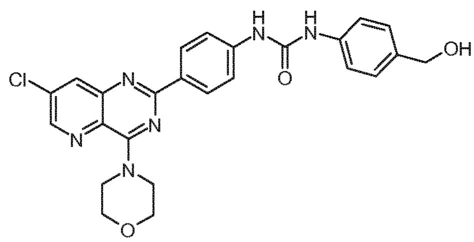

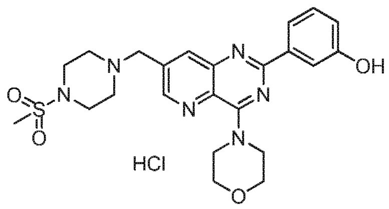

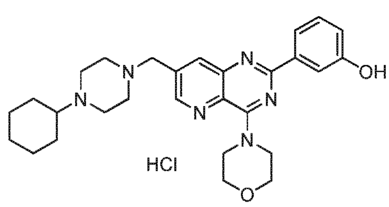

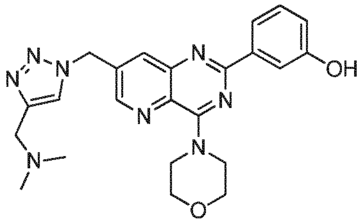

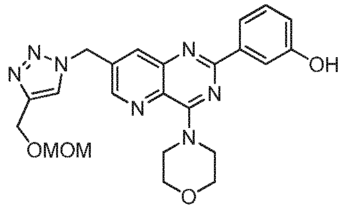

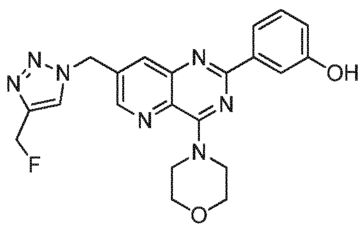

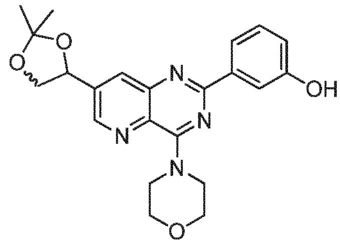

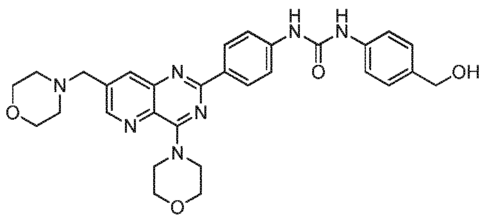

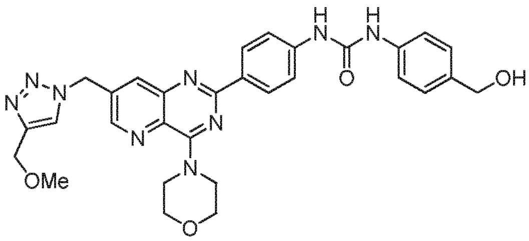

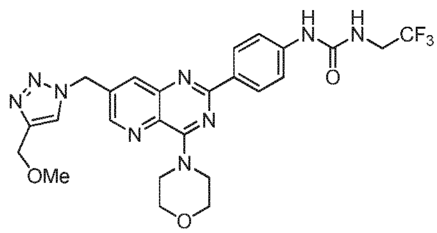

in a free or salified form, notably a hydrochloride.

8. The compound according to any of claims 1 to 7, for its use as a drug.

9. A pharmaceutical composition comprising a compound of formula (I) as defined in any of claims 1 to 7, or one of its pharmaceutically acceptable salts in association with a pharmaceutically acceptable carrier.

10. The compound according to any of claims 1 to 7, for its use as an inhibitor of PI3K and mTOR enzymes.

11. The compound according to any of claims 1 to 7, for its use for treating and preventing diseases related to deregulation of the PI3K and mTOR enzymes.

12. A compound, for its use according to claim 11, for treating or preventing pathologies selected from the group consisting of cancers, such as cancers of the lungs, of the kidneys, of the ovaries, of the pancreas, of the skin, of the colon, of the prostate, leukemias, non-degenerative diseases, such as arthritis, inflammation, sclerosis, glomerular nephrites, psoriasis, allergies, asthma, diabetes, thrombo-embolic diseases and auto-immune diseases.

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

### Littérature non-brevet citée dans la description


- **S.M. BERGE et al.** Pharmaceutical Salts. *J. Pharm. Sci,* 1977, vol. 66, 1-19
- **KAD et al.** *Synlett,* 2006, vol. 12, 1938-1942